# EUROPEAN PATENT APPLICATION

(11) **EP 3 978 514 A1**
(43) Date of publication of application: **06.04.2022**
(21) Application number: 20199513.1
(22) Date of filing: 01.10.2020
(51) Int. Cl.: C07K 14/005, C12N 7/00, A23L 3/3571

(54) **A NOVEL PHAGE FOR LISTERIA, INCLUDING LISTERIA MONOCYTOGENES**

(71) Applicant: Micreos Food Safety B.V., 6709 PA Wageningen (NL)
(72) Inventor: HAGENS, Steven, 6709 PA Wageningen (NL)
(74) Representative: Nederlandsch Octrooibureau

(57) **Abstract**

The invention relates to the field of microbiology, specifically to a bacteriophage or a combination of bacteriophages, to a composition comprising said bacteriophage or comprising said combination of bacteriophages for preventing, treating or controlling contamination with and/or growth of *Listeria,* including *Listeria monocytogenes* and *Listeria* serovar (SV) 1/2 mutants that are resistant to bacteriophage P100 and/or *Listeria* serovar 3. The invention further relates to the a method for controlling contamination with *Listeria,* including *Listeria monocytogenes* and *Listeria* serovar (SV) 1/2 mutants that are resistant to bacteriophage P100 and/or *Listeria* serovar 3, in a food product, on food processing equipment, or on food storage containers by applying the bacteriophage or the combination of bacteriophages, to the composition comprising said bacteriophage or said combination of bacteriophages to a food product or food processing equipment to reduce the amount of *Listeria.*

SEQUENCE LISTING

<110>  Micreos Food Safety B.V.

<120>  A novel phage for Listeria, including Listeria monocytogenes

<130>  P6062393EP

<160>  9

<170>  PatentIn version 3.5

<210>  1
<211>  131384
<212>  DNA
<213>  Unknown

<220>
<223>  Bacteriophage P100

<400>  1
aaggttgtgc taaattaata cagtagaact atgtggaatg gaggaaatat tatgaacaag      60

ccagacaaaa atttctttat gaaaatgatt atcgttattc tatcaggatt acttgtgtta     120

tttgttataa acctagcatc tatctacttt gggtactctc ctcacgttgt agctacgtac     180

tttatgacag ggggctttat tgcgtatatc gtgataaact tatacgaccg aattgttttc     240

caaaaaggtt ctcctaggga agaacgagag aaacaagaga gacaaaagat agcatctaga     300

caaaaagctt tagaaagcgt agtaactcaa cgactaggta tccctataga ggagctagat     360

ttagaagtac tcattcagat ggaaaaggat ttagcagaca aaactgctaa aggtaataaa     420

aatgaaaaaa ctgagtagag acgagataaa gaaaagacga aaatatttca ttaacgaagc     480

aaaaagagtt acacatatac gagcaggaga ccgtagaata gcaacaggtt caagaatatg     540

taccttatgt ggtagaagtc tatcatctca gaaacggtct acaggagaga cctttgcctc     600

agtaaaccac atccattgtt actttaatga acttcttatc gttaacttat gtgaagatgc     660

aacttcttgt tatagaagac atgaaaggag ttagatagta tgaatttaga caacatagaa     720

agaaacttaa gcaagaaaaa aactacagga gatgccgtaa aggatgagtt caatgacatg     780

gctatgaagc tactagcagg ctttaaagca gatgtttcgg caggagataa aaaattagaa     840

gatgtagcag acatggctag gctgttacaa acctttatac aagttaacaa agaagatagt     900

ggtagcggag gaacatccac ttctttacca gagcttagta gcttgcaaca agaagcaata     960

accaatcaga taaagacaga gagcgtagtt tcaggtgata aagagcaaga actagtagac    1020

ctacaggact tagctaacat gacagaagag gacgtacaac ggatgataat ggataaagaa    1080

aaagcaatga acatggaaaa tgaggagacg ttttaatgga tggcaacact cttttaaaga    1140

tggcgcaaga aacttttcaa acagaaaatg taactacaga gcagttaaac tatgttctgg    1200

atatgctaac tccatcaaca tatttactaa gaaaccacaa agtaagaaat cataacatta    1260

cgttctctat tgcaaacaag tctactgaaa aggcgcaggc acatcgacct tggcaaccta 1320

agattataaa tgactcccac ccgaatgtag ctgttattaa gtcaagacaa ttagggttaa 1380

aattagcccc tctataaagt aatttataaa gaaaactctg ttaaacgggc ataggaaaat 1440

aaccaatatc ctgataagag accctaaatc ctaagtagga cagtgggaat cccgtgctaa 1500

atcaggcgca agcttgtaaa tgcctaacga ctaaatttct aggtagctaa ccaaatgggg 1560

ttagtgagaa ctagataaga aaatatattc tcttacgata ccaactgaaa aactctatca 1620

gatgttaaaa aattcttaga agaaaatata aagtaaagca gagcaaccta aaccaaaaaa 1680

ttaggttgat gatatagtct agtcccctaa taaatatcgg gaaaccgagg gtataacgta 1740

agtgagatgg gtgtagggag tatgattcat tttgcagata cacacagcta tgcagctgtt 1800

aaatgtttat acacgtttcc tacaggtgaa cagatgcgta aatttgtaca gtcacggtta 1860

gaccctgtac ttgagtctag ttactatagt tctattctag acaaagataa caacagtctg 1920

aactacaaaa gaatacgaaa tagtcacctt tactttagaa catctagtaa gcccggagct 1980

gtagagggtg tagatattga ctacttatcc atggatgaat atgacagagt tcctgttttc 2040

tcagaggctt ccgctttaga gtctatggct agttcaccct ttaaaattgt tagaagatgg 2100

tctacaccat cagctcccgg tatgggaata caccgattat tcgaaaactc agaccaacat 2160

tggtacttgc ataaatgtga aaaatgtaac cactggaacc aactagacta cgaggactac 2220

gacagtagct ctgtagaagc aggaggaaat atactatgtg taaatccaca aggtgttgat 2280

accttagcta aaacagttgt agaaggttcg ttccaattcg tatgtaaaaa atgtggagcc 2340

ccattagacc gttggtataa tggtgaatgg gtagcaaagc accctgatag aacaaaaaat 2400

ggagacggta ttagaggata tatgatttct cagatgaatg ctgtttggat aagtgctgat 2460

gacttaaaac gtaaagaact aaattcactt tcaaaacaag cattttttaa ctatacacta 2520

gggtacccat tcgaagatgc taaactagca gtttatagtg aagatattat agaaaatcca 2580

agcccaagag taaaggttcc aatgcacagc agaggagatt acaagtacat ttctgttggt 2640

atagactggg gaaatacaca ctgggtttct gttcatggta tgacggaaag aggagaaata 2700

gacttgataa gactgtttag tgttgagaag agtcgaggag taggtaatat tgagtctgat 2760

ttagataaga ttatcctaga agtatctatg tacaaccctg atatgattgt agctgacgta 2820

ggggatagcg ggaactacgt ggacaaatta gtaaaacatt tcggagaaga ccgagtattt 2880

ggttgtattt ataagtccag ccctaaatct acaggtcagt tgaaaccgca gtggaatgaa 2940

gcagggaatg tagttactgt agacaagtta atgcagaaca aaagatatat tatagaaatg 3000

aagacaaaga aagttaactt ctatagcttt atagacccta tgctaaaact attggtagac 3060

cattggagaa acgttgtaat acaagacgaa gaggacgaaa aagatggaac tttctaccaa 3120

gttattggca gaagaggaga cgaccattta gcgcaggcat cagtgtacag ctttattggt 3180

ttagacagac tagctgatat gtatttgaga ggctctaagt atgagttcaa ctctactttt 3240

gtaacaagca actaccaaga tgtttcgtca acaccaccag atatattcag taaatattaa 3300

attaatttag ttagggggct atgctccctt tcttatttgt gttataatat agacaaggag 3360

gttttactgt gacaaatgaa gagaagtttg acaaagtaat gtctatcgtt aaagaagaca 3420

actctaccgt cctacctgac tccgcttcta ttagtaaagg aattttaaga gagttgacac 3480

aggaaagttt acaagactac ggagaggtca ttgaaaagaa tgatttttta agcagggagt 3540

ttaaacatat tgtttccctc tacggttttg acgactttta ctcactatat gcctatgctg 3600

atttttgcga tactatgagt aatctagcta ttaaaggcgg gaacaaagat ttatctaagt 3660

taaaaaaggt aaaaagaacc gttatgagaa acggaaaacc tacagagatg actttttatg 3720

agtcaactac cgaagaagag aataaagaca agaaagaaga aagtaatacc aagcgccaag 3780

gagtaagcac agtatctgca aaagacttac ttgcagagct actaaagtct aaaggggaac 3840

cagaaaaagt aactcagtta ataaaaataa ttcttaaatt ccctgataca cctgctaacc 3900

ccaaagtatc tgattatttt taccagttaa aagacgactc gggagaagta gtcgctgtgg 3960

caggttacag tataggagcc caatacgtct ctcttgattt ttataccagt aatggctcta 4020

ttagcggagt agcatcaaga gcgttctttg aattactacg gatagcaata gaaaaagact 4080

taggagctga attaaatgac gaggataacg agctagctca agtactattt tcaaaattta 4140

attttcagct agatgaggga aaacagagag tgtctgctag aaacttaaaa aaacacttta 4200

aggggtaact atatgttgcc attctattca atatatgcta taataatagc atgtgttata 4260

tacacagtta tctttatttt agtagtacgc agaatgtatc gtaaagcaga caaacgagag 4320

aaggaaaaag aagacattct taacactatg acagaaataa cagaggaaat aaaaacattt 4380

attggaggag aaaataacaa tgacaattga gaacacgaag actttagaac aaacactaaa 4440

tactgtagcc gaggaaaaag attatgactt attcctatca acgttggcgg gggtagttaa 4500

atcactatac aaagagtacc aatttcttag acaagaccca gcacccgagg ataaaacatc 4560

aggaaacatt ttgattttcc aaattttccg aggagaccct gacggttatg ctaaagattt 4620

tgatacactg tttaaaattg cagtagacag aaatttagag cacagaaaaa tatcccaaaa 4680

attctttgaa tgggtagaga aaggtaagtt cttagagttg ggtgataaca tcgttattag 4740

cagggatggt acaggagtaa gctctattgt tgagtctgtt gaacttaaag tttttattac 4800

ttttatgact aaaagttatg ccgaagaacg agaagatttt ttagctaaag agaaggaaaa 4860

gcaagaagag gctaaaaaga aagaagaaga gcgactagct ctaaaagagg aatcgtataa 4920

atctttagga gaggctacag aaatgctaaa tactctcatg gaagagcaag gtgtatctcc 4980

tgaggagctt gctttagctt tagaacaggg gaaggagagt tatcgtaatg cacgataatg 5040

aatttgaagt acctgagagt gttattaatg cttctgctac accttttgag gcgcctgata 5100

gaacaaaaga tacctaccaa agttcattaa agaactggtt ggacagagta aaatcagtcg 5160

gagatgcagg aatgtcacga gagtttgtta ttcgtattcc agcaaaggca ctagaggaga 5220

acaacccaga agctacctta tttttaaaac agtgtaacga aataaaaaga aaccctaagt 5280

gcaaccttcc tattaaaatt gaaaaaacag tagagaagta caaagacaag aaacacttaa 5340

tgttagactt ggtgttcact tatagtaata aaacagatat tgatgcagta aatagtgtaa 5400

taaaataaca aacaggttcc tctttacgtc tgctatatta gatagtagaa aacgtaagga 5460

ggaattttta tatggtaaaa tataccgtag agaacaaaat tattgcagga ttacctaaag 5520

gtaaactaaa aggggctaac tttgttattg ctcatgaaac tgcaaatagc aagtctacta 5580

ttgacaatga agtaagctac atgactagga actggaagaa cgcatttgta actcactttg 5640

taggtggcgg aggtagagtc gttcaggttg ctaatgtaaa ctatgtttct tggggagcag 5700

gtcagtatgc taactcttat tcctatgcgc aggtagagtt gtgccgtaca agtaatgcaa 5760

ctacatttaa gaaagactat gaagtgtact gtcaattact agtagaccta gctaaaaaag 5820

caggtatccc tattacactt gactctggta gtaaaactag tgataaaggt attaaatccc 5880

ataaatgggt tgctgataag ctaggaggaa caacacacca agacccatat gcttacttaa 5940

gctcatgggg tattagtaaa gcacaatttg ctagtgactt ggctaaagta tctggcggag 6000

gaaacacagg aacagcgcca gctaaaccaa gcacaccagc acctaaacca agcacaccat 6060

ctactaacct agacaaactt ggcttagtag actacatgaa cgctaagaaa atggactcta 6120

gctacagtaa cagagctaag ttagctaaac agtatggtat tgctaactat tcaggaacag 6180

ctagccagaa cactacactc cttagtaaaa ttaaaggagg agcacctaaa ccaagcacac 6240

cagcacctaa acctagtaca tctacagcta agaaaattta tttcccacca aataaaggaa 6300

actggtctgt gtatccaaca aataaagcac ccgttaaggc taatgctatt ggtgctatta 6360

accctactaa attcggagga ttgacttaca ctatccaaaa agatagagga aacggtgtat 6420

acgaaatcca aacagaccaa ttcggcagag ttcaagtcta tggtgcacct agtacaggag 6480

cagttatcaa aaaataaata atggtaaact agaggagact tttatagtct cttcttttct 6540

tttgtaacag tagtgaaata aaacttaacc cctttgtaat attatggaat tacttctgta 6600

acacatctat gttatattta gttggtaatt taaaaacgca tatgcggaat atagaaagga 6660

aaccagctaa tgaaaaaagg aagtaagatt gcaattgcta ctgtagtagc tgtacttatt 6720

gtcgttacag ttagcctagg gtaccataca gttaagttga atgagacaaa tgaggcaaag 6780

acagaagagg tagttaagct aaaggagaat atctctagtg tatctaaaga tttagataat 6840

ttaggtaaag aacatgaaaa gaaagtctca gaatccgaag ctcttaagaa agaccttgac 6900

aataaaaaga aagagttaga ggataagaac aagaaagaag agcaacttaa aaaagaaaaa 6960

gaagaactac aaaaacaagt aagctataaa aagagcttaa agaaaaaggc agtagcagaa 7020

aacattccaa atgcaactag tagtaataaa agtgctaaaa cggcagaaag caaaggaaat 7080

ggaagaagtt ttaacgtaga agttacttac tatgctattg gtgacggttt tactcccggt 7140

gtacataccg caaatggtac taacatcaaa aacacaataa caacatcaga aggacatcga 7200

ataattgctg tagacccatc agtaatccct atgaactcta ttgttaaagt aactacaggt 7260

aacggagaga gttttgtagc taaagcttgt gatacaggtg gagctatcaa aggtaacatt 7320

attgatattt tagtagaacc tagcgcagat atttatcaac tagggagaac aacaggtact 7380

gtagaaatta ttggataatt taaagaaata agagtctgca aggtataaca accttgtggg 7440

cttttttatt atgctatatt atatatgacg gattatgaag tttgaggagg tatattaacg 7500

tggttataac acagatagtg atgattatag cgattattat tactacattt cttattattt 7560

atggtatata ctcttctagg agaaaaacgt ctaagtcgtt tagactagta ctcagatatg 7620

ctataatggt agtaggtctt cttattgtct actatctatt tcaagtttcc tctattgaca 7680

ttcaacagga ttttttacag cacttaaaaa caggaggtaa cgggatagaa gaagtttttt 7740

tcacaaattt ctttcttatc atggtaggag cagtaggtac acttgtagta caactattcc 7800

aaaatagaaa gaaggattaa tgtgtcatat acaaaagact ccttgtggtt ggagacaaaa 7860

aaggttgcta gagataatgg gtttttaggt aactggatag agatggttga ctactatgag 7920

agtataggag gaagccatgt attcgtttat gcggttatgg aaggtcagaa gtaccgaatt 7980

ttaggcgttc ttagcacgga tgaggtatta ctactaaaaa gagatttaac gctctcaaaa 8040

gccctatatg aagatgttat gaatagcaaa aagatgtttt tctataaaga ggacagtgaa 8100

aagaaaaaaa taaaattgac agatgaaata agtaagttag aagataaaga tagtatcata 8160

gtatatatgt aggtaggtga aaaataaatt gggactattt gaaagtataa gattagcggg 8220

ggtaaataag agcgatgcag taaaacacat tgaggttgat gataactact ccattgcaat 8280

tcaacaaagg gaacaggaac aaatatctaa agcaatgaat aacaaagaag tagcttattc 8340

tcaaccagta attggctcta tgtctgctaa cccggggttt aaaacaaaac ctagtattcg 8400

taataaccaa gatttacacg gtgttcttaa aaagtttggt ggaaacatca tcctgaacgc 8460

aataatcaat acaaggtcaa accaagtttc tatgtattgt aaaccagcta gacactctga 8520

gaagggtgtt ggttttgagg ttagattaaa agatttagat aagaagccta cttctcatga 8580

tgaagcaaca attaaaagaa tagaaagctt tatcgaaaaa acaggagtag ataacgatat 8640

taaccgagat agcttttctt cctttgttaa aaaaatagta cgagacactt atatgtacga 8700

ccaagtaaac tttgaaaaag tatttaacag gaaccaaagc atggttcggt ttgtagctaa 8760

agacccaaca actatatttt ttgcgacaac agcagatggg aaaattcctg ataacggtaa 8820

tcggtttgta caggttatag accagaaaat agtagctacg tttaacgcaa gagagatggc 8880

atttgccgta aggaacccac ggtctgatat ttatgctaca ggatacgggt accctgagtt 8940

agagatagca cttaagcagt ttatagcaca tgagaataca gaagcgttca acgatagatt 9000

ttttagtcac ggtgggacaa cacgaggtat tctacaaata aaagcagcac aacaacagtc 9060

gcaacatgct ttagagatat ttaaaagaga atggaagaac tcgttaagtg gtatcaacgg 9120

ttcatggcaa atccctgtag tatctgccga agacgttaag tttgtaaata tgacaccttc 9180

tgcaagagat atggagtttg aaaagtggtt aaattatctc attaatgtta tctccgcact 9240

gtatggcatc gacccagcag aaataaacat acctaacaac ggaggagcca caggttcaaa 9300

aggtggtagc ctaaacgaag gaaactctgc cgaaaaaaat caggcatcca agaacaaggg 9360

cttacaacca ttattaggat ttattgaaga ctttataaat aaacatattg tagctgaatt 9420

tggagacaag tacacattcc aatttgtagg cggagacata aaatcagaac tagagtcggt 9480

taagatacta gcagaaaaag caaaagttgc tatgacagta aatgaagttc gtaaagaact 9540

aaacttaccg ggagatgtaa ttggagggga cattccatta aacggagtaa tcgttcagcg 9600

tataggacaa cttatgcaac aagagcagtt tgagcatgag aaacaacagt ctaatttaca 9660

aatgttacaa gaacagacag gtaacagagt aagtactgat gtagaagaca ttcctgatgg 9720

gaaagataca acaggagaca taggaaaaga cggacaaaga aaagataaag ataacgcaaa 9780

cgctggtaaa caaggcatga aaggtgataa acctaacgat tggcagacct aatattttgt 9840

agttctgcct tttaggtaac taactagtag aggtatatgc tatattaata acagaatgtg 9900

ttataataga tatgagaggt gatttcttaa ttgaataacg tcgtagagaa agagatgaaa 9960

atctttgtac ctattgatat tgaaggttct ttagaaaaat cccataaggg agatgacaag 10020

gactggtatg ttaaaggtta cgcatctaca cctgctctag acttacaggg agaaatggtt 10080

aaccctgctg gaatagacat tggttatttt gtagataatg gttacattaa ctttgagcat 10140

agacaagaac cagactatgt agttggagtt cctacaagta actgttatgt agacttccaa 10200

aaaggtctgt tcgttgaagc aagactgttt aaagacaacg aacacgccca atctatgtgg 10260

aagttagcta acagcattag taagacaggt attgatagac aacttggttt ttctatcgaa 10320

ggtgcaataa gaaagagaaa cacagatgac agccgaatta ttgaagaggt tgtaatcaaa 10380

aatgtagcag tcacaatgaa cccagcaaac ccagaagcta cgtgggaaac atttatgaaa 10440

tcatggacta ctggacatgg aacaacacct gatacgcaag taaatgcagg agctttaaga 10500

agagagagct tagctaacag catatcagta ctagcatcag catacaaaat ttctgaccct 10560

attgaatttg acaacttatg gaaagatgta agtaagtttt tagatgaaaa aggagagtat 10620

aacgagaatg aggcaatagt tatgttacaa ttagctaaag gcgtttctcg tgtaagtgca 10680

tgtgagtctt taatgaatag acgaaagggg aaacaaaatg aataacagtg aagaaaagtt 10740

cgaagatgtt ttaaacgaat ttggtaatac agtagaaaaa gataaagaag aaatttctaa 10800

gtctaccgaa gaaaaagagg aggttacaga tattaataaa gataaaaaat cagaagaaga 10860

aactgctgag gaagaagtaa ctgaggctcc cgaagtagac gttgcagaag acaaagaagt 10920

gtccactgag actcccgaag aagagtctac tagtaagtca gctaaggaag aaaagaaaga 10980

aaaagatagt gaagaagaca aggacgaaga cgagaacaaa aaaggtaaga aagacaaaaa 11040

agacaaaaaa gaagaagaca gcaaagaaga aaaaacggag aagtctgatg aagaagattc 11100

cgaagaggct gtaaaaaata ctaactctga tgcagatatt gcaggagcat tgaatgctgt 11160

tgctaagtct tatgagaatg ttaaagaaag ccaaagagag caaaatacaa ctgtagaacg 11220

cttagagaag aatatcggta gcttagtcag taaattagac agcttggtag atttaatgag 11280

tgaacgtgta tctaagagtg tagtagaaga tattgaaact cttgaaggag ctagtgaaaa 11340

agctgttggc tatgtagcaa aaaacgtaga tagcagtgta gtagaagtta ttgaaggaga 11400

agaaacttct aagagtgtag atgcagaagt gagtcctaga gatgagtttc tagctaataa 11460

gagtgctttc ctagagtctt ttgaatatgc aagtcgtaac ggtgctgagc gtagagattt 11520

aactgatgtt cgtcacgctc atgggaacat tacaaatgat attgctagta aagcagatta 11580

taaccgagtt aacgacttta tgaaaaaatg gagtaagtaa taaaggtaac tgtatagagt 11640

attttttgta aacagtatgc tatattatag gaacatggga aacatgttac gttatgagag 11700

agcttgcccg ttttaggact cctcccctag ggcaggttat ctcactaaca aaaaaataaa 11760

actatgaaac tgaaaggtga taaatataaa tgccaaaaaa taacaaagaa gaagaagtta 11820

aagaagtaaa ccttaattca gtacaagagg acgcgttaaa gtcctttaca actggttatg 11880

gtatcacacc tgatacacaa acagatgcag gagcattaag acgtgagttc ctagacgacc 11940

aaatctcaat gcttacttgg acagagaatg atttaacatt ctataaagac atcgctaaaa 12000

aaccagctac atctacagta gcaaaatacg atgtatacat gcaacatggt aaggtaggtc 12060

atactagatt tactcgtgag attggggtag caccagtaag tgaccctaac atccgtcaaa 12120

aaacagtaaa tatgaaattt gcttccgata ctaaaaacat cagtatcgca gcaggtctag 12180

taaacaacat tcaagaccca atgcaaattt tgactgacga tgctatcgta aatattgcta 12240

aaacaattga gtgggcttca ttctttggag attctgactt atcagatagc ccagaaccac 12300

aagcaggact agaatttgac ggcttggcta aacttattaa ccaagataac gttcatgatg 12360

ctcgtggagc tagcttgact gaaagcttgt taaaccaagc agcagtaatg attagtaaag 12420

gttatggtac acctacagat gcttacatgc cagtaggggt tcaagcagac tttgttaacc 12480

aacaactttc taaacaaaca cagcttgttc gtgataacgg aaacaacgta agcgttggtt 12540

tcaacatcca aggtttccat tcagctcgtg gatttatcaa acttcacggt tctacagtaa 12600

tggaaaacga acaaatctta gatgaacgta ttcttgcttt accaacagct ccacaaccag 12660

ctaaggtaac tgcaacacaa gaagcaggta aaaaaggaca atttagagca gaagacttag 12720

cagcacacga atacaaagtt gttgtaagtt ctgacgatgc agagtctatt gcaagtgaag 12780

tggctacagc tacagttact gcaaaagatg acggcgttaa actagaaatc gagttagctc 12840

caatgtacag ctcccgtcca caattcgttt caatctatag aaaaggtgca gaaacaggtt 12900

tattctacct aatcgctcgt gtacctgcta gcaaagcaga gaacaacgta atcactttct 12960

atgacttaaa cgactctatt cctgaaacag tagacgtatt cgttggtgaa atgtctgcta 13020

acgtagtaca cttgtttgaa ttactaccaa tgatgagatt acctctagct caaattaacg 13080

catctgttac atttgcagtt ttatggtatg gagcattagc tctaagagca cctaagaaat 13140

gggtacgtat tagaaacgtt aaatatattc ctgtaaaaaa cgttcatagc aactaataat 13200

tataggataa ttgaataaaa acagtataga gagcagataa atactgctct ctattttact 13260

aataaggagg atttaaattg ctaaaaaata caaacttagc taattataaa aaagtgaata 13320

cacgatttgg aaatcttagt tttgatgata aaggtatttc taatgaccta acggaagagc 13380

agcaaaaaga attaggtaag cttagaggat tcgaatatat taagacagaa cagaaaacga 13440

aagaagaacc taagaaagaa gaacctaaga aagaaagtac agaaaatgaa ttagacagct 13500

tcttagctaa agaaccttca atcaaagaat taaaagaatt tgcgagtaaa aaaggcatta 13560

aaattgaaaa aactaagaaa aatgatataa ttgaagaact aaagagaggg taatgtacaa 13620

tgtatggagg ttatgaagga caagattctt acgaataccc ttactcacac gggaacccta 13680

agcatgtaga gccagaaaaa gttgacgaat atgttctttc tgattatggc tggactgcgg 13740

aaacaattaa agcatacatg tatggtgttc gtgtagtaga ccctgaaaca ggagaggaaa 13800

tgggagacac cttctacaat catattatag aggttgccgt tgataaggca gagaaagaac 13860

tagatatagc tattctccca agacgggagt cagaacacca cgattataac caaacagaat 13920

ttagtagtta tatgtatacc catgttcata aaaaacctat cttacaagtg gatgacttaa 13980

aactagaaat gaatggtagg gcaatatata gataccctgc taactggtgg aaagtttatt 14040

gcttagctgg acatatagaa ctgtacccga cagccttgat gcagacttct caaagtatga 14100

catatgactc tgtatttgga ggatacccgc agttggcagg aacagcccct tctataggta 14160

gaacatatgc ccctcaaatg attcatgtag aatatatagc aggtatgcta ccaaggaaaa 14220

gagcaggtgt tagtcgtgac tgggaagtac ctgctgcatt agaacagtta gtaactaaat 14280

atgctttagt agaaatattc caagtttggg gtagacttat catcggagca ggtattgcta 14340

gtagaacact agtttacgac ggagtttcag agtctattga tacaactcag tctgccatgt 14400

atacaggttc agcagcagat attgagctta taaataaaga catagacagt ttagtagctc 14460

aactgcgttc tcactttgga ataaacatga ttggactata agagaggaga atgtttaaat 14520

ggcagagcct actagatttt caagtgatac tcctattaaa aacctaacaa cacaaaaagt 14580

agagtacaac atatcacagc atagaaagtt tattaaagac cgtagtatcc atacgttatg 14640

ggaaagagct tatttatgcc cttgtatgaa caagtcctcg ggttctgctg actcttcttg 14700

tcaaagatgc catggtaggg gatgggcgta tttaccagct aagcccgcaa atatgattat 14760

tcaaggacag gaaaaagggg ttactaacac agacctagga ctatatgact caggaacggc 14820

tatcggtaca aatgattttg atgctagaat ggcttttagg gacagaataa cagtacctga 14880

tgttaaaata ggtcaatccc ttatctactc tataaccgaa agcagacaaa aacgaggtat 14940

atttttaaca tatgacgttg atactataga ctttataaca agtatggaaa gagaactaat 15000

agaaggtaaa gactacacct ttgataaaca cagtaataaa ctattccctg atattagttt 15060

gataggtgaa gttatatctt taaacgtatc tgttactcta aggtatattg ttattgactt 15120

actaaaagaa agtcgctatc agtacaccac attaaataga acagacccta agtttgagca 15180

gttgcctcag aagttattac taaaaagaga agacactatt gtattacctg aaccatttgt 15240

acttgatgct gatacagaga ctagagtaga gttagttaat gaccctaaaa ggaaaccaag 15300

acaacaaggc gggttctacg aaggatgttt tgactagtga aacagagtac acagagacca 15360

tctctattca ataatccaaa gcaggtaatg agaaatatgg caagagctat agaatcagat 15420

gttattgcaa caggagctca agaggcatct cgtataccca atgtagagat taaaattatg 15480

cctaagtatt tagaagtaac tgaaaagaga atggaaaaaa atggtgtaat tgaccttaaa 15540

ccttattttg ctaaaagtaa taaaaagaaa tggaatcaag aaggtaactg gtacttagta 15600

atccctataa gattaaagac aagagatatg tctcgaaaat tatatgatga cttaaggaaa 15660

cagtcttccc caccagttgg aagttcaaca acaattgtat cagatttcct atatgaccgc 15720

agaagaacga gccctagtgt cccttctata aactataatc caaagtcaaa taatgttaca 15780

aaaaaacgta ttagtaacag tagaacaaca tatacagtat accgtacagt aactgctaag 15840

tcacctgcta atagttggat tgttaacagg gacagggtta atgaagagga tatgagtaaa 15900

acgatgttag acagaataga caggctaatg aaatggaagc ttaaaaactt gtagaggagg 15960

agtacaatgc ctattccaag ccttgattct tatgtagcaa gaagagtaag agagagcctt 16020

aaaattatgc tttcagagtg ctatattata gaggaagctt tgagtgacat ggatgtcgaa 16080

gcaaaaagta actttatgaa gacatattgt ggagataatc ctaaaaaaga aatagaagtg 16140

atatacgagt tccctcaggc aaaaacatca gtagatgcta cttatgtaat tcagctagga 16200

ggaggttccg aagatactaa aagcatcggc ggtattgaag gtacttacga gtataaagag 16260

gattctttaa gaaaagagac agtagttgct actagagaag gaaccgagtt atttctagaa 16320

acaaaagagc taattggaag tatagaggga atacctaatt tatccttttc agaaagtgat 16380

aataaacgaa tagaaggtaa acgattatac ttctcttatg aaggtaatgc agatattgag 16440

ggagtttcct tcgatattat ctatacaagt aaagtagacc tagagagcga ccccgttgga 16500

ctaaaaaaag ggtttacttc tagagacaat atcactgttg taggtttaag cactaacatg 16560

gatactgcaa ggtgcctaga tgctatctta aaagttatcc ttattatcat gagagaaaac 16620

ttagaagaga agagttacta ctccctacaa acaactcagt tttccgatat gtctcctgta 16680

attatgggag aggaaaaacc tgtatttggt agacctgtaa caatgagtta cactatatca 16740

tatactgtag actttaatct agtcaaccaa ataaataaaa ttatagtaca ggagcgtaat 16800

aagtgatggc agaaaaaaaa gaggtaacta aagaacctat tgtagacgaa gtaaaagtaa 16860

caaagaagaa ccttaaggaa cctacaacag aagaaaataa agtagtgaag aaaaaactta 16920

aagaagaaca aacagatact gtagagaaag ttactacagg atatgttcat atagatgtat 16980

tccttggaac agccaagccg tggtacggtt tatcagaaat gcaagtacaa gggtttaaat 17040

cttttatgca agggaaacaa taccagttcg gtgaacaggc ttttgttcct tatttagaaa 17100

aatatctagg aagggaagta aaataaaata tggcaatcga aatttaccca agaaaaccag 17160

tttctagacc tcatacagaa atttctgtag atacaagcgg tattggtggt tctagctctg 17220

gaagtgaaaa aatactttgt ttagtaggtt cagctacagg aggaaaacct aatgctgttt 17280

acaaagtaag aaactattct caagcaaaat ctgttttccg ttcaggagaa cttttagatg 17340

caattgaaag agcatggaac cccggtgaag gaacaggtgc aggagacatt ttagctatgc 17400

gtgttgagga agcaaaagaa gctacctttg aagctgaggg ggttaaggtt agctctacta 17460

tttatggagc tgatgccaac gacatccaag tggcactcga agataacacc atcacaggta 17520

caaaacgctt aagcattgta tttgcgaaag aaagagtcaa ccaagtttat gacaacttag 17580

gtagcatctt ctctatcaag tataaaggta ccgaagcttc tgcaacattt acagtagctg 17640

tagacccagt aacatttaaa gctacaaagc taactcttaa agcaggggat aaaacagtta 17700

aggaatacga tttaggttca ggagcgtatg cagaaactaa tgtacttatt agtgatatta 17760

acaacctacc tgactttgaa gctaagtttt tcccaatcgg agataaaaac ttaacaacag 17820

acaacttcga tgcacaaatc gatgtagata ttaaaacaaa agaagcttat gtaaaagctg 17880

ttggcggaga cattgagaaa caaacagcat ataatggtta cgtagacttt gaatttgacc 17940

gctcaaaaga aattgctaat ttcccactaa ctaagctaac tggtggagac aacggtacaa 18000

ttcctgagtc ttgggctgat aaattcagtt actttgcaaa cgagggtggc tactacctag 18060

ttccattaac atctaaacaa gctgttcatg cagaggcact tcaatttgtt agagattgct 18120

cttataacgg taatccaatg cgggtattcg taggtggagg aatcggagag tcaatggagc 18180

agttgttcac aagagctatc ggcttacaga atgaacgagc aggtctaatc gggttctcag 18240

gaacagtgaa gatggacgat ggacgttctc tgaaaatgcc gggatatatg tttgcagcac 18300

aagttgcagg acttacatgt ggactagaga taggagaagc aatcactttc aaaaacattg 18360

cgattgagac attagataca atttacgaag gttctcaact agaccaatta aacgaaagtg 18420

gtattattac cgcagaattt gtacgtaata gagcagttac caatttccgt attgtagacg 18480

atgtaactac atttaatgat aaaacagacc ctgttaagtc agaaattggt gtaggagaag 18540

ctaatgactt cttagtttct gagttgaaga taagcctaga caacgagtat attggaacta 18600

aaattattga cacctctgct tctttagtta agaacttcgt tcaatctttc ttagatagaa 18660

agaaattagc taaagaaatt caagactaca gcccagagga agttcaagtt gtaattgagg 18720

gcgacgttgc tcgtatctca ctaacagtat tcccaatccg cagtatgaag aaaattgagg 18780

tatctctagt ataccgtcaa caaatcctta ctgcctaatc taatacaggg gagggcttag 18840

ctctccctat gtaaataaaa aataaaacag gagttgaaac ataaaatggc aagtgttact 18900

aatcagacgg ttcatacagg taatacggtc taccttatga taaaaggtaa aatcgtagga 18960

agatgtcagt ctgcatcagg tgaacgttct tatggaacaa ctggtgtata cgaaatcggt 19020

tctatcatgc cacaagaaca cgtttactta aaatacgaag gtagtttaac agtagacaga 19080

ctgcgcatga gaaaagaaga tttagctaag ctaggtatta cagcattagg agaagacatt 19140

cttaaaagag atgttatcga cattgttatg atggataata ctactaaaga agtaatcatt 19200

gcttatcgcg gatgttctgc tgagacatac aatgaagaaa tcaaagctaa tgagattgtt 19260

tctgaaagtg caagattctt gtacctaagt gcagcaaacg taaaataata ctaataaaag 19320

gctaccttaa tctggtagtc ctttttttat agtttactaa ccttagcaaa tatggtataa 19380

taaagtaaga gaacgaaaaa gtattactag tttgttatat tagataatga gagagtaaac 19440

aaggaggaga caaattacat gtcagaagaa caaaaagtaa atgaagaaca aatagaagaa 19500

agtaaagaga agatgttgac gaaagaagac atcgaccgta tcaactatga acaacaaacg 19560

aagcaaaaga aaacacttga ccgtattatt aaaggggtaa atgatgtttg gacacatgag 19620

tatgacttta aggagttaga tttaaagttc accattagat tacgagcacc taatgcttta 19680

gaacaagcaa caattcaagc taacagagaa gcttatttaa aaggaacagg tgcattagtt 19740

agtgactaca tttatacaat ctatcataca ttagccacta taagaacagt tggtgtagat 19800

attccaaagg aactagcaga agacgataac atttacaata ctgctatttt ataccaaatt 19860

ggacgtgatt ttgccgaatg gctagacacg tttcgctact gagataaaag atttaggtgg 19920

aataaaagct ttagtaaaaa ctccctatgc aagaaacatg tggggaataa tgaaagagtt 19980

caatgtttta cctacagacg agaggttcag agcgcttact gaggagcaaa taaacttcat 20040

ctactataac aaagagagag actacaaaga ggcagagaga gcttctagag gcgtgtctat 20100

ggagtctgaa tttgaagact ataactcgga tgactggtgg gaagcttctc atgaggactt 20160

ccaagcaatt caggaaggtc atgatgaaga tgacatcgct caacaagtag agaacatgac 20220

tagtatcgaa gaccaaaaac gcctaagaga cagattccag tctcatgagg agtacgaaga 20280

gtttataaga aatggtggta aagatactag acaggaaatg attgatgaac acatcaataa 20340

caaactgaaa gaactgtttg cagatgccga agagttaaat aaacaaggct tgagtaagtg 20400

gggagaagta tcagaaatga caactatcca agaagctgta gcagacgagt tagaagagct 20460

aacatcagat agtgttaagg aagcaatcag tatgttcgaa gatgacgaag ttcaggtaat 20520

accgggtcaa gatgaagacg atgattttga aatttaatac taaaaagaaa gggtagagct 20580

gtgtactcta ctctttttta ctaaggaagt gaaagtaatt ggaaaacact aactatcgct 20640

tttcggtaga agccttaacg gcagatgcag agaaaaagtt agagcgtatc gctagaataa 20700

tggacgacat tgagggaaca aataggaaag gtgcaagtaa tttcttcacc agctctcaaa 20760

aagatataga caacaacata agagctatga gagaatatgt ttcattagtt aatgaagttg 20820

agaaggaaat gaaaaacgcc cagtcttcta tgtctcgttc tggaaataac gaaggtgttc 20880

aaaaattaat aagagaccag caaagactaa gggaagaagc ggaaaaaaca cggaaaacat 20940

tccaagatac tacacaaaat aaaacaggta gtaacgctta tcttaaagac ctgttaagag 21000

accaaaaagc atacaacgat gaaatagacg aaggtactcg tagattaaaa gagttgaaag 21060

acttacaaag agatgtagcc agaacagata gcagactggc tgccagaaca aggcaagcta 21120

cttcatcagg taatatgaac tcacaacaag caggaacagc taggtctgac ttagcaaata 21180

aaaccgcata tatgagtcaa ttacaagcca accaacaacg aagagatgag ttaagaaaag 21240

ggctagagtc cgaccaagca aaaaaaagaa acttaaatgc taactttaga ggtaacgacc 21300

aagaaagaca gaatgagttg tcagcgttaa accataatat tgagactcgt agaaaagaaa 21360

tagaagcttt aaaagagctc gatagtcaaa taaataagac agtaagtaat atggaaagct 21420

acagtaacca attaaataat gtaaatgtaa aaccagaccg taactcgtgg caaggggtcg 21480

cttatgaaag agcaccatct atcgctatgg ctgcattagg tggagcaggt gcaatatttg 21540

gcggtctata tgctaaaggt agctctataa aagaccagat tagacctgac actatctcta 21600

tagggcaacg aacaggtaac tatgatttcc gttctatcag aaaacagaac gaagaagtag 21660

gtatggatag gtctcttggt tacaacgcag tagacatgat tgatttccaa aacacctatt 21720

tgtcttctgc tgggtttaaa aaccaaaaag acttagatgc aggaactaga caaatggcag 21780

aagggtctag agctgtaccc gtagataaag attctctaaa tcagtttatg ggtgggctaa 21840

tgacgaatgg agccgtatca ggagcagacc aagtaaaagc ggtacaacaa ggttttattg 21900

gagcagtaaa agcttctgga atggaaggta gggaaaaaga ccaactaaaa gctttagaag 21960

ctatgagtac ccaaaccttt gcaggaagaa atggttctaa tgctgagcta aataacctgt 22020

tagctatcca gtcactgttt tcttcatcag gtgaccgttc tatgcaagga acagcaggag 22080

cagaagcctt atctagtcta gatgcaggaa tgaaacaatc tgcatataac ccacaagcaa 22140

gattactatt tggtatgggg agtgagtacc aaggagtagg tggtatgtat caagttcaag 22200

gacaaatgga aaaaggtctc ggagatacag acaatttaaa tagactactt aaattctccg 22260

caggtgcagg aagaggtaaa gaagaacaaa aaggttactt caagaacatg gctgagaatg 22320

tatttggtac tacattaaca aatgaccaag tcgatgcaat gtataaacaa gtaggctcag 22380

atggaacaat agataaagac aaaatgaaaa acatccaaag tattattgat ggaggaaaag 22440

acggatacga taagaacaaa aaagggtatg aagactctaa ggaagctaca gaaaacgcct 22500

ctaaagcagt aactgataag caggctacag cgatgaacga catgggggac atagttagaa 22560

aactaaacag tgttctcggt ggtatgcccg caccattgta tggtttggca gccgcagcag 22620

ttgcagcagc aggagcctta gctatgtctg gcggaatgag tattctatct ggtggtatta 22680

aaggaaaact aggtaagaaa tttggaggcg gaataggtaa agcaggtagc agtacagtag 22740

cttccgcagg agcaggcgca ggagcctctt ctacagctag taaagtattt accaataaaa 22800

catggggagc aggagcagaa ggagctacaa aggcttcctc ggggctctca tggggtagta 22860

aagcaatgga aggcggaatt tctgcaacta aaggattagg tggagcagga aaactagccc 22920

agtatggaaa aggagcctta aaaggtctcg ggccgcttgg tcttctaata ggcggaggag 22980

cctcaatcgc caatattgca ggtgcagaaa acaaaaccga agccatcgga gaggaagcgg 23040

gtggctgggg aggcgccatg ggtggtgcag caggcggtgc agccatcgga gccatgtttg 23100

gtggagtagg tgctataccg ggagccctta ttggaggggt actgggaggt attggaggaa 23160

gcaaagtagg taagtgggct ggcggaaaaa taaagagtgg ctgggattct ctatggggcg 23220

gagacaaagt agatgctgct gagataaacg gcacaggagg agctaaaaaa gctcaaacag 23280

agaaaaaaac caacatgaac ggtgcttcta tggctaactc tggtgtagga actatgctag 23340

gaaatacagt aatgcccggt gtaggaggaa ttatcggagg agctatagga ggttcaggta 23400

tcctagaaca actttctaag ctaactggta ttggaagaaa cgcacaagaa gaaacaagtt 23460

caggaaaaca agatgaaaaa gacacagtag gtaagaaagc tacagcagag aaagagagac 23520

aaagtaatac aagagaagaa gcagcaaacc tttcttttta cagtaaactt cttgacagag 23580

ccgaagcatt attgatgcaa gctagaaatc aaaatggatt attcggaaaa gaaggcttag 23640

gaactactgc tacaggtagc ggtggaggag atgctctcaa ttcaggtctt aacttcttaa 23700

cagatggtaa aaagtggact aacaaagatt taacaaagca tgacttaggt tcaactattc 23760

aaggtattac tgctgatgag ttagacgaat ggattaactc gaaagcacct gagggttctg 23820

caatgagagg gatgggttca actttcctaa aagcagcaca agaatcagga ttagacccaa 23880

gatatttagt atctcatgca gccacagaaa ctggttgggg aacatcgaac atcctaaaag 23940

aaaaaggtaa ctggtttggt attggggcat atgataactc acctatggca agtgcctaca 24000

aatacgggga caaagaaact ggtgtagtag aaggagctaa atggattgct gaaaactact 24060

acaataaagg tcaaacaaca ctagagagta tgcgacataa cggaggacaa cacgagtatg 24120

ctaccgaccc taactgggat aaaacgatag ctagcataat ggggggctca gagaaatacg 24180

tgcaaggtaa aggtgcttct ttaacaactt ctgctaatat aactgttaac atgaatggag 24240

actcatcgaa agataagaag acagcaggaa aagcagtagg agatgccgtt gctaaatcgt 24300

ataaagaaca atcagagttc ttctctaaac aatacaagag aacataatgt tctcttgtta 24360

ttacatataa gataaggagg ataaaattat atatgactac agttaaaaga atgcctgaat 24420

ttgatttaaa gtttgtaaca gaaaagaatg actatcttat aagatatgat gcaaggaacc 24480

cttcctcaga tacattagct gagaaagtaa taagtgtaac tactaaaaat gcaatgtctg 24540

acgacagcgc cgttttttct attgtagtag ctggtgatat ggaatgggat aaaatattag 24600

actctaatga tgtagtaata ctaaaaatat acccaaactt aagagtaatg atgcctgaca 24660

acgttgttgt tttagtgggt ctcatatccg aagttcgaag agaaggcgac tactctaata 24720

actctattat ttatcgtata acagggcaat cttttgctaa atccttcatg cagttccaat 24780

taggggtaat tcaagaggta tctgtagtaa ttaccgatat tgggtggttg cctgactcta 24840

aggcagatgg tgttgagttt acaggaaaaa cagcagcaga gataggtaag tctatcacag 24900

ataggtttaa aaaatatatg aaatacaatt ttaacagaga atatacaatg gaaaacttct 24960

tagactactc tttcagcagt tggaaagatt acgaaaaatt agcagaccct accccattta 25020

ttaactatga gggtagccta aaacaacttc tagatgatgt tacagcaaag ccatttaatg 25080

agttattctt tgagtccaca agtgatgaga agtgtaaaat gattatgaga agaacacctt 25140

ttaataaaga agactgggac aagttaccat cttataaaat atctacagaa gcagtaatat 25200

cagattcttt agctaaaggg gacacagaag cctactctat ttttaacgtc actagtggaa 25260

atatggcagg agcaacatca gtagacttaa attcattccc tcaatatcac caagctttag 25320

tagataaata tggatacaaa aagttagagg tagacaacag gtatttattt gagtcctcaa 25380

cagatggctc tagtaccaca gaaaaggcag atgtaggaag taaggaaaaa acaaaaacag 25440

taattactta cagcaagttt aactctttta tgcgtagtta tacttccgac caagttcgta 25500

tgaatcaaag tagtattgca aaaagtttag tggacagcta cgacaaattg acaacttctc 25560

aagccaacca actgcttgct aaatattctg ctgtaggtgc gataagtgag gcagatttta 25620

aaaaaattgt aggagacata gcggagggag acaacacagg aacagctact ttagattttg 25680

atagtgtaaa ctcttggttc tctctaaatt atagttcttt gtctgaggtt tctacaaata 25740

gagatgctac aataaaagct tttgtaaaaa actttgctaa cacagatgaa gaccaagcaa 25800

ctaagatagt agcactatat atatcaagtc aaggagtcat gacaaaagaa aaatttgatg 25860

caatcataaa ggagtctact agttctagca ctaaagaccc agataacaca acaggcaact 25920

ctagttcagc actacagtac ttctctaaaa cgatatataa ctggtacagt gaaaacgcta 25980

atttttatgc aggggatata aaggttattg gtagtcctgt atataggtta ggaagtaaac 26040

tccttgtaga agataaacaa caaggtgatg agtgggagtt ttatattgaa agtgtctctc 26100

atgaatatag ctatacagca ggttatacta caacactcgg agtcactagg ggattgaaca 26160

ataaagggaa agatagattt actcatttat ggggaaaaag ttcggacttt aaaggcgggt 26220

tacttggaga aaaaacaagt gctgaactta tacaagaagc aggttctacc tcgtcaggtt 26280

ccgatggaag tggcgatgta agcgcaccag atgtacaagg tagtgatgtt gccgtagccg 26340

ctttaagata cgggctagct cataaaaagc cagagaaaaa atcagtatat tcttttggtg 26400

gaggtagagg tagtagtaac ccaatggagg gtaaggaacc ttacgcaatg gactgttctt 26460

cttttgtatg gtggtgttat aaagcctgtg gtgttacttt agcaggagca caaacacaag 26520

cgatattagg ggacgatagg tttaacacag taagctcaag aggttctaag agtaaagaaa 26580

tatttaaaaa aatgcaagta ggcgaccttg tttactttta cgataacaac actcatattg 26640

gtatgtatgc aggagaagga aagttcttag gatgtaatgg agatggaagt tgggacacca 26700

acggaggagt acagttgaaa ccaatggact ctggttattg gtggacacag tttcaaggac 26760

atgtaatacg ttttgtttaa aaaggaggaa tgactagatg agtgatgtag aatcaggagc 26820

accacgaaca caggctagtt taggtagtga gtgggaacga gtatacaaag caggagaaaa 26880

agtagacaca atcgtattag cccagataat aaaagtaaac tataaatata acaccgcaga 26940

ggttatgata ctaaaaaata aagaagtgat gtctaagtcg tatatttcta aaggtaagtt 27000

ctcagctcca ctacctcaag attttgctgg aagaactcta gaaggacttc catatggaca 27060

aattaaacca attcaagaag gagctattgt tcttataggg ttcctaaata gcaagaagga 27120

caacccaatt attttagcag tctacgggga ctcagatgta aataataagc tagctagaac 27180

tccttatgaa agtgcagacc cattagaccc taagctagct agaataaata accagttttt 27240

taacttgtac ccttccttga cgtatgataa tatagacggg gacggaaata gaatatccac 27300

ttaccccggc aagacatttt ctattatgac aagtttaata aacgagtctt ccaacatttc 27360

agacagcaat aatgggactg aatatgcaga cttacccgaa tcatactaca cagactacaa 27420

actgatagag ccgagaagcc ctttagcacc atcaatgcta tttaaacacc aaggaataag 27480

aacactagac gggaaggaag acaatcattt agtaactgcc tttataaata cagatggaac 27540

aacaaggctg tctttcctaa ataaggttat gaactggcgt tcctattatg aggctgatac 27600

tgagggaggt caaaagttca gacttcaaag agacagtaag gtaccaaacc aaggaagcat 27660

attttcagag tttgggttaa caaaaaacgg agacttcttt attgtaaatg gaaaccataa 27720

gtttgaagta agagagaacg gtatttttat taatgactct gctttagcag acgacttagg 27780

ggaagtttta gaagaaattc aaaatagaat actagggata aacgaggaga ttgctaatat 27840

catccgtatg ttggatgaga ttgatgttga gaacctagga aagttatttg accgtatgac 27900

aaaagtagaa acagacatta ttagcttaag gaatgattat aaaggaatag acagtagagt 27960

gaacgatatg tcagaagacc ttaacagtct gattaaatcc tttgataact ttaaacagac 28020

aatgtctgcc tacaccgagg ctaatactat aagggttact gctattgaga ccgctactgc 28080

aaataataca gtatctatag gaaacattaa taatagtatt actgcgattg agcagaagaa 28140

cacagcccaa gatacgaaaa taagtagcct agagagtaaa gttacgtcac tagagacaag 28200

taatgctagc ttagtaacaa aagtagggga gttagaatct gatgttactt tcctaaaagc 28260

agaaaatata gatttaacta atgcactagc ccaattagca gaaagagtta ctgctttaga 28320

aaatagctga tagtagtact atattataaa ggagacgagg tggaaaaatg ccacaagcag 28380

acggaaaagg tggagtatta agacgagttg ctttccaaat aggagagaat aaatggtatc 28440

gttttgcgat aaaccccggt agttactctt ttacaggagg acaaagggtt gctgtgttta 28500

aaacgaagtc cttaaacgta gtagaagact ttggtagaga tattcctact ataactttct 28560

cgggaacgac aggtcttaga aaagactcta acggtaaaac aggagcagat aggttaaaag 28620

agcttaaaaa atatattgaa gattatgcag actcatcagg aaacggaaat agacctaata 28680

cagaccttac ttttcataat tttacagacg atgaaagcta tattgtacat ttatcacctg 28740

aggcatttaa gatagaaaga agcgcagaaa aacctctgtt atatgactac acaataactt 28800

tagtagtttt aaggatgctt tctcaaccag accaaggaga cattgtagac ccagacttag 28860

gtaataaaca acctagtgta ggggacaaga caaaggataa tgacgatgtt tatggggatg 28920

taggaggcgc agacaagaca ccagaagcta atattactgg aagagtccct ctagaagtag 28980

gaggtaacgg gggaggggca gtaaaccccc gaacaacagg taaagcatac tcctacggtg 29040

tagatgaatt aaaaaaacaa attggttatg ggagaggtta acaatatgga ggtatatcag 29100

tcatcagatt taatgaggtt tattacaggg ttaaaagtta acccagataa cactattgta 29160

gtaaacatct tagaccagca atcaccattt atttctaggt tttacactcc taagcttatt 29220

ctaagttctt tatcagtaaa gcttattcct gaattgcaag gagtaaacat tatacaaaca 29280

aacaaagcat taagtaaaaa tacagttaca gctaaaatat cagcaagtag gttaaaaata 29340

gaggctcctt acatgtatct aatcgcacag tctatagtca tggaatgctt tgctctaatc 29400

tacagtatag aagaaaagca agatatgcta aggtacattg aagtaggaga agttaaacaa 29460

gttagagata acataaatta tttagctgac tacttagctg acaaaccaga atacagcagt 29520

attgttacgg acttacatga tatggaaatt gctgtagggt atattgagaa ccaaatacca 29580

attatacaga gggagataag attaaatggc tcgttataaa aaacacgtag tacaatatgg 29640

cgatactatg cagtctatct ctcagctaga gactggtagt gttggaaact gggtagatat 29700

agtagaatat aataagttaa agtatcctta tattgtacaa acaaatgagg aaagaaaaga 29760

aaatcttgag agtacagtta ctattgggga tgttattatc atacctgtag catcagacct 29820

aacagaaata gacccttatg ctctaacaaa ccaagataag aacttaataa tgagtttagc 29880

tctaggtagg gatttaagta tgacctccaa tcaagattac tataataaaa gaggaactag 29940

tgacgagata ctagaagttg tagctaacgg caagggagac ttagcgactg ttgacggagt 30000

agaaaaccta aagcaggcac taatagctag actattaact cctaaaggag ccttgatgct 30060

acatccagaa tatggctcta acttacacaa gatgtttaaa aaagctacac atgaacaagc 30120

atcactaata gatattgaaa taacaagaac attgttacta gacggtagag tagaaacagt 30180

agaaagtctc ggatacaaag tagaagagga cgtatatgta ggaaaattcc tagttactat 30240

agagtcttta cagacagcgt ttgagttcgt tgttcaaggt gatactacag gttctattat 30300

tttgctagat aattgaggag gaaaacaggt tgagattaaa aagaatttca gaaattttat 30360

ctcgtttaat agataagatt atgattaaca catcagagct aaatgatttt tctgtagggt 30420

ctactatctt gtctatacta gaggcagttt ctatggagtt ggagcagtac tacatcctat 30480

ctagagagaa cattgtgtgg ggtattcagc aaggggtatt tcaggctttc gactttttta 30540

agagagagcc aaaaagagct tatggagatg tcctaattga gtttcatagc gcagttcaaa 30600

gtgatgtata tattccaaaa ggaaccactt ttagttcaac aattaacgga tacgaccagc 30660

aattcgagac agtcatagac tactacgttc ctaaagattc catattagct cgtgtagaag 30720

tgtattgtaa agtagcgggg gaaataggta atgtcccagc aggaactatt aatttgatta 30780

tgaatagtgt aagtaacgtt aagagtgtca taaatgagag taacttccaa acaggaactg 30840

atgaagagtc cttagaatca gtaaaaaata ggtttcatgc tttcgtagag agcagaggga 30900

gagctacagt ccctgctata aactatggta cccgtttagt agaggaagta tcaggcgtat 30960

atgtaaagga atatgtaggg tatgtaattg tctatgcaca cgatagaaac ggaaaccttt 31020

cagctaacat ccaagataac attaaaatag cattagaaga ctatagacca gcaggtataa 31080

aattagatgt aaaacccgta aacaaaaggt tgctacctgt cgaggttact gtaactatta 31140

ctaacaaaaa cagaataggg gatacactac aggcacacat tcaaggagtg ataacagact 31200

acttaaatag tatgcaagta tctaacaact taattttatc agagcttatt caaataatta 31260

tgagtataga cgactccctt atttatgact gtaagattga aaactttaca gaaaatgtta 31320

taataaatag tgaggatatt attagagcag gagaagtaaa ggttaactta aaataacata 31380

gttaacaaat gagaggagct gacaactacc cttgccaaac tttttaaaaa atatacaccc 31440

tttattaaaa agaaaaaaag atgacaacca agtaagtagc gtaaactacg ctgttttaca 31500

agcgctcgaa acagagctga gtgaagtaga acaggaaacc ttagagagta aagtacagtc 31560

atcccttgga acagctacag gagactacct agatatttgg ggagactggt ttaatgtata 31620

ccgtagagaa gatgaaggag acgaacgtta cagagatagg attattcggc atttgttatt 31680

aaagagaggt acaataccag ctataatcga ggcaattcgt gattttctag aagactatag 31740

cgcagatatt actatctatg agccgtttaa aaatatcttt tacacaaata aatcagactt 31800

aaacggtgaa gactacttga tggggtacta ctatcgtttt gctatcattg atgttacaat 31860

tggtagacgg tttcctttag aagtaattga cattataaac gcatttaagc cagcaggtgt 31920

aaaattctat ctgacttatg acggtagtgg agaaattgat gaagaaaaca gtatagttga 31980

actaccacct tatagtatag aaattgaaac atatcaagag gtagatattc taaacgggtt 32040

aaatacaaca cttcacgggc acttcacttt aggagagtct aatattgtag agggtgttat 32100

ccttgagttg tttcgaacaa atagaagtcc actaaactct aatgatgtac tatctggctc 32160

ttttaatgca ggtagacgag tatacaatct agcaacaatt ggagacaact cctataaacc 32220

tacgatgagt tctcttattg gggaaataaa aaattcgttc gaggaattac ccgaagactt 32280

ctacgtagaa actaaccgct tagatgatag gtttgctagc atagacatgg caaataatac 32340

acaagatact gcattctatt tcaacatgga tttaggaact tatatcaata ctaactacgc 32400

aggatattta gctgagttag caggaagcca gattataaat agagcacact atgtatcctt 32460

agctaaagag ccaaagttaa ccgtggcgtt taaagctaaa atatccccag caaacccagt 32520

aaattacaat atagaaggtt atgacttagt ttctaaaaaa tgggttgttc ttgagacagg 32580

agctatagcg gttgaaaaga aaatatcaca gataccttta ggttcattag aagactatct 32640

taatagtaac tatatatgtt ttttccgaat tgtagtgaaa cctaaacggg aaacctactc 32700

ctttgaatta gactatttcg aattaggatt tttaaacaac tttaaagaag gggagtatac 32760

aattaagcct tttacaggta ttgtagaaac ttatgcagag ttgttcggta gagttataga 32820

agttcctatg tatcggataa taaattcagg aacagaaaat attatttcta aacaaggtca 32880

tatacctgtt agatatatta gagatacttt agagggtggt agtactacaa acactgctaa 32940

cctatggata gaaattcagg ctaaaaataa agacaatatc aatgttgcgc ttaataagcc 33000

tgtatataga ccacaagctg aacctacaaa tggtaatatt cttctatatc cacagttcct 33060

taatattgga gctacttaca tgaatggtaa gtggagagga aaactaacta gtgaggtaaa 33120

catggcatat actgtagacg gtgtaagaac ttacggagga aacatgaacg cagatggaac 33180

ctatacagtc tatatcggga acaaagtaac ctctactagc caaaaagtag ttgtggaggt 33240

atatgcagac actgcaagaa cgaagttaat agacaaagct aatgttccta ttacaaatgc 33300

aggggacgga agccctatag aggctatcac agacggagta attagtggct ctaatatatt 33360

ctatattgca ccaacaagcg ccaaatactc agtaactgta gacttagaag ctacctttgt 33420

agacttatca gaggtaagga tatggcacta cccaacacga acgtttaagt ctgttttgac 33480

agaagtatca gcagatggtg taaactggac tactatatat aactccgctg ttgatgggac 33540

ttatctagaa actagtgaag gaaaagcgtt tcctgtacca gagatggact tatatggatt 33600

gaaagctact cctctaacaa gtgtactagc aggttctgaa agacaaatta ttacattcct 33660

agactacaaa atagaggact ttgagaaccc agctaatact attgaaaacc taaacaatga 33720

ttactttaac gcagtgtggc aagagattga ccgacttgaa gagtttgttc taggcaagtt 33780

cactatttta gctgatgttg gctctgacgg agtactagta aatgactggg gtcttattga 33840

ggctaaaata cctacaggtg ctacagcata ccaagaagcg gacagtagaa gtgaattgtc 33900

ttccgtgtac actgtggagc tcacagcggg ctctcacgag gttattatgg atgttgctaa 33960

taaagttgtt actacagagg cagtaaccct aaaaacggat tacagggtgt ctaacgagct 34020

taccttggaa gggctaccta caaaaatacc tgtgagatac attcgagact ccttgtgggg 34080

aggaaacagt ataaatacag caaaccactg ggtagagcta caggctagag acttgaaagg 34140

ggacaatgtt gcccttaata aaaaagtggt agagaacaac ggagttagct tactatctcc 34200

tgaaaatcta atgttagact cgaagacaga gattactagt acaacaaaca atggttatac 34260

ttttgcttct gatatagctt atatatttga taattatgga gtagataaag agtatactat 34320

tagttttgac atcaagtcca ctacggtagg cacagttact gtttatagta ggtacaaaga 34380

tgaagctaaa tattctatag gaaacaaaac tgttcctgta acgactgctt acacaagaca 34440

gcaagttgta gtaacaccta ctagaaacac agcagaacaa gctaaatatg cagtcagctc 34500

tattctaggt tttcttaata gtacaggagc aatatctaca gttaaggata taaaaatatc 34560

agtaggaaac atctcgaata tgatttggac acctgcacct aaagactaca gtattcttac 34620

tgatggagta acaacaagtg ctaacggatt cttgagtgga gttaactcct taactattga 34680

cttaggtagc cctagatatg atattgacaa catcactatg tggcactatt ggcaaggggc 34740

tgaacctagg tactacaaat atgtaaaact agaagtatca gcagatggtg tgaactggac 34800

tacactgttt gatagtgatg taagtggaac ttattacgag actagtggag gtaagcaggt 34860

tagcgtacct gctataaaag aataccttaa gagtactaat gctatatttg cacctagcgt 34920

agcaggaaca ggtacttttg agagagtaac cgaagcaaac ggagtaagtt ctataaaagt 34980

gacacctgat actgataaga cagctagaat gtacaagttt ttcggaactt ctacagaccc 35040

taacgctaat tcgagagacc ttctagaatc agggctaact acaacgaaga aatacacagt 35100

agcagtatcc ctaaaacctc aaaataatag ttcggttatc ttagcggtaa aagatggaag 35160

taacactaac atagagtcgg gctccttaaa tattgcaaaa gatgtgtgga caagagtttc 35220

ttacaatgta acgttacctg caacagctat tgataggcta gctttatgtt tgtacaatgc 35280

taacacagga gaatatatta attataaaga ttggagcatt aaagaaacga tgtttccata 35340

aagtaaaaat tatggtataa tagagatagg gcacaaagta gtgccttatc tttgctatat 35400

tagtaatgag agtacgaata caataccgat tttaaaataa ggaaatgagg taaataaatg 35460

gcaatagcaa catataatgc gcatgtatat gcagcactaa acctaaaaag taaggtcgat 35520

acaacattta tggcaattgg taaaacaaca gcgtggactg atgagactaa ccctccagaa 35580

ccagacccaa atgctacagg gttaacagaa gttatcggtt acaagaagct aaaaactatg 35640

tctctatgta gaccacaaag aacaggtgaa acaccaacac tacctacagt ttcttacgga 35700

aataaaacat gggttcttgt acctgatgca caagcttata cagaaggagc taaatggctt 35760

tattgtgagg cagagttcgt aggggatgaa ctaccagtag gaacatacag acaagtaggt 35820

gtatttacag acttagcgcc aaaatctggt gtaacaaaac cgaatttatt accaagtgaa 35880

gtagctaatg taggtgtact acagtttttt gagaacaagc agttccaaaa cagaacacca 35940

caagttacag ctagagaaag attcgtagcg gaattataag aaagggtgaa taagtttggc 36000

agatttcaat tttagttcag caccatatca taataggttc aatgccttga atgattggac 36060

attagcactt ttccaaccag acagacccct tcaacagtcc gagttaaacg aaatgcaatc 36120

tattttgcat tattacctaa aatctgttgg ggatgctgtt ttttctgatg ggaacatgca 36180

aacaggtatg gagtttgtct tggataactc aggaaactta actgttggtg atggtcaagt 36240

ttatgttgag ggcaaagtaa gaaacttcaa aaaacaaact ataaaattaa ataagatagg 36300

tagagagttt gtaggagtaa aagtagaaca gtctattatt accgcagacc aagacagtag 36360

tcttctagac caaacagcag gagttcctag tgctttttct aaaggagctg acagactaaa 36420

agaaactgtt gttttaacgt tagacgaccc tcagtcagca actatctacc agtttgataa 36480

cggaaaactt tttatctcct ctgataaccc agagatgaca aaaataaacg aaattctagc 36540

tgaaagaaca tacgatgaat caggctctta tcgtgttaac ggttttaaca tgtggacaga 36600

acctcatccg acagacacta gtaaaattca gttagttatt gacacaggta gagcttatgt 36660

gttaggatac aaggtagata aaccaacatc tacacgtata cctttagata aagctagaga 36720

tactcgtagt gtagccaatg aagggcaata ctacgataca gcagttaacc gtattaagct 36780

aggaaacagt cctgtagaaa cagtaacacg agttacaggt caagttcaag tacctaagga 36840

gcaagtgagc agaggttctc aatcaggagg caccgacttc ttgagtagaa cttccgtatt 36900

ccagatagac cgtgtgtgga cagaaaaccc aacatcagga gaagttatta aggaatacac 36960

tcaaggacaa gacttccagt taataaatgg acaagcggtt tcatgggcac ctaacggagc 37020

agaacctacg gcaggagcaa cttactacgt gtcttaccgt tataacaagt ctatggtact 37080

gaatacagac tacaagataa ctattgaagg tacaggggat acaaaaactt ggtatattga 37140

ttttagtgat atgacaggaa gtaaacctat ccaacaaagt ttagtaaacg ttgattacaa 37200

ctactacttg gctcgtaaag acctaatagt acttgataga tacggagaag ttacagtaca 37260

caaaggacaa ccagataact taagattagc ggaagcacct gcacaggtag acccctcaac 37320

acttcaaatt ggggtagtta ctatttaccc tgaaagtgag accgctagtg ctatgtcatt 37380

ctctgttact cgtctttcta tggctgattt acaaaaaatg agaacacgta ttgataatat 37440

tgagtataac gaagcagtaa atgctttaga taatcctgca atggcaggag aaaacccagt 37500

gacattacgt tctgtattct ctgacggttt tatttcacta gacaaatacg atagcacgca 37560

tccagatgct attatagctt ttagttttga ggatgcagaa attaccctac cttaccaaga 37620

agttaacaag gtagttccaa aaatgttaga gaacgcatca gaagcttatg tttggggacg 37680

tttagtatca gcaccattta cagaagagaa aacaatttct caaccttttg catcagaagc 37740

tatgaacgta aacccatata atgtgtttaa taaacaaggg gttattactt taacaccttc 37800

tgcggataac tggattgagg aagaacgagt aactgtaact caggacgaag ttaagtctta 37860

tacggtaaac cgttggtggc tacatggagg taactactat tctgatgaag ctagatactt 37920

gcataacaac ctagcactag atgcagggca gtcatggagt gggactcctt ctcagaacaa 37980

acaagtaaca ggaacaacat taacagcagg tggacaacaa acacttgagt ctatgataga 38040

gtttatgcga caaattgata tagtggtaga aggtaaaaac tacttgccta acgctaacaa 38100

cttatacgca ctattcgatg gtgttcgggt acctattaca ccagcttctg gttaccgaaa 38160

aggtacagac acaggaacat taatggctaa tgcagatggg acagtaaaag gaactatcac 38220

tattccagca ggtatccgtt gtggtgtgag agaagtaact ttacggaatg cagataactt 38280

aggtatttct acattcactg cacaaggtag aaagaaaacc gtgcaagaca tcattatcag 38340

aactcacgta acagctaact tgtatgaccc gttagctcag tcattccaat tcacagatga 38400

ccgagttata actagcttag gattacattt tgctagtaaa gatgcaggag aaaacaacgt 38460

tatcgttcag attagaggta tgagtgacgg aggacaacca aataaaacta tttatgcaga 38520

aagggtatta accccagctc aagtaaatgt gtctgctgac agtagtaagg aaactaaagt 38580

cacttttgat gacccaatta tgtgtaaagc aggtcaagag tactgtatcg ttattgtaac 38640

agactctgat agttacacta tgtggttagg tactagaggt aaacctagaa tagactcacc 38700

tagcgactta attacgtcaa acccttatgt acaaggtgta ctatttagtt cttctaatgc 38760

ttctacatgg acagcacacc aagattctga tttgaagttt aatgtatata ctgctaagtt 38820

taacgaacaa gctgtactag aatttgacac aatgaaaaat gttaaagttg atagagtagt 38880

actaatgagt acgtacctaa ctcctaataa cacaggatgt atctgggata tgaagattgt 38940

tctagacaat gaacctacag gagttacagt agcagacaaa gaatggcaac caattggtaa 39000

ctaccaagat ttggatgtag agcaattagc tagagaagtc aagttaagag ctacttttgt 39060

tgctaacaac tacatttcac cattgttatc attggacgac atcatgtttg caggtttctt 39120

aactgcttta aaaggtagtt atgttagtag aaatattaat atgacagact ctccttataa 39180

tacagttaag attagttacg aagctttcct accagctaac actacagtaa aagcacagtt 39240

tagtactgat gcaggtatta catggaaaga cttgacgtct attcctgtag taacacaaca 39300

aacagccgat ttctacaagt atgaatacga agagaaagtc atccaatcag gcacatataa 39360

aggatttaag gttcggctga acttagcttc acaaaacagt ttcttaagac ctcgtgctcg 39420

gagactaatg tgtttaatga aagatgaata ggggaacttt tgttccctta tttttaacta 39480

aaaaggggcg gtaaacatgc cagagtcata tagagacccc cattcagggg ctttaaagtt 39540

tattcctacg aaacaggaga aaataattaa agatgtgact gaaaaactaa gagaagacga 39600

agaaaaaatt cgaaagaaaa tagaagagtt agatattaaa ctagcacagg cagacagcag 39660

gttgcaagaa ataactaact tgtatgaaga attaaaagaa ctaaaggagg gtaactaaat 39720

ttgtctagat acgaccatag tactgtagag tacacagaca aaattaagga cttaacagat 39780

tcggtaaacc gagtaggaaa ctacttatca ggagacaata gcccttatga cgatgttcag 39840

aaattaaaag caattacaca aaatattaag ctaactaaag atacaggttt agctaaaagc 39900

attacagcag gaacaactgc gttgaggtca gtagtcgaag taggggttta ctatattaat 39960

agcacagaag ctctagccct aactgataaa cccccagagc taacaggagc ctttatacta 40020

gtaaactacc cgacaacagc ctctacttct gttaagcaag aagtacacat gtttgctaca 40080

ggaacaacag gtagctacgt agggtaccga tggatttctg cttctagtgt cagctcatgg 40140

tggacttatg aaaacacact aggttcacaa gctaaagcag ataaagcatt agcggacggg 40200

aaaacatata ctgatagctc agtaaactcc gctttgcaag caattcaaaa ctcagcacaa 40260

atgtacaagt taacagcgga tgatggtaag cctattgatg cttctgctat ggctactcca 40320

ccaactagtg tagcttctct aacgaagaca ggaatatatt actttactgc cgcttttgga 40380

aatacaatgc cagatacccc ttgcacaggg caaccattct ggttggttgt tttacagcat 40440

gtaacagata acagtatatc acaatctgtg acagccaaca ctgtagcagt agagagggta 40500

gtcgcagata ggattattac tactctaggg gtaccgagta agtgggaata cagagctaaa 40560

gcctctaatt tcttctttag tgcttccaat tcgtcacgta taacactagt atcgacccaa 40620

cagaatatta taacacctaa taaatttatt aataaccccg gtagtttacc actatctgcg 40680

ataaaccccg caataactat tccagaagat gggatgtacc aagtgatggc ggccctaaac 40740

attaacggta ttttagaaaa agtgtaccta gtaagtgagc taactctact aataaatgac 40800

gtagtacacc cagctacatt tggaatggta aaaacagcag ataatgctaa cgggcagtac 40860

tctttagcag ggaatggcat atatcaacta aaaaaagggg acaagttaaa actaagaagt 40920

tactgtaaca atacagggaa taacccttac ttggatgtag acaaacttta tatatctgtt 40980

ggtaagattg cagacatttc attatttaaa taaacttagt atgctgagaa aggaagtcac 41040
aataatggca gaagaaaaat tttcattaga cggaaggcag tatgtaacag cagtcctata 41100
tatgtaccct aacctagacc ctcagtacga ctttggagtt gtatcatccg aagggaaggt 41160
agaggctttt gaatggaacg taaaaaatat aacaaagcct tctattgcac agttagacca 41220
agcatataaa gactatgtaa aatctatcgg aagtccaagt aacccaccag ccctagaacc 41280
agaggtctca aaagaagcct caataggtaa gattctaact gaaaaattta gtgcgttaga 41340
ggaagttaat gctaatatgt tattggaaaa tttagagctt caagctagcc tagataaaac 41400
aaaacaacaa aattcagaac tagtatttga gctagtagac aggggagtct tatgagtgta 41460
aattggtatg agttagtttt agattactac acaaaaggtt attacacacc aagtcaggta 41520
aaaagatttg ctgaggtagg aaaactaact ttcgagcaag ccgaagaaat tatagctaca 41580
aaatcataga gtatgtttaa actatatagg taattaagac taggactaat atcctagtct 41640
ttttatgtta taataaagat atagtaaata tatttggagg agtacattaa tgagaattgt 41700
tgtagatata atgaatacac gagttttatt cgataaagaa gaagaggcaa ttaaaaaaag 41760
atttcttagg gatatgcacc aaaaattagg ggtaaaagta gatgggttct tctttagccc 41820
agcatataaa tcaggtcatt gggacggtat ccaagacttc tatgacttta aaacaaatac 41880
gttccctaca gggctgttgc cacaagtgca agagatacta ggagctatgc aaaatgatgc 41940
cgtatttcaa tttgagatta ttgatgacag accagacccg tttttaacag tagaggactt 42000
agatagtgag ataactctta taggagatga cggaggagat atacagctta gagattatca 42060
gtatgactct gtaaagtcta tcattgaaaa ctatagtggg attatcaata ttgcaactaa 42120
tggaggaaaa acggagattg cttctggtat cattcagcag attttaccgc atttagagtc 42180
tggggagaga atagcctttt ttactcactc ctcatctata tttaaccaat caattgaccg 42240
tatagaaaaa agactaggta ttaaagtagg taggctagga aatggtaaaa aggacatcag 42300
acaagttact tttgtaatga tacctacgtt aacagcctcc ctaaaagacc ccgaagttgg 42360
tttgaaatta acgcataagg aacgagttat taagaagtta gctaaagagg tagttcctcg 42420
tttcactatg caaaaaaatc atagaaagct tcttaatatt tatattgaga acaacagacc 42480
gaaaaataaa gtagaagaag atataaaagc tcgactagaa gacttgttat attcctgtgg 42540
aacagactcc gaagttctaa tgggactaaa aggttttaac gcagaatatg aaaaaattat 42600
tgagaaaaaa aataaaaaac tatatgacaa gaacaaacaa gctagagaat ttctatcatc 42660
tgtatctgtt atgattgcgg atgaggcgca ccataccagt tcagaaactt ggtacaactc 42720
tttgatgtct tgtgagaatg ctatgtacag ggtggctttg acaggttcta tagacaaaga 42780
aaataaaatg ttatggcaga gaatgcaagg agtattcggt caaattacgg ctagaacaag 42840

taatgagacc ttaattacac ttgggcactc tgctaaacca acgattaaaa tattccctat 42900

caaggctcct acagatattg aaaatgtaga taactatgct actgcttatg aaaaaggaat 42960

agtagaaaat gattatcgta atttacttat tgcaaaatta gcaaagatgt ggtatgataa 43020

agacaaaggt gttcttatta tagtaaatag aatagagcac ggtaacagat tatctgaact 43080

tcttgagtct ctcgaagtgt cgcactactt cattcatgga gacttagaca tagatttaag 43140

agaacaacaa ctccaaaata tgagagatgg gaagcttaaa gtgatgattt ctacatctat 43200

tgtagatgag ggagtagaca tctcaggtat tgatgcgctt attttaggtg caggagggaa 43260

gtctttaaga cagacacttc aacgtgttgg cagggcttta cgtaagaaga agcaaggaga 43320

aaacacttgc caagtatttg actttaaaga tatgactcat agatttttac taagtcactc 43380

agaccaacga agacagattt acgtagatga aaactttgaa attcaagaca tttaggggga 43440

agaacaagaa tggatttagt atatgtagga tgtcagatgg aaataggaat gaggaaaggt 43500

ttacgagact ttctagtgga tgttgctaaa acagctcagc aatggggaga aagcgtggct 43560

atggttactc aaaaacatag cctagctgag caaacaggag taaacattag aacagtctca 43620

cgttatatag ctgaacttga aaacaaaggt attattaaaa cagaagcaaa aagaggaaga 43680

aacggtggac tagttatcat gtttaataaa aatatggtta cttttaaatt aacagacaac 43740

cctataacct ctgattcacc tgatgcggta gaacttagag accgtatgtt ccctaaaaag 43800

aatgaagacc cgaataaaga gaaaagacct cgtagaacaa aagaacaaat ggcagaggaa 43860

aaagtactaa aaaatcgtaa gagaagtctt aataatgagt taaacgatat gttagacgat 43920

acattattcc ctacagaaga gatatttagt aaaacagagg agcctgagag gtactttaaa 43980

gcttatttac tgtctcgaat ggttaatgct tatgcgtata tttatcctac agagagagag 44040

ctagacatgc taagcagagg ggtgcaggta gaagcagaga aggcacgaaa aaatagagta 44100

cgagcttact cttttaactc actacctgct agattttttg ggacatctca gtttacttgc 44160

tacttaaaac tacaagactt attagaaaaa gagaacatta acccattatc ttatttaaca 44220

gttcaatttg actacttaga ctacagatta gataacagtc ttggaggaac tccactagca 44280

tatagaggta tccctgaggc aaactgctta ataggtgaga ctgcctttag tcgttacaca 44340

tctgctaagg acttctacac tgttatgcga ggtaaaaaca tgttacgcac cagtacagca 44400

gatgttgcct ttaaaggtgg aaaataccct attgtggagg cgttattaca cgcttacaat 44460

gtaggtaagc aagatattac tgcactaaac caacaaatag aggatatgaa gacacaagct 44520

agtgtgttcc ataaatccga agccctagca gacttttatg aaacaacaaa actattagtt 44580

gaagaaagtg ctttagaaac aaatgaaaaa gacgagctat acaaatttgt tactgaacaa 44640

gttgttttac actctcgtaa gaatgcaatg agtcttagcc aacagttgct aagcttccca 44700

cttcaaattc aacgtgtaag acagacagcc gaattaaaag gattagacaa aaaggactac 44760

tatacttttg taggcaactt agagaaacaa gtgaaagtaa gtagaaacac gtttaactcg 44820

tttactgaaa aaggtaagct tttacacgag tctctagagg caaacagtac tttttatagt 44880

accattcgta tgctaggaga ctaccgagga ctaaccgtaa acatcccgtt actcggagaa 44940

gctatcaaga aattaggaga ggaacatatt cctctagatt cttatggaat gttagatata 45000

acacaggttt acgacaagta tttaacagac ctgcaaatgc aagaagatga agagaactat 45060

gaaacaccta caagttttat tgttaagtaa ggaggaggag atatacgttg agttccatta 45120

ttcaaaagca agttatatac cgagctatta gagacccttt atttggtaag gatgtgttag 45180

cacagttacc tagtaatgta tttgacgaga atgagcacta caaaaatcta gtaattatta 45240

ttaaaaagca ttaccagaca agtaacaagg cgcttgatga aagtacgttg ctaactatta 45300

cggaggaaca tttagttaga cagaaaaaga gcttagaagt acaggaaagc tttaatgaga 45360

caattcatga tttgtataaa attgatgaac taggtataga cgatgaggct gtcggagaaa 45420

gcattaaacg attcgtcaaa aaaaatctgg cagctcactt gataaaggag actttgtcta 45480

agggtgaagg gctagacaac gaagaaacat tagaaaaact atacacaggt atcaaagaaa 45540

tctctatttt agatattaac cgtagtgaaa acactttatt tagtttcttt gatgatgtag 45600

aagccaagaa aaagatgcta caaagtatcc accaagataa gttctctaca ggattcacag 45660

acttagatgt tattgctgag ggtggtattg gacgtggaga gctcggtcta gttattgccc 45720

ctacaggtgg tggtaaaact atgtgggcag taaaccaagc aaaaaactat gttgctcaag 45780

gtttaaacgt tttgtatgta gtgttagagg aacgattaga acgtatgatt ttacgttttg 45840

aacaaaatat gatgggtatc cctaaatctc gacttatgcc agataatgaa cttgacgaag 45900

aagctttcga caaattacaa acgatttatg atattaaacg taaacaagga ttaggtaatc 45960

tttacattga aaagagaatg cctcaggaag ttagtcctac aatgttagag caaattatcg 46020

ttgatgctag tgttcgtaaa gggaaacatt tagatgtggt tattattgac tatcctgact 46080

taatgaaaaa ccctcatgaa agagataata gtgagtcccg tgcaggagga aaactatacg 46140

aagaaatacg taagttagct caaacctata agtttgctgg ttgggtactc tctcaggtta 46200

accgcggagg atattcagag gatgtactta ctgctaaaag tatcgagggt tctaaacaga 46260

aactaaatgc tgtggaaatt gctatgacct taaaccaaaa acctattgag tttgaaaaag 46320

gttttattag agcatatgcc gacaagattc gtaataataa cggtaaatct tataaccgta 46380

tgcttagctt taaggttgtt ccagaaacaa tgactatcag aaatataaca cctgatgaaa 46440

gagtagaaca cgatatgatt ttaaataacc tcgaagaaaa agaaggtcgt gtatttaaaa 46500

atacgaaaaa ggctacacct gacccccaag cagttgtaga cgaagtaaaa gcactaaata 46560

gtagattata aggaggagat agtttttgga taaaattata ttttttactg attttcatgc 46620

ccatattttt accgaatttt ctaaaccaga tgtggagtat gaaacagaca gatttagaga 46680

acaaatggaa gtcttacaac aggtatttga cttagctagg gagaataagg cagatattgt 46740

ttttggagga gacttattcc ataaacgagg agctgtagat actagagtac taaaccatgt 46800

ttttcgtgta tttgaaagaa acacggatat tgtagttcac ctactgagag gaaatcatga 46860

tagctttaca aactcgatga acacagagag tagtttggag acacttagag gtttacctaa 46920

tgtggaagtt tacagcaacc cttctattgt agagactgaa aaatataagt tatatatgct 46980

accttatggg gatgaagtgc aagagatgaa agactggatt aacagtttag acattgaagc 47040

aggtaaaagt aatatcttag ttgctcatat aggggtagag ggagcgttaa caggtaaagg 47100

ttcccacaga ctagaaggtg catttacaac aggagattta agaacaagcg actttgatgt 47160

tattgctctt gggcattatc acaagagaca gctactacta gatgttccta atatatttta 47220

tggggggaac accattcaga acactttttc tgatgaaggg caagagaagg gtgtatggct 47280

aattgactgt tcagatgtta aaaatacaac aagtactttt attcctatta gtagccgtaa 47340

gtttattact attgacggag ataacattcc tgatgatata gaagaactta ttgaacaagg 47400

caactttatt aggtttattg gaagtaaaga acaggctaaa aaaattgact tatatggaga 47460

agaaagtaca ggtattagag tagagctacg aaaagagtat acaactgatg ttcgactagg 47520

gattgatgaa aagtcctctc ccgaagctat ttctaaagcc tatgcagaga aatttagtcc 47580

ctctgcaaca agtgaaatag tagagtgtat aaaagaggca cttgtgagag aaaaataagc 47640

ctaaagctga ggatattcct cagcttttct tattgacttt tttagtaaag tatagtatac 47700

taaagttaca aataaaaaga aggaggaaaa gaatgttcac ttttaaaaaa gtaaagataa 47760

aaaacttctt atctatcaaa gacatggagc ttaatttaga caaacaaggt ctagttctga 47820

tagaaggaaa gaataagaca aatgaagcct ttaagtctaa tggtgcaggt aagacaagta 47880

tgattgatgc tattacttac gctatctttg gtaagacagt aggtggcctt aaatcggatt 47940

cagttgttaa taacaaagag aagaagaaca ctgctgtaat tttagacttc gaagttgaca 48000

agaataaata ccgaattgaa cgctatagaa aagacaaaaa agagggtaat atagtaaagc 48060

tctaccaagg taaaaaacaa tattacaaag agtacagtgg acaacacaga taaagagatt 48120

ttatctctat tcggtattga ttttaatacg tatgtaaatg ctattatgta cggtcaagga 48180

gacatgccta ttttctccca agcaacggat aaaggtaaaa aggagatact agagagaatt 48240

actagtattg acatctatca gaaggcacaa gaaatcgcta aagagaaagt taaagagaag 48300

caagctgagc aacaagagat agaaaatgaa cttgtaaaat atgactatga gcttgtagct 48360

attgacaact tagaagagca agaaaaagaa aactataaag cagtccagca aacaatagaa 48420

ggtatccaag caaccctatc agtagctagt acagagctag aaaactttat gacagaggct 48480

taccctaaga aggcagaatt agagaatagt attgcacagt ttatagtccc tcaagtagat 48540

aatacaaaag ttaacgagct aagagctgac ttaacggagg cagaacgtaa attattacaa 48600

attaacagta ttaaagaagg ttttgacaga gacttacgaa agctagctaa agactatgaa 48660

aacctttcta gtagcaagat ttgtcctaca tgtggaacag acctcggggt ggatagtgaa 48720

gcacacattg cagaaacaaa agcagatatt gaaaaacaaa tgagtgttct tggtgcaaaa 48780

acccataaaa ttttagtagc gttaccttct gtaaatacaa ctattgagag caaaaaggag 48840

ctcgtacaac aagagatagc taaaaatgag gagatacaaa gaaattacaa tcagctagtg 48900

tcaacagtca ggggatatga acaagaggta caacaactag aaaacacaaa aaatagttta 48960

gaagagaaag tggcttcaca acaaacatat ttatcacagg tacaaagtat gcctaagcca 49020

aaaagtagag acaaggatag agaagaagta cacaaaaaaa tagaagcaac taagttgcaa 49080

gtgctagaca aacaaaaaga gactgagaag tatgaggaag cagtaaaagc cttctcaaat 49140

gctggtattc gttctatggt tcttgactta gtaactcctt ttttaaatga gaaagctaat 49200

aaatatttaa cagtgttgtc aggttcagat attgaaatta attttactac acaagttaaa 49260

aactctaaag gggaactaaa agataagttt gatgtagaaa taatcaatgg tagtggaggc 49320

tctaactata aagctaactc tgctggtgag aagaaaagaa tcgaccttgc aatttctttt 49380

gcaatccaag atttagttat gagtaaggca gaactatcta caaatattgc attgtatgac 49440

gaatgttttg acggtctgga cagcataggt tgtgagaacg ttattacttt gttaaaagaa 49500

agattagaaa cagtaagcag tatatttgtt ataacgcaca atgagaactt aaaaccttta 49560

tttgagaagg taatcactgt gcagaaagag gatggggtta ctaccctaat aaactaagga 49620

tttggagagc tataaaatga aagtaaaaat tatgacagaa gaaaaagggg tactcacctc 49680

agaagtaagc aagaggtcaa ctaagaaaga ggctatctac tccgcaaatg tttttcctat 49740

tagtagcttg tacaagtggt acccctttaa tactaactac gcctatgtag tagacaagca 49800

tggggtatac agactcactg aactagatga ctgtattcat acatcgtata acttggtaag 49860

tgaagaaaaa gctaagttag acccgacaaa gtggcacaat aaaaccgtaa cacaaagcaa 49920

cttattactt tgtagactag caaataagct agggataaaa agcattaaag atgattttaa 49980

ccaacaagat gtagaaaacg gatttgaggt tatcagtaca gagttagcct atatcaaaga 50040

acaagcgaca gcacgttgct acggggataa agatgtcttt gaaagaagtg aactactcaa 50100

gaaaagtaaa gaagaaagag aacaagcata ctttgagtac ttaactgtag aggaggtaaa 50160

cttccaatat aagttaggag agctatacgc tatgcttgta cttatgcgga aggtggtaaa 50220

ctactaatgt ttttagacta cattacaagt gagctaggaa cacataaaga tatagggcat 50280

gaattacggt tctgttgccc tttttgtgga gaagacgact acaaatttta tgtgaaggta 50340

tcaggggaca aaacagaagg tctttggcat tgtaaaaaat gtaatgaaaa aggtaaccct 50400

gttacttttg ttatgaagta tcttaacatt aattttaaag atgccttaga tatactagag 50460

gtgtacgact atgagataga cgaaaacagc ctatctagca gagacgtagg ggtaacagag 50520

gaagagtttc tgcttatact attaaagaaa tctagtgagc ctgaggaagt agaaattagt 50580

gaggaagtga ctctcacgcc acctaggcta cctaccaatt ttaaaagact agcagacaac 50640

atgaataacc ccgaggcatt acctttctca aactacgcac actcccgagg tataacttta 50700

gaccaactat tcctacacaa cataggatat gttatagagg ggcaagtaga aaaagtagat 50760

ggtaatatga tgacacttag aaatcacctt gtttttctaa cacatgggaa tgatggacaa 50820

taccaatatt ggaacacccg agccataaca cctatgggga taaaagcttt taatgctcct 50880

agcaaacagg acgagtactc taaaagaaca actgttttta acctaaatat tgctaagaat 50940

acgccttatg ttattgttca cgaaggggta attgatgcct taacaatggg agagtcggga 51000

gtagctactt ttggtaaaca gataacaaga gaacagatag agttactatt aaataatgtt 51060

agagaagacc agcctattta tttattccta gatacagagg cttatgaagt tactactaag 51120

atagctagca gtctttatga aaagcataaa gaaacctaca tcgttatcca tggagatgag 51180

gatgccaatg atatgggata tgaaaaatct tgggactgtt taagaaataa ttcagtaaga 51240

gctaatgagg aaggtataag tattttttta gtatcttgca ttcagtaata aagtatgata 51300

taatagagca gacaagtaag aaggaggaat tttattgagt aataaagtaa atgtaaaagt 51360

taagttagag ggaggagcta ctatgccctc acaagggtat gataatgatg tagccttcga 51420

tttatacaca tcagaggatg ctattatttg ccctgatgca gtgggagcta cactaattcc 51480

tacaggatta agaacagagt ttgaccccac taagtttggg ctattcatca gtcctcgttc 51540

aagtattatg aacttacctt taaccctagc taacaacaca ggaattgtag aaggaacata 51600

tcgtggaggt attggtattc ctttaaaaaa tacgttgtct atgacaggtt tttctgattt 51660

tgcactaaca tggaacccag acaagaaggt agtagaacgt atccctgtag agaagttacc 51720

tacaggtaaa cttagaagtg cggaaaaaat gttttacaca gaccaagcat tattgtatga 51780

taacacacta ccagaagaac tagcagacca attgtttgtt actttggtgc cgactgggac 51840

attcttttta ccaaaaggct tccgtattat gcaagcttac cttctaccta aatatcatct 51900

agacctagaa aaaacagaca aactatcaga cagccctaga ggggataaag ggtttggcag 51960

ttcaggacat ggaggaaagt gattccttta gaccctatta tccttattgc cgactctata 52020

ggcataggtt attcaaaagg aaatataaca ctgtctttaa aactacggga taagtatacg 52080

ggtaccgagg tagaaaaaat agctttatta gccgttggta gcttctcaga aaaagtaacg 52140

cttgttagta gtgagctagt agataatgtg tataaaataa aagttaagat ggaggaatta 52200

tgaaagattt tactcagtat tttaaagaag tacaagatgc acaaaaaaga gaggactctg 52260

aaactgtaga aagacttgca gtagaatatg gagataaaac tattacgtat aaagaactta 52320

gacatacgtt aggttccgtc ttgttgccac taattattgg aacaatggaa atcaataagc 52380

agttggcaga cacctcttta gacatcgtta tcaatatgtt agaaaaagag aatgtattat 52440

cagaggatac cttaattgat ttaaggcaac taattactag catgaaacaa tcagtagaag 52500

gaggagaagg agaaaatgag taaagaacaa aagcagttaa gtaaagagga tttaacttta 52560

tgggttaact ttgtagtaac tggtaaggtt gaaaaaggga cggagaaaca gctaaatcgt 52620

atttctaaac gagaggtatc tgtaggagat gtaaccaccc taatccgtgc tcttacaagt 52680

catcatgatg cgtacattag ttctcttatt gaagctaatt cagttcttag agtggtaatg 52740

cgagatgagt taaatatctc agaagctcaa atggacaagg cgttaaatac ctataagcaa 52800

caagtaggta cagcactcca aaagacacat gatgctatca tggaaaaagc acaagaaaat 52860

gtggagttca cagaagaaga actagaggta cagaaaaaac tagaagaata tgatactaaa 52920

aataaaaaag aggaataaga atggctagta caggaagagg aagtcaagta aaaggttcta 52980

aatttgaaag agagaccgct aaagctttat ctgtttggtg gggagctacc tttaatagaa 53040

caccgggttc tggcgggcta aaatgggcgg aagaaaacaa tgtaggtggc gatatagtag 53100

cccctccctc cgcaggtttc cctttcattg ttgaatgtaa aaaacatgaa gggtggacac 53160

tagacaatgt attccttaat acaggagagc ctaagtcttg gtggaaacaa gtagtatcag 53220

atgccacaag aacgaataag atacccttgt taatgttctc tagaaacagg gctaaaagct 53280

ttgttatgct accttatgat gacttgctat ataacgtatt acgtgataaa agaagtacag 53340

tagctagaac tacagtgtca tttaagaata tcagggaaga aagtgtgttc tatgatgtaa 53400

tggtgttcac tatagaagag ttaactagct tctctcctga ttttttaaga gaacactata 53460

gtaagtatag ttgggatttc ttttacaaag aagtggacga gaactctgta gaagatttag 53520

aaacaggtat agataaaatg ttagacaacc tagaaggtta aaataaagaa aaacactaag 53580

taggtctttt gtggtgacat atacataagc ctacttttag tgtattgtgc tatactagag 53640

tagaacggaa aaacaaaata tgtttggagg aatatcgttg acaataatag taaaagacaa 53700

cggaagtaga aaactacctt ttgaggaaga cagactaaac aggtacctag ataacataaa 53760

aaaagagttt cctaagttaa atatagagag ttatcgaaaa aaggcaatac actatatttt 53820

aagcaaggac gagtattctg tagacgaaat ggtagactac ctaataagag aagcagaatc 53880

tcgggtagac gttaccacac ctgaatggga gttttttgct actagactat atttacaaag 53940

attgtataag aaagctagta aaaacaggtt ttacgatgta attatgaagt atggttctta 54000

cgtgggtttg catgagagtc tagcacaaag aggaatatat tccgtagata ttttagagaa 54060

gtattcaaga gaagagctta tggaagctga aacgtttatt gacagtagca aagaccttct 54120

ctttacttac aatggtttgt acctactagc tactcgctat ctagcaacag atgaggataa 54180

gaaagtattt gagttaccac aagaacgatg gttaactatt gctttatttt tgatgcaaga 54240

tgaaaaagaa aatagaatga agttagtaga agaagcttat tgggcgttaa gtaacttgta 54300

tatgacagta gcaaccccca cattagcaaa tgcaggtaaa gtaggcgggc aactatccag 54360

ttgttttata gatacggtag atgatagttt gcaaggtatt tacgatagca atacagatgt 54420

tgctagagtt tctaagcaag gcggaggcgt aggagtctat ataggcaatg taaggtcgtc 54480

aggagcacct attagaggag ttctcggtgc tagtggggga acggtacctt ggattaaaca 54540

attaaacaat acggctgtta gtgtagacca gctaggagca agaaaaggcg ctataactgt 54600

aacgttagat gttttccaca aagatataga agcctttctg gatttaagac taaacaatgg 54660

agaccaacgt ttacgggctc acgacatttt tacttctgtt tctattcctg atttatttat 54720

ggaagctgta gagaatagag gggaatggta tttatttgac ccttacgagg ttaagcagaa 54780

aaaaggttgg tacctacaag actactatga taaagaaaaa ggtagtggtt tattccgaga 54840

aaaatatgaa gaactcattc aagatggcac gattagtaag aagagagtga aagctcttga 54900

tatgatgaaa cgtatcatga ttagtcagct agagacagga acaccattca tgttttatcg 54960

agatactgta aatagagcta accccaactc ccatgctgga atgatttatg ctagtaattt 55020

gtgttaattc tatggcacct ttgggtagca atactcatcg aaaacttttc ttaaacgggc 55080

atagctgaat acgatgaata agctggtaag agagcctaag tccttgagtg aggatagagg 55140

taatcccgtg ctaaattata ataaaatctt tattgtataa atgcctaacg actatcgaaa 55200

agctactttt tagtagaact tagtagagta cactcaagtg agtggaaaca gaaaagactc 55260

ccatgtggag tatgatatag tcttttcttt acagaaatgt aaagcagtta gtacactaac 55320

gggtagggaa ttaacgaccc ttactgaaag caagcaagaa atcgcacaaa atatgtcagc 55380

tacggtagtt acagaggaaa ttatagatgg agataaaatt gttgtaacaa aagaagcagg 55440

agacttcgta gtttgtaact tgtcttctat taacttaggt tctgtatttt ccgaagaaga 55500

cgttatcgac cgactagtaa ccattcaagt taggatgcta gacaatgtaa ttgagttaaa 55560

caaactacct gtaccacaag ctctaattac taaccagaag tatagggcag tgggattagg 55620

aacttttggg tggcaccacc tactagctaa aaaaggtatt cattgggaca gtgaagaggc 55680

tactaatttt gcagatagcc tatatgaaag aattgctttt cacactatcg aagcaagtat 55740

gaaactagcc gaggaaaaag gagcttaccc atactttaaa gacagttcgt gggatacagg 55800

ggagtacttc tctagtagaa actatactag tgatacttgg gttgacctag ctaaaaaagt 55860

gaaagagaaa ggtattagaa atggttactt aatggcagta gcacctaata tgtccacagc 55920

gcagatagca ggctctacag cctctattga ccctatctat gactctttct attatgaaga 55980

gaaaaaagac tacagacgac ctgtgatagc ccctgagttg aacttgaaca cttacgcttt 56040

ctataaaagt gcttacaaag taaaccagat tcaaagtgtt aagcagaatg gtaaaagaca 56100

aagacacata gaccaagcta tcagttttaa cttctatgta cctagtggca ttaaagcaag 56160

taaactactt gagttgcaca ctacagcgtg gagtgaaggt ctaaaaacaa cttactatgt 56220

tcgttctaat gacattgatg tggaagaatg tgagtggtgc tctagctaac agctattgac 56280

aacatgaccc ttttatgata tgcttatagc ataaagtaag agggtttttc cttgttgcag 56340

agaggtgtga gaaataattc aaggtatttg acaaacgatt taatgtgtgg tatacttgcc 56400

ctaaatgtac aggaaaacta ctaaaaatag aggaggagaa ctaattgtta aaaaaaataa 56460

aaatattaga accactaaac cctaacaaat caacagcgat tattaatgga gaggctagtg 56520

gtatcgtaaa ctggaacgat attcggtatc cgtctttcta tcgagcttac aaagagttaa 56580

ctacaaatta ctggttgcct gatgaggtag atatgaagag cgatgccaaa cagtacaacc 56640

acttatccga agaagagaag tatgcgtttg actccattat cggactttta gctacacttg 56700

actcacccca aacacgtttt atttataaca ttgcagagta tgtcagtgat gcctccgttc 56760

atgctattac agctatcatt gcacaacaag aagctattca taatgagagt tattcttatg 56820

tacttgcttc tattactaac ttacaagagc agaatcgagt atttgaggaa gctagaacac 56880

accctacgat tattaaacgt aatgcaccta ttatgaagtc ttatgacgat ttcatgaata 56940

acaaaacacc tgagaatatg gtaaaagctt tagtacagtc atctatctta gagggtatta 57000

atttctacag cggttttgct tacttctaca acctcgttcg tcaaaacaag atgacgggga 57060

caggtaaaat cattagcttc attaatcgtg atgaattagc ccattcgaaa tttatttctg 57120

agctaattag agctattgta ggggaaaacc ctgaactaca aacagatgaa ctaaatgact 57180

acgtgtacgc ttcttttaga catgccgtag agctagaaca agcgtggtct agtgaggtac 57240

tacaaggcat tgaaggaatt gatgtggaag aaatgttaga ctatgtaaaa tatcgtgcta 57300

ataaaatgtt agggatgtta ggtcttcaac caatgtatca aggtcacaca gaaaattcta 57360

tgacatggat taaagcttac gccgataact ttacagaaac aaaaaccgac tttttcgaaa 57420

tgcgtaattc tagttacaag aaaacaaatt tagacaatgg gttcgatgac ttatgagggt 57480

agggatttta tattcttcat tgagtggtaa cactagtgaa ctagctcaag gaattaaaga 57540

taacttggag gaactagcag tagaggtgaa atcttttcat attgaaagag aagaccaaat 57600

gactaatgaa gtaatccctt atctatctaa ttgtgacctg attatggtag gtacatggac 57660

aacagattac ggagtcatcc ctgaggagat ggaagaggct ttagaaaagt atgactatac 57720

ttgggtagat aagcctgttg ctgtatttgg aacaggagaa acccagtggg gtatggaata 57780

cttctgtgga gctaatgaca aaatagccaa aaagtaccac tctccttttg atacacttaa 57840

aatagagcag tttattatca cagaagagca agaaaataag ctattaagtt gggttactga 57900

tatagttaag cactggaggg agtagctatg tacgagctag aaaaacttct aggtaaagat 57960

aaacatggaa atcagataag aattggagat agactaagtt tacaactaga tgatggggag 58020

taccgagagt gcgtagtagc ggttagaaaa gttaatacta tcgtgcttaa ccctgtagct 58080

ttaactagta aaagtgaaac tgtaagtgta acaggtgtat tctttgtagt tactgaccca 58140

catacaggag aagaactacc tttactacct aaagtagata gtaacggtgt ggtagacact 58200

caaaaagcag aaataatctt ataattttat agaaagaagg aaaacaagtg cattcagtag 58260

aagtagaaaa gaaagaacag ttagcggaca ttgtatcaca agacaaggtt gtattaaagt 58320

tttataagga taactgtagc ccttgtaaga tagtaggtat gtctattgag gaagctagta 58380

gtaatataaa agaggatatt actatcgcta atgttaagtt agaaacttta ggagaggaag 58440

tcttctttga tttcggcatc aaaggcacac ctacactgtt gttagtagaa aaaggaatag 58500

agaaaaagag acatagtggg tttatttccc ctgatgactc caagagtttg ttaaataaga 58560

gaggcttagg cttctctttt tttgttgaca tttacaagtt tatatggtat agtaacagta 58620

acagagggga ggtaaaaaaa atgacaaaaa agaatattaa aataacgagt aagacagagg 58680

taaaaaatag ctctgttgag gcaggagaca tcttctacat tgaaggggtg tctaaggggc 58740

ttactaattt gtctggtttg tatatagtga catttgattt tcatgcaaca gaagagtcgc 58800

tgtctaggta cgctctagtt tctttggaga cagggatgtt aaacaccatt tccatcaacc 58860

agttttctct agaagaaaaa atagggtgta tagtgaaggt gaataatata gaaatagtag 58920

caaatactaa ggaggtataa ctatgaaagt aggagaaaaa gtagtaatta aaggtgaagt 58980

agttcacgta aatggttctt tggtaaaagt agagacaaaa aatagttctt tttgggtaaa 59040

caaagaagac ctaaagaaag ataagagtag tggtaagtag gtatggcaca cttaattgaa 59100

aagctaagac tatataaaaa accagaagag gtcgtaagag gaacatcagt aaaagcacag 59160

catatttatg ttgcaaatga tgaagcaaag tcattttcag gagccgaaaa atgggcggga 59220

acgtctgcct ataccttgac aagtaagaac gaatttacaa atgtaactat tcactctcta 59280

gacattagag gagagggagg cagagcttac caagttacac taaatcacga taataatact 59340

tatttatgtg acttaaggga aactcaatta atggaaacta ttcgtaacgt aggaattgag 59400

gctggcggaa agttaattgg aacttacagc tttattgctg acggctcaca gaaagttcta 59460

gtaaggatag gttcagacgc gtatgcaaaa gcaagtgaac aagcggagac taaaaataag 59520

gcaaagagaa ttcccaagaa ggagctagaa gtaggaggag tatacgcacc taaggctact 59580

tatttaggaa caaatgactc cctagactta tatatagggg acttgtatag aaatgaaatt 59640

gtagacgata acccttggta ccgagggtac tacgaacgaa gaatagaatt agggaagcgt 59700

aagaagttct ctgtaagctt aaatgtagca ttacatgagg gacaggtatg tgttccaaca 59760

tacagagaag ctgaaatcat acctgtattt ctaacagacg atggggttac ttcactaaat 59820

attagcaacc agctagggcg tttatcccca agaatggact taaaatcaag ccctaatttc 59880

tttggaaagg taggggaggc tcctgaaatt aaaacaacag cagagttctt agaatgttta 59940

agacgtggct tagaagctcg cattgaagct ttacctagta aaatagaggg caatatttta 60000

aagggaaact taaggttaca aaacgttatc cctgaaatgg agactatact caagttaata 60060

ggactcaata ctacgattga aaagcctaaa tactccgcta gttttgttaa caaaataaaa 60120

aatataaaat atatagttat agcagaacgt aaccaaaaag ggacgttaac cgcatcggaa 60180

agtacagact tccatcagtt tagtcgtgct tttgatgact taatgtccaa tattttagag 60240

gttattatag tagaggagga aaataattga cgttatatat gaatggtaaa aaagtacatg 60300

agagtacttt ccccaatggg gagattaata tcatgcctga tgaaaattta tgggtacttg 60360

aggaggagaa agcagagatt acttggcagt actcaggaaa cggtgcagaa gacttgctag 60420

ctgtcagact actagtgaac agtataagag agcacaacaa gaaacataat agcattaatt 60480

taacagttct ttatatgcca tacagcagaa tggatagact tcaaggaaat cagcccttta 60540

gcttactaga gtattttaaa atgttccctc aagttgacaa actagaaata gtggaacctc 60600

attcagacgt tacagggagt attgctagaa aacttattga cagtggaact tactactcag 60660

agcggtttgc tacagtagag atgctaccag aagtgttagc agaagagttg tctaatactt 60720

ggtgggaacc tgtattagtc tatcctgata aaggagcaaa agacagatat gacaaattag 60780

caactgattc tgattattca ttcagaagag ctactgaaaa cagacgagta ctatttggaa 60840

ataaagtacg ggactttgat acaggtagaa ttaaaagttt agagctagta ggggaaccac 60900

ctactagaaa aggatggaca gctattattg tcgatgattt aacttcttat ggtggaactt 60960

ttgtaaaaac agctacagag gctataaagt taggagcaag ccgagtagta ctactagtag 61020

ctcatatgga aggtagcgcc actaagggag aattatttga atctattgat aaggtgtata 61080

ctacgaacag tatggaagct agctacacac cagaagagct aaaagaagta gaaagatata 61140

aaaaactagg gttactagac gtaactaata ttataaaata aggaggaaaa tatgcgtaaa 61200

ttaaatgaaa tttctatgtt aatgagtgat tgctataaat ttggtcacat gtctttatac 61260

ccgaaaggta tcacacaggt gtatagtaca cttgttcctc gggataatac ttactttttt 61320

ggaggtcgta cagataaaat ggttgtcttt ggataccaga tgtttgtaca acgtttcctg 61380

ttagaacatt ttaataaaaa ctttttcgag ttacctattg agactgtaaa agaggactac 61440

cactatgtag ttagtaacgc tttaggggag aagaatgctc ggacagaaca tatcgaagca 61500

ctgcacaagc taggatactt acctataaaa gtaagagccc ttccagaggg taccttagtt 61560

cctatgcgtg tacctgtgtt aaccattgaa aatacacacc ctgactttgc ttggttaact 61620

aatttcttag aaacagtatt actatctcaa accttcttac ctgcaacagt agccactact 61680

gctttagaag ctaagaaaat tatgactaag tatggagaag aaacttgtga taacttagac 61740

catattatgt tccaaggtca tgatttttcg gagagagggc aacactctaa tgaggcttcc 61800

ttgttagcag gtatgggaca tttagtaaac tttgcaggtt cagatactat tcaagctagt 61860

gtgatggctc acaactacta cagtgctgat ttagagaagg aactcgttct taagtcggtg 61920

acagcatcag aacactccgt tatgcaaagc tacggtacag atgaagtaga gacattctca 61980

acactaatag acaataaccc tgatggtatc ttgtctctag ttagtgatac gtatgactat 62040

tacggtgtac ttacagaagt tcttccaaaa cttaaagata aaattatgaa tcgaaatggt 62100

aaactagtaa ttagacctga tagtggaaac cctgtagaca ttattgctgg acgtgactat 62160

atagagtttg gaactaattg gtttaactat catataacta aagagaactt agtaaaagca 62220

gtaaccactc gatacgagca gtcatgctca aacaaaccct caataggtca aagagtcgat 62280

attgtagact acctaaatct atttgtagat agcgactata tttataataa tgatattgtt 62340

gataaagaga aaaaattagt atcagggtat tataaagtaa cattaagtgc cgttttcctt 62400

ggtgaagacc tgtgtaaccc ttataaagta gacaaacttg aactagaaga ttaccaacca 62460

acccctgagg ataaaggttc tctacaacta ctatgggaaa cctttggtgg aagtgtaaat 62520

gagaaagggt acaaagtttt agacgagcat attggtttac tgtacgggga gggtattgac 62580

ttagaaatgt tagagaagat ttttactcgt atgggtgaaa aagggtttgc ttctagtaac 62640

gtagtatttg gtcttggtgc ttatatgtac tccttctctc tttccagaga cagttttgcc 62700

caagcaataa agagccaaat ggtagttatt aatggggaag aaaaattaat ctttaagaat 62760

cctaagacag atactaatca tattaagaaa tctcttaaag gaagagtgtg tgttacaaaa 62820

aaagaaggaa catatgaagt tacagatgct ttaacagcta aagaagaagc caaccttgaa 62880

aacttaaacg agctgaatgt aatttttgaa gatggtaagt tagcctatca agactccttc 62940

catactatta gagagcgttt agacagagaa gcaaaaaaat attagaaaaa cgaaagagcc 63000

tttagtgggc tcttttttgt tgacaattaa gtaaaagtgt ggtaagatac tagtataaat 63060

caataaagga gatgttttat ttgctagtta aagaaagaaa attacgtacc tcaaaaaata 63120

gtatgctaga ttttttaaag gagggggagt ttgtaagagc cttcggtgac ttatggtata 63180

gaaaagataa gaaactatat aatgttacac ttaaagaaat atttgacatt tatctggagc 63240

ttgacgtttc tatgctagag gctactatgc tagcttttag cactaaagaa ggattctcac 63300

ctaaaaagta tgcgttagaa gatttaaagg aagaatatgg tactaatata gtaatcgact 63360

ttactcttcc tgagaataaa gaggactttg tagtagataa tacaaaaagt ctgcctctat 63420

tatgggacac ttacagaagt tatatgccta aaacccaagc agagtttcac tacaaaaaac 63480

ctatttctaa gaaagtgttg aaagaaactt gggggcacgt cctagacaca ggctcactaa 63540

gtgaagttag aaagaaaatg gttttaggta caactgctag cattcgaggt ctacgtaatt 63600

cggtaagttc gtcaagaata aaaactagcg gagaactaac cgttacctct gctaaaatgg 63660

attttaaaac aggagtactt acttccgttg ttaagcgtgc aatggtagac gaatacgaac 63720

tatctgcgaa cgaatataca gtaaacttta atctttataa tgctatggag cttattaatg 63780

acatatcgga ctataaagta ttagacaaag gacacagaga ccattacgat tctaacatga 63840

tgtgggagat tgttacaaag agtataaccc atatatacaa aaatattgag gataagtaca 63900

ccctcttaag gctagtatcc tctcttacct caggtgtgca aggaagatac tgtagaagtg 63960

ttcgttatga gatagaaggc tcagagttgg aacactcaga agtacctatg cctttagtac 64020

gtgaagtagt agctaatcta aatttaggtg aaaatatact tgacagagca ggaaactggt 64080

tttaccaaga cgtagtaggc agagaaagtg ttgcatctac attaaaaaca aacaattatc 64140

aagagttagt agagcgtgca ggttctttag tagcagtttt tcctcttttc gacaggattg 64200

cttcttatat gaacaaccaa actaaaaaag agcagaagat ttttgagaga gctattgaac 64260

gacattggct aggtggatac aactaccttg agcgcagtaa tttaaaaatt attggtaatg 64320

cactagtacc caatgaagta ttaagagacc tactcactta taatgcagta caactcgtca 64380

ttgagaagtt tacacgctac caacacaatg tggttgctta cgattactta ccagataaag 64440

ggtatgaaca ggagattgaa aaaatacttc caagccttca aaaagaaagt aagcggattg 64500

gagaaaaaat tcctaagtac attgaggata cattccagaa cataaaggaa aatatggtag 64560

aggatgtaaa aaatgtacaa aataagatta cacctctaaa gaaaccatgg gaatatatta 64620

tccatgttaa ccaaggtatc tggaaaaaag taatggaatt agggaatttt gagaaagagt 64680

cctatattat tatctggaaa gctttgtacg aaaatacgga agagaatgta ccatttctta 64740

aatataaatc caagatagaa aacaagttac tattcgaaag ctctgtttta gcaaaccctg 64800

ctatacaatc atcatttact gaggcatttc gagctattgg cgggtatctt gagagactag 64860

gaagaacacc tgatgagttt atcaaagtca ttaactactt gtctacagac tgtacattaa 64920

gacagagaat gagtcttaga aacgcaacag tccaatatag agactatatt aattcctatg 64980

tagcattaaa ctcattagac ttgaacgagg ctaaaagatt cgatttgtgt cctttctcct 65040

tgaagttggc tcatgatatt gcaacatctg attaccaaac tatgcaggat tctagagcgg 65100

atgagtttat ggtagcacga tacaaagtta tgcctgacga gttcagctta aacaacagaa 65160

aattctctat tgataaagag gaatatgttt tagtacaacc taaaggagta gacgacctaa 65220

aagcggaagg aagttcttta aaccactgtg tcggaacata tgcagctaga attattaaag 65280

gggataactt gatttatatg ttacgtaaga aagacaacat agaagagagc tatcttacaa 65340

ttgaaatcct tgtccacgat gactcccgag tatcattagg tcatctacag ggagagtctt 65400

atcgcagtaa gattccaaca aaaataacag ataaattaaa ggaatttgtg gttaaagtaa 65460

atgaggctat gttagctcaa agagaaggca gaaattttac taaagacact aaatccgaaa 65520

ctttagttag agccagttaa cttgtattta aactcttaaa ttatggtata ataaaggtag 65580

aggctagcta ataatatagc tagccttata attatactga aagaggaaat taaatgaaaa 65640

aagataatac tcggaaaaaa aggttaaaca tttataacac cgaccgatat tttaatatga 65700

accttatgaa aagaagtgct attgagaaaa gtattaaagt aacacggttg aacgagaaag 65760

aaatcgaaga agaaatggac aagctagcag aaggttctac ccgatttaac aagaagaata 65820

aaaagagcta cttactctat caagagaaat atacaaatga tagactaatt caaaagctct 65880

ttgctcatgg aggtagtgtt acatattaca ctaactctat cataccttac tatgtaataa 65940

aacatatggc acagtccact acaagtaaag ccgtatactc tgtaaagaaa gagttcacag 66000

aagaacaggt aaaaaatata gagctaacca aaatggctac tacagtgata attgacgttc 66060

ctgttgttgt tcctgatgtt agcccctatg atgttctgtt ctcactacac cctttaaaaa 66120

cagatgtaga cggtgtacag ttttcctttc caagattagg taaaagtgaa atacaaaaaa 66180

ggcacctacc gttctatgag ttgaaaggga aagtatacga attacgaccc gaaattaaat 66240

atgaatatgt aaaatacact caagtatctt tatctatttg ggggatgtat ctctggttag 66300

tctgtaatag tgaagaagac ttccaagaaa tagacaagct actacaaaaa gacctaaaaa 66360

gaagagacag tggcagacga cctagtgcaa gtcagcaact aggagggaaa acccatgcct 66420

aagaagatga taaacagaac agatatagcc cgtgaagtag cacaaagaac aggtttttat 66480

atcaaagatg tagaacagat actagcagaa agagacaccg ttattataga tgcccttcaa 66540

ggcggagtca ctaagattaa agagcacaaa ctgtatcaga ttgaagtaga aacaaaacca 66600

gcaaagaaaa atgcttggaa tgggttagac aaagtatact ttagtatacc agagaagaaa 66660

ataattaagt ttaaaccctt agtagcagta gaagaggtaa tagatagctt aaataaaaaa 66720

gagacgtaat agtctttttt tttattgtac ttgacaggat aatcaactca tgctatacta 66780

aggtataaga aaataagagg agaggtgcca tgtgaagata ctttttttac aagagtacat 66840

tagggtaaac cacgtccttg tacaagatac aggtacagtc aatatttttc ttaaaactga 66900

cttagggaag aagctagtac aaaagcttaa atcagtaggt ataaccaaac aagactttta 66960

tattgactat gcttatgcta agatacctga accaaaaaaa caagtaaacg gtaaaatact 67020

aacgtacaag gagccaacag cacaagagaa aaaggaacca caaacattac taacagagag 67080

aattatacaa gaaaaacctg atttaatcgt acctatgggt aacattgggt gtaaatacct 67140

tcttaatgtt gcatccatta ggtctttaag aggtatacca gaaaaggtga ccttacaatc 67200

agagcaaaca ggggaaaagt ttactccatg ggtaatgcct atgtattcta tggaatatct 67260

aatatcaagt cctaacgtag agggtatgtt tgatgcggat ttaacaacac tcagtcggta 67320

caaaacccaa ggagacgagg cgtttactcc tgcggaagta tcttatgagc tagttacttc 67380

cttagaaaga gtacaggata tttttgaaaa tgaagttaaa aactatcctg ttattgcatg 67440

ggatttagag actaacacct tactacctga gatgttagga gccaagcctc tagtcatgtc 67500

agttagttgg aaagaagggc aaggtgttac tattccgcta gaacaccgag agagtcattg 67560

ggaagcctat gaacttgaag agatttataa ctatattaaa gaatttgtag cagataaaaa 67620

tattgttaaa gtaggtcaca acatttaaca caatgggtgt tgtaaaactc ctctccatcg 67680

gtgaacgcta agtgcaaagc atatgccaat accgagccta cattactggt gacagtagat 67740

ggggtgtaga gactgaaccg agccattgta gggcaaggtg agaagtggta catatcactt 67800

aaacgaggag cattgttcca tataggtaac tacaaataaa acctattggt aaggggcata 67860

aaggtatagt ccacgcttat atgaaaatat aagactacgt gcagtatgat atacgttttc 67920

ttatgtcgtc taaagggttt actaagttta ataataatcg tgatacgaag atagggtact 67980

acttgtctgt aacacaacag atagaggact ccttcaaatt atcagattta gcctatgaga 68040

tgacagatat gggtggttac gacaaaccac ttgccgattt caagaaaaaa tatattaaag 68100

actacaacga taaaaaaaca gcagaacagg aagaaagaaa aaagaaagta gtagaggaaa 68160

taaacaaaca gtttgtagaa gaaacactaa aatttaaaga agatactaag aaatacaacg 68220

aagaaaaggc aaataataag tattctgtac tagttaagcc tgtaaaacct aaaaaaggta 68280

aggcacctac aaaggcagaa ctagcttacg agcctttagc taacgaagta gatggaggaa 68340

actttaacta tgactggatt cctctagaga tactacatcc ctatgctagt ggagacactg 68400

actgttgctt acgtattcat aatgtactta cggagataat tgcaaaaaac caaaaaatgt 68460

ggttccagtt tgctaatttt taccctagtg ctattgtggc attagctaga attgagtcta 68520

acggtatcta tgctcagacg gactatatgg aagatattaa tgagaagtac actgaggagg 68580

aagaacgcct cgtagaagaa atgcgtaaga tagcagaggt atccgagtta gaagagcaac 68640

acatggagtt gtacgaacgc ggggtagagg agatggcaat acctcctgca aaaagagata 68700

aggatatagc taagttaaga gacaaataca agaagaagct aaaatttaat cccaactctg 68760

caacagacaa agggaaagtc ctttatcaaa ttatgggtat tacacttccc tacgataaag 68820

agcatattaa agagtctgct tttgatgtag ggaaacctga gaataagcta acatgggagg 68880

actacaaaac agataagaaa gcactaaact atattgtaga ccatttcccc gaccaaaagg 68940

cgtttgcttc tctcctacta gagcactcct tagtaaaaac aagaaaaaat aacttcacag 69000

ccaagcttct aaacttcatt agtaataagg acggaagggt acatgggtcg tttaacgcta 69060

caggaacaga gacaacccgt ttaagctcta ataatcctaa tatgcaacag ctacctgcaa 69120

aaacttccga ccctaaaagg ttcgactatc agtacccgat aaaacgtatg ttccagactg 69180

catttgagaa tggagcacta ctacaactgg attactcttc actagaaatg cgtatcttag 69240

ctttagcagc aggtgacgaa gctatgacac aagcattctt tgatgggcca gacttgcaca 69300

aagaaactgc ttctattgta tggggaatgc ctgtagacaa agttccagca gaatttcgta 69360

aaaaggctaa agcagtaaac ttcggtattg cctacggaga aacacctttc agttttgcac 69420

caaaacatga tatgagtgta gaagaagcag aagacatctt ccaacgttat tttgcaaata 69480

aacctaatat caagacattt attgatgaaa cacacagaga agctctacgt gacggttttg 69540

ttgatacatt acaaggtcac agaagactta tccgtgatgc cttttctaaa gataagcaga 69600

tacaaaatgg tgctttacgt aaatcagtta ataccaagat tcaaggaaca ggtgcttact 69660

gtacaaatat gtctctaatt tatatcgacc agtatataca ggataacaat atgaaatcta 69720

aattagtatt aactgtacat gatagtattg tagtagactg cccaccagaa gaggttgaga 69780

ttatggctcg ggtgtgtaaa acaattatgg aaaatctacc aattgacttc ttgagtattg 69840

aatggaaagg tgaaaaaact aagtatccta ttgaagcaga catcgagatt ggtactaact 69900

ataacgatat ggtagaatac aatcctagtg agttaaaaga gttcaactct ttaacgggtt 69960

atgtgaagta ctacaaagat ttagggaaaa tttccgatta catggaggct ggtataatta 70020

cagaggagag tatggattta ggacaaacta gaattaaaga atcaaaagaa atttacaaaa 70080

aaattgccta aaaggttgct tttgttttaa aaatagtgta taatgataca gaaagctatt 70140

tctaaaaaca gaggagggta aaatatgact taaggaggga tttgtttgga catacatgta 70200

gattcattag aatttagcca attacgaatt atagatggta acggacaagt tacagagctt 70260

gatttagaga gagagttgag agtaaatgaa tactcgatgc aggaggagtt tctcaaccaa 70320

gcaccaaaat atatctattg ggcttctatt ctagagaaag tacgtatgta tcaggagtct 70380

gcatcactag ctttagaggt agtgggagca cagatgaatg agaaggcaag agagtattac 70440

aagactgtag acactaagcc aacaaaagac caagtagaag cctatatact tcaacaagag 70500

gaatatcaag tggcttacaa acagttgtta acctataatc acttggttaa acaactacag 70560

tacgtagtaa aagcgtttga acaaagaaaa gacatgctaa ttcaatacgg agcagattta 70620

agaaaaaata aagataacgg tataagccca aacttcgggt aaaaaaaaat aattataaac 70680

gataggagac aataacagta tggtaaactt taaagaattt ttaaaacagg agaaccaaga 70740

aaacgaaagt aataataatg aagaggtagc atttccaaaa ggaaaacatc aacgcttatt 70800

cttgtctaaa aaatcaaata ccgtggtagt tcgtatttta ccaccagcta atttaacaga 70860

taactatgca caaaagtatc gtaaaattat gctgaacaac gttatgacag atacaggaaa 70920

agcattaaat ccgtcattta cgttaccatg ggagcctaac gaggattccc cactagaaca 70980

ggctttagct gattggtata accgtggagt agttcctaac tactatggag gaaaatcacg 71040

accaagcaaa gctactgtag taaacgctgt ggcattagta acagatgcaa acggaactgg 71100

ggtacatgag gttgatgaat acggtcaact agtagttcga gtgttggact taccttattc 71160

agcatataca gctatcaatc gtaaattagc agacccaatg ctaaacacta caggctctga 71220

ctatagcttt atctctgatg taggagctaa cccaatccgt attactaaac ctacatcaaa 71280

tacagagaaa gcatatcaag ttgaagtata tacaggtatt acattaccac ctcttcctga 71340

gaattggaga gaccaattgg aggacttagc ttatcaagtt agcccgtcag aagactataa 71400

cagcaaattt gttaactttg ttattgaacg agttaatgct gtaactagtg gaggagctca 71460

acagcaacaa gctaaccctt acggaggaca gcaagcacca cagcaacaag gtggttatca 71520

acaacaagtt cctcaacaac aagcttacca acaacctgcc caacaagcac agacgtatca 71580

acaaccagta cagcaacagc aagcacaaac ataccaacaa ccaacacagg cgtaccaagc 71640

cccaacacaa caagtacctc aacaacaagc gtaccaagca cctgtgcaac aaccaattca 71700

acaagctcct gtagcccagc cacaacaggc tcctgtacag caacaaacgt atcaggctcc 71760

tgtacaacca caggcacaac aacctattac gcaaggaatg gtggactcag caatgagcgg 71820

gttagcatct cctaaggaac ctgtacaaca acagaaacca gtaaccccac caccatctcc 71880

acaagcacag caaccagtaa gcaatgattc aagcttacct gatgtagatg agttactacg 71940

cagtatgcaa gaaggttgat aagaactaac atgaggtcta ggatttattt tctagacctt 72000

aataagaatt ttagagaggg ttgttttcat ggcaagagct aaacagagtg aaaaagataa 72060

caagacagaa attgatttag gtaaaaaaat tgggttaact actttgcagg atagtaaatg 72120

ggcggagatt tcagataagt tacctacatt tatccctcag ttagactata ttctaggggg 72180

agggattcct tttggtcgtt tgacggagat tatgggtaaa aacgcaagtg gtaagtctac 72240

tcttgcagtc cacttaacta aggtagcctt acagctagat tgtaaagtta tttggattga 72300

cactgaggga acggcagacc ctagtaggct tagtcagcta ggcgtagatg taaacaaaat 72360

attttttgtt caaccaaaaa aatctaagaa tgctgaggta aacaaagagg attctattac 72420

agtagaattt gtagcagaac gagtagaagc tattttaaat gccttctcta gctctgaaca 72480

acgaattgtg ataatttggg attctattgg acatacacct tctatcaaag agattgaaca 72540

aggggtagca ggtaaacaac cgggtattaa agctaaggca atggctcagt ttgctaccat 72600

tattgctcct atggtaacta aatctcattg tgcctttatt ggtatcaacc aagctagaga 72660

cgaaatggga agcatgtttg gtgcagtaga cagccccgga ggaaatgcac tacaccattg 72720

ggcttctcta agactagagg ttaaacaggc atccaaaata gaagaaccga tgcaaaatgc 72780

ttttggtact atggataatg tatatgtagg acataaatta cgtgttatta ctaaaaagtc 72840

taaggtatct acacctagac aaagtgcaga aatgttcctt ttatccgcat caggtttgca 72900

atatgaagag aatatttatc gttcttgtgt ggacagtaaa atgtacggtc ttatcaaagg 72960

actacaatac aagtcttata ttgcagatga tggaaaagaa tacaaaatgt atggtaagga 73020

ctgggttcct ttcttacagt caaaagacgg ggaaaagata cgaaaagagt tattccagaa 73080

aatgatgtta attagtttcc ctactggata tagtccactt gataatgagg tagtggatgt 73140

taacttatgg gaagatatga aggggttaac agaactgtac aaaaagaata aagtaaagca 73200

acctaaggtg aacgagggag caaaagaaga gtctattgag gaagtactag caggaattga 73260

ctcggataaa taactttata ggggagggaa acttcccctt ttttctttta gtactattga 73320

ggaggaagta aaatatgaga gctaatagaa atattgattt aaccgagcat gtggaagttt 73380

ggtctgaaaa aacaagaagt attccaccct ctaagttaaa tccatttcta gatattaaaa 73440

aaagatttct atatgcaaaa gagcataata tcccaattat tctagaaaac agcgagctaa 73500

ataactctca atctgtggtt ctagacctaa gctttgtggg agataaatgg gcaatgggat 73560

acgtaacaca ttattcagct attcgtacag agtctactcc atacactatc cattacagtg 73620

atttgtattg tgcaggtaaa caaagtaaaa agaataaaag agtaaaagta atatttaaag 73680

gggataaccc ttatactatg gaggaagcct gatgcgtaat attgataaag aaaaggaata 73740

tattagaaac ggcaatcgtt atgtacaaga gaacagtggg agaggggtat ttgctagaga 73800

tagtgaccga ctattccatc agtacagtaa tctacggtgg agtgtctaca ataagcataa 73860

agactccttc aatgacgaaa ctactagagc agaattacaa agctatattg acgagcagtt 73920

tattaaacta gtgaaggagt acgaaattaa cggtgcagta gacttcccgg ggtacattaa 73980

aacaaagtta aaccagagag taaagcatac ttttgttaag aatgtattcc gagacaaagc 74040

tagagaacgt ctaggtgctg atgagtttga agtagctaat ctagtagacc gacagcatac 74100

tgatgaagca ctactagaag accagatgtt acaagagtat gtatttggta agacagagtt 74160

cacacctatt caaaaagagt tgcttgtcta tttactagaa aaagggatgg tttctgatga 74220

acgagtaatt atcagaaatg tggcatctaa attcaaagtg cctagagaaa aaataaatga 74280

ggaattatcg gaactacgct tttttgtttc ttctaagata gctaagtatg agtcagaaaa 74340

aggctattaa tattgaaata acgtctgcta tattagagtt agaaattcag tataggaggc 74400

tttaaacaat gacagaaatt catacaccgc aagacgaaga agtaaaagat gaagtagttc 74460

aagaagttat tgtagaagaa caacccgaga acgtagaaga agtagtcgtt cctagtgacg 74520

atgaagtaaa agcaaaattt atgtcttatg ttccgttatt gttactttag ttcttttctt 74580

gaacacctta ttagcatcgt tgggttggga accattagca gtttctcaag accaattgta 74640

tctagtaggt tcttctgttg caacagttat tgcaactgta tggtcttggt accgtaataa 74700

caacgttact aaaaaaggct tgaagagaga aaacatcgca aaccaagtat tacctaaaaa 74760

agaggacaaa taagaaagga tggaatatta atggctatta agaacattaa agatttgctt 74820

aaagagtcta cagtcgtttt agatggaaag tatctacaag ctagaacggt aaccgatggt 74880

tttacaggaa cctatcaagg taaccactcg tttaaagtag aaaaaacagg gagtaactac 74940

tctgtatacc ctatgtattt tgacaaagat acaaaacaaa tggagaagag aaaagacccg 75000

attatctata cagatgacgg agaaattttc tttgtagttc gtactacagt agacccttac 75060

aattacccag ttattgagca agactcctta acaggaaaaa atacagagaa acagattcta 75120

caagcattta ttgctttctc tgctgacgag tatagttttg ctaactataa cttgtttatc 75180

aatgacgatt taaagttcta tgacggttct gctataaaag aagttcaaag tattaagttc 75240

cctaatgcga cagaaacaat aaattctttt ggagaagata aaacattaac tgttatcttt 75300

acacctgcgg atgctagcga taaaacagta acattcacaa gttctgacga gagtgttatt 75360

aaagtgagca acacaggtaa gttaactggc tctgtcaact ccactgaaaa gtctgctgtg 75420

gttactgctg aaacttctaa tggaaaaaaa gctactgtta ctgtaataat gaaagcatca 75480

gagctaccag aaccgtgata aactcataga aaagaaaggg actttcgagt ctcttttttt 75540

tattgacttt acaggtaaac tgtgttatag ttacatagaa gatacactaa gaaaggagtg 75600

acaagatgga agacaatttt gtaacagcct ataataaagg agaacagaca tgggatgact 75660

tagcattttg ggaggagtct tggaacaacg accctaatca tattgccagt ctagcggagt 75720

atctagggat gacagataga gactactgga attatgtagt aatggacaaa gaagtaccta 75780

aagagctcct acctcgaagt gagcaacccc aactagaaac tgaatacatc taccacattt 75840

gccataaaaa gaatgaagta caaattttta aagaaggttc tttagtctct cataatggaa 75900

aagtggtaca agtagataaa ataatctctg taatcatcac agagaataac tcagtagcca 75960

ttcatcttaa gattaaggag ggaactaaat gattcagcta attacaaaaa ataacatgca 76020

gggatactat gcagactatc tgttacgaaa agtatctaca catgaagtga gaacgactta 76080

cttagagaag ttcagactgc aagacatcac tagttatagt gcagatgcta ctattattat 76140

cgtactagga tttagctaca gtgacagaga ggcgggagct actgttagta acttcgaaga 76200

gtatagtgca acatttaata acccattcac tttcttttac cactttgcta gttttggtga 76260

taagtttaaa tacccaaata ttgactctct agttgacgaa aaagtaagtc ctgttatggt 76320

aatgtacaac accttgaagg aaaaaaatcc tgatttctta atagcagaga aagacaaaga 76380

agctatgttg gttgctactg ctgtagacga ctaccactgc taccgttttg cagaaacagg 76440

gaatgggctg tctctacctt taaaagagct aggagacctc tatggacgtt atttagctga 76500

attgtacgga aacatgtctt tggtggagct aattcagtca aacaagacca ttatcaaagt 76560

gagacaagag tatcggaatt attacatcaa gaaaaaaata gagcagtcta aacgacaact 76620

tgttaaagat gttatgcttt attttgtcta tgcagaggaa tacgagaatg agattgcaca 76680

ccatattatt gataaggcta aagaagttaa cgatagggtg cttgtcttag taggaaagca 76740

tacaacaaat aatgatatgt tccatgtacg ctcccataat attcacgcag gagaggtagc 76800

taagctaata aataaaggtt caggtaaaga aagtacagcg gtggtattct taggggattc 76860

caaaacacct acatttaatg ctgttgtgaa agttttgcta gatgctagtg atatttaacc 76920

actaaatgtg gtataataga agagaagtac acactaatta gttaggagaa aataatttga 76980

aaaatttaac gaaggcagaa agaaaagcta ttgagaaagg acacttagga aaaacagccg 77040

ttattctagg agtgctaaat gcttctcacg gcaaaattgt tatgtctcag tataataaaa 77100

tagcggaaag tttaggaatg gaaaaggtag ctaaaccaga cttcttagca ctaaatgaaa 77160

ccattcaaga agataaggtt ttacttggac tgtaccaaaa atcgtctgta ggaaagatta 77220

cactagatga tttaacagga ggaagtaacg aagacaagta tagccagttt aatgctataa 77280

cagcttatac agtagataat gacaaagtat tagcagactt acgagaattt agaaaaattc 77340

aacgagatgg agcttataca caacatcttt ttaacggagt gaaagcagat ttaaagaaag 77400

agctctcttc tattgctaaa cctaaattta ttattaaaga gaacaagaaa aaaacaggta 77460

aagaaatgat tgtagcctta tctgattggc acatcggagc tttagtaatc aactctgata 77520

caggaggata cgactacaaa aagctagtaa gctatctgac atatttacta ggtgaggttg 77580

ttcagacagt agaagagcat gatattgacg tatgtcatgt atatcatatt ggagatttta 77640

ttgagcatat taatatgaga agtgtcaacc aagcatatga agccgagttt aatgcaacag 77700

agcagatttc taaatctatt cgtacaatgc ttgacttctt aaacgtatta gccgaccatg 77760

tacatgtaaa atttggtatg gtaggcggta accacgatag gttccaagga aataaaaatg 77820

aaaaaattta taatgataat gtagcttaca ttattctaga tacattgttt ttactacaag 77880

attccaaagc gctacacagt aacattacgc taattgataa tcggaaagat gtttacagct 77940

ttaaagataa tgttgcaggt aagaatatta aagtagtgca cggggacaca gaaggtaaga 78000

aagtagatgt aaaaatacca aaacacatta agagtgaagt agtagactat ctattcatgg 78060

ggcatatcca tacaactaga attgtacaag aagattatag cagattccac gtatatgtag 78120

gtagcccgat gggagctaat tcctattctg cggaaaataa cctaccgaca acacaaggtt 78180

cccaattatt agttattcta tctaaaggta gtaacaatcc aatgttttta cctatcttta 78240

ctaaataaag gaggaggaaa acaaaatatg gcatttttaa tcacagtagt gctagtaacg 78300

taccaagtta ttgtactttt gcaggcggtt agtggaacta tccgtaaaga acgaattatg 78360

gtagaagaag gcggagaccc cctaccaaca tcctcctatg ttttcttaat cattaatcaa 78420

ggagctattg caggattcct attcttgtgg ggggcagtac taagctttat tctagctatg 78480

gttactaatg ttatcctagc tgtaatacta tttacgctag cagtaaccat cttatcaatt 78540

gtaagtgcaa cgattctaag tacaatcgtt cagacaattg cagttattat tgtaagacga 78600

aagcagatag cacatacgaa aaaagttatt gctgaaaaag aaagagagta gataaaatat 78660

gtctgagtat gaagcaagca gagcagactt aagtagagag ttcctacaag gtgatttaca 78720

gcctagtatt atgagacgag ggattctcta catcagaaag aaacctgaat atattgttga 78780

gtatgtagaa gaggctagta gcgatacatt aatggtaagt gtgtttgcag ttcaaccaaa 78840

gtctaaaccg cctttaaagg cttggacatt agttgataaa acattccagt ataacacagg 78900

aaccactcta ggtttactag ctaaggagct gaaacatgta cctaaaaaaa cagagaaaag 78960

agaaaatccc cctttgttta tcgcacctgc actagatggt gtggacacct ttacaggaag 79020

agcaggtaag ggtttctatg accgagaacc tgatagagcc acaagcgacg gtaaaatcgt 79080

tcaaggaaag cacacaggtg tatttgttgg gtttgactct attacttggg agaaagctat 79140

tgtgctaact gaggagatag ctagtaggta tttaactgcc cgagcccaat ggtataacat 79200

ttagtaatgc tatattaaat agcatgttaa caattgttaa caggagagga tggaagatat 79260

gacctttaca gttgttttat tcctagctgt aatactcttg tttttatccg cattatgttt 79320

taccagtcat aaacagagtt ggaacgtagt aggcggtgta ctattagcct ttggtctaat 79380

cgcattgttc tatgtgctta taatggcaag tgctgtttca ggtataaatg gagaatggtt 79440

tgggatgagg ttataataca cacattgggg gctctgtaag gggctccctt ttttatggag 79500

gttggaatga aaaaatttta tgaagatgat attaagcagg tactactaaa aaaaggaaag 79560

atgtttggtg atataggaaa gagcactatt gtatttgaga aaggtattac tatctcctct 79620

acaatcgcag actgtcttat atttacagag aaaaaaggag tcattggtgt ggagataaag 79680

acagagagag actccactgc aagattaaat aagcaactaa aaaactactc cttagtgtgt 79740

gattatgtat atgtaatgtg tcatgataac catattaaaa aggtagagca gataatgaaa 79800

aggtaccatc atgaacatgt aggcattata tcttatattg agtttatggg agagcctgct 79860

attggtgtct ataaacctgc aagaaagtca ccgttgaaag atgtatacca cacgattaat 79920

attctatgga aggaagaaat actaaaaata ttaggtgcct ttagacatta tggtgcaaga 79980

gctcctaaaa gctcctacac agtagcagat agaagcggag gactaaatgg gttatccata 80040

aaatccagtt tctctgctcg tatgagtaag ccagaactaa taagaaattt gattgctagg 80100

gttggacgac cagaggctaa tcggattgtt tgtgatattt tcattgaaca tagaaacaat 80160

cctgccaagg ctataaagta ccaccacttc tttcctagca aactagaaag gtaagtgaag 80220

aatagtggca aacaatacta gaggtaatct acctaggggg tatactgcac taggagacgg 80280

gagtgagtct cacaaaacag tagaaagagc gctaagaggt tactatacag tagactttag 80340

taatatatac gaacacatga cagaaagaga cttagaagta cgacaggcat acgctaaaga 80400

cctcatatct caaagaacag gcatcccttt agacatgatt ttatttagaa agaagacaga 80460

gaagcagaca ttaacagcta tagatgaagt cttttatgta aaacaagggg taaaaacaat 80520

aggaaaagta tctatcaggt cacaaagact actaagcagt aatatgctga ttattattta 80580

catgtctaag agccatatgc ctcgtgtagt tagaaacaca ggaatgaaaa cttatacaaa 80640

acgtagtaag tcagacatac gtgtagcaaa aagacaaagg aaaacaacaa aaagacagag 80700

ctattgacaa cagagtctac ttatgctatg ctaatattag aaaaataaac gaaatgaggt 80760

acaagtatgc aaatagtaga aaaggagagt tccttaaaca gctttttaag cagtatgaga 80820

aatgaaaaag gtacagtggt aggggtctat tataaaaatg caatgataga cgaaagtatc 80880

ttagcagaag ccagtcttca attagaggtg cctaataaga cggagaaggt cttacaagcc 80940

acggtagcac taaaacctat gccaagtcag gtaaaccagt taacgacctt agagagcctc 81000

ctagaggact ctggaatggg atggtttttt gatagagaac gacctatcac accaacaatg 81060

agtaccccct tgtttaacga cttagctatt agtcaaagtg cactatcaaa tatacaaaat 81120

tatagactag tagttaacat ggctgaccct aaagtaacaa aacaaacctt tactttttat 81180

aaaaatacct atacattgtt taaagtagag gacagacagt tggatagtaa ggttgcaggt 81240

aacattattc acagtatcca tgtcttatat aaggaagagc agtatgacgg tgaaacctta 81300

ttcggtagat tgtttgtaga aagtggtagt aaagaagcta aacttatgaa gagagatact 81360

aaaatcttgt tggacttgtt atgtgatgaa aacatcacta gtctttttat tggaggaata 81420

gagttatctt tagaagaccg tttatccaat gctaagcttt tccctctgta cacagcagtt 81480

acagctacaa gaacaaacta tgatgcttat agattcctaa cagattcagg agagactact 81540

attgtttcgg atatgattct agagagtaag ttagttaaag tagctgacta taagtttacc 81600

tttgtagtaa aattaaaagg aaatgcacaa ttaactatat atttaggata gaagtaaaag 81660

aagtcagcat ttaattgttg actttttctt ttatatatgt tatgatgtag ttaataaaaa 81720

agaaaagggg ttgacagaag tgacgaattt tttaacatta gtatttattc tagctattat 81780

tgtagctgtt cctacgttat tagctaaagc gatgagaaac cactatacca acaaggagac 81840

aggtgtttgg tggatgccat atacacactg gtttgtgtac aactttttat atacacagaa 81900

gctaggatgg ctaattttag gctccggtat tattatctcc atttatattc catttattgg 81960

tggattttta ggaacagtca ttggatttgc agggggacta cttatgttat ttggtgggct 82020

accttatgta tttggtttac tagggggagc agtctttggt gtttttgtag acaacattgt 82080

gtatcatcta ccagaatggt atcgagcttg gtttagaaac cgatttggga ttgagtttcc 82140

ttacctagaa gacaaggaag atgaaaacga ggagtgaaag taatgaaaaa tgaagataca 82200

gcttggagtt tactaggttt aatttcagct attgccttgg tagttatttc ttttctattc 82260

ttagcagggt gttcacaaga gacatcacag gttaaagaaa cagagcaaga gaggtatgag 82320

aagttcactc ctatgattga atatatttac tctcagaagt taattaaggt gtataggaat 82380

gaatcagacg agcttgtaga gtacacctac tatgcccgca cagaagagga agaagaagaa 82440

gggctcatcc acgaagagga gtataattat tacaaaagtc aaggagttac tgatagaggt 82500

gttattaaaa caacaataaa taggtatggt aagggtaagt acccattaaa actatgggat 82560

gtgcaagagt acgaaggaaa tgtcagctta gatagaatag ataatgaagt ctactttgag 82620

ggtgattaat atagaaacta caagtacagt aggatactgg aacatagata ccttttcgta 82680

tgaatatttg tactgtgcta cagagggaga gccttggagc atagggacta tgctaacgga 82740

gtactacact gattataaac aagtaaggca actaatgcag gacaaagagt ttggtaataa 82800

gaagtttagc ttcacgtctt caaaggtaag caaggcaact aataaaaaga agctaaagga 82860

caaagagacc tattttcagt atatatttga aaaagggcag tggttctgtg tgtatactaa 82920

agggttcatg gctaacgtag agatacctgc tatagaaaac ccataccgat ttaataagtg 82980

gttagacaag aatgctaaca agttaagtaa caaaagagta aaactgttac gagaactagt 83040

tgtaagtagc agtgaggtta aagaggaaac agagagagta gttcctgcta tggttaaatt 83100

agaacctccg ttacgattat tgctagacga cactcatagt gtcagttctc ataatatact 83160

ggaattttta aagtcaggga atttgtactc tttagctaag agagagttct atgttgatga 83220

ctatggaacc gaagtagact gctttactat aaatgagtta acaaaaccaa gttcccaagt 83280

acctagtcaa ataacggtta gtaaagagca tacatctaat tggtatcctc tattagacct 83340

acttattgac tgtggttata tatcagacta tgtagttgag gatacagggt ctaatagtaa 83400

agaagtaaaa atagagataa aagaaggagg ataacgggat gtctatatat gagtctatcg 83460

aggggctaga acaaagtatt atagagggat tacgtacaac tttagaaaca gaaggtaaga 83520

atatagccct agacgttatc agaaaagaat tagaagacag cccgttctta cggttttttg 83580

agttgtctca aggtagccct gcgggagatg tataccaagt agatactact aagatagcta 83640

gtaaccctac acagcattac gaaactgccc gtgtagctag tatcgtgtct gctaacgttc 83700

ctgtgtggtt gtgggggcag gctggaacag ggaaaacttc tattgctcaa agtattgcca 83760

aaatgctaga tttagaattt taccctatgc agaaaataat ggacgagttc gaacttaaag 83820

gttttgtgga tgccaactct agattcgtac caactccact gtatgaggca atgaaaaatg 83880

gtgggttgtt atttattgac gaaatagatg cttccgtacc agaagcacta atacttatta 83940

acactgcttt agctaataat tttatgcgat ttgctgacga gactgtatat gcacataaag 84000

actttagggt tattgttgca gggaacactc taggtaaagg aggtaacaac tcctacgcac 84060

gtaataaaat agactcagct actttagaca gatttgtacc tattgagata ggatacgatg 84120

aaaaaattga gttattaatt acaaatggag accgagaatt agtatatttt gttgcactag 84180

ttcgaataga gatggctaga acaaagttag accatgttat ctcttacaga gcgttaagtc 84240

attataaaaa atttgagtct tttggtgcgg atttcttgat tgaaaatgtt ttgtttaaaa 84300

cactatcttt agaggatatt actagtgtag ctcgttctct tagcagaata atcaaaaaaa 84360

cagataagga ttctaagtgg aagaacaaca aagtgtggaa gcagttacag acataccttt 84420

caaacaaagg agtagttgta gatgacacga actaatcata cgtattcatc taaatttata 84480

gcagaaaacc aaggaaatgt ctataggttg cactatgcag gagaggacta tacaaagata 84540

atagatgata tgtcagctat cccccactat gatatagata atatcaaaaa ccctcaaatg 84600

aaactctcat ggtctacagg aggggaacct attccagaat ttatacacaa gttccaaaca 84660

aattatatgg aacctgagaa ggcacaagag cttaatgtaa tgctctctaa agtatctgaa 84720

ttagtaagta gccaaccacc tatttatcat ttttacggta atactatatc agttcctaga 84780

gccattcaag ggcaccctaa aagttttaaa catcggaaaa aagaagtaac tccttatatt 84840

agtatatttg tagctatcaa tgttctttat gacgttagtg tagagaacca taataagttt 84900

ataaaaaata tgttgaaagt tattttgata ctgtcaaaag accatactgt aaaagtaaca 84960

ctaggtaaag ttagtgcctc taggggaaga atagctgtgg agagctttgt actaaaagag 85020

gctaatcaga gttttaactt atctagactt tggtaccctt tatctagtat agccacatat 85080

agaattgttc tttttgagat gctagaaagg catatgacac tagagcctaa actatcctct 85140

tacaaaggta attatgggtt tacaatacac agtacggaag gaatagcaga agcagtaaac 85200

ttgtttgaga aatacgacta tgtagtcggt tattacgaaa caataggcat gacagaaaaa 85260

gaaattatag aaagatattt tgtgtagttg actttatctt tcatatatgg tatagtagct 85320

atgtaaagta caaagaggag gtaatactat ggaagtagca gagttagtag gtaaactggt 85380

aggtaaagaa gttatgatta gtacagaaag gcatgagcgc caacaggttt atagttttag 85440

aacacactta atagaacgtg ttgagttgaa gcctgcccct aggacgattg acaataaatc 85500

agttattggg aatgcctttt ttgtagtcta tttaaaagga ctacctaatg aatatcgact 85560

agaacagtta agaattagaa tggatatact agacagagga tgcttattac ctgttgagat 85620

tgacacagaa acaggagagg taacacttgt tgtaagcgac tctctagcta aatatttctt 85680

tgctagtctt ttagaagttg aagcacgtac tgacgaggac aagaaaaagg agctagtaca 85740

ggagtttcta gcagtgtcta aattatttca cggtggcggg tggactaacg gtaactatta 85800

tgcagaggag gattaaaact ccgtatataa gaaatgaggt gaatatgtat gattaaagta 85860

gataaagtag aatgcaagga tggaagagtt tactatgtct ataatagtaa taatggatat 85920

aataaaaaga acatatgtat taatggaaag gtgcaaacag tacctaaaga taaatatgag 85980

aagtatttac ctaaactttc agagataaaa aaactagata cattgtatat gagtaacaaa 86040

aaccaactgt acaaagaagt tgtatgtgcc agcagtggaa atacaagtaa taaagacaaa 86100

aaggtacgaa tcaatataaa caatgaagta gtcagctacc tgccgaagaa ggcaaataac 86160

ttggcgatac ttctaacgcc tgacaattgc catctggtta gtagggtaga gccatctata 86220

ctagtagaac ttctttctaa aagaacagtc attatctcta gagatggcgg tggggggaat 86280

gtaagaagaa taggttctgc ttgttgtggg gacgtactaa cgttagcata taaatatata 86340

aatggtaaac agcaggtaag tattaatgta ctatctgtta aggattttta ccaatatgaa 86400

ctaggagggt tatttttatg attaaagtag agcatattag atataagcct gtagattttg 86460

gcttgtccag tacaactcgt acatcagaac actacgtatt taaaagtaag agaggtcaaa 86520

aggtggttac aatgaaaatt gtgggtaatg gtattcgggc aatgcctttt gctaagctta 86580

aaaagcacca tttagacaag gttactgtat tgaaaacatt ttatgtacct aatcgaaaac 86640

agctagctaa gcaactaggt ttaagttact acaatagtgg gaaagcagta caaaaaacag 86700

taaaacgttt agatgatatt caggactcga ttgcatttag taatgctatt caggcactac 86760

ctgaccactc agcattgaga tttaagttag taggggagga gaaaacccct aaaactaaac 86820

ttaacacaga tggcagtaca aactacctta ttagaacttc cgctaagcta ttgggagagg 86880

atgtaaacca aattaactta cttaacgtat ctggtgtgct aaataaagcg gaggcagaag 86940

cagagaagtt cttacgagta ctaaatctgt ataaggaggc taccaaagat gattaaagta 87000

gaaaaatata cagctagtac cacaaaaaaa gcaggttcta ttagagggac ttatgtagta 87060

ggtggtacta gaaaccagaa cgaatcagat ttacacttaa cagggactac acactacaag 87120

tataaaaaca aaaaaggtaa tacgatgatt acatttgtca attattatac aaaagaatta 87180

gtctctattc ctgaaaagga cttaagccct caagacctag atacctttta tagtacagta 87240

aataacagac taactactgt gttctatgtc cctaactgga agcaaatgaa taaaaatgta 87300

ctaaaaaagg gtacaaaata gcaaaattat aggtagttca taaagatttt atgatactac 87360

ctagtactgt tttctaatct gtttcactta tcttgtttca tttacttctt ttatactcta 87420

gattgtttca aatgcttata agtagttatg cctagtaaac cctactctct agtacacttt 87480

atgggtgttt cacttacaaa tgagataata caaagtgtat gctatattat atattaagaa 87540

cactgggagg caacagataa ccagtaggtc ataaaatagt ataaatgtag aggagttaca 87600

tgtgaaatgg cagataacaa agataaaaac aaggatttag aaaataaaaa agaggcacct 87660

acaacatcgg ttttacttag actgcatgag atgcctatct tagcttctaa ggtaaatgat 87720

gctttagatg aggacaagtc gtatgactat attgtaaact tatgtagtca gtataacttt 87780

gtaatctcta aatcagcaat cagtagatac aaagtaaaaa gagaagaatc tatagaaaca 87840

ggaattgatt taggtgcttt attagataaa aggcgtaaaa acgaaacaac agctactgtg 87900

tctatgattg cccctaaaga gaaaaagtca tctcccgaag ataagagata cgaggaaact 87960

tttagtactg tagacaaggt atacaacgat gtaatggttt tggaagagat tatccaaaaa 88020

ggaatggaag gcttacaata cacagagact atagaggcac ctattgcgat gaaagctatt 88080

gaaataaaag ctaaaatcac aggaaaccaa cttcaaggac taagtattat aggactacgt 88140

gaattgaaac ttagaagtgt agctaaagag acagcaatca cagaaatttt gatgcagtat 88200

atccccgaag ataaacaagg agaagtgctt actaagatga gagaagtaga aaaggaattc 88260

tataagaact tagacttatc agatgaggat agaagaataa ctgaggcgct taaagtcgca 88320

ggtataaaca gctagaagga ggttttttta tggagatagt agtgccagaa ggttatacaa 88380

aaataagcat gactaagttt gataacctag aagataagtt actagcacta ttaacaggag 88440

agacactgtt agtgcgtggt ttagaaaaaa gtacgaacgc agacgtacta gttaagctag 88500

atgacagaaa gtaccctgtg acacagataa gctatgaaat tagtattaca gacaatgtgt 88560

ttcgagaacg ttattggact atatatgaca tatctattaa tgcactaagt ttgtatgatg 88620

ttttcctttt tgaggaagac aaccctgcat atgagctaaa gatacaagta tcagacaatg 88680

tgcagtatac tagtatcagc ggtgttaaag acacagctaa agtaatagct atttatgaag 88740

atgaagaaaa aaacttgttt tgcgagttat caagggaaga cacattgtac ccattagtag 88800

aacttacaaa aatttagcaa aaggtaaaat cccttgaaag ctctaagtag ccgactaggg 88860

atttttttgt tgacattata tagaaagtat gctatactac atttataaca tagaaggagg 88920

agatttaaat tatgaacaac aaaaaagaat ttgataaaaa ggtgactgaa ttaaagaacg 88980

cgacagagat tattaaagag cgggtagcaa aagtaacaca cggtctaaac caagcaacaa 89040

acctgttgaa cttaaatgta aaagaagtaa aacgagttgc ttcaacactg caacctctag 89100

aggaagttgt ttatgagttg cagaaagagg ttgacggatt agcacagtat agcaaagcgg 89160

aggttgttta ctacgatgga agagaggtta accttgagga gattgactta gaagaggacg 89220

actatgaaga ggatactaca gaagaggaaa tccgagatac cgtctctact gatggagggg 89280

gagcacggat tagagtatat cctcctgagg aaactttagt atatatggca gagcatattg 89340

gacaaggagt agagactgat gaccttgctg tttatgaagc tgttcgagac acaaacggtc 89400

acacaggttt ccgacaagaa ggctattatg tagaccctac ggaaacatat gctaaccgag 89460

ataaacgatt aaaagaaacc tcaaaagtag aagaggttga ggaagtagaa ctagaagtta 89520

ctattattag accgaaaagt aaatctaaat ctaaatctaa acctaaagct aagaaaaagc 89580

aaaatagtaa aacaaagtct accacaactg agtttaaaaa tgatacagaa aaaggtattg 89640

taactgacta tattaatgga gcaggaacaa gtgctattgc acgaacttac aatgttagta 89700

tgggtactat ctacacagcg cttaatcgtc ataagattcc taagcgtaac atcgctaata 89760

acagcgtaga ggttcaaatg gttcctgtaa tcaataataa gaaactacta aatgcaatta 89820

ttgacagcta caacaaagga gtaccagtta aagacatcta cgaaaaatac agtattcata 89880

aaaacgggct atactacttg ctagattatg taggtgtaga ccgtaaagta caccataaag 89940

ggaaaaaagg agacgtagaa cttaagatgg actaacctcc atcttagttt tataattgta 90000

gttaccgaag gaggttagtt agttgaaagt ccatttcaag aggtctgtga taatagttgc 90060

tttagtagta actagtttta ttctaggagt agctttaggt tatactatcc atgtagataa 90120

tgctgttcaa gaacaaatag cattacaaaa taaaaaagat tcttctacta gtaaaaagga 90180

acctagcacc aataaagaca ttgcagacac aaaagctgta gactatgaga ctgacgtagc 90240

tgtaaggttg aacctcctac gagtaacagt aaaagaattt tctgagaagt ttaacgaggt 90300

agaggataat gtacaagtac ttaatgatat agaatactat aatagttata ttgcactaat 90360

tggagacgtg aaacgccaag ttagtgatat acaaagaatg aagtatcgta gtagtgagtc 90420

tgctttagct agttctgtaa accaagcagt agacaacttc tatcaagcct cctccctaga 90480

agaaaataac gttcagtcaa gacagacgga catttctact atggttgtta acaagctaaa 90540

tacaggtaat gatgaattag aaaaagtctt tagaataata ggaggtaact aaatttggga 90600

attgaagcaa tagtatgcgc cttatatggt gtttgggtag cacggaacct gatgaccgtg 90660

acaagtgttg atgaaacact gacggaaaag attattctag aaagcagtaa cacacaagta 90720

gcccacttag caataagtga agccaaaaaa cttctagaga aagaaacaaa agagaaaaat 90780

agaaaaatta ttattgatta cattgaaaaa tggcatcggg cactaagtga cgatacttta 90840

atagataccc tagtagagaa tgaaagaaaa ggggctagga atgctagaat tattgctaat 90900

atatttgtaa aaattgggac attacttgga atcctagtat taaatattcc gttctttctt 90960

gcattagcta acttcacagt agcaggttgt gtggcttttg ttatattagg cgtaatatat 91020

ggttgtcaga cggaagcaaa gcaagaagga tacaaagcaa gtaaggacta tattgtagtc 91080

tcttatgtgc taaagcaggc tttattcttg ctggtagtgt taatctagta ggaggtttta 91140

ttatgagaga atatgatgag gaaaaagaac gagagctagc taagaagaac cataggataa 91200

aaagagaacg agctgaggca cttgaaagaa aacgagagat aatagagtat gtaagagaac 91260

tagataataa ggagaaaaag ttagtagagg aacaggcaaa agaaaaaata aacggtaaga 91320

aaagtagtac tgtagaaaag tatggtaacg cactggatat ggtaaagaac acagactttg 91380

aagctttgtg tcgaatacag acagcttacc acctacagag agaatatcct atggttttaa 91440

gctctctaaa taacaaaggt tcttatattc atctaggagt agctaagcaa gttgcagata 91500

agaaaacctt gtcaggagat gctacttacg agtgtgagct attattttca gttcaagtac 91560

ctaaagcagg tcaaaagaaa caaaaagatt tactactaga gctagaaaag agaaaaacac 91620

ttgagggtaa ctatactaac gtagaaggta gttttactag tcaccaatac attccttttc 91680

taactttctt aaacagaaga aagattgatg caaagattga ttctatgcta gatgactact 91740

ctctagctaa aacgaaggca cagaagaaga aaatccaagg agaaataaat acttgggtag 91800

gataccaaaa agtagtaaaa aataaaggat acgttcttta tagctatgca agtgccaaag 91860

tggtagcagt ttatcacaga gaaataagtg tatacagtta aggaggggtt aaattgagcg 91920

gactaaccgt atctgataag caaagaaaag aaatgctttc tatgatagag actctatggt 91980

taaagagaaa tcccagccaa aggtttaatc aatttatgtt taacttagaa agagagtttg 92040

cttcctataa aaagaatgaa atttcaaaac gaaatagagt tttctaccaa atgagccact 92100

tagggtacaa caaaggatac gtctatgtag cccaccaagt ccctgattta tttgatacat 92160

ctgaacaaga cttctattct tatttgaagg ataggatagg ataggatagg ataggatagg 92220

ataggatagg ataggatagg ataggatagg ataggatagg ataggatagg atagaccttc 92280

ctatcttttc ttttgttcta ataaagtagt gggttacagc tacactttca gttgtttaca 92340

agttgtagga ctctctaagt agccagctat agttttatct gtaaaaagag ataacccaga 92400

ccccattata gagcatatat accctaagta gcaggctaca caaaataacc aatctataga 92460

taactaatgt cattcagaga ctatagatga cacagcatcc ttttgtaaat ttatatgttc 92520

gggctagatt aagaggcgat accccacccc tcctcttaga gtacttgtag agggggtata 92580

cagagtttcg ttttcctacc tagtatttat atagaatata taagtatctt ccttaaaaag 92640

cctgtggtgg cttgtgagag catacaggaa ggtattgtac cttgtattta tacggtagca 92700

ggtaccttaa cccctcatac aagaggaagc tttctacccc atataaacga taaaacacta 92760

agtagcaggc tatactttct gatataggac agtatgactt atcaatatgt aaaagggaca 92820

aagctctaag tagcaggtta cagaaaaaag gaaaaagctc taagtagccg gctacagaat 92880

ttcctaagta gcaggttata gatattaaaa ccatttatgg ggtaggtgaa tgcaggtaga 92940

tggcggggtg gtgaggactt tgaaatacct tgtgaacgtt ggtgttatgc cgtttactgc 93000

tagatgataa atagtttctt ctatataata caacgaaagt aagctaaaaa gaatttaaaa 93060

atgatgtata aaaactgttg acacttttta tattgtatgc tattataaaa gtagttaaag 93120

aaagcaacca aataaataaa aaaaaacttt ataataactg ttgactttta aaagtaagca 93180

tgttattata agagtataga aaagagcaac aaactaaaaa agttaaggtg gtaatctcaa 93240

tgaaaaaatt aaaagcaaaa agtaaactaa gctttatcct cccaaaatta acaaacgaca 93300

acaaaaaaat tgactactct aaaattctta catcagcttg caacgttttc ggaggctaca 93360

cattaagaga ggttcagcgg ttgggttgac aataatacat tatatactga cgaaagcaac 93420

gaactagaaa ttttacacga aggcttaaac gaagacatga aaaacacagt agaggaaatt 93480

cttgattttc ttttcagtga agaagaagcg ggacagcttg cagtatacgc aaacaacaat 93540

aacgaagttt acattttaga aaacgaagat gtaaaagaat ttaaaaatat tttataataa 93600

ctgttgactt ttagaaaaga acatgatata ataaatgtac agtaaaaaac aaataaaaaa 93660

aaattaaaag gtggtaatat taatgaatat tgaaatttgg gcgtacgcaa cgaaagaaga 93720

cgaaatttgt agtgcagagc aattcatttg ttcagtaagt aatgaagaag aggcagaaca 93780

agaattaagc tacgcagaga tggaaaacta ccacgcttgg aaaacttact aataaattta 93840

tatagcaaaa ttataataag tgttgacttt aataaataat tttgctataa taagtttatc 93900

agttagatag gtaaacaaac taaacaaagg tggtagttaa aatgaatgta gtaactaaaa 93960

tcagtacaac gatgaagcta gacgactata gcaacttgac aggacaagaa tttacaacat 94020

ctttctatca gttagcaaaa ttgcataaaa acgaaattaa agacggagta atgaaagaac 94080

tatctaatta tcctgaggag ttacagaaaa aaatacagga ttgccagaaa gaagcagaag 94140

gacttaaaaa agtagtacaa tttgttagtg aagacgtgaa gaacaataaa gtaaaattac 94200

aaaaaaattc ttatggtgac cttaacactg tttctttgaa agcaagtgtg cggagagctt 94260

tagcgcaata cggtgacaat attaataatt gggaaaaaaa ttacttacta gaggattgtg 94320

ctatacgcct tcaatacaat gtttctaatg tgtatctttc ggaactagct aacttgcggt 94380

cagatgcaag tgaaaatatg attgcatgga gctataaaaa agattattca ttagaagcag 94440

agtttgaaaa aattgatgac tacatggcgg taaaacttat agacttagtt acaaaatcac 94500

agcatgagca tagaaacacg catgacgata taagaatcaa actgttagca attctagaat 94560

ttcaaaaaaa atacgaggtt attagctggg acttatacaa cggtaaaaag ctttctgatg 94620

aagaaatcat agcaattcat agtaaactac cagaatttat ttcaaagttt catgctgttt 94680

acactgatat tgaggagtta agaaattatt tagaaattga gtaaaaaacg cttgacttag 94740

tgatgataca atgctataat ataagtatag taaaggttat cactaagtca agtacctaca 94800

taaaattaaa aaaaagttat aaaaggtgtt gactttrgak actaaacatg wtataatraa 94860

kgkatrgtaa ragttaagga cgtgttaatt atgaaaaaac ctattcagga actaagacaa 94920

ttaattgagg aggaagaaag agtattggta gactacatca gtctagaaga cgacaacggc 94980

agaaacatca tttttattga aaacctaaac gcagaaaact cagatgacag cactatgcta 95040

gaaacttact ccccttatga agcattagag tttttagaaa atgaggggtt gcttgcttaa 95100

taaatttata taacacagtt gtaaaaggtg ttgactttaa atagtaactg tgttataata 95160

agtttatcaa gtaaaggtaa acaaattaaa aaattaaaag gtggtaatat taatgaaaaa 95220

tcaaattgtt gggtataaaa aattaacggt tcaaaaaatt tggagagcaa caaaagcgca 95280

attagtattt gaaggtgcta gaatggacta cgctagatac ctaaaaatga aaaaacaagg 95340

gaaagaaatg ggtattacag taccaaaatt cacgttgaaa gagatgtacc aaaatcgtgc 95400

tagcatgaaa cgtgatgaac taagaaaaga catgttcctg tgccttgata ttttaaatga 95460

ccgagtagga aaataaaatg tggtttgtaa ttattggtat cagctttgga ctaattttag 95520

ggctacgaaa atacaataag gggaaaggag cggaaaaagg tgagcaaaaa aaaccttagt 95580

attagtaaga tgaaagaact ctgtcaaaaa gaagggagta ccttctttga acctagcgcc 95640

atgaaattct tcgggacaaa aattgtaacc aaaccaaaaa ataacttatt tattacggaa 95700

gatttagcaa aagaccacag aactaaaaag tttacggtaa gaaaatttga gaatggtgaa 95760

gtgattagtg ttaacggttt tttgaagtac agttccttgg cagaagctaa agaagttcta 95820

aaaactattt tataaattta tatagcatag ctgttgactt ttaataataa ctatgctata 95880

ataagtatat aagggaagag ggtaatttaa atggcrgacg gtatttggga tttaggagta 95940

taccctattg acaggaaccc atcaaggcta tatagcacaa atcaattttg acggyacagg 96000

aaacgaatat cctttaccag ctacatggga tgagttcatt gaaacattta aagatggttg 96060

gaatggtact tatgaagacg aacaagcgtt cttcaacgat atgcaggaag ttgcattaaa 96120

agaaatctta gacgaactaa tagacgtatt atataatcta gacattacta catataattt 96180

tactatagat gatgttaaaa agaaagttat tacattaaac aaacctacag acgaagaaga 96240

tgcagaagac ttagtaatag aatttgattc tacttgcttt ygggatgcta cagtagaaaa 96300

tgataaaata aaggttactg taagaaacaa aagtaaataa ataaataaat aaataaataa 96360

ataaataaat aagggttatc actaatatat ttatatagcg tatctctacc tttcacttgc 96420

aatactagcg gaggtatgct acaataaaca tattagacct aaataaaaag gagaaactta 96480

tatgatgaca attgaagagt tagcaggtct acaaaaagta aaaaataagc tagaatgtgc 96540

atataaacaa ctaaacgatg ctacagaatc actagaaacc actctaatta atgaacctct 96600

cgctataata aataccratt actcttttct agctaataac atagaggatg ccagagaggt 96660

attaaacaaa acaatcacta agttagacag gtataacgaa gcactagcaa ctaataaaga 96720

agagatagac aagtaccttc atcttactaa gtctaaaact aaataataaa tagtataata 96780

gacaaaaaaa aataataaaa ccaaaacctg taaatcacct cagcattttt ttcacacggt 96840

gtcaactcaa aaggcttata tatgcgcagt ctttctacct attttttaga ggggataata 96900

gtactatata aataacctct ataggtataa cttcttatat ttaggattat ttcaaactaa 96960

ttaggtctaa actcacatta ttataatgtc aatagggtat aactcctaaa atatataaat 97020

ctatgtacct attgactaat aatggtgtgt aaggtataat agagggggga ggtgtaggca 97080

tcttatgaaa aagaagaaat tcagtttwgt tatttctyta ttactacytc ttcgattata 97140

ytagtgaggg tmcgcttgta gatctaaaag aaacaaaagt aaaataaaac tgttgactta 97200

ttaggttaac tatgctatac ttaaggtata aacagttaca agtgggataa gagggtatag 97260

gagctcgtat ataggctttt actttattag ataggtaaat agcagtaaat aaactaatcc 97320

cgctcagaaa ggcttgtagg aggttagaga agatgtttgt attaaatggt ttactatgta 97380

ctttactagt attgatgatt ctagtggggg ctattgcttt aaacgcagat aagcagacta 97440

agcttattgt aggaattata caggtggtat ttatgactat gtgcgtagtc ctctatatca 97500

ctattatagg gattagcatt actattattc cttttggtgt attatttgcg atttgtgggg 97560

ctgttgcttt gttcaatcta cttactttta ataaggagga gatatgatga caaaatacaa 97620

gttaaatacg gttagtaata ataatttaaa agcaggggac gtggtacttg tcgagaactt 97680

agaatgctac gaattaaaat caggtgttta caagattatt agtgagtgcg acggtctata 97740

taattttgtg ggtattgatg tagacgtgta cgaacctgtt tctgtagaaa acattaaccg 97800

actgcttggg agctgtgtgt tacgtgtatc cgaattaact ttggatgtag tcgttcaaaa 97860

ctaagattag aaaaggagga agggactatg ttgttattta tcatgaatgt tatcagtgta 97920

gctatattaa tgagtacttt ttactatata cgcaatatat cccctttcta ttacgagcgg 97980

acgactagac tagttaatca cttagtagat atagagctga ttttaggatt atttatctgg 98040

ttagtgttta ttatctctat tacttttgta tagggaaagg agtagaacta tatgcttatt 98100

ataaagtctt taattgtagc tattatattt actatcttac tagtttatac tatagacggg 98160

ttttctcgta ttcgtaaagg ggctaaaagt aatggtattt tagctgttgt ttgcgggatt 98220

atagggataa ttaccttttc tattctagta gttattatct tttagaggta taccaatttt 98280

ttaagggttc atttagctaa aaatagcttt ggggtgtgcc aacgagtttc gcgttttgga 98340

gtgaaaatat ccctaggggg gaattcaaaa agttcaccta taccaatttg aaatgagggg 98400

gttttattgt ttattgtact gatgggcgtt ttgggaatag tgttagtcgg cttgggtaac 98460

tattatacaa tgaaaggtct agcgatgtca cataagctta gtaaagacgt agctagtgaa 98520

gaatgggtaa atgaacagtt tgaagattct atagaaggtc gtataaagct aaataactat 98580

gctagagcta ctatgaatcg ttcagactat gtatttaaca aagggatgtt attactattt 98640

ataggtgcct tattacttat cccgctagta actttaattt aaaaggaggt agaaaatggg 98700

tttaacatta gtaaatgacg atgataagta tctacgtgcg tttaaactag attcttttca 98760

atcagtaaaa ggaattatct atgtatatat tgttattact cccgaagtat taacagagta 98820

cctctcacag agctcagatg gacagtattt taagagtagt aaagattatc aagtaaatgt 98880

acaggaatta gctagatggc ttaaaacgtc taatagaggc tttatagaga cactatctca 98940

taatgagaac attatggcaa gtgtaggaga aaataatata taaaagagtt gacttctttc 99000

ttttgatgtg ctatagttag tgtatcaagt aaaggagttc tttttatata cttaaaaaaa 99060

agttataaaa ggtgttgact ttataaaaga actatgctat actgaatata taataaaaac 99120

aaaggtggta atattaatga acaacaatct taattactgg acggatacac tagcaatcaa 99180

cttggcggga ttaacattag tagaacggga agaggtaact acacaggaag cactcatatc 99240

agagatgtat acaagagact tacaagctaa caaacctatg tttaataact tattagataa 99300

cctagaagag ttaaacgaca aagcacgaga agctcactca gaggctttct atcatgcagt 99360

agatgctatt atatcgctgt gggacggaat aagcaaaaac actttataaa ggaggaatag 99420

ttataatgac aaataaaaag aaaccaactg tacagatgac aggggaggac ggaaatgcct 99480

ttcttatctt aggaagagtt actagtgcgc tgaaacgggc gtgatacacc gaagaagaaa 99540

gggaacagta ctataccgaa gccacatcaa aggactacga ccatctactg gcggttacgg 99600

ctgaatgggt gaatgtagag taagcatata aaccaatact attagaaaat tattagataa 99660

aagggttgac aagttcaacc ccttatggta tacttagtat atagtaaaga gaggggacga 99720

gttacatgct aacactaaag aaactattga aagctaacaa aggacaaaag aaagagattg 99780

agaaagtatt atccgaaaga gaacaagcta tcgcaaactt agaaaagcta gttaaactac 99840

aacaatctgc ttaagcattc agttacgtca ttagtggtag gcatatatac ccaccccgtg 99900

gttctgtgac cgtactttgg ggctatgcta cacagggggc tcctctcggg gcacgttttt 99960

cgggaacttt ttttaaattt ttttattctt aaagtcaaca aggaatggct ctatagaggg 100020

ctttacaaga atctttataa aaagggcacc ccgttgtttt ttcttaggta attttaagga 100080

gaggcttcta taaataaagt gaaaaaattc cggtaaaaat ttttaaaata gggaataagg 100140

aggtgttcct atagatatga ataaagataa agacaacgca ttagagtttc gagcttggtg 100200

taagataaaa cagatgatgg cagtggttat ctctattaat ttttataaag gagatataca 100260

aggggaggat gtatttctct cttaccctgc acttgggtca tttaacttaa gaggcttttc 100320

cgaggttatc cttatgaggt acacagggtt acgagataag ctaggacaaa agatattcga 100380

aggggatatt gtagaggttc ctttagaggc agaccatgga tatgcaggct ctgtgggggt 100440

tgtcacgtat cgtaactatg gtttaggggt tatgacacct caagggttct tttatttaga 100500

ggaacctgag ttgcttcttg ttacagaaga taaatatacg aataaagtag aggaggttta 100560

agaatgaaag cacctatttc agaatgtcct tattgtggtt ctacagaagg gtacacactt 100620

aggtcacaag caagtggtac tatctactgg cggatgaaat ttaatgaaaa agaggcagat 100680

aattcagagt cacatgataa tttacagtat agtccccata agtacgctta ttgtaccgta 100740

tgtgagagta aacttttcaa agcagaggag gtgaatgggt aagatggagc ttcctaagca 100800

cattatagta aaaattaaac gagcaggcca ccataataaa aaagcaaatg acttacaaac 100860

ggaagtacgc aagtggttag agagtagatt aacagatggg gaatctctcc ctgactatat 100920

aggagaccaa ctgatagatg cgttggagct tggtgtagat ggtagtgagg cgttacttaa 100980

attcttagct gaatatgagg agtgaatctt aatggaaaaa ttgaatgaga accagcagat 101040

tgtactaaag tggttaaaga attacttatt agatgattct gatttttata acacggtata 101100

tgcttctaaa caattatacg tgaacggctt tcctagtgat aaaagtaata ttgcttttat 101160

gaaactaaca gacaaggagt tcgctagcgt actactagcg ttctctacat ggtacttact 101220

agaattagaa aaggaggatt taacatgaaa gagaaatgta gtatagagat ttcttactat 101280

aacggtgaag acaagtttac gggaaaagta gtgtgggttg tactagacaa agaagaaggt 101340

atccaatttg tacttactga gaaaccagag gacgaatact ttgtttttga cagtgaggag 101400

tcggcaggta ttgtaggtgg tcttattaga ctaagactag catgtgacaa catagaagat 101460

gactcccctg taacaattaa ttaccttaag gaggaggaga ataatgaata gctgggagtc 101520

tattttaggg ctaggtagca cgcacaaaat ggtaatagca caagagttgg taagttttct 101580

ttataaggaa aattgtgata ctactacgct aggggagaat gagcttttca tactaaagtt 101640

tctcctcgat aacaaagtgg agtttaacag aaaagaaact tattatggaa caacgttcta 101700

cagaggttat actgtaaccg taaatgttag ggactctgtg ttagcaacta taggtaagga 101760

tatagcttcc gtagataaag acgaagataa ggctactcca gaagcagtaa gttggttaga 101820

agtggaatgt tgtgatatac tgtatgagtt cctgtctact aaactaactg ctgagaaggg 101880

tagtggtaga cttagtgaca cgcttaaaat aagtcgttct tatgaagaaa aggtaaaagg 101940

actagaaata actataacaa aaacaaaagt ttttaacaaa ttaggaggag agtaataatg 102000

aaaaagagtt tatggagtac aatactagaa gacacagtac tagaggataa taacaactca 102060

atagcatacg caagtgttgt taacacacat cgcaagttta gacgattact agagaatgag 102120

gactatgagg ctactatttt acgtatgctt cttgaaagga aacaagaaat tgacggggtg 102180

tttcaaagca acgttgagtt tagtccccta agtgtacgcc catcagcgcc acctgtaaaa 102240

gaacgagggt acgaaatagt tattgatgtg aagagctttg tagccgaagc attagatatg 102300

cctaacgacc tagatattaa cctaggtatt aatggctttg agcctgtctt tgctaatatg 102360

ctacaaaatt atttaaatga aaagattttt gaagaaaacg aggtaatata tgagtttgag 102420

ggtagtacat ctgtggagca atctataaaa gtagagggtg acggttgcat cgtagttgtg 102480

aaaaacataa ctcgtacttg gtaaggagga acttatgaat agaaaaacgt ttagacctag 102540

ttggcttgga gaagcgtata aggggtatat accaccaaaa aatgattttc ctgtgatacg 102600

tataaaagga gacatggttt cttggtttac tatcctttta gaacaaaatg aagaatttgt 102660

acgggagtgg atgttaaagt ataaaagaac aacacgtaca aaagaaacag gtgctcatta 102720

tataggggat agtgactact ccacttattg cgtgacaacc taccttgtta ccacacgtac 102780

agcatcattc aatcccaaaa ggcttgttat agctctattt actaaagaac tagctctttt 102840

ttctagggaa tcgacatcag taacactaac agaagaaaat ctaaatttaa atgagcgtat 102900

tactgaatat gttgaggcat tgagtgagta tctagagtct gtaggtattt ttccgttctc 102960

tcttcctagt tggtgtcatc ccgactatca ttacacatac gatggctgtc gttttactat 103020

ctccaagaga gaaagggaag taatatctag cactaaaatg aacttagaag aatataacca 103080

acatttacta aaaagcaaag gagagaaact aatgaaagta actcaattag aaacatttaa 103140

cgattttatt gactacatgg ttaaaaagaa tgaagaaggc tggaagtggg aaaacaaaga 103200

ccctctagta cctgtaagtt tcgaaaggtg gcaaatagat agagaccaaa cacttatagc 103260

taaaaacttt agaaataagg ttatttatac agttagggtg gaagaccttg accaaacacc 103320

tacaattgta tcatataaga cagatgcgga ctgggtcggt agcttagacc caaaagaata 103380

tgaatgggag acgatgaaac ctaaaaaagt acctattgtc cacccgtgta cattaactgt 103440

tgaagaactc gaaagggatt ggaagactaa tacaaaagag tatgacgaag ttaataatcc 103500

ttctcattat acgtctgggg gcattgagcc tattaaatat atccagagtc acaaaatgaa 103560

ctacgaaaaa ggtaatgtta ttaagtatat tactcgggca ggtaaaaaga aaggtcagag 103620

cgagattaaa gaccttaaga aagcaatgaa atatattggg ttactgattg aagatataga 103680

gaaatatgag aatagctaat ttaataacaa aagtaatagg gttgtggatt ctgtgtagac 103740

tagtaagtgt ctaagggttc acaaccctaa tttaaaatgt gaaaacaaac gaagtataac 103800

tgttgacttt ttacttattt catggtatag taagtatata ttaaagaaca ggagtgatta 103860

catgagtaga gcaggctaca aaattcgtta tgtaaaccag agaggtaaga tggtttgggt 103920

taaaagtatt gggtttggaa caaaggcatt agaagtcgat actactacca acaaagagaa 103980

ggttaaagta tttttggata agaaatgggc acttcaagta tgtaaactca ttgatggcga 104040

cctagatgtt aattgtatga tagacgacat tacttataag tattttatct tctatgaagc 104100

agaagataag aagtactttc taaagtcgct aactgtagtt caagataaga ttgagcctga 104160

atggacagaa aacgagccag aacgagcggt tattcataaa tacaatgatt tagatagaat 104220

tttagggcta gttaacgata gaattgataa ggaagcaaga gcacgtcacg atatgttttt 104280

actttaaacg aggaggtaat aaaaatgatt ttatatggag tgtctcttta tggggaggta 104340

actgagtgga ctacagataa agctactgca aataaatggg tagataatgc agtacaatct 104400

atagaggata atgatataga tgaggactgt tatgttattt tgttaagact agacttagaa 104460

aaacttctat atggcacatt cgaccgtgaa gtagatttaa acgaccaact gcatgatgaa 104520

gctacagtac taaaagaatt acgttggcag gtggacgagg aagggctata taatattgtg 104580

gaggtggcgg aatgaataat aaaaagacag attataaagt atacaaaata acctacaagc 104640

aacgttttat gggggaggtt cttattgact cgtatgaaag agtagtgagg gatgataacg 104700

aactacagtc tgcaattaac gctttatatg atgacccaca tgtgttctcg gttagtagtg 104760

aggaggtgac agaataatgg agtttaaatc agtaaaagaa ataaatacag agattagttt 104820

gttagctgac aaaggaggta tctcagacgg ctcacacaca tttgaggagc tttactttca 104880

tagaatggtt ttatttgcaa caatatgtaa tgctaatcac tctaaaagtt ggaagtctaa 104940

gaaacatgag gacggttcta tgtttgataa ctattttatc gttggtattt ctacatcgaa 105000

aggtatgttt tcttaccatt atcacttaga caattgggac tattttagtg ttccagaact 105060

agaatttgcc cctacatggg atggacatac agctaatgat gttactcgac ttttagatat 105120

ataagaaaat gtaagtaaac aaaggaggta ctactatgga tttaaataaa tttttaactg 105180

aggtagataa agttacttct gcttacgggg aggatgcaaa ctacgagatt tcttttattc 105240

gtgagaaata tgcggactta gacgaaggtg ccaaacagga ctgtgaggaa acggacttct 105300

ttgaggaggt agaatacatc agtcagatgc aaacaggaga agactgtttt gagggctata 105360

tttttagaaa aataaaaaac acagattggt gtctttgtgt cacgtatgct atctgaggtc 105420

ttggaattac tactaacttt ttaggttggc ttaaaatgta gtcaattagt aaacatatta 105480

aggaggtaaa cataaatgat acaagcttat agagttcgtt cacaagaaga ttttgttaac 105540

ctaatggtta acttagaaaa aaaggatttt aaatggtata gtgggttacc tcctctagac 105600

ggtgtatact acagacaaaa cccagataaa gacttagcta gtgattctct tcttgttgtt 105660

ctaaactatg aacatcgtac tattagatac tatacagttg ctaatttaag ggaagcttac 105720

ccactagcaa ccatctatac gtacacccct agtagaacat ctaaaacaaa ctcagtagac 105780

ggttttactc ctactcagtt agtctatgac gaagaacttt acctataaat gtactctaca 105840

caactaaagg cattatacat gtcttttttt gttgacttta tttatcttat atggtatact 105900

agtattataa ataaaggagg tgtctttatg aggctatccc taagaaatat attggaaggt 105960

tttcttctat tctgcctagc tttaccacct atcctagcag ttatgggatt tttattaggt 106020

attatatgga ttacatttta ctttcctatg tttggtctag ctttttggag tattttaagt 106080

gcatgtttag tagagaactt gctagaaaaa tgggataaca gaaagactct tagaataagg 106140

aactttagta tgtggtatgt tatagcctta cttattgctc ttactattac tggtatagct 106200

atactagtgc tgtttttatt ctagaacata aagagttatg tcttaagcta gtttggtaat 106260

aaaataaaca taagaaggag aggttatttt gaaagctttg ttttttgtaa taggcattct 106320

agtatgcctt ttaggtgttg ctatcttagc tacagcaata ctgtggatta ctactagtct 106380

tcctgcaatc ggtgtccctt ttctaactat tactttactt gtaggaatcc tactagttct 106440

gcttatagaa ggaaataaga aatactaaag aagtaatgga ggttgttaaa atgagaaaga 106500

tagacataaa ccaagcaagt gtggatttac aactactaat cgaatttgag aatgaggact 106560

atacagtagc ctatagagga gaagagagta ggcatacaga agaaatgaag gaagcaatcc 106620

tttcagctca caaagataca atagaccgtt tagaggtagc taacgataaa ctactagaga 106680

cttatgaaga cctgatagaa ttgaaaaaag aatatcacac cacgtataag aaaaatgaga 106740

agttagctgt aaaaaataaa gaactaaaaa aagagttacg agatattaca aatgaaaaag 106800

aaaagttaga aaaagagcta ctccgcataa aagtgtctaa gggggaactt taatgctagt 106860

atcccttact tttttagagg tacttttatg ggtactgact ttgttatact ttggtatggt 106920

aatctacgta ctcttatccc acctagttac tagcgtagct taccccgagt ctgatggagt 106980

tgctagttct tttatagact atagttattt atggggggaa tctgatgaga cgagcaaggc 107040

tagtaactat aaatgctacg accatataaa ctacagaaaa acgagctatt ttaaccccac 107100

ttataaaagc ggtgaatttc taactagcac agaaaaggtg tctaagcata aaagaaaagt 107160

ctttaaaaat aaacaaaaag ttaacttaga tgaagaagaa gagttataaa agacacataa 107220

gaaaaggtac ctgctaaggg taccttttac ttttatacta ttttttgttt tactggctct 107280

actatgttaa attccccttt atttgttata gtgatagcat taagttgccc ttcaaaacag 107340

caaccgccat ctattcctat aagttttttt ctgtcgtgaa cccacacgtc aaaattagaa 107400

taattacggt ctctaaacgg taagtaacag gtaggtgtgt gaccgaatac aattactttt 107460

cctgtgaggt tctcttgctt ataccagtcc ctaatccaca taaactcgtg gtctgttgtc 107520

ttcttccagt cttccccgta taaagattga atacctgcat gagtaaacag aacgtcacca 107580

aactcgtaat acggcttaga agctctcagg agctctatat gatgtctata tttcttttgt 107640

atgcaaatac ttgtaaagtg ataaaaagac gatagcctat gcctagaacg ctcctcctgt 107700

ggcatattat tgaagtagct agacaaggtg tttcctcctc cactagcaag ccatctgtta 107760

atagtgtact cattctgctc ttcttctata aaatcaagca acatttggtc gtggtttcct 107820

cttataacaa ccacttggtc accgtactcc atctttaaat cacaaataat atctaaaacc 107880

tctctagact cgtcccctct atctatatag tccccacaca agacaagcaa ttgtttatcc 107940

ctatcccaat tgtttaacat ttctaaaaac tctaagctac aaccatggat gtcactaatt 108000

gcaaaagctt ctttgaattt agtcaccgag gcacctcctt aatcaaaatt aatttcttct 108060

ttccaacctt ttatataaga tgttaggtct ccttcgtacc ccataagacg tatttccatt 108120

accaactgac ctatcgtagc atttgaagtg ctcataccct ctaaaacctc tataaacggt 108180

cttagtcttc tgtccataat ctcgtttgca gtttctatag cactctcttt gtttctaccg 108240

ttctctatga gtaaatttac gtaatcttca taagtcatgt taacatgggt tacaccgtcc 108300

accacactag aaaacctaca gtatctacca tttggttgtt gtgagataaa gctactcatc 108360

cttgatattc atctcctctt taacagtact ttttagcctt ttgttttccg ctttaccttt 108420

ttcgtataac cagagagctg tagacaagtc ctttcgtgtt acctcgctgt tgtttcttac 108480

ttttgctagt agctcctcat acgttttatc tttaaagtcc atagtagctc cttcttctgc 108540

attgtcccaa gcgacctcgt aagcaaccat tcctgtcatt ccttgcatca cacccacaat 108600

agtacctata gatgtgttat aacgatgttg taccagttca aactttttaa acttattcat 108660

tcttatctat cccctagcaa ttatttgtaa aagtaattcc aaaagattcc atcctaccat 108720

atctaacaga aacacaagct gttaggtagt cacttactcg tatctttact ttaacaatgt 108780

caccatctat aggagactta aattttattt ctatgtcaac tgcaaaatga ttgttgtgat 108840

taatccaaga ttttaaatgg aatattcctt cacgcatctt tgttacagtt gtgtatggat 108900

actctagtga atgtttgtgt atatttcttg gaaattctcc aaacattgca attacttctt 108960

ttgggtttgt tggtgctaac atgtgatgtc tattttgatt tgataataac tctctgcctt 109020

ttttgtcaag ctctaaaaag tcttctcctc tatttttcag taaatcaaat aataattttt 109080

ctgcaattac ctttccttct ttttgtcttt cttttctctg ctgtttatca tatttcttct 109140

tttctttaat tatcctattc cttgcatgac taaatttttc cataattact gtcacctcac 109200

atctttattt acttataaaa tctactaata ttgccaaaat aataagaaat aatatcacta 109260

tccaaacaaa caccactatt actacccaaa taattattgg aataaacact gtccaccaaa 109320

caatattaat aaaaccagtt aattgaagta ttaataaaac taggaaagct accccgcata 109380

ctgtacttaa tctcatactt attattcacc ttccacactg gcttctttta ataattttat 109440

tgtaacgttt tcatcatctg ttgctgttcc cgcaagaacc tgcttaacca tctcttcaaa 109500

attctctctt tttggctctt caaaatcttc ttccgtatac acctcaactt ctacttcctt 109560

tttaggaacc aaaagtagtc caccaaattc atcattttcg actccataag tcatcttacc 109620

tgcttctaca tcttcgtttg ttttaattgc cattattgca attacatctt taatagtatc 109680

caccatattc actctcctat tagtttagta agcttagccg ggtaccacag tttttcatta 109740

ttattaatat cccattctat taaataggca aaatcttcac cttgtactct agtgtatcat 109800

tataaacttc ctctactgta cctaactgtt ttttactctg taaaatgaac tctacttggt 109860

ctcctacttt aaacttcatg cttcttcctc catttctaat atttcgtgag ggtctacctt 109920

tacaaaatca gataaagagt acctaaatac aaccctgtct ttttgagata caagggcttc 109980

ttgtttttct tttttatgac ccctacaatg tttgcaaaca agaagtacat cacgagtgta 110040

aatattcgtg ttctctatag atttccactt agaagccgta ccctcccata tgtagacgtg 110100

actgcaagta ggcttacttc taactaaact agctagttta ctagtgtata ttttcatagt 110160

gtaaacacct ccaactacat tatatcataa aaaaagaaga gtgtcaataa acactcttac 110220

tttagatgtt cccacccgta ggcactaaat atagcaatta ccgctaatgg taaaaacgta 110280

ataaaggaca cacgtaagat aaatcttgag ataggatttc tgtagtaatt ccatagatat 110340

gttactttaa catcatggct atcatcttta gactctaaat aaataagtaa caggaacagt 110400

gggtatattg ctacataaat ttgaaggatt agtaacacca tctatttagt atcctcctta 110460

agagacaaat tatcgaggaa ggctactaag tcttttttag ctaagtttcc tttgtgattg 110520

cctacaaagt cctttatagc agccaccagt tcttcgtgaa gtcttctgtt ctcttcgtaa 110580

acctcattct ttaccgtccg ctctaggtta cttcttgcac tctcttctct tttaagtaaa 110640

acgcaagctt ccccctttag tctagtgttg tcttccttta gagtctctat ctctttttcc 110700

ttttcttcca tctgcccgac aaccctttgt aactctctta gttgccgttc tttctcaccc 110760

aaaataactt caagagcgct cttttctcct acagatttcc ctagtttgct ggttacctct 110820

tctagttcag tagtcatatt tttaatagct tccttatctt tcttttgttc ttcataagca 110880

tctacaatat attcttttaa attcttatat gggatttctt tctgttcttt ttcctttagt 110940

ttgctatttt ttcttttaga actaattacc tctaaaaact tcattcggat tcctccttca 111000

ttgcttcaat ctctctatgc acttcttcat agtaactagc tgtaaaagtt actcttgaaa 111060

actcctcgta agcctttagc tcccgctcgt ccctctcagt tatctgctcc catgtaaaag 111120

ttttgtcaca ctccttgcag tataaatcgt aatctaagtc ataattacat tgtaaacact 111180

ctactgattc gtctccttcg tagtgcatat ctatttacca cctttagaga tactatcgaa 111240

cttttccgct aaatctgcat acatatctga tagggagtca taccagactg ttctagagaa 111300

agcaacagag tatacaatgg ctttttggta gtaaagtctg tcaactctta ccgcaagacg 111360

tctaaaacta tctctactct cttctaggaa atatgtgtta ctagggtcaa ctgcttcttt 111420

tatcacaggg taaatctctt taacaaaata agcttcttct ttggagtcaa ttcccttttg 111480

agtcagtata cctaaaagaa tacctgcacg ttgttttacc tcctctggtg tatctcctga 111540

tgctactcgt ttaactaatt ctttaatatc ttcttctgta cctacgccta taaggtgaca 111600

ataaacttct aagtagaatt tatttactcc taggtgatat tcttcgtctt caacattttt 111660

ccagataaac tctttaaggc tttcagcagg taagtctagc atttttaaaa cacagctaac 111720

gttaagggct ctgtacttgt ctaataactc ctcttctttt tttctatcat aagcaaggga 111780

agctgtatgt ctgctaaatg tatctacgaa acattggtct ttgaaaagat agttcagctt 111840

ttcttcacca gattgtttta cgcatacctt aaaaagttta tacagacggt cataatcagc 111900

agaggataag taagtaaacc aatcagctcc tactttaggt gtgaacccta taaccttata 111960

actgttctca ttacaaatac gaaactcttg tcttttaatt atactagcgt aaacgaaaaa 112020

tttacctttc ttttccacac caaaaatatt tttgtcttta acaggttctg tagtgacctt 112080

aatatcccaa caggacttca actccacccc taaaccacct aagatactgc ccgctacatt 112140

ttctatctta atactatcat ttaatttgaa ctgactgtac ttaagcccca attcctcagt 112200

aactacttct atcaccaaag gcattatatc ctgtatacgt tcctctgggt tctcacttga 112260

cttgcattca actataataa gaggtagcca tatagtccca ttaccccctt cacttgtact 112320

acccgcatct cttttttcat aattatcctt aatacgtttt ttatttagtt cttctactat 112380

acctctgtag taaatgttca tcattcgagt gttatctttg atgtctacta acggaatgtt 112440

cttatactcc ttatgcttag ccatactatt cctcctcact acctattatt tctttgtaga 112500

agtcctttac aaagtctgct tgctcctctt taggaacaat actattaatt tcctcctcaa 112560

agcaaaatag aataaagccc actgttttga tagagccctc attatatagc ttactgctta 112620

gaaccatgta gtccaacaat tcttctttct tcgtattgtc tctatgaagc ttgtcaaagg 112680

tattaacaga aaaaccaaaa ggactcagct cctttataac taacggcttt aactcaccaa 112740

actctacttt agggttagtt ttacttccga aagaaagctc caactcttta gtgtactttt 112800

taacaagttc ttctacttct gtgtacgttc ctacgctaca gttgtttttc tcctcctgtg 112860

agctatattt tacgtaatac tctttactca tactatcaac tccttattct aattgtatca 112920

caaaaactct attaagtcaa caaaaaaaga agatgttact cttcttttaa tggcagtcta 112980

gtttttagtt ttcgtgttct tcctttgtat ctgagattgc tagtatcagt gtgtactgac 113040

cttactttta caatactctc tttgacacta tcatatagaa aatcccctac agctacctta 113100

cctaacaagc tgtcatctaa actaaataaa tatgttttag cataggtaca gcttaggtca 113160

tcctgaacat agcttatgta aacgtacttg ttctcataat ctgtcgctcc ccaagactcg 113220

tctacttccc atccaccaag tatagtttca atattggcat ttagtatctc tgcaatgctt 113280

ctagcttgcg ttaagcttgt cagcggtctt acctgattag gttctcgaac tttttcccag 113340

tttaatacaa aagtacctct tttgttctta ataaatgatg ttgtgtagta tgcgtatgga 113400

aaccccttct ggcgtagaac atacatattt ctttttttta ccatataaat caacctccta 113460

gtatttttgg taagtcctct gataagttta cagatacgtc ttttcctagt aaattaagta 113520

actctttgat agcttcttct agttctacaa agtactgtgg tgacctattt cgactatctt 113580

gttgcaactt aatcacgcaa tccatattca aatcagggca tgaaccctct cttattctta 113640

taatattcag tttattattt aacgcatgtt cgcacttctg tctatctttt tcagcatttt 113700

ggtgccaacg acctccatca tattctacca ctagtttcac agaaggtacg tagatgtcaa 113760

atctttttcc tgctatattt ttttctaata caattcgagg gaacgctttt tttaaatagt 113820

aggcaattat cttatttggt ttagaaacag tagaggaaca cttagggcaa gatttaaacc 113880

ctacaaactt agacttcaca ctctgctgat aagagccatg ctccttacat ttccaccaaa 113940

cctttatgtc gcttcctcga cttacgttct taggttttat acctatagca ttgttttttt 114000

catagtccca tagttttctt agaacagagc tatgggactc actaataggt atacaccctc 114060

tcttaagagc tttagtagcc atagttatct cctccgctct ctactatatc ttaggttgca 114120

aaggtctact tctgcaaaga acatgtagtc tacctccgca tgagacatcc ctaagtctct 114180

taactcatat actttatctt ttacagtttc ccttacacgt tccagttctt taataccttc 114240

ggaagttagg ttaccttccg catctcttaa tttaatagcc atagaatttt ctcttattgg 114300

agttagttgc tctcctaaaa ttgtctgaat ctgataatat agcggttgcc tttaataact 114360

ctccttttgt aagccagttg aataggtcat caacagatac cttcataaga acagctagtt 114420

gctccataat gtcatcttgg tctgtatgac atgacctgct tgcttggtct agtagtccgt 114480

aacaagcttt tttaagtatc ttcatgggtt tatctgagat agtgctagaa ggctcacgag 114540

aggataaaga aacaacacca gagcttgctc tacaactagt gcacatgtag cacattccat 114600

taccgtactc cttgccatat aaatcagcat tagatgtgta ctttatatta ccttgacata 114660

acgggcactt tgtaggaatt tccttcttca tacgtgccaa cccccaatcg tcagtccatc 114720

gaataacata ccagccgtta ttgctacaga gacaatcaaa atcattaaaa ttattgaccc 114780

ccataaggta agccctctgg tgaagaactc cttaaccgta actaacagcc aaataaatga 114840

aataataaaa attatccaag tagttaatcc cataactttg tctcctctcg gtatttctta 114900

atcttcaatc ttgcatctga tataactgtt gacaaaggta ctagtagcct tcctgccatg 114960

taatcactat ctacaccaat gttaatttgg tctttacttg gactaggttt actatgaatg 115020

tgtccatgaa tgttgaatct acttttatta catccaagtt ccataggata atgggaaaaa 115080

tgaaactcta ctgagtatgc gtgaataatc ataccaatat cgtgtacttc tactacacta 115140

gggcataggt cactattgta taagtttact ttttttcgtg tatcatggtt ccctcttata 115200

ataatctttt tacctcgtag agcattcata attttaaccg ctttttcttc gtcacctaat 115260

gcaaagtctc ccatgaagat aacgacatca ttatcattaa cagtatcgtt ccactccatt 115320

ataaactgcg agttcatgtt gtcaataaaa acgtatttat ctcctctata atgttcaaac 115380

ttaagtatgt tatcgtgatt aaaatgtgta tctgatgcta agaaaacatt gttagaactt 115440

gaaatgttat atttaaccaa aaaatcagct agactaatca attaaaaaca cctgcctcta 115500

gaggtacttc tttataacct cctgtataaa aacttaagtt gtactccgtc atagacttat 115560

ctttactata tacttttact acacgattac tggttactag aatactagct acctcttttt 115620

tacctactac atgtttatgc tttcttccgc caccatctgt gatttctact tctagtaatt 115680

cacgaatacc gaacatacca aacaactcct atctattcta taataatatt ttttctgatt 115740

tctaaccagt ctacctcttt gccaactgct tttaaagcct tatcatcaat gtataagtca 115800

gcgccaattt ttctagtatc atttttccat attgctatct tatctgggtt gttttcattt 115860

acaaagtggt acggtatact gttattggca agaaaatcca aacacaaatc cataccaagc 115920

tcttctctac atgtccaaat agtgataact gccccaagtt caacaagttc ttctattgtc 115980

tctttagcat aaggtaggag ctcccctact aaggggtagc tctcctcctg ctcactagca 116040

atagttccat caaagtctat agctatatac ttatagcgta aacttctcat ctctacgcct 116100

ttggtttgct atctttaaat tttaactgct cttttgttgt agatggtaaa cgataagact 116160

catacaagtg cgggtggtct ttctctaact tctkaccgtt aaatcgtaaa ccttccacct 116220

ccgcttgata agttagttca aaatctccta aaatctctaa tgattttacg tttgccttca 116280

ccatcatagg cattaattca tctttaagtg catctaactg tttttttaat atctcgtagt 116340

tacgttttgt tgttgcataa tttttagctt tgtcctgcaa ggttttagta atagacaccc 116400

ctgcaccctc tttaagttct accgcttgta atgcaccttg ttttgccatt tataatctcc 116460

tcctgtctta tttataaatt aactataaca catattttat tataagtcaa caaaaatgta 116520

tattttttat ttactatcct ctacttttag gattatttct ctgagatgta ctggaacact 116580

acgtccaaga gactttttag ctgtatcctc atcgtaaaca aaagctaagt catctggtga 116640

ttcctcactc ataatatcat catacacaat tgagggtact accttcatta gttgggaaaa 116700

gttagaaaat tctaagttag gcaacttatt ttcctcttct agtctctgta gagctttcct 116760

aaccctagtc tcagtgacga attgtcgaat ccactcggta ctttcaggta aatctttaac 116820

ttcccttttt ctagattgga ctctttctgc gtgttttggg gaaacacttt taaatagttt 116880

ctggctacca tcgatgtcta cttcgtctaa aaatttaaca ataatacctt ctccttcatt 116940

agtttcctta gtaaatctgg actgacctac aaattcgtta actgtatcaa agtcagtaaa 117000

tacccctaag taaaacatat ctggcatagg tagatttagt gtaaaagcaa tactacgtac 117060

ctcatctaaa ggtaagtatc gcccttctac gttactgtac acgtcaaata agtagaagtt 117120

atttctaaat tgctccttat acggtgtatt taacttctta gaacctagcc actcaccaaa 117180

gaaagttagg tttactagac ctgaatggac aacatcagga atacttacct tgtctttaat 117240

gtaagtagtg aaacctgata gtgtgtttac ctctcctact tcattaggaa tgtctaactc 117300

tatatttctt gagaatttac gcatagcata gccaccatca ggaagtttct ctacaaaaaa 117360

actagcgttg ctaccatcta acttctctag aatgataatt tcacctgtat atgtttttgc 117420

catttgttga gctcttccaa ttctttgaat ttttctatat ctattcatat taactctcct 117480

cctaaatttt taagtctttg aacgaaatat tatctagatt gctgggttca agctctcctg 117540

tacgaatata agtagtcatg agcttgctgt ctaactcagt atcctctttt aataattctt 117600

cattaacttt atttataaga atgccaatat cttttccttt agcgggaata ggtatattta 117660

atgatttagc tatcttgtct acttctaact taattgcctc agggagagtt tcctgataag 117720

gcaagaactc atatagtaag ccaatctcca tacccaagtc atcagcttca ctctctgttc 117780

tagcaaaatc aatttgttga cctatttgtt ttacctcctg ctttactgct tggcgtacct 117840

gctcatcagt aagttctgat acacttttaa ctttaacttc tactcgcatg ttatcaagtc 117900

tagcaattaa attttggtat acattgatga aatcactttt actttttctg gcatgtataa 117960

gactttcttt cactctttct accaaactag ttcctttgtc tttttcttct aacttcataa 118020

attttcccta ctttctctat attctctttt tgtatcatct tttataagtt ttctctcata 118080

tctgtttctt agttttttgt ctcttccttg gcagtgtgga aacatcccat ccttagtgaa 118140

gagtagctcc tcgtacactc tattagcctt attcttaaaa tatttatttg tagatttttt 118200

actcatttag ttatctctca cacgttgtaa aattttacct aacaagtttg aacctctcca 118260

taaatctttg tttaaaatac gagggtcact ttctcgtagt cctacgcccc atatagtgtc 118320

ataaggcttt gcctctacta aagtagttcc cttagtagct tctagtttat cttttaattc 118380

cttattttga gagaacttag ccattaagat aataaaaaca atagcctctt tgttatcatc 118440

ccacgtagcc tcgttaaaac ctctaacttt tctacccaga gatttaactg ttttagggtc 118500

tccactagga acggatagaa tttgtgaagc cactaactca tcattgaata gacgtgcctt 118560

ctcatacatg aatgcctgtt ctgcattatg aaacataata gacttgttag cttctttccc 118620

ttcttgcttg tactctaaag aaaaatttga tgggtgccaa ttactaaagg ggctttcctt 118680

tccccaaaaa aatgtgtatc tatctctcat tttataactc ccctccttga cttagtaaat 118740

aagtaggcgt ttctgtgtta gccattctgc ttactcgggt agctaatgtc tcaataaatt 118800

ttttattatc accataaata ttaatagtct gcgcttcttt tttagttagt gtagtgaact 118860

ttgtagtttt gtcaaactgt acaaaacggt aataaaattt ttgtgcttct tccacagaac 118920

tagctaatac ccaagttttt tctttatttc gttctgaaat ttctactact aattgtttca 118980

cgtgaaacaa ccttcttatt tattttagtt tgtgttctct tatttaatta atgcgcctta 119040

aaattataga ttggtttaac aatgaagtca atgtcaaccg tatctgtaat attctctatg 119100

atactctctt tctttttata ggcaaatggt gcctcatcta gtgtagactc caacacagag 119160

gaagaccaaa catctttcat tgtattctta aaatcttcca tttctagtgt ttctttagct 119220

ttagaacggg acagtaccct acctgcccca tgaggtgctg aattgttcca gtcttcgttt 119280

ccctttcctg tagcaataat gctaccatca cgcatattta gtgggataac tagttgtttt 119340

cctttagagg catctgtcgc acctttacga ataaccttat tagggatgtc aatataatta 119400

tgaatagagt caaactcaga aactaccaag ttttcccaac ccatttagaa cataattact 119460

ttatgaatag cttttcggtt atcctccgca aatttttgcg caatagccat atcatttaga 119520

tagtcctcta ctaatttacc gtctagataa gcaaggtctt tattaaaagg tgctgtactt 119580

agttttaagc gcaataactc ttcctgtatg tctttttccc taccttgctc ttttagagct 119640

ctaatgagct cctgtggctt ctcagtgtta gaacaatact taacagctag ttcttggtgt 119700

gttttagcta cttgaacgcc taagttacgg gaacctgtgt gtgtcataag gaagattcga 119760

ccatcgtagg cacctagttc aatatagtgg ttccctcctc ctagtgtacc ttctgataaa 119820

gcgattctat tttttacctc ttcttttaag tttccataga tagtcagatt cttaagtgac 119880

ttacttacct tctcctgatt tagtgctact cctcttacgg agtttcccga aggaacaaac 119940

tctttaataa cactatctaa tttttctaat gtagtattgt ttacctctaa atctaattcg 120000

gttactcgta ttccgcaacc aatatctact ccgataaggt taggtactac tttgttagta 120060

taagtcatag tggttcctac aacacaccct ttgcctgcat gaatatcagg cataaatcta 120120

acacgctctc ctttaacaaa ttcctgactt aacaattcta ttacttgacc cacagcggtt 120180

tcttctacct cactagtaaa aaccttagct gtattatacc ttccaagtag ttctatcatt 120240

ctgctcacgc tccttttatt atacttctta ctaaaaacca ttataccata tgtcagttta 120300

taagtcaaca ctaagttaaa aaaagaggaa cacgtatgcc cctctatttt agttctttaa 120360

agttcttaga gaagaactcc tcataatatt ggtcttctgt agtaactttc ctacctgagt 120420

ctgatactac tgttatatct cctactagaa caccgtcttc tgtgatacta tttacagtaa 120480

acgttctgtt cttgttacct ttttcatgaa atgtcttatt taaatatttt tttccttttt 120540

tagtcataca taacatcctc ttttgttaat tgtgctagtg ctttacaata tttttctttt 120600

tgtgaggcaa aagcaatagt gttatttgaa aaatagtcta gaatctctgc tagttttact 120660

tttttagcta gattatcctg cttcaatctc tggatatagc aagtatacgc ctcattttga 120720

atgcgtgtta ggtgaactaa acttccaagt aaataatcct tgtccactag agataaaaaa 120780

gtttggacat cactaaatct gacggcggta tcctcaaata tatcatgcag taaacttaaa 120840

taataagcat cttcatcttt gtctttccta aagttttctg ctactcggta cacgtgcgct 120900

agctgatttt cctcataatt agctaaaaca gtctttaaat gattacttac gttgtcttta 120960

attgtaaaca atacgtagcc tcctttaaaa aataattggt agaaccaata gtacacatat 121020

tcctagagaa acaagtaata acataatagt atctcttact gaaagtagga acttaagttt 121080

aggtacatat ggcatagata cccctgctct gcctaaaaga ttagaagcaa taggcacaag 121140

gttatactca ataagaaaag ctaaatataa ataagagaat attaaaataa aagtgattat 121200

aatccccata gatgtctcct ctctactccc ctaaggagct attttcatgc ttatctattt 121260

taaaaagctc actagaagga acaccaaaat atttttctag tttcactcga tttctctctc 121320

taggaaactt cttacctgtc tcccaataag aaattaggga gtagttaaca tctaaatcct 121380

gcgctaattg tcgaatagac aactgctgat tctctctaat tcttcttaaa tttcttactt 121440

tcccaatatc ttctttattt gtcatttttt ttttcacccc ttacttttta tatatttatt 121500

ataacataaa agtcaacgtc tgtcaagcgt acaaaaaaaa agagcacgta ttaggtgctc 121560

tctaggttac tatagaatgc tgtcaataaa gctgtaggct tcacttctta aaacagatac 121620

cataaaatct tttggtgtat ttttttctaa agacattaca tgtttaaaga cagtaggtgt 121680

aattccagat actaaagcaa ctccgctttc agaaaaccga actggtgagt tatcattgcg 121740

ggcatttaca ttccaaaaag ttactgttgg aagtaagtaa cctgcttttg aaaacttacg 121800

tctcatactt tcgtagaaag agctgtcgtt ctctttttgt gaactacact ggtcgaactc 121860

catgtcagag ataatgacaa tatcagtagg tagctcactt tgtggcagtt tgctctttgt 121920

tgcggtattc agaattaagt taaaaacagc ctctaggtta gtggacatgc cccagtcgct 121980

actaatagca gtagaaaccc gctcccgtaa gcttaactca ctaggaacat gaactaaagc 122040

tggtttagaa ctgaatgtaa taaagtggtc tttgaacact ccatttagtc tctctgcgca 122100

gtatagccct aaagaaatag aaacgtccat aggtgacact tcttttgccc caccatacat 122160

agaacctgat acgtcagaaa ctacaatagc atttctagtc atattttcta cgtagttagg 122220

tagtgatttc caagttgcct ctactgtgtt gtctacagag tcgtcccagt caccacttgc 122280

ggtgtacttc ttaactaact cataaggcat aatgttgctg gcatttagtt ttttatctgt 122340

ggagtttact ctgttaataa acatttcata ggatgtgggc aggtgttttc taaaagcccc 122400

tctatattta aacagggcac gagaaggtaa cttgtcaaaa ttgatagtat cgtaatcctt 122460

gtcggctaag ctagtctcca ctaagtttaa ctttgctcgt aaagcggaaa gtaatttacg 122520

atactctcga ctggtgtagc ctagggaagc agacaagtct ctagctgtat gtttcttctc 122580

cttgtttggt gaagactccg aaggtaacca cttagctaat aaagatatag attcatctga 122640

ttttaagttc tttttatcta gttctagttg ttcagaaatt agttccacta gcaccattgt 122700

taagactttg tttttagttt cacaactata gcgatataga gatacaacat cgtcccaacg 122760

accaatttca ctcataaact ctgtaaactt aattagagtg tctaagtcaa gctcagctag 122820

ccccttaatg gactcgttat acactgctcg ttcacctaag ccactaagtc ggatattacg 122880

aagatgtact aagttcttga gtgctagctc tcggtcttct atatatgcct tcttaaataa 122940

cagcttagca tcatttgttc tatcacgcat tgctcctgct cgtgagaaaa aatctaagtt 123000

atagtctaaa gtagtgctaa atgtgttagc cccctttctt gttttactta aagtagtctc 123060

ttcttttaat ttatttaaaa attctgacat aataatctac cccttttctt ttttataatc 123120

ttatagctga cctaagctat attcaggtca gctatcgtat gtacaggaag cgtttttttc 123180

tcgtacattt tttttttaca tggttagaca ccatgtcgta ctaactgcta atatgcgctg 123240

ttttagtttt tagtatttgc tgtgtgcttc ctaattaatt tttacagaga gcgtttttac 123300

tttttcaaat agtataggtt agtaattgct gttagctctc tattttatat tctgtagatt 123360

atttttttat cacaggaatc atttttatat tttatccaat aatatagtag taattgctgt 123420

tagattccta agtttatttt atcttaaatt tatgataaca tattttgtct agtatgtcaa 123480

catcttttta catttgtttt ttgttgtcat tctgccagtt ttcccaaaat tgctcttttt 123540

cactttttcg gctctcgaca aaaccttgtg tcttcttaca atatgggcaa tacatatgtt 123600

taatatgacc gtccttcttt tgcttagctg accttctgaa tatagagact ttttccccac 123660

actcagggca tgttaacgtg gtttgtactg tctttaccat agttataacc agattgcccc 123720

gttgggaatc gaacccaaac cgaggagtta tgagctcctt gctcgacctt ctagctacgg 123780

gacaatgagg gaaacttctc ttagttagag aaggctccaa aatgttttag ttttccaccg 123840

agtgcctttg tcttgtctga tagttctatt tttgatgtta gacgagactt accttctgcc 123900

acgtacagaa aatctttctc aacagtaact aacttcattt cttttttgcc agtctctttg 123960

ttgtatcgtt cctgtaattc tttaacaggt tcggaaatta cgccgtaaaa ttttttcatt 124020

gtaaaacaac tcctaatttt ttatttttta actaactttg taatttaata atacaataaa 124080

agaaatttgg agagattcca ctctcaacta cttcgtgcat caatatattc agtcacggct 124140

aaactagatt cttggtaagg agctaccttg tcctccatcc agtctgtcaa ttaagactaa 124200

gtttaaacac atcacgttgg aagtgattaa aaatttaccg acaggactat tttacctgta 124260

catttcacca agcatggaag agtatatttt tattaatacc aattttaaag atgtagattt 124320

tggctacttt gactcccatc atcccttata ctcggctttc accgaatcaa ctacataaat 124380

gacttttagt aagtagtttt aaaagagtac ttacattccc ttctaaaacg cttcatcacg 124440

ggcaggtagg atacttttat cttataacac acgtagcagt acctttgtct cacactaacc 124500

ataactatgg ctcagtcccc aacgcatact gatatttacc acatatacta atcgaggcgg 124560

aaaatttaag tacattagaa gagctacctt cttgttatgt tacaaatttc ctttattcta 124620

ttatcaatgt tcttagtaac actataccat agatttcata tagtgtcaag taataaatca 124680

aggtggtagg attcgaacct acgacttccc gtctccaaag cgggtactct aaccaaactg 124740

agctacaccc tgtcggttcc gtacacaaac cttttaattg cacggcatat ttttttgact 124800

agctacggac gtctagaacg ccagccgaca tctcctgtat cagagaagat gaagcagata 124860

tatagtatta tctgtagttt aaggtatgag gatactagaa ccagaaagaa gcacctctat 124920

tagttttcct gttcctatag tatcggaagc aaaaccgaag ttataggtgg atttcctatc 124980

ttttttactc gttgtaactc actttagtta ccgtttgctt ttagcggtgt gagtggtaat 125040

ctctttactg tacacctagt gtaccacact ttgcgtttat tgtcaacact tattttctaa 125100

aatttttcct gtaccaattt tctatcatac tatttatata gttattcatg tagtagtcag 125160

taactataat acccttgtct gctgctaatg tatactcatg aacccgtaat atatagtaag 125220

tacttatagg aagtacaacg ttacctgaat aaggtttagt agttctctct acatgtttta 125280

taaatgactc tatttctttc atttcttgtt ggaaagatag gtattttcca tgtctagtcc 125340

taaaaactag tttgcggtaa aacccaccat cagggatatg ctcttctcta gatattctag 125400

acctttgaag tttattcaac cttattttgt ctagtctcct ctcctttgaa tgccagtccg 125460

ctttactctt gggcgtcttc ttataacttc tgctcatagg acacctccta atgattatct 125520

acctagtatt tctctctgat agtcatttta ccattctttt cttctgcttt acgaaaattc 125580

tctctaactt tagctactct agcttgctct tcttttttct cttcgggtgt cagctccatt 125640

aaaccgagaa ctttcatagc atggtcatac tcttcttgtg agttacacac tactgtatgt 125700

ttaaaattaa tctctgtcaa tattgaagcc tcccttgttg ataattatgt acctatggct 125760

gttaactcct attcatttaa cttccagtct ataagaagca ccacagtttg aaacagattt 125820

ataaggttcc tcaacctaca tataagttaa ctttattatg tatttacaaa gtcctcagca 125880

ggattcgaac ccgcgctaag ggagtagaaa tcccttgtgc atccgctaca ccataaggac 125940

acgattctcc tatacacaca gccccgctct taaccagatg agacgtaacg gtcaccgaag 126000

caaccgtaag ggagtcgaac cctttccgag ctgtgcaaag gaaagtaaca taggtctctt 126060

attatgttac ttagtctagt gtccgtccac ataggttgag actacctcgg gacacgtccg 126120

cattctgtta attcagattt atttagtatt gtgttgcaac acaacgctgg gatgactaat 126180

ccctaaagcg ggtaggggga ttcgaacccc cactacatgg ttggaaacct tgggtactac 126240

ccttatacta tacccgcaga ggcaacctac ttagggcaag taagttacca atacaaaagg 126300

agtaaaaatt aattaaaaca aaaaagcgta tcctgctgga tttgaaccag cgacttccga 126360

cttaacagga cggcactctg acctctgagt taaggacaca aaatactcgg caaggatttt 126420

caccttgcaa taaccttcat cattcgtttc agctatcaat tcaagcatcg gatatgctct 126480

cagcttactt ctattatagg ttataccagt cctactgtgc gatatttttt ccgccacgag 126540

tataaaaaat agtaatttaa gtacaatagg tagcaagttt ttgtaagtgc tagcaagcgc 126600

aatactgtac cactcagtat taaaaaaggt gggatataca attgtaattt cattttactg 126660

tgtttgtaat tgttatcgta catggtttca tacacattgc tattgagtta aataacttgc 126720

taccgaggta actgtactat ttttgcctgc tattcctaaa gctcttacaa ttgacaggta 126780

aggagtcgaa ccttaataac taccactagc ttgtctttaa tacttatcgg tgcaccttat 126840

agtagactta gggttactag acaaactaaa gtatactaca tacttgactt actacccgac 126900

atttgaaggg aggttagaaa ttgattttag cctcttttta tttgctgata atagtttttt 126960

ctactatagc agtaaggtgc accgtttgtt tgtctcacca catagtaatc ctaattttaa 127020

ggaaggtaag ggatttgaac ccttgtaccc tgtattccaa gggcaaacta gttttcaaga 127080

ctagcgtatt accaagctct accaaccttc cattttgttg ctatggagtc cgcaggtatc 127140

gaacctgcct ctcctgcttt tcaagcaagc gctaatccat ctcagctagg actacgttaa 127200

tggagccact aggattcgaa cctagaataa cggggtaaaa acccgcagtg atagccattt 127260

caccataact cctttgtata gaggttagag ggatttgaac cctccgtaac tagctatata 127320

gtctagtatg tcgtcatttc atcttaacct ctataataac attataacat actttaagat 127380

gtttgtcaac tgttttacag ggatagtagg acttgaaccc acctcattgg agttggaaac 127440

caatatttta ccagataaac tataccccta agctggtcta gttggattcg aaccaacgta 127500

tcacggaatc aaaatccgtt gccttaccgc ttggctacag accaatagta ccaagtaagc 127560

tatgcttgta gtaggacttg aacccacgtc tgaggcttac aaaaccactg ttctaccaat 127620

tgaactatac aagcttattt ttttattaag tcagctctct aagtctccgc tctttacgtt 127680

agtaattatt agctgactcg aagagctaac ctattatgcg ccatcaagga ctcgaacctt 127740

ggaaccccga agggagggga gttacagtcc cctgcaattg cctctatgcg aatggcacgt 127800

tgtaaagcta gagtacatac ttcttgctta ttaagatacc ctagctaatt tttactactt 127860

gttatatact tcccagtcat tagcaaatac atctaagttg ctaatacacc acattacatg 127920

acttccagta gctgttctca tttgcaagta aggttctact ttaaatagtg caccttcctc 127980

taactcataa gcatctgctg tttgtttatt agcagggata ccctctggat agcctttctg 128040

agctactaca aacatgtctt taccattcca acctgttctg ctagctttct ttcctttttt 128100

aattaaaggt aaaatctctt cgaatgtcat aattttctcc tccttctatg tcttattaat 128160

accgagagtg ggattcgaac ccacatatcc ttgggacagc agattctaag tctaccgtgt 128220

ataccagttc caccatctcg gcaagtgttt ctactagtct ttgattacat tataatagtt 128280

agcataatga gtagagataa caaacccacc tcgaatgttt aagtatggag ggattctttc 128340

taagtcctgt aaatcaatac catctactaa gtaaccgtca ttaggggcat ctgttaatgg 128400

ggatacagca agagagttgt ctaccgaaat agttttcacg ttaggatatt ttttatcaaa 128460

aatagttttc tgaaactttg tgcttacaat aacagagtat ccaaattcct cagctatctc 128520

cattatagcc tgtgtttttc cttttctcct atacttaaca gtactgataa agaatagagg 128580

ctccattcga tacatagcct tcttaaatcg ccttacttct ttactctctt taaacttaga 128640

cttttttact ttcttatgag tgttcttttg gttcagcata ttcatcagtc cctttcattt 128700

ttattcaagc gcacgtaagg ggaatcgaac ccctatttct cgatagacag tcgagtataa 128760

tagccattat atcatacgca cagtactgag gtacttctag ctaaaaagtt acctcagagt 128820

ttaacttggc aatagactgg tcggctgtgt ttctttagct aaggaagcct tctacgcact 128880

attagtacca acacttggaa ttgaaccaag gactaccgat tatcggtcgg ttgctttgcc 128940

tcctaagcta tgctggtata gtatactctc tcgttgacta ctatacgggg gaatgtatta 129000

ataggaacga gagaatacct atttttaatt ataccacact ctaacagctt tctgagattt 129060

taatctcttg ggttttcctt tttcaaggtc aaggtttctg gtttagtgtg tctactacaa 129120

accttttttc gaaaggagag taagggattc gaacccttgg tactattact agtacaacga 129180

gttagcaacc cgtatcatta agcctctctg acaactctcc aagaaacaaa atttaaactt 129240

atatttatat tataacataa attagtacgt ttgtctacta ataacttatt ttttttcata 129300

aagcgggtaa tgggaattga acccatatac gtagatttgc agtctactac ctttccagtt 129360

ggttataccc gcaagttcta gaagactaat aagactccta gagtttttaa atatttagtg 129420

aaggtaaaga tataaacctg atgataagaa aaacgtgtta ctaacaatcg tctaacttac 129480

ctgtatatac aatatagcat acttactgat aaaaagcaac agttttctat aatatttttc 129540

atactgagct aagaaaatgt atagcaaccc taaaactata taattatctg acaactgtcc 129600

tatttaattt tattcgtccc ctatattata ttctaatata ttatataatt atttaaagat 129660

atgtaattag acttagaata taaaaggaat ataatatagg agaaccttaa aattaaatag 129720

gacaattatc tgaatatttt ataacgacta ctattacttg agtaacaata gaaacttgat 129780

actagtatca aataaaaagt aatttaatat tgatagtaac ccacctcagg tacctaaata 129840

tattcagaaa atttatacta ctgtcctatt taattttatc tacctcctat attatattct 129900

aatatattat ataaagacta taataaatta ttattattat tattatttat aatatagaag 129960

agtcctaaaa ttaaatagga caattatctg aatataaagt ttaattaaca taaaaagaca 130020

cctgtaaagg catctttatt aaacatctaa aaatttcttt tcgtaggata ctacaagtga 130080

ggcatttaca taaacttctt ctccatctaa tgctttagta gttctaacct taataaatgg 130140

taactcagaa cctcttacag cagagtttaa cttagtacta gggtcagtaa cagtatcaaa 130200

tacatcttca ctgaaaagat aaaatacatc tcctgcaata gtctttagtt ctaaaacagt 130260

ttgtcttcta gccattttta ttgttcctcc tctgttatgt ggtataatta aagtatatca 130320

cacattactt cttaactaaa ctgaaaacat aggtaactac cgcacctagt ataatcataa 130380

atactttttc tactaagtct tttttacctt catcttcttt ttggttcgtt tgtcttagcg 130440

cttccacacc tttctctaat tgacctatct gatagaagat gtgtgactgc ttttcagatt 130500

gtagggcaga atctttatct agagccacta caatatctcg taattccttg atagcttttg 130560

ttagctcctc attgtcagtc ttacgtgtta gttcacccgt ttgtacttta tcttcaaggt 130620

tttttatttt atctagcaga ctttgaatat ttgtatcttt ttccactcac atcctcctcc 130680

tctcagttct ttatttttgg tagggtttac gaatgtgttc tgatacatcc gagctttcag 130740

ggtagttatc taagaagttc ctaattatat cttcataatc aggtgttaca attctcccgt 130800

aatgtgtaac aaagtttagg tgaaagtcaa ttctaccgta ggaacctagg tcaaggtact 130860

taccaaccat gtcgtcttca tcaactttaa aataaccaaa gtcaaaacct ataagggttc 130920

ccattgatag agccctacta ataaagtaga tatgatattc ttcacgattt tccgagagga 130980

agttacgtaa tatatctcta aattctactt ttagcatatc cctgtattct ttcattgcag 131040

actcctttct ttacatactc catgcaacca ctactataac cataaaagat acaactatta 131100

caaaatatat aataaaggtg taaactaacg acataagtag tacaatgtta gccacacctg 131160

ctataaacgt tgctataatt agcgtaaagt tttgcgtaat accttgtata tctcctagag 131220

aaacatggcg ttgcaagtca cttataacgt ggtatgaagt ccaaaaaata cccacttgaa 131280

acaagacaga ggctagtaca gctataaaca ttatactcat agtaagacaa cctccttaaa 131340

gctagttcta atatagtata cgacatagaa taatttaaag ttag 131384

<210> 2
<211> 135028
<212> DNA
<213> Unknown

<220>
<223> Bacteriophage P100+

<400> 2
aaggttgtgc taaattaata cagtagaact atgtggaatg gaggaaatat tatgaacaag 60

ccagacaaaa atttctttat gaaaatgatt atcgttattc tatcaggatt acttgtgtta 120

tttgttataa acctagcatc tatctacttt gggtactctc ctcacgttgt agctacgtac 180

tttatgacag ggggctttat tgcgtatatc gtgataaact tatacgaccg aattgttttc 240

caaaaaggtt ctcctaggga agaacgagag aaacaagaga gacaaaagat agcatctaga 300

caaaaagctt tagaaagcgt agtaactcaa cgactaggta tccctataga ggagctagat 360

ttagaagtac tcattcagat ggaaaaggat ttagcagaca aaactgctaa aggtaataaa 420

aatgaaaaaa ctgagtagag acgagataaa gaaaagacga aaatatttca ttaacgaagc 480

aaaaagagtt acacatatac gagcaggaga ccgtagaata gcaacaggtt caagaatatg 540

taccttatgt ggtagaagtc tatcatctca gaaacggtct acaggagaga cctttgcctc 600

agtaaaccac atccattgtt actttaatga acttcttatc gttaacttat gtgaagatgc 660

aacttcttgt tatagaagac atgaaaggag ttagatagta tgaatttaga caacatagaa 720

agaaacttaa gcaagaaaaa aactacagga gatgccgtaa aggatgagtt caatgacatg 780

gctatgaagc tactagcagg ctttaaagca gatgtttcgg caggagataa aaaattagaa 840

gatgtagcag acatggctag gctgttacaa acctttatac aagttaacaa agaagatagt 900

ggtagcggag gaacatccac ttctttacca gagcttagta gcttgcaaca agaagcaata 960

accaatcaga taaagacaga gagcgtagtt tcaggtgata aagagcaaga actagtagac 1020

ctacaggact tagctaacat gacagaagag gacgtacaac ggatgataat ggataaagaa 1080

aaagcaatga acatggaaaa tgaggagacg ttttaatgga tggcaacact cttttaaaga 1140

tggcgcaaga aacttttcaa acagaaaatg taactacaga gcagttaaac tatgttctgg 1200

atatgctaac tccatcaaca tatttactaa gaaaccacaa agtaagaaat cataacatta 1260

cgttctctat tgcaaacaag tctactgaaa aggcgcaggc acatcgacct tggcaaccta 1320

agattataaa tgactcccac ccgaatgtag ctgttattaa gtcaagacaa ttagggttaa 1380

aattagcccc tctataaagt aatttataaa gaaaactctg ttaaacgggc ataggaaaat 1440

aaccaatatc ctgataagag accctaaatc ctaagtagga cagtgggaat cccgtgctaa 1500

atcaggcgca agcttgtaaa tgcctaacga ctaaatttct aggtagctaa ccaaatgggg 1560

ttagtgagaa ctagataaga aaatatattc tcttacgata ccaactgaaa aactctatca 1620

gatgttaaaa aattcttaga agaaaatata aagtaaagca gagcaaccta aaccaaaaaa 1680

ttaggttgat gatatagtct agtcccctaa taaatatcgg gaaaccgagg gtataacgta 1740

agtgagatgg gtgtagggag tatgattcat tttgcagata cacacagcta tgcagctgtt 1800

aaatgtttat acacgtttcc tacaggtgaa cagatgcgta aatttgtaca gtcacggtta 1860

gaccctgtac ttgagtctag ttactatagt tctattctag acaaagataa caacagtctg 1920
aactacaaaa gaatacgaaa tagtcacctt tactttagaa catctagtaa gcccggagct 1980
gtagagggtg tagatattga ctacttatcc atggatgaat atgacagagt tcctgttttc 2040
tcagaggctt ccgctttaga gtctatggct agttcaccct ttaaaattgt tagaagatgg 2100
tctacaccat cagctcccgg tatgggaata caccgattat tcgaaaactc agaccaacat 2160
tggtacttgc ataaatgtga aaaatgtaac cactggaacc aactagacta cgaggactac 2220
gacagtagct ctgtagaagc aggaggaaat atactatgtg taaatccaca aggtgttgat 2280
accttagcta aaacagttgt agaaggttcg ttccaattcg tatgtaaaaa atgtggagcc 2340
ccattagacc gttggtataa tggtgaatgg gtagcaaagc accctgatag aacaaaaaat 2400
ggagacggta ttagaggata tatgatttct cagatgaatg ctgtttggat aagtgctgat 2460
gacttaaaac gtaaagaact aaattcactt tcaaaacaag cattttttaa ctatacacta 2520
gggtacccat tcgaagatgc taaactagca gtttatagtg aagatattat agaaaatcca 2580
agcccaagag taaaggttcc aatgcacagc agaggagatt acaagtacat ttctgttggt 2640
atagactggg gaaatacaca ctgggtttct gttcatggta tgacggaaag aggagaaata 2700
gacttgataa gactgtttag tgttgagaag agtcgaggag taggtaatat tgagtctgat 2760
ttagataaga ttatcctaga agtatctatg tacaaccctg atatgattgt agctgacgta 2820
ggggatagcg ggaactacgt ggacaaatta gtaaaacatt tcggagaaga ccgagtattt 2880
ggttgtattt ataagtccag ccctaaatct acaggtcagt tgaaaccgca gtggaatgaa 2940
gcagggaatg tagttactgt agacaagtta atgcagaaca aaagatatat tatagaaatg 3000
aagacaaaga aagttaactt ctatagcttt atagacccta tgctaaaact attggtagac 3060
cattggagaa acgttgtaat acaagacgaa gaggacgaaa aagatggaac tttctaccaa 3120
gttattggca gaagaggaga cgaccattta gcgcaggcat cagtgtacag ctttattggt 3180
ttagacagac tagctgatat gtatttgaga ggctctaagt atgagttcaa ctctactttt 3240
gtaacaagca actaccaaga tgtttcgtca acaccaccag atatattcag taaatattaa 3300
attaatttag ttagggggct atgctccctt tcttatttgt gttataatat agacaaggag 3360
gttttactgt gacaaatgaa gagaagtttg acaaagtaat gtctatcgtt aaagaagaca 3420
actctaccgt cctacctgac tccgcttcta ttagtaaagg aattttaaga gagttgacac 3480
aggaaagttt acaagactac ggagaggtca ttgaaaagaa tgatttttta agcagggagt 3540
ttaaacatat tgtttccctc tacggttttg acgactttta ctcactatat gcctatgctg 3600
atttttgcga tactatgagt aatctagcta ttaaaggcgg gaacaaagat ttatctaagt 3660
taaaaaaggt aaaaagaacc gttatgagaa acggaaaacc tacagagatg actttttatg 3720
agtcaactac cgaagaagag aataaagaca agaaagaaga aagtaatacc aagcgccaag 3780

gagtaagcac agtatctgca aaagacttac ttgcagagct actaaagtct aaaggggaac 3840

cagaaaaagt aactcagtta ataaaaataa ttcttaaatt ccctgataca cctgctaacc 3900

ccaaagtatc tgattatttt taccagttaa aagacgactc gggagaagta gtcgctgtgg 3960

caggttacag tataggagcc caatacgtct ctcttgattt ttataccagt aatggctcta 4020

ttagcggagt agcatcaaga gcgttctttg aattactacg gatagcaata gaaaaagact 4080

taggagctga attaaatgac gaggataacg agctagctca agtactattt tcaaatttta 4140

attttcagct agatgaggga aaacagagag tgtctgctag aaacttaaaa aaacacttta 4200

aggggtaact atatgttgcc attctattca atatatgcta taataatagc atgtgttata 4260

tacacagtta tctttatttt agtagtacgc agaatgtatc gtaaagcaga caaacgagag 4320

aaggaaaaag aagacattct taacactatg acagaaataa cagaggaaat aaaaacattt 4380

attggaggag aaaataacaa tgacaattga gaacacgaag actttagaac aaacactaaa 4440

tactgtagcc gaggaaaaag attatgactt attcctatca acgttggcgg gggtagttaa 4500

atcactatac aaagagtacc aatttcttag acaagaccca gcacccgagg ataaaacatc 4560

aggaaacatt ttgattttcc aaattttccg aggagaccct gacggttatg ctaaagattt 4620

tgatacactg tttaaaattg cagtagacag aaatttagag cacagaaaaa tatcccaaaa 4680

attctttgaa tgggtagaga aaggtaagtt cttagagttg ggtgataaca tcgttattag 4740

cagggatggt acaggagtaa gctctattgt tgagtctgtt gaacttaaag tttttattac 4800

ttttatgact aaaagttatg ccgaagaacg agaagatttt ttagctaaag agaaggaaaa 4860

gcaagaagag gctaaaaaga aagaagaaga gcgactagct ctaaaagagg aatcgtataa 4920

atctttagga gaggctacag aaatgctaaa tactctcatg gaagagcaag gtgtatctcc 4980

tgaggagctt gctttagctt tagaacaggg gaaggagagt tatcgtaatg cacgataatg 5040

aatttgaagt acctgagagt gttattaatg cttctgctac accttttgag gcgcctgata 5100

gaacaaaaga tacctaccaa agttcattaa agaactggtt ggacagagta aaatcagtcg 5160

gagatgcagg aatgtcacga gagtttgtta ttcgtattcc agcaaaggca ctagaggaga 5220

acaacccaga agctacctta tttttaaaac agtgtaacga aataaaaaga aaccctaagt 5280

gcaaccttcc tattaaaatt gaaaaaacag tagagaagta caaagacaag aaacacttaa 5340

tgttagactt ggtgttcact tatagtaata aaacagatat tgatgcagta aatagtgtaa 5400

taaaataaca aacaggttcc tctttacgtc tgctatatta gatagtagaa aacgtaagga 5460

ggaattttta tatggtaaaa tataccgtag agaacaaaat tattgcagga ttacctaaag 5520

gtaaactaaa aggggctaac tttgttattg ctcatgaaac tgcaaatagc aagtctacta 5580

ttgacaatga agtaagctac atgactagga actggaagaa cgcatttgta actcactttg 5640

taggtggcgg aggtagagtc gttcaggttg ctaatgtaaa ctatgtttct tggggagcag 5700

gtcagtatgc taactcttat tcctatgcgc aggtagagtt gtgccgtaca agtaatgcaa 5760

ctacatttaa gaaagactat gaagtgtact gtcaattact agtagaccta gctaaaaaag 5820

caggtatccc tattacactt gactctggta gtaaaactag tgataaaggt attaaatccc 5880

ataaatgggt tgctgataag ctaggaggaa caacacacca agacccatat gcttacttaa 5940

gctcatgggg tattagtaaa gcacaatttg ctagtgactt ggctaaagta tctggcggag 6000

gaaacacagg aacagcgcca gctaaaccaa gcacaccagc acctaaacca agcacaccat 6060

ctactaacct agacaaactt ggcttagtag actacatgaa cgctaagaaa atggactcta 6120

gctacagtaa cagagctaag ttagctaaac agtatggtat tgctaactat tcaggaacag 6180

ctagccagaa cactacactc cttagtaaaa ttaaaggagg agcacctaaa ccaagcacac 6240

cagcacctaa acctagtaca tctacagcta agaaaattta tttcccacca aataaaggaa 6300

actggtctgt gtatccaaca aataaagcac ccgttaaggc taatgctatt ggtgctatta 6360

accctactaa attcggagga ttgacttaca ctatccaaaa agatagagga aacggtgtat 6420

acgaaatcca aacagaccaa ttcggcagag ttcaagtcta tggtgcacct agtacaggag 6480

cagttatcaa aaaataaata atggtaaact agaggagact tttatagtct cttcttttct 6540

tttgtaacag tagtgaaata aaacttaacc cctttgtaat attatggaat tacttctgta 6600

acacatctat gttatattta gttggtaatt taaaaacgca tatgcggaat atagaaagga 6660

aaccagctaa tgaaaaaagg aagtaagatt gcaattgcta ctgtagtagc tgtacttatt 6720

gtcgttacag ttagcctagg gtaccataca gttaagttga atgagacaaa tgaggcaaag 6780

acagaagagg tagttaagct aaaggagaat atctctagtg tatctaaaga tttagataat 6840

ttaggtaaag aacatgaaaa gaaagtctca gaatccgaag ctcttaagaa agaccttgac 6900

aataaaaaga aagagttaga ggataagaac aagaaagaag agcaacttaa aaaagaaaaa 6960

gaagaactac aaaaacaagt aagctataaa aagagcttaa agaaaaaggc agtagcagaa 7020

aacattccaa atgcaactag tagtaataaa agtgctaaaa cggcagaaag caaaggaaat 7080

ggaagaagtt ttaacgtaga agttacttac tatgctattg gtgacggttt tactcccggt 7140

gtacataccg caaatggtac taacatcaaa aacacaataa caacatcaga aggacatcga 7200

ataattgctg tagacccatc agtaatccct atgaactcta ttgttaaagt aactacaggt 7260

aacggagaga gttttgtagc taaagcttgt gatacaggtg gagctatcaa aggtaacatt 7320

attgatattt tagtagaacc tagcgcagat atttatcaac tagggagaac aacaggtact 7380

gtagaaatta ttggataatt taaagaaata agagtctgca aggtataaca accttgtggg 7440

cttttttatt atgctatatt atatatgacg gattatgaag tttgaggagg tatattaacg 7500

tggttataac acagatagtg atgattatag cgattattat tactacattt cttattattt 7560

atggtatata ctcttctagg agaaaaacgt ctaagtcgtt tagactagta ctcagatatg 7620

ctataatggt agtaggtctt cttattgtct actatctatt tcaagtttcc tctattgaca 7680

ttcaacagga ttttttacag cacttaaaaa caggaggtaa cgggatagaa gaagtttttt 7740

tcacaaattt ctttcttatc atggtaggag cagtaggtac acttgtagta caactattcc 7800

aaaatagaaa gaaggattaa tgtgtcatat acaaaagact ccttgtggtt ggagacaaaa 7860

aaggttgcta gagataatgg gtttttaggt aactggatag agatggttga ctactatgag 7920

agtataggag gaagccatgt attcgtttat gcggttatgg aaggtcagaa gtaccgaatt 7980

ttaggcgttc ttagcacgga tgaggtatta ctactaaaaa gagatttaac gctctcaaaa 8040

gccctatatg aagatgttat gaatagcaaa aagatgtttt tctataaaga ggacagtgaa 8100

aagaaaaaaa taaaattgac agatgaaata agtaagttag aagataaaga tagtatcata 8160

gtatatatgt aggtaggtga aaaataaatt gggactattt gaaagtataa gattagcggg 8220

ggtaaataag agcgatgcag taaaacacat tgaggttgat gataactact ccattgcaat 8280

tcaacaaagg gaacaggaac aaatatctaa agcaatgaat aacaaagaag tagcttattc 8340

tcaaccagta attggctcta tgtctgctaa cccggggttt aaaacaaaac ctagtattcg 8400

taataaccaa gatttacacg gtgttcttaa aaagtttggt ggaaacatca tcctgaacgc 8460

aataatcaat acaaggtcaa accaagtttc tatgtattgt aaaccagcta gacactctga 8520

gaagggtgtt ggttttgagg ttagattaaa agatttagat aagaagccta cttctcatga 8580

tgaagcaaca attaaaagaa tagaaagctt tatcgaaaaa acaggagtag ataacgatat 8640

taaccgagat agcttttctt cctttgttaa aaaaatagta cgagacactt atatgtacga 8700

ccaagtaaac tttgaaaaag tatttaacag gaaccaaagc atggttcggt ttgtagctaa 8760

agacccaaca actatatttt ttgcgacaac agcagatggg aaaattcctg ataacggtaa 8820

tcggtttgta caggttatag accagaaaat agtagctacg tttaacgcaa gagagatggc 8880

atttgccgta aggaacccac ggtctgatat ttatgctaca ggatacgggt accctgagtt 8940

agagatagca cttaagcagt ttatagcaca tgagaataca gaagcgttca acgatagatt 9000

ttttagtcac ggtgggacaa cacgaggtat tctacaaata aaagcagcac aacaacagtc 9060

gcaacatgct ttagagatat ttaaaagaga atggaagaac tcgttaagtg gtatcaacgg 9120

ttcatggcaa atccctgtag tatctgccga agacgttaag tttgtaaata tgacaccttc 9180

tgcaagagat atggagtttg aaaagtggtt aaattatctc attaatgtta tctccgcact 9240

gtatggcatc gacccagcag aaataaacat acctaacaac ggaggagcca caggttcaaa 9300

aggtggtagc ctaaacgaag gaaactctgc cgaaaaaaat caggcatcca agaacaaggg 9360

cttacaacca ttattaggat ttattgaaga ctttataaat aaacatattg tagctgaatt 9420

tggagacaag tacacattcc aatttgtagg cggagacata aaatcagaac tagagtcggt 9480

taagatacta gcagaaaaag caaaagttgc tatgacagta aatgaagttc gtaaagaact 9540

aaacttaccg ggagatgtaa ttggagggga cattccatta aacggagtaa tcgttcagcg 9600

tataggacaa cttatgcaac aagagcagtt tgagcatgag aaacaacagt ctaatttaca 9660

aatgttacaa gaacagacag gtaacagagt aagtactgat gtagaagaca ttcctgatgg 9720

gaaagataca acaggagaca taggaaaaga cggacaaaga aaagataaag ataacgcaaa 9780

cgctggtaaa caaggcatga aaggtgataa acctaacgat tggcagacct aatattttgt 9840

agttctgcct tttaggtaac taactagtag aggtatatgc tatattaata acagaatgtg 9900

ttataataga tatgagaggt gatttcttaa ttgaataacg tcgtagagaa agagatgaaa 9960

atctttgtac ctattgatat tgaaggttct ttagaaaaat cccataaggg agatgacaag 10020

gactggtatg ttaaaggtta cgcatctaca cctgctctag acttacaggg agaaatggtt 10080

aaccctgctg gaatagacat tggttatttt gtagataatg gttacattaa ctttgagcat 10140

agacaagaac cagactatgt agttggagtt cctacaagta actgttatgt agacttccaa 10200

aaaggtctgt tcgttgaagc aagactgttt aaagacaacg aacacgccca atctatgtgg 10260

aagttagcta acagcattag taagacaggt attgatagac aacttggttt ttctatcgaa 10320

ggtgcaataa gaaagagaaa cacagatgac agccgaatta ttgaagaggt tgtaatcaaa 10380

aatgtagcag tcacaatgaa cccagcaaac ccagaagcta cgtgggaaac atttatgaaa 10440

tcatggacta ctggacatgg aacaacacct gatacgcaag taaatgcagg agctttaaga 10500

agagagagct tagctaacag catatcagta ctagcatcag catacaaaat ttctgaccct 10560

attgaatttg acaacttatg gaaagatgta agtaagtttt tagatgaaaa aggagagtat 10620

aacgagaatg aggcaatagt tatgttacaa ttagctaaag gcgtttctcg tgtaagtgca 10680

tgtgagtctt taatgaatag acgaaagggg aaacaaaatg aataacagtg aagaaaagtt 10740

cgaagatgtt ttaaacgaat ttggtaatac agtagaaaaa gataaagaag aaatttctaa 10800

gtctaccgaa gaaaaagagg aggttacaga tattaataaa gataaaaaat cagaagaaga 10860

aactgctgag gaagaagtaa ctgaggctcc cgaagtagac gttgcagaag acaaagaagt 10920

gtccactgag actcccgaag aagagtctac tagtaagtca gctaaggaag aaaagaaaga 10980

aaaagatagt gaagaagaca aggacgaaga cgagaacaaa aaaggtaaga aagacaaaaa 11040

agacaaaaaa gaagaagaca gcaaagaaga aaaaacggag aagtctgatg aagaagattc 11100

cgaagaggct gtaaaaaata ctaactctga tgcagatatt gcaggagcat tgaatgctgt 11160

tgctaagtct tatgagaatg ttaaagaaag ccaaagagag caaaatacaa ctgtagaacg 11220

cttagagaag aatatcggta gcttagtcag taaattagac agcttggtag atttaatgag 11280

tgaacgtgta tctaagagtg tagtagaaga tattgaaact cttgaaggag ctagtgaaaa 11340

agctgttggc tatgtagcaa aaaacgtaga tagcagtgta gtagaagtta ttgaaggaga 11400

agaaacttct aagagtgtag atgcagaagt gagtcctaga gatgagtttc tagctaataa 11460

gagtgctttc ctagagtctt ttgaatatgc aagtcgtaac ggtgctgagc gtagagattt 11520

aactgatgtt cgtcacgctc atgggaacat tacaaatgat attgctagta aagcagatta 11580

taaccgagtt aacgacttta tgaaaaaatg gagtaagtaa taaaggtaac tgtatagagt 11640

attttttgta aacagtatgc tatattatag gaacatggga aacatgttac gttatgagag 11700

agcttgcccg ttttaggact cctcccctag ggcaggttat ctcactaaca aaaaaataaa 11760

actatgaaac tgaaaggtga taaatataaa tgccaaaaaa taacaaagaa gaagaagtta 11820

aagaagtaaa ccttaattca gtacaagagg acgcgttaaa gtcctttaca actggttatg 11880

gtatcacacc tgatacacaa acagatgcag gagcattaag acgtgagttc ctagacgacc 11940

aaatctcaat gcttacttgg acagagaatg atttaacatt ctataaagac atcgctaaaa 12000

aaccagctac atctacagta gcaaaatacg atgtatacat gcaacatggt aaggtaggtc 12060

atactagatt tactcgtgag attggggtag caccagtaag tgaccctaac atccgtcaaa 12120

aaacagtaaa tatgaaattt gcttccgata ctaaaaacat cagtatcgca gcaggtctag 12180

taaacaacat tcaagaccca atgcaaattt tgactgacga tgctatcgta aatattgcta 12240

aaacaattga gtgggcttca ttctttggag attctgactt atcagatagc ccagaaccac 12300

aagcaggact agaatttgac ggcttggcta aacttattaa ccaagataac gttcatgatg 12360

ctcgtggagc tagcttgact gaaagcttgt taaaccaagc agcagtaatg attagtaaag 12420

gttatggtac acctacagat gcttacatgc cagtaggggt tcaagcagac tttgttaacc 12480

aacaactttc taaacaaaca cagcttgttc gtgataacgg aaacaacgta agcgttggtt 12540

tcaacatcca aggtttccat tcagctcgtg gatttatcaa acttcacggt tctacagtaa 12600

tggaaaacga acaaatctta gatgaacgta ttcttgcttt accaacagct ccacaaccag 12660

ctaaggtaac tgcaacacaa gaagcaggta aaaaaggaca atttagagca gaagacttag 12720

cagcacacga atacaaagtt gttgtaagtt ctgacgatgc agagtctatt gcaagtgaag 12780

tggctacagc tacagttact gcaaaagatg acggcgttaa actagaaatc gagttagctc 12840

caatgtacag ctcccgtcca caattcgttt caatctatag aaaaggtgca gaaacaggtt 12900

tattctacct aatcgctcgt gtacctgcta gcaaagcaga gaacaacgta atcactttct 12960

atgacttaaa cgactctatt cctgaaacag tagacgtatt cgttggtgaa atgtctgcta 13020

acgtagtaca cttgtttgaa ttactaccaa tgatgagatt acctctagct caaattaacg 13080

catctgttac atttgcagtt ttatggtatg gagcattagc tctaagagca cctaagaaat 13140

gggtacgtat tagaaacgtt aaatatattc ctgtaaaaaa cgttcatagc aactaataat 13200

tataggataa ttgaataaaa acagtataga gagcagataa atactgctct ctattttact 13260

aataaggagg atttaaattg ctaaaaaata caaacttagc taattataaa aaagtgaata 13320

cacgatttgg aaatcttagt tttgatgata aaggtatttc taatgaccta acggaagagc 13380

agcaaaaaga attaggtaag cttagaggat tcgaatatat taagacagaa cagaaaacga 13440

aagaagaacc taagaaagaa gaacctaaga aagaaagtac agaaaatgaa ttagacagct 13500

tcttagctaa agaaccttca atcaaagaat taaaagaatt tgcgagtaaa aaaggcatta 13560

aaattgaaaa aactaagaaa aatgatataa ttgaagaact aaagagaggg taatgtacaa 13620

tgtatggagg ttatgaagga caagattctt acgaataccc ttactcacac gggaacccta 13680

agcatgtaga gccagaaaaa gttgacgaat atgttctttc tgattatggc tggactgcgg 13740

aaacaattaa agcatacatg tatggtgttc gtgtagtaga ccctgaaaca ggagaggaaa 13800

tgggagacac cttctacaat catattatag aggttgccgt tgataaggca gagaaagaac 13860

tagatatagc tattctccca agacgggagt cagaacacca cgattataac caaacagaat 13920

ttagtagtta tatgtatacc catgttcata aaaaacctat cttacaagtg gatgacttaa 13980

aactagaaat gaatggtagg gcaatatata gataccctgc taactggtgg aaagtttatt 14040

gcttagctgg acatatagaa ctgtacccga cagccttgat gcagacttct caaagtatga 14100

catatgactc tgtatttgga ggatacccgc agttggcagg aacagcccct tctataggta 14160

gaacatatgc ccctcaaatg attcatgtag aatatatagc aggtatgcta ccaaggaaaa 14220

gagcaggtgt tagtcgtgac tgggaagtac ctgctgcatt agaacagtta gtaactaaat 14280

atgctttagt agaaatattc caagtttggg gtagacttat catcggagca ggtattgcta 14340

gtagaacact agtttacgac ggagtttcag agtctattga tacaactcag tctgccatgt 14400

atacaggttc agcagcagat attgagctta taaataaaga catagacagt ttagtagctc 14460

aactgcgttc tcactttgga ataaacatga ttggactata agagaggaga atgtttaaat 14520

ggcagagcct actagatttt caagtgatac tcctattaaa aacctaacaa cacaaaaagt 14580

agagtacaac atatcacagc atagaaagtt tattaaagac cgtagtatcc atacgttatg 14640

ggaaagagct tatttatgcc cttgtatgaa caagtcctcg ggttctgctg actcttcttg 14700

tcaaagatgc catggtaggg gatgggcgta tttaccagct aagcccgcaa atatgattat 14760

tcaaggacag gaaaaagggg ttactaacac agacctagga ctatatgact caggaacggc 14820

tatcggtaca aatgattttg atgctagaat ggcttttagg gacagaataa cagtacctga 14880

tgttaaaata ggtcaatccc ttatctactc tataaccgaa agcagacaaa aacgaggtat 14940

atttttaaca tatgacgttg atactataga ctttataaca agtatggaaa gagaactaat 15000

agaaggtaaa gactacacct ttgataaaca cagtaataaa ctattccctg atattagttt 15060

gataggtgaa gttatatctt taaacgtatc tgttactcta aggtatattg ttattgactt 15120

actaaaagaa agtcgctatc agtacaccac attaaataga acagacccta agtttgagca 15180

gttgcctcag aagttattac taaaaagaga agacactatt gtattacctg aaccatttgt 15240

acttgatgct gatacagaga ctagagtaga gttagttaat gaccctaaaa ggaaaccaag 15300

acaacaaggc gggttctacg aagggatgtt tgactagtga aacagagtac acagagacca 15360

tctctattca ataatccaaa gcaggtaatg agaaatatgg caagagctat agaatcagat 15420

gttattgcaa caggagctca agaggcatct cgtataccca atgtagagat taaaattatg 15480

cctaagtatt tagaagtaac tgaaaagaga atggaaaaaa atggtgtaat tgaccttaaa 15540

ccttattttg ctaaaagtaa taaaaagaaa tggaatcaag aaggtaactg gtacttagta 15600

atccctataa gattaaagac aagagatatg tctcgaaaat tatatgatga cttaaggaaa 15660

cagtcttccc caccagttgg aagttcaaca acaattgtat cagatttcct atatgaccgc 15720

agaagaacga gccctagtgt cccttctata aactataatc caaagtcaaa taatgttaca 15780

aaaaaacgta ttagtaacag tagaacaaca tatacagtat accgtacagt aactgctaag 15840

tcacctgcta atagttggat tgttaacagg gacagggtta atgaagagga tatgagtaaa 15900

acgatgttag acagaataga caggctaatg aaatggaagc ttaaaaactt gtagaggagg 15960

agtacaatgc ctattccaag ccttgattct tatgtagcaa gaagagtaag agagagcctt 16020

aaaattatgc tttcagagtg ctatattata gaggaagctt tgagtgacat ggatgtcgaa 16080

gcaaaaagta actttatgaa gacatattgt ggagataatc ctaaaaaaga aatagaagtg 16140

atatacgagt tccctcaggc aaaaacatca gtagatgcta cttatgtaat tcagctagga 16200

ggaggttccg aagatactaa aagcatcggc ggtattgaag gtacttacga gtataaagag 16260

gattctttaa gaaaagagac agtagttgct actagagaag gaaccgagtt atttctagaa 16320

acaaaagagc taattggaag tatagaggga atacctaatt tatccttttc agaaagtgat 16380

aataaacgaa tagaaggtaa acgattatac ttctcttatg aaggtaatgc agatattgag 16440

ggagtttcct tcgatattat ctatacaagt aaagtagacc tagagagcga ccccgttgga 16500

ctaaaaaaag ggtttacttc tagagacaat atcactgttg taggtttaag cactaacatg 16560

gatactgcaa ggtgcctaga tgctatctta aaagttatcc ttattatcat gagagaaaac 16620

ttagaagaga agagttacta ctccctacaa acaactcagt tttccgatat gtctcctgta 16680

attatgggag aggaaaaacc tgtatttggt agacctgtaa caatgagtta cactatatca 16740

tatactgtag actttaatct agtcaaccaa ataaataaaa ttatagtaca ggagcgtaat 16800

aagtgatggc agaaaaaaaa gaggtaacta aagaacctat tgtagacgaa gtaaaagtaa 16860

caaagaagaa ccttaaggaa cctacaacag aagaaaataa agtagtgaag aaaaaactta 16920

aagaagaaca aacagatact gtagagaaag ttactacagg atatgttcat atagatgtat 16980

tccttggaac agccaagccg tggtacggtt tatcagaaat gcaagtacaa gggtttaaat 17040

cttttatgca agggaaacaa taccagttcg gtgaacaggc ttttgttcct tatttagaaa 17100

aatatctagg aagggaagta aaataaaata tggcaatcga aatttaccca agaaaaccag 17160

tttctagacc tcatacagaa atttctgtag atacaagcgg tattggtggt tctagctctg 17220

gaagtgaaaa aatactttgt ttagtaggtt cagctacagg aggaaaacct aatgctgttt 17280

acaaagtaag aaactattct caagcaaaat ctgttttccg ttcaggagaa cttttagatg 17340

caattgaaag agcatggaac cccggtgaag gaacaggtgc aggagacatt ttagctatgc 17400

gtgttgagga agcaaaagaa gctacctttg aagctgaggg ggttaaggtt agctctacta 17460

tttatggagc tgatgccaac gacatccaag tggcactcga agataacacc atcacaggta 17520

caaaacgctt aagcattgta tttgcgaaag aaagagtcaa ccaagtttat gacaacttag 17580

gtagcatctt ctctatcaag tataaaggta ccgaagcttc tgcaacattt acagtagctg 17640

tagacccagt aacatttaaa gctacaaagc taactcttaa agcaggggat aaaacagtta 17700

aggaatacga tttaggttca ggagcgtatg cagaaactaa tgtacttatt agtgatatta 17760

acaacctacc tgactttgaa gctaagtttt tcccaatcgg agataaaaac ttaacaacag 17820

acaacttcga tgcacaaatc gatgtagata ttaaaacaaa agaagcttat gtaaaagctg 17880

ttggcggaga cattgagaaa caaacagcat ataatggtta cgtagacttt gaatttgacc 17940

gctcaaaaga aattgctaat ttcccactaa ctaagctaac tggtggagac aacggtacaa 18000

ttcctgagtc ttgggctgat aaattcagtt actttgcaaa cgagggtggc tactacctag 18060

ttccattaac atctaaacaa gctgttcatg cagaggcact tcaatttgtt agagattgct 18120

cttataacgg taatccaatg cgggtattcg taggtggagg aatcggagag tcaatggagc 18180

agttgttcac aagagctatc ggcttacaga atgaacgagc aggtctaatc gggttctcag 18240

gaacagtgaa gatggacgat ggacgttctc tgaaaatgcc gggatatatg tttgcagcac 18300

aagttgcagg acttacatgt ggactagaga taggagaagc aatcactttc aaaaacattg 18360

cgattgagac attagataca atttacgaag gttctcaact agaccaatta aacgaaagtg 18420

gtattattac cgcagaattt gtacgtaata gagcagttac caatttccgt attgtagacg 18480

atgtaactac atttaatgat aaaacagacc ctgttaagtc agaaattggt gtaggagaag 18540

ctaatgactt cttagtttct gagttgaaga taagcctaga caacgagtat attggaacta 18600

aaattattga cacctctgct tctttagtta agaacttcgt tcaatctttc ttagatagaa 18660

agaaattagc taaagaaatt caagactaca gcccagagga agttcaagtt gtaattgagg 18720

gcgacgttgc tcgtatctca ctaacagtat tcccaatccg cagtatgaag aaaattgagg 18780

tatctctagt ataccgtcaa caaatcctta ctgcctaatc taatacaggg gagggcttag 18840

ctctccctat gtaaataaaa aataaaacag gagttgaaac ataaaatggc aagtgttact 18900

aatcagacgg ttcatacagg taatacggtc taccttatga taaaaggtaa aatcgtagga 18960

agatgtcagt ctgcatcagg tgaacgttct tatggaacaa ctggtgtata cgaaatcggt 19020

tctatcatgc cacaagaaca cgtttactta aaatacgaag gtagtttaac agtagacaga 19080

ctgcgcatga gaaaagaaga tttagctaag ctaggtatta cagcattagg agaagacatt 19140

cttaaaagag atgttatcga cattgttatg atggataata ctactaaaga agtaatcatt 19200

gcttatcgcg gatgttctgc tgagacatac aatgaagaaa tcaaagctaa tgagattgtt 19260

tctgaaagtg caagattctt gtacctaagt gcagcaaacg taaaataata ctaataaaag 19320

gctaccttaa tctggtagtc ctttttttat agtttactaa ccttagcaaa tatggtataa 19380

taaagtaaga gaacgaaaaa gtattactag tttgttatat tagataatga gagagtaaac 19440

aaggaggaga caaattaaca tgtcagaaga acaaaaagta aatgaagaac aaatagaaga 19500

aagtaaagag aagatgttga cgaaagaaga catcgaccgt atcaactatg aacaacaaac 19560

gaagcaaaag aaaacacttg accgtattat taaaggggta aatgatgttt ggacacatga 19620

gtatgacttt aaggagttag atttaaagtt caccattaga ttacgagcac ctaatgcttt 19680

agaacaagca acaattcaag ctaacagaga agcttattta aaaggaacag gtgcattagt 19740

tagtgactac atttatacaa tctatcatac attagccact ataagaacag ttggtgtaga 19800

tattccaaag gaactagcag aagacgataa catttacaat actgctattt tataccaaat 19860

tggacgtgat tttgccgaat ggctagacac gtttcgctac tgagataaaa gatttaggtg 19920

gaataaaagc tttagtaaaa actccctatg caagaaacat gtggggaata atgaaagagt 19980

tcaatgtttt acctacagac gagaggttca gagcgcttac tgaggagcaa ataaacttca 20040

tctactataa caaagagaga gactacaaag aggcagagag agcttctaga ggcgtgtcta 20100

tggagtctga atttgaagac tataactcgg atgactggtg ggaagcttct catgaggact 20160

tccaagcaat tcaggaaggt catgatgaag atgacatcgc tcaacaagta gagaacatga 20220

ctagtatcga agaccaaaaa cgcctaagag acagattcca gtctcatgag gagtacgaag 20280

agtttataag aaatggtggt aaagatacta gacaggaaat gattgatgaa cacatcaata 20340

acaaactgaa agaactgttt gcagatgccg aagagttaaa taaacaaggc ttgagtaagt 20400

ggggagaagt atcagaaatg acaactatcc aagaagctgt agcagacgag ttagaagagc 20460

taacatcaga tagtgttaag gaagcaatca gtatgttcga agatgacgaa gttcaggtaa 20520

taccgggtca agatgaagac gatgattttg aaatttaata ctaaaaagaa agggtagagc 20580

tgtgtactct actctttttt actaaggaag tgaaagtaat tggaaaacac taactatcgc 20640

ttttcggtag aagccttaac ggcagatgca gagaaaaagt tagagcgtat cgctagaata 20700

atggacgaca ttgagggaac aaataggaaa ggtgcaagta atttcttcac cagctctcaa 20760

aaagatatag acaacaacat aagagctatg agagaatatg tttcattagt taatgaagtt 20820

gagaaggaaa tgaaaaacgc ccagtcttct atgtctcgtt ctggaaataa cgaaggtgtt 20880

caaaaattaa taagagacca gcaaagacta agggaagaag cggaaaaaac acggaaaaca 20940

ttccaagata ctacacaaaa taaaacaggt agtaacgctt atcttaaaga cctgttaaga 21000

gaccaaaaag catacaacga tgaaatagac gaaggtactc gtagattaaa agagttgaaa 21060

gacttacaaa gagatgtagc cagaacagat agcagactgg ctgccagaac aaggcaagct 21120

acttcatcag gtaatatgaa ctcacaacaa gcaggaacag ctaggtctga cttagcaaat 21180

aaaaccgcat atatgagtca attacaagcc aaccaacaac gaagagatga gttaagaaaa 21240

gggctagagt ccgaccaagc aaaaaaaaga aacttaaatg ctaactttag aggtaacgac 21300

caagaaagac agaatgagtt gtcagcgtta aaccataata ttgagactcg tagaaaagaa 21360

atagaagctt taaaagagct cgatagtcaa ataaataaga cagtaagtaa tatggaaagc 21420

tacagtaacc aattaaataa tgtaaatgta aaaccagacc gtaactcgtg gcaaggggtc 21480

gcttatgaaa gagcaccatc tatcgctatg gctgcattag gtggagcagg tgcaatattt 21540

ggcggtctat atgctaaagg tagctctata aaagaccaga ttagacctga cactatctct 21600

atagggcaac gaacaggtaa ctatgatttc cgttctatca gaaaacagaa cgaagaagta 21660

ggtatggata ggtctcttgg ttacaacgca gtagacatga ttgatttcca aaacacctat 21720

ttgtcttctg ctgggtttaa aaaccaaaaa gacttagatg caggaactag acaaatggca 21780

gaagggtcta gagctgtacc cgtagataaa gattctctaa atcagtttat gggtgggcta 21840

atgacgaatg gagccgtatc aggagcagac caagtaaaag cggtacaaca aggttttatt 21900

ggagcagtaa aagcttctgg aatggaaggt agggaaaaag accaactaaa agctttagaa 21960

gctatgagta cccaaacctt tgcaggaaga aatggttcta atgctgagct aaataacctg 22020

ttagctatcc agtcactgtt ttcttcatca ggtgaccgtt ctatgcaagg aacagcagga 22080

gcagaagcct tatctagtct agatgcagga atgaaacaat ctgcatataa cccacaagca 22140

agattactat ttggtatggg gagtgagtac caaggagtag gtggtatgta tcaagttcaa 22200

ggacaaatgg aaaaaggtct cggagataca gacaatttaa atagactact taaattctcc 22260

gcaggtgcag gaagaggtaa agaagaacaa aaaggttact tcaagaacat ggctgagaat 22320

gtatttggta ctacattaac aaatgaccaa gtcgatgcaa tgtataaaca agtaggctca 22380

gatggaacaa tagataaaga caaaatgaaa aacatccaaa gtattattga tggaggaaaa 22440

gacggatacg ataagaacaa aaaagggtat gaagactcta aggaagctac agaaaacgcc 22500

tctaaagcag taactgataa gcaggctaca gcgatgaacg acatggggga catagttaga 22560

aaactaaaca gtgttctcgg tggtatgccc gcaccattgt atggtttggc agccgcagca 22620

gttgcagcag caggagcctt agctatgtct ggcggaatga gtattctatc tggtggtatt 22680

aaaggaaaac taggtaagaa atttggaggc ggaataggta aagcaggtag cagtacagta 22740

gcttccgcag gagcaggcgc aggagcctct tctacagcta gtaaagtatt taccaataaa 22800

acatggggag caggagcaga aggagctaca aaggcttcct cggggctctc atggggtagt 22860

aaagcaatgg aaggcggaat ttctgcaact aaaggattag gtggagcagg aaaactagcc 22920

cagtatggaa aaggagcctt aaaaggtctc gggccgcttg gtcttctaat aggcggagga 22980

gcctcaatcg ccaatattgc aggtgcagaa aacaaaaccg aagccatcgg agaggaagcg 23040

ggtggctggg gaggcgccat gggtggtgca gcaggcggtg cagccatcgg agccatgttt 23100

ggtggagtag gtgctatacc gggagccctt attggagggg tactgggagg tattggagga 23160

agcaaagtag gtaagtgggc tggcggaaaa ataaagagtg gctgggattc tctatggggc 23220

ggagacaaag tagatgctgc tgagataaac ggcacaggag gagctaaaaa agctcaaaca 23280

gagaaaaaaa ccaacatgaa cggtgcttct atggctaact ctggtgtagg aactatgcta 23340

ggaaatacag taatgcccgg tgtaggagga attatcggag gagctatagg aggttcaggt 23400

atcctagaac aactttctaa gctaactggt attggaagaa acgcacaaga agaaacaagt 23460

tcaggaaaac aagatgaaaa agacacagta ggtaagaaag ctacagcaga gaaagagaga 23520

caaagtaata caagagaaga agcagcaaac ctttcttttt acagtaaact tcttgacaga 23580

gccgaagcat tattgatgca agctagaaat caaaatggat tattcggaaa agaaggctta 23640

ggaactactg ctacaggtag cggtggagga gatgctctca attcaggtct taacttctta 23700

acagatggta aaaagtggac taacaaagat ttaacaaagc atgacttagg ttcaactatt 23760

caaggtatta ctgctgatga gttagacgaa tggattaact cgaaagcacc tgagggttct 23820

gcaatgagag ggatgggttc aactttccta aaagcagcac aagaatcagg attagaccca 23880

agatatttag tatctcatgc agccacagaa actggttggg gaacatcgaa catcctaaaa 23940

gaaaaaggta actggtttgg tattggggca tatgataact cacctatggc aagtgcctac 24000

aaatacgggg acaaagaaac tggtgtagta gaaggagcta aatggattgc tgaaaactac 24060

tacaataaag gtcaaacaac actagagagt atgcgacata acggaggaca acacgagtat 24120

gctaccgacc ctaactggga taaaacgata gctagcataa tggggggctc agagaaatac 24180

gtgcaaggta aaggtgcttc tttaacaact tctgctaata taactgttaa catgaatgga 24240

gactcatcga aagataagaa gacagcagga aaagcagtag gagatgccgt tgctaaatcg 24300

tataaagaac aatcagagtt cttctctaaa caatacaaga gaacataatg ttctcttgtt 24360

attacatata agataaggag gataaaatta tatatgacta cagttaaaag aatgcctgaa 24420

tttgatttaa agtttgtaac agaaaagaat gactatctta taagatatga tgcaaggaac 24480

ccttcctcag atacattagc tgagaaagta ataagtgtaa ctactaaaaa tgcaatgtct 24540

gacgacagcg ccgttttttc tattgtagta gctggtgata tggaatggga taaaatatta 24600

gactctaatg atgtagtaat actaaaaata tacccaaact taagagtaat gatgcctgac 24660

aacgttgttg ttttagtggg tctcatatcc gaagttcgaa gagaaggcga ctactctaat 24720

aactctatta tttatcgtat aacagggcaa tcttttgcta aatccttcat gcagttccaa 24780

ttaggggtaa ttcaagaggt atctgtagta attaccgata ttgggtggtt gcctgactct 24840

aaggcagatg gtgttgagtt tacaggaaaa acagcagcag agataggtaa gtctatcaca 24900

gataggttta aaaaatatat gaaatacaat tttaacagag aatatacaat ggaaaacttc 24960

ttagactact ctttcagcag ttggaaagat tacgaaaaat tagcagaccc taccccattt 25020

attaactatg agggtagcct aaaacaactt ctagatgatg ttacagcaaa gccatttaat 25080

gagttattct ttgagtccac aagtgatgag aagtgtaaaa tgattatgag aagaacacct 25140

tttaataaag aagactggga caagttacca tcttataaaa tatctacaga agcagtaata 25200

tcagattctt tagctaaagg ggacacagaa gcctactcta tttttaacgt cactagtgga 25260

aatatggcag gagcaacatc agtagactta aattcattcc ctcaatatca ccaagcttta 25320

gtagataaat atggatacaa aaagttagag gtagacaaca ggtatttatt tgagtcctca 25380

acagatggct ctagtaccac agaaaaggca gatgtaggaa gtaaggaaaa aacaaaaaca 25440

gtaattactt acagcaagtt taactctttt atgcgtagtt atacttccga ccaagttcgt 25500

atgaatcaaa gtagtattgc aaaaagttta gtggacagct acgacaaatt gacaacttct 25560

caagccaacc aactgcttgc taaatattct gctgtaggtg cgataagtga ggcagatttt 25620

aaaaaaattg taggagacat agcggaggga gacaacacag gaacagctac tttagatttt 25680

gatagtgtaa actcttggtt ctctctaaat tatagttctt tgtctgaggt ttctacaaat 25740

agagatgcta caataaaagc ttttgtaaaa aactttgcta acacagatga agaccaagca 25800

actaagatag tagcactata tatatcaagt caaggagtca tgacaaaaga aaaatttgat 25860

gcaatcataa aggagtctac tagttctagc actaaagacc cagataacac aacaggcaac 25920

tctagttcag cactacagta cttctctaaa acgatatata actggtacag tgaaaacgct 25980

aatttttatg caggggatat aaaggttatt ggtagtcctg tatataggtt aggaagtaaa 26040

ctccttgtag aagataaaca acaaggtgat gagtgggagt tttatattga aagtgtctct 26100

catgaatata gctatacagc aggttatact acaacactcg gagtcactag gggattgaac 26160

aataaaggga aagatagatt tactcattta tggggaaaaa gttcggactt taaaggcggg 26220

ttacttggag aaaaaacaag tgctgaactt atacaagaag caggttctac ctcgtcaggt 26280

tccgatggaa gtggcgatgt aagcgcacca gatgtacaag gtagtgatgt tgccgtagcc 26340

gctttaagat acgggctagc tcataaaaag ccagagaaaa aatcagtata ttcttttggt 26400

ggaggtagag gtagtagtaa cccaatggag ggtaaggaac cttacgcaat ggactgttct 26460

tcttttgtat ggtggtgtta taaagcctgt ggtgttactt tagcaggagc acaaacacaa 26520

gcgatattag gggacgatag gtttaacaca gtaagctcaa gaggttctaa gagtaaagaa 26580

atatttaaaa aaatgcaagt aggcgacctt gtttactttt acgataacaa cactcatatt 26640

ggtatgtatg caggagaagg aaagttctta ggatgtaatg gagatggaag ttgggacacc 26700

aacggaggag tacagttgaa accaatggac tctggttatt ggtggacaca gtttcaagga 26760

catgtaatac gttttgttta aaaaggagga atgactagat gagtgatgta gaatcaggag 26820

caccacgaac acaggctagt ttaggtagtg agtgggaacg agtatacaaa gcaggagaaa 26880

aagtagacac aatcgtatta gcccagataa taaaagtaaa ctataaatat aacaccgcag 26940

aggttatgat actaaaaaat aaagaagtga tgtctaagtc gtatatttct aaaggtaagt 27000

tctcagctcc actacctcaa gattttgctg gaagaactct agaaggactt ccatatggac 27060

aaattaaacc aattcaagaa ggagctattg ttcttatagg gttcctaaat agcaagaagg 27120

acaacccaat tattttagca gtctacgggg actcagatgt aaataataag ctagctagaa 27180

ctccttatga aagtgcagac ccattagacc ctaagctagc tagaataaat aaccagtttt 27240

ttaacttgta cccttccttg acgtatgata atatagacgg ggacggaaat agaatatcca 27300

cttaccccgg caagacattt tctattatga caagtttaat aaacgagtct tccaacattt 27360

cagacagcaa taatgggact gaatatgcag acttacccga atcatactac acagactaca 27420

aactgataga gccgagaagc cctttagcac catcaatgct atttaaacac caaggaataa 27480

gaacactaga cgggaaggaa gacaatcatt tagtaactgc ctttataaat acagatggaa 27540

caacaaggct gtctttccta aataaggtta tgaactggcg ttcctattat gaggctgata 27600

ctgagggagg tcaaaagttc agacttcaaa gagacagtaa ggtaccaaac caaggaagca 27660

tattttcaga gtttgggtta acaaaaaacg gagacttctt tattgtaaat ggaaaccata 27720

agtttgaagt aagagagaac ggtattttta ttaatgactc tgctttagca gacgacttag 27780

gggaagtttt agaagaaatt caaaatagaa tactagggat aaacgaggag attgctaata 27840

tcatccgtat gttggatgag attgatgttg agaacctagg aaagttattt gaccgtatga 27900

caaaagtaga aacagacatt attagcttaa ggaatgatta taaaggaata gacagtagag 27960

tgaacgatat gtcagaagac cttaacagtc tgattaaatc ctttgataac tttaaacaga 28020

caatgtctgc ctacaccgag gctaatacta taagggttac tgctattgag accgctactg 28080

caaataatac agtatctata ggaaacatta ataatagtat tactgcgatt gagcagaaga 28140

acacagccca agatacgaaa ataagtagcc tagagagtaa agttacgtca ctagagacaa 28200

gtaatgctag cttagtaaca aaagtagggg agttagaatc tgatgttact ttcctaaaag 28260

cagaaaatat agatttaact aatgcactag cccaattagc agaaagagtt actgctttag 28320

aaaatagctg atagtagtac tatattataa aggagacgag gtggaaaaat gccacaagca 28380

gacggaaaag gtggagtatt aagacgagtt gctttccaaa taggagagaa taaatggtat 28440

cgttttgcga taaaccccgg tagttactct tttacaggag gacaaagggt tgctgtgttt 28500

aaaacgaagt ccttaaacgt agtagaagac tttggtagag atattcctac tataactttc 28560

tcgggaacga caggtcttag aaaagactct aacggtaaaa caggagcaga taggttaaaa 28620

gagcttaaaa aatatattga agattatgca gactcatcag gaaacggaaa tagacctaat 28680

acagacctta cttttcataa ttttacagac gatgaaagct atattgtaca tttatcacct 28740

gaggcattta agatagaaag aagcgcagaa aaacctctgt tatatgacta cacaataact 28800

ttagtagttt taagggatgc ttctcaacca gaccaaggag acattgtaga cccagactta 28860

ggtaataaac aacctagtgt aggggacaag acaaaggata atgacgatgt ttatggggat 28920

gtaggaggcg cagacaagac accagaagct aatattactg gaagagtccc tctagaagta 28980

ggaggtaacg ggggaggggc agtaaacccc cgaacaacag gtaaagcata ctcctacggt 29040

gtagatgaat taaaaaaaca aattggttat ggggagggtt aacaatatgg aggtatatca 29100

gtcatcagat ttaatgaggt ttattacagg gttaaaagtt aacccagata acactattgt 29160

agtaaacatc ttagaccagc aatcaccatt tatttctagg ttttacactc ctaagcttat 29220

tctaagttct ttatcagtaa agcttattcc tgaattgcaa ggagtaaaca ttatacaaac 29280

aaacaaagca ttaagtaaaa atacagttac agctaaaata tcagcaagta ggttaaaaat 29340

agaggctcct tacatgtatc taatcgcaca gtctatagtc atggaatgct ttgctctaat 29400

ctacagtata gaagaaaagc aagatatgct aaggtacatt gaagtaggag aagttaaaca 29460

agttagagat aacataaatt atttagctga ctacttagct gacaaaccag aatacagcag 29520

tattgttacg gacttacatg atatggaaat tgctgtaggg tatattgaga accaaatacc 29580

aattatacag agggagataa gattaaatgg ctcgttataa aaaacacgta gtacaatatg 29640

gcgatactat gcagtctatc tctcagctag agactggtag tgttggaaac tgggtagata 29700

tagtagaata taataagtta aagtatcctt atattgtaca aacaaatgag gaaagaaaag 29760

aaaatcttga gagtacagtt actattgggg atgttattat catacctgta gcatcagacc 29820

taacagaaat agacccttat gctctaacaa accaagataa gaacttaata atgagtttag 29880

ctctaggtag ggatttaagt atgacctcca atcaagatta ctataataaa agaggaacta 29940

gtgacgagat actagaagtt gtagctaacg gcaagggaga cttagcgact gttgacggag 30000

tagaaaacct aaagcaggca ctaatagcta gactattaac tcctaaagga gccttgatgc 30060

tacatccaga atatggctct aacttacaca agatgtttaa aaaagctaca catgaacaag 30120

catcactaat agatattgaa ataacaagaa cattgttact agacggtaga gtagaaacag 30180

tagaaagtct cggatacaaa gtagaagagg acgtatatgt aggaaaattc ctagttacta 30240

tagagtcttt acagacagcg tttgagttcg ttgttcaagg tgatactaca ggttctatta 30300

ttttgctaga taattgagga ggaaaacagg ttgagattaa aaagaatttc agaaatttta 30360

tctcgtttaa tagataagat tatgattaac acatcagagc taaatgattt ttctgtaggg 30420

tctactatct tgtctatact agaggcagtt tctatggagt tggagcagta ctacatccta 30480

tctagagaga acattgtgtg gggtattcag caaggggtat ttcaggcttt cgactttttt 30540

aagagagagc caaaaagagc ttatggagat gtcctaattg agtttcatag cgcagttcaa 30600

agtgatgtat atattccaaa aggaaccact tttagttcaa caattaacgg atacgaccag 30660

caattcgaga cagtcataga ctactacgtt cctaaagatt ccatattagc tcgtgtagaa 30720

gtgtattgta aagtagcggg ggaaataggt aatgtcccag caggaactat taatttgatt 30780

atgaatagtg taagtaacgt taagagtgtc ataaatgaga gtaacttcca aacaggaact 30840

gatgaagagt ccttagaatc agtaaaaaat aggtttcatg ctttcgtaga gagcagaggg 30900

agagctacag tccctgctat aaactatggt acccgtttag tagaggaagt atcaggcgta 30960

tatgtaaagg aatatgtagg gtatgtaatt gtctatgcac acgatagaaa cggaaacctt 31020

tcagctaaca tccaagataa cattaaaata gcattagaag actatagacc agcaggtata 31080

aaattagatg taaaacccgt aaacaaaagg ttgctacctg tcgaggttac tgtaactatt 31140

actaacaaaa acagaatagg ggatacacta caggcacaca ttcaaggagt gataacagac 31200

tacttaaata gtatgcaagt atctaacaac ttaattttat cagagcttat tcaaataatt 31260

atgagtatag acgactccct tatttatgac tgtaagattg aaaactttac agaaaatgtt 31320

ataataaata gtgaggatat tattagagca ggagaagtaa aggttaactt aaaataacat 31380

agttaacaaa tgagaggagc tgacaactac ccttgccaaa ctttttaaaa aatatacacc 31440

ctttattaaa aagaaaaaaa gatgacaacc aagtaagtag cgtaaactac gctgttttac 31500

aagcgctcga aacagagctg agtgaagtag aacaggaaac cttagagagt aaagtacagt 31560

catcccttgg aacagctaca ggagactacc tagatatttg gggagactgg tttaatgtat 31620

accgtagaga agatgaagga gacgaacgtt acagagatag gattattcgg catttgttat 31680

taaagagagg tacaatacca gctataatcg aggcaattcg tgattttcta gaagactata 31740

gcgcagatat tactatctat gagccgttta aaaatatctt ttacacaaat aaatcagact 31800

taaacggtga agactacttg atggggtact actatcgttt tgctatcatt gatgttacaa 31860

ttggtagacg gtttccttta gaagtaattg acattataaa cgcatttaag ccagcaggtg 31920

taaaattcta tctgacttat gacggtagtg gagaaattga tgaagaaaac agtatagttg 31980

aactaccacc ttatagtata gaaattgaaa catatcaaga ggtagatatt ctaaacgggt 32040

taaatacaac acttcacggg cacttcactt taggagagtc taatattgta gagggtgtta 32100

tccttgagtt gtttcgaaca aatagaagtc cactaaactc taatgatgta ctatctggct 32160

cttttaatgc aggtagacga gtatacaatc tagcaacaat tggagacaac tcctataaac 32220

ctacgatgag ttctcttatt ggggaaataa aaaattcgtt cgaggaatta cccgaagact 32280

tctacgtaga aactaaccgc ttagatgata ggtttgctag catagacatg gcaaataata 32340

cacaagatac tgcattctat ttcaacatgg atttaggaac ttatatcaat actaactacg 32400

caggatattt agctgagtta gcaggaagcc agattataaa tagagcacac tatgtatcct 32460

tagctaaaga gccaaagtta accgtggcgt ttaaagctaa aatatcccca gcaaacccag 32520

taaattacaa tatagaaggt tatgacttag tttctaaaaa atgggttgtt cttgagacag 32580

gagctatagc ggttgaaaag aaaatatcac agataccttt aggttcatta gaagactatc 32640

ttaatagtaa ctatatatgt tttttccgaa ttgtagtgaa acctaaacgg gaaacctact 32700

cctttgaatt agactatttc gaattaggat ttttaaacaa ctttaaagaa ggggagtata 32760

caattaagcc ttttacaggt attgtagaaa cttatgcaga gttgttcggt agagttatag 32820

aagttcctat gtatcggata ataaattcag gaacagaaaa tattatttct aaacaaggtc 32880

atatacctgt tagatatatt agagatactt tagagggtgg tagtactaca aacactgcta 32940

acctatggat agaaattcag gctaaaaata aagacaatat caatgttgcg cttaataagc 33000

ctgtatatag accacaagct gaacctacaa atggtaatat tcttctatat ccacagttcc 33060

ttaatattgg agctacttac atgaatggta agtggagagg aaaactaact agtgaggtaa 33120

acatggcata tactgtagac ggtgtaagaa cttacggagg aaacatgaac gcagatggaa 33180

cctatacagt ctatatcggg aacaaagtaa cctctactag ccaaaaagta gttgtggagg 33240

tatatgcaga cactgcaaga acgaagttaa tagacaaagc taatgttcct attacaaatg 33300

caggggacgg aagccctata gaggctatca cagacggagt aattagtggc tctaatatat 33360

tctatattgc accaacaagc gccaaatact cagtaactgt agacttagaa gctacctttg 33420

tagacttatc agaggtaagg atatggcact acccaacacg aacgtttaag tctgttttga 33480

cagaagtatc agcagatggt gtaaactgga ctactatata taactccgct gttgatggga 33540

cttatctaga aactagtgaa ggaaaagcgt ttcctgtacc agagatggac ttatatggat 33600

tgaaagctac tcctctaaca agtgtactag caggttctga aagacaaatt attacattcc 33660

tagactacaa aatagaggac tttgagaacc cagctaatac tattgaaaac ctaaacaatg 33720

attactttaa cgcagtgtgg caagagattg accgacttga agagtttgtt ctaggcaagt 33780

tcactatttt agctgatgtt ggctctgacg gagtactagt aaatgactgg ggtcttattg 33840

aggctaaaat acctacaggt gctacagcat accaagaagc ggacagtaga agtgaattgt 33900

cttccgtgta cactgtggag ctcacagcgg gctctcacga ggttattatg gatgttgcta 33960

ataaagttgt tactacagag gcagtaaccc taaaaacgga ttacagggtg tctaacgagc 34020

ttaccttgga agggctacct acaaaaatac ctgtgagata cattcgagac tccttgtggg 34080

gaggaaacag tataaataca gcaaaccact gggtagagct acaggctaga gacttgaaag 34140

gggacaatgt tgcccttaat aaaaaagtgg tagagaacaa cggagttagc ttactatctc 34200

ctgaaaatct aatgttagac tcgaagacag agattactag tacaacaaac aatggttata 34260

cttttgcttc tgatatagct tatatatttg ataattatgg agtagataaa gagtatacta 34320

ttagttttga catcaagtcc actacggtag gcacagttac tgtttatagt aggtacaaag 34380

atgaagctaa atattctata ggaaacaaaa ctgttcctgt aacgactgct tacacaagac 34440

agcaagttgt agtaacacct actagaaaca cagcagaaca agctaaatat gcagtcagct 34500

ctattctagg ttttcttaat agtacaggag caatatctac agttaaggat ataaaaatat 34560

cagtaggaaa catctcgaat atgatttgga cacctgcacc taaagactac agtattctta 34620

ctgatggagt aacaacaagt gctaacggat tcttgagtgg agttaactcc ttaactattg 34680

acttaggtag ccctagatat gatattgaca acatcactat gtggcactat tggcaagggg 34740

ctgaacctag gtactacaaa tatgtaaaac tagaagtatc agcagatggt gtgaactgga 34800

ctacactgtt tgatagtgat gtaagtggaa cttattacga gactagtgga ggtaagcagg 34860

ttagcgtacc tgctataaaa gaatacctta agagtactaa tgctatattt gcacctagcg 34920

tagcaggaac aggtactttt gagagagtaa ccgaagcaaa cggagtaagt tctataaaag 34980

tgacacctga tactgataag acagctagaa tgtacaagtt tttcggaact tctacagacc 35040

ctaacgctaa ttcgagagac cttctagaat cagggctaac tacaacgaag aaatacacag 35100

tagcagtatc cctaaaacct caaaataata gttcggttat cttagcggta aaagatggaa 35160

gtaacactaa catagagtcg ggctccttaa atattgcaaa agatgtgtgg acaagagttt 35220

cttacaatgt aacgttacct gcaacagcta ttgataggct agctttatgt ttgtacaatg 35280

ctaacacagg agaatatatt aattataaag attggagcat taaagaaacg atgtttccat 35340

aaagtaaaaa ttatggtata atagagatag ggcacaaagt agtgccttat ctttgctata 35400

ttagtaatga gagtacgaat acaataccga ttttaaaata aggaaatgag gtaaataaat 35460

ggcaatagca acatataatg cgcatgtata tgcagcacta aacctaaaaa gtaaggtcga 35520

tacaacattt atggcaattg gtaaaacaac agcgtggact gatgagacta accctccaga 35580

accagaccca aatgctacag ggttaacaga agttatcggt tacaagaagc taaaaactat 35640

gtctctatgt agaccacaaa gaacaggtga aacaccaaca ctacctacag tttcttacgg 35700

aaataaaaca tgggttcttg tacctgatgc acaagcttat acagaaggag ctaaatggct 35760

ttattgtgag gcagagttcg taggggatga actaccagta ggaacataca gacaagtagg 35820

tgtatttaca gacttagcgc caaaatctgg tgtaacaaaa ccgaatttat taccaagtga 35880

agtagctaat gtaggtgtac tacagttttt tgagaacaag cagttccaaa acagaacacc 35940

acaagttaca gctagagaaa gattcgtagc ggaattataa gaaagggtga ataagtttgg 36000

cagatttcaa ttttagttca gcaccatatc ataataggtt caatgccttg aatgattgga 36060

cattagcact tttccaacca gacagacccc ttcaacagtc cgagttaaac gaaatgcaat 36120

ctattttgca ttattaccta aaatctgttg gggatgctgt tttttctgat gggaacatgc 36180

aaacaggtat ggagtttgtc ttggataact caggaaactt aactgttggt gatggtcaag 36240

tttatgttga gggcaaagta agaaacttca aaaaacaaac tataaaatta aataagatag 36300

gtagagagtt tgtaggagta aaagtagaac agtctattat taccgcagac caagacagta 36360

gtcttctaga ccaaacagca ggagttccta gtgctttttc taaaggagct gacagactaa 36420

aagaaactgt tgttttaacg ttagacgacc ctcagtcagc aactatctac cagtttgata 36480

acggaaaact ttttatctcc tctgataacc cagagatgac aaaaataaac gaaattctag 36540

ctgaaagaac atacgatgaa tcaggctctt atcgtgttaa cggttttaac atgtggacag 36600

aacctcatcc gacagacact agtaaaattc agttagttat tgacacaggt agagcttatg 36660

tgttaggata caaggtagat aaaccaacat ctacacgtat acctttagat aaagctagag 36720

atactcgtag tgtagccaat gaagggcaat actacgatac agcagttaac cgtattaagc 36780

taggaaacag tcctgtagaa acagtaacac gagttacagg tcaagttcaa gtacctaagg 36840

agcaagtgag cagaggttct caatcaggag gcaccgactt cttgagtaga acttccgtat 36900

tccagataga ccgtgtgtgg acagaaaacc caacatcagg agaagttatt aaggaataca 36960

ctcaagggac agacttccag ttaataaatg gacaagcggt ttcatgggca cctaacggag 37020

cagaacctac ggcaggagca acttactacg tgtcttaccg ttataacaag tctatggtac 37080

tgaatacaga ctacaagata actattgaag gtacagggga tacaaaaact tggtatattg 37140

attttagtga tatgacagga agtaaaccta tccaacaaag tttagtaaac gttgattaca 37200

actactactt ggctcgtaaa gacctaatag tacttgatag atacggagaa gttacagtac 37260

acaaaggaca accagataac ttaagattag cggaagcacc tgcacaggta gacccctcaa 37320

cacttcaaat tggggtagtt actatttacc ctgaaagtga gaccgctagt gctatgtcat 37380

tctctgttac tcgtctttct atggctgatt tacaaaaaat gagaacacgt attgataata 37440

ttgagtataa cgaagcagta aatgctttag ataatcctgc aatggcagga gaaaacccag 37500

tgacattacg ttctgtattc tctgacggtt ttatttcact agacaaatac gatagcacgc 37560

atccagatgc tattatagct tttagttttg aggatgcaga aattacccta ccttaccaag 37620

aagttaacaa ggtagttcca aaaatgttag agaacgcatc agaagcttat gtttggggac 37680

gtttagtatc agcaccattt acagaagaga aaacaatttc tcaacctttt gcatcagaag 37740

ctatgaacgt aaacccatat aatgtgttta ataaacaagg ggttattact ttaacacctt 37800

ctgcggataa ctggattgag gaagaacgag taactgtaac tcaggacgaa gttaagtctt 37860

atacggtaaa ccgttggtgg ctacatggag gtaactacta ttctgatgaa gctagatact 37920

tgcataacaa cctagcacta gatgcagggc agtcatggag tgggactcct tctcagaaca 37980

aacaagtaac aggaacaaca ttaacagcag gtggacaaca aacacttgag tctatgatag 38040

agtttatgcg acaaattgat atagtggtag aaggtaaaaa ctacttgcct aacgctaaca 38100

acttatacgc actattcgat ggtgttcggg tacctattac accagcttct ggttaccgaa 38160

aaggtacaga cacaggaaca ttaatggcta atgcagatgg gacagtaaaa ggaactatca 38220

ctattccagc aggtatccgt tgtggtgtga gagaagtaac tttacggaat gcagataact 38280

taggtatttc tacattcact gcacaaggta gaaagaaaac cgtgcaagac atcattatca 38340

gaactcacgt aacagctaac ttgtatgacc cgttagctca gtcattccaa ttcacagatg 38400

accgagttat aactagctta ggattacatt ttgctagtaa agatgcagga gaaaacaacg 38460

ttatcgttca gattagaggt atgagtgacg gaggacaacc aaataaaact atttatgcag 38520

aaagggtatt aaccccagct caagtaaatg tgtctgctga cagtagtaag gaaactaaag 38580

tcacttttga tgacccaatt atgtgtaaag caggtcaaga gtactgtatc gttattgtaa 38640

cagactctga tagttacact atgtggttag gtactagagg taaacctaga atagactcac 38700

ctagcgactt aattacgtca aacccttatg tacaaggtgt actatttagt tcttctaatg 38760

cttctacatg gacagcacac caagattctg atttgaagtt taatgtatat actgctaagt 38820

ttaacgaaca agctgtacta gaatttgaca caatgaaaaa tgttaaagtt gatagagtag 38880

tactaatgag tacgtaccta actcctaata acacaggatg tatctgggat atgaagattg 38940

ttctagacaa tgaacctaca ggagttacag tagcagacaa agaatggcaa ccaattggta 39000

actaccaaga tttggatgta gagcaattag ctagagaagt caagttaaga gctacttttg 39060

ttgctaacaa ctacatttca ccattgttat cattggacga catcatgttt gcaggtttct 39120

taactgcttt aaaaggtagt tatgttagta gaaatattaa tatgacagac tctccttata 39180

atacagttaa gattagttac gaagctttcc taccagctaa cactacagta aaagcacagt 39240

ttagtactga tgcaggtatt acatggaaag acttgacgtc tattcctgta gtaacacaac 39300

aaacagccga tttctacaag tatgaatacg aagagaaagt catccaatca ggcacatata 39360

aaggatttaa ggttcggctg aacttagctt cacaaaacag tttcttaaga cctcgtgctc 39420

ggagactaat gtgtttaatg aaagatgaat aggggaactt ttgttccctt atttttaact 39480

aaaaaggggc ggtaaacatg ccagagtcat atagagaccc ccattcaggg gctttaaagt 39540

ttattcctac gaaacaggag aaaataatta aagatgtgac tgaaaaacta agagaagacg 39600

aagaaaaaat tcgaaagaaa atagaagagt tagatattaa actagcacag gcagacagca 39660

ggttgcaaga aataactaac ttgtatgaag aattaaaaga actaaaggag ggtaactaaa 39720

tttgtctaga tacgaccata gtactgtaga gtacacagac aaaattaagg acttaacaga 39780

ttcggtaaac cgagtaggaa actacttatc aggagacaat agcccttatg acgatgttca 39840

gaaattaaaa gcaattacac aaaatattaa gctaactaaa gatacaggtt tagctaaaag 39900

cattacagca ggaacaactg cgttgaggtc agtagtcgaa gtaggggttt actatattaa 39960

tagcacagaa gctctagccc taactgataa acccccagag ctaacaggag cctttatact 40020

agtaaactac ccgacaacag cctctacttc tgttaagcaa gaagtacaca tgtttgctac 40080

aggaacaaca ggtagctacg tagggtaccg atggatttct gcttctagtg tcagctcatg 40140

gtggacttat gaaaacacac taggttcaca agctaaagca gataaagcat tagcggacgg 40200

gaaaacatat actgatagct cagtaaactc cgctttgcaa gcaattcaaa actcagcaca 40260

aatgtacaag ttaacagcgg atgatggtaa gcctattgat gcttctgcta tggctactcc 40320

accaactagt gtagcttctc taacgaagac aggaatatat tactttactg ccgcttttgg 40380

aaatacaatg ccagataccc cttgcacagg gcaaccattc tggttggttg ttttacagca 40440

tgtaacagat aacagtatat cacaatctgt gacagccaac actgtagcag tagagagggt 40500

agtcgcagat aggattatta ctactctagg ggtaccgagt aagtgggaat acagagctaa 40560

agcctctaat ttcttcttta gtgcttccaa ttcgtcacgt ataacactag tatcgaccca 40620

acagaatatt ataacaccta ataaatttat taataacccc ggtagtttac cactatctgc 40680

gataaacccc gcaataacta ttccagaaga tgggatgtac caagtgatgg cggccctaaa 40740

cattaacggt attttaaaaa aagtgtacct agtaagtgat ctaactctac taataaatga 40800

cgtagtacac ccagctacat ttggaatggt aaaaacagca gataatgcta acgggcagca 40860

ctctttagca gggaatggca tatatcaact aaaaaaaggg gacaagttaa aactaagaag 40920

ttactgtaac aatacaggga ataaccctta cttggatgta gacaaacttt atatatctgt 40980

tggtaagatt gcagacattt cattatttaa ataaacttag tatgctgaga aaggaagtca 41040

caataatggc agaagaaaaa ttttcattag acggaaggca gtatgtaaca gcagtcctat 41100

atatgtaccc taacctagac cctcagtacg actttggagt tgtatcatcc gaagggaagg 41160

tagaggcttt tgaatggaac gtaaaaaata taacaaagcc ttctattgca cagttagacc 41220

aagcatataa agactatgta aaatctatcg gaagtccaag taacccacca gccctagaac 41280

cagaggtctc aaaagaagcc tcaataggta agattctaac tgaaaaattt agtgcgttag 41340

aggaagttaa tgctaatatg ttattggaaa atttagagct tcaagctagc ctagataaaa 41400

aacaacaaca aaattcagaa ctagtatttg agctagtaga caggggagtc ttatgagtgt 41460

aaattggtat gagttagttt tagattacta cacaaaaggt tattacacac caagtcaggt 41520

aaaaagattt gctgaggtag gaaaactaac tttcgagcaa gccgaagaaa ttatagctac 41580

aaaatcatag agtatgttta aactatatag gtaattaaga ctaggactaa tatcctagtc 41640

tttttatgtt ataataaaga tatagtaaat atatttggag gagtacatta atgagaattg 41700

ttgtagatat aatgaataca cgagttttat tcgataaaga agaagaggca attaaaaaaa 41760

gatttcttag ggatatgcac caaaaattag gggtaaaagt agatgggttc ttctttagcc 41820

cagcatataa atcaggtcat tgggacggta tccaagactt ctatgacttt aaaacaaata 41880

cgttccctac agggctgttg ccacaagtgc aagagatact aggagctatg caaaatgatg 41940

ccgtatttca atttgagatt attgatgaca gaccagaccc gtttttaaca gtagaggact 42000

tagatagtga gataactctt ataggagatg acggaggaga tatacagctt agagattatc 42060

agtatgactc tgtaaagtct atcattgaaa actatagtgg gattatcaat attgcaacta 42120

atggaggaaa aacggagatt gcttctggta tcattcagca gattttaccg catttagagt 42180

ctggggagag aatagccttt tttactcact cctcatctat atttaaccaa tcaattgacc 42240

gtatagaaaa aagactaggt attaaagtag gtaggctagg aaatggtaaa aaggacatca 42300

gacaagttac ttttgtaatg atacctacgt taacagcctc cctaaaagac cccgaagttg 42360

gtttgaaatt aacgcataag gaacgagtta ttaagaagtt agctaaagag gtagttcctc 42420

gtttcactat gcaaaaaaat catagaaagc ttcttaatat ttatattgag aacaacagac 42480

cgaaaaataa agtagaagaa gatataaaag ctcgactaga agacttgtta tattcctgtg 42540

gaacagactc cgaagttcta atgggactaa aaggttttaa cgcagaatat gaaaaaatta 42600

ttgagaaaaa aaataaaaaa ctatatgaca agaacaaaca agctagagaa tttctatcat 42660

ctgtatctgt tatgattgcg gatgaggcgc accataccag ttcagaaact tggtacaact 42720

ctttgatgtc ttgtgagaat gctatgtaca gggtggcttt gacaggttct atagacaaag 42780

aaaataaaat gttatggcag agaatgcaag gagtattcgg tcaaattacg gctagaacaa 42840

gtaatgagac cttaattaca cttgggcact ctgctaaacc aacgattaaa atattcccta 42900

tcaaggctcc tacagatatt gaaaatgtag ataactatgc tactgcttat gaaaaaggaa 42960

tagtagaaaa tgattatcgt aatttactta ttgcaaaatt agcaaagatg tggtatgata 43020

aagacaaagg tgttcttatt atagtaaata gaatagagca cggtaacaga ttatctgaac 43080

ttcttgagtc tctcgaagtg tcgcactact tcattcatgg agacttagac atagatttaa 43140

gagaacaaca actccaaaat atgagagatg ggaagcttaa agtgatgatt tctacatcta 43200

ttgtagatga gggagtagac atctcaggta ttgatgcgct tattttaggt gcaggaggga 43260

agtctttaag acagacactt caacgtgttg gcagggcttt acgtaagaag aagcaaggag 43320

aaaacacttg ccaagtattt gactttaaag atatgactca tagattttta ctaagtcact 43380

cagaccaacg aagacagatt tacgtagatg aaaactttga aattcaagac atttaggggg 43440

aagaacaaga atggatttag tatatgtagg atgtcagatg gaaataggaa tgaggaaagg 43500

tttacgagac tttctagtgg atgttgctaa aacagctcag caatggggag aaagcgtggc 43560

tatggttact caaaaacata gcctagctga gcaaacagga gtaaacatta gaacagtctc 43620

acgttatata gctgaacttg aaaacaaagg tattattaaa acagaagcaa aaagaggaag 43680

aaacggtgga ctagttatca tgtttaataa aaatatggtt acttttaaat taacagacaa 43740

ccctataacc tctgattcac ctgatgcggt agaacttaga gaccgtatgt tccctaaaaa 43800

gaatgaagac ccgaataaag agaaaagacc tcgtagaaca aaagaacaaa tggcagagga 43860

aaaagtacta aaaaatcgta agagaagtct taataatgag ttaaacgata tgttagacga 43920

tacattattc cctacagaag agatatttag taaaacagag gagcctgaga ggtactttaa 43980

agcttattta ctgtctcgaa tggttaatgc ttatgcgtat atttatccta cagagagaga 44040

gctagacatg ctaagcagag gggtgcaggt agaagcagag aaggcacgaa aaaatagagt 44100

acgagcttac tcttttaact cactacctgc tagatttttt gggacatctc agtttacttg 44160

ctacttaaaa ctacaagact tattagaaaa agagaacatt aacccattat cttatttaac 44220

agttcaattt gactacttag actacagatt agataacagt cttggaggaa ctccactagc 44280

atatagaggt atccctgagg caaactgctt aataggtgag actgccttta gtcgttacac 44340

atctgctaag gacttctaca ctgttatgcg aggtaaaaac atgttacgca ccagtacagc 44400

agatgttgcc tttaaaggtg gaaaataccc tattgtggag gcgttattac acgcttacaa 44460

tgtaggtaag caagatatta ctgcactaaa ccaacaaata gaggatatga agacacaagc 44520

tagtgtgttc cataaatccg aagccctagc agacttttat gaaacaacaa aactattagt 44580

tgaagaaagt gctttagaaa caaatgaaaa agacgagcta tacaaatttg ttactgaaca 44640

agttgtttta cactctcgta agaatgcaat gagtcttagc caacagttgc taagcttccc 44700

acttcaaatt caacgtgtaa gacagacagc cgaattaaaa ggattagaca aaaaggacta 44760

ctatactttt gtaggcaact tagagaaaca agtgaaagta agtagaaaca cgtttaactc 44820

gtttactgaa aaaggtaagc ttttacacga gtctctagag gcaaacagta ctttttatag 44880

taccattcgt atgctaggag actaccgagg actaaccgta aacatcccgt tactcggaga 44940

agctatcaag aaattaggag aggaacatat tcctctagat tcttatggaa tgttagatat 45000

aacacaggtt tacgacaagt atttaacaga cctgcaaatg caagaagatg aagagaacta 45060

tgaaacacct acaagtttta ttgttaagta aggaggagga gatatacgtt gagttccatt 45120

attcaaaagc aagttatata ccgagctatt agagaccctt tatttggtaa ggatgtgtta 45180

gcacagttac ctagtaatgt atttgacgag aatgagcact acaaaaatct agtaattatt 45240

attaaaaagc attaccagac aagtaacaag gcgcttgatg aaagtacgtt gctaactatt 45300

acggaggaac atttagttag acagaaaaag agcttagaag tacaggaaag ctttaatgag 45360

acaattcatg atttgtataa aattgatgaa ctaggtatag acgatgaggc tgtcggagaa 45420

agcattaaac gattcgtcaa aaaaaatctg gcagctcact tgataaagga gactttgtct 45480

aagggtgaag ggctagacaa cgaagaaaca ttagaaaaac tatacacagg tatcaaagaa 45540

atctctattt tagatattaa ccgtagtgaa aacactttat ttagtttctt tgatgatgta 45600

gaagccaaga aaaagatgct acaaagtatc caccaagata agttctctac aggattcaca 45660

gacttagatg ttattgctga gggtggtatt ggacgtggag agctcggtct agttattgcc 45720

cctacaggtg gtggtaaaac tatgtgggca gtaaaccaag caaaaaacta tgttgctcaa 45780

ggtttaaacg ttttgtatgt agtgttagag gaacgattag aacgtatgat tttacgtttt 45840

gaacaaaata tgatgggtat ccctaaatct cgacttatgc cagataatga acttgacgaa 45900

gaagctttcg acaaattaca aacgatttat gatattaaac gtaaacaagg attaggtaat 45960

ctttacattg aaaagagaat gcctcaggaa gttagtccta caatgttaga gcaaattatc 46020

gttgatgcta gtgttcgtaa agggaaacat ttagatgtgg ttattattga ctatcctgac 46080

ttaatgaaaa accctcatga aagagataat agtgagtccc gtgcaggagg aaaactatac 46140

gaagaaatac gtaagttagc tcaaacctat aagtttgctg gttgggtact ctctcaggtt 46200

aaccgcggag gatattcaga ggatgtactt actgctaaaa gtatcgaggg ttctaaacag 46260

aaactaaatg ctgtggaaat tgctatgacc ttaaaccaaa aacctattga gtttgaaaaa 46320

ggttttatta gagcatatgc cgacaagatt cgtaataata acggtaaatc ttataaccgt 46380

atgcttagct ttaaggttgt tccagaaaca atgactatca gaaatataac acctgatgaa 46440

agagtagaac acgatatgat tttaaataac ctcgaagaaa aagaaggtcg tgtatttaaa 46500

aatacgaaaa aggctacacc tgacccccaa gcagttgtag acgaagtaaa agcactaaat 46560

agtagattat aaggaggaga tagtttttgg ataaaattat attttttact gattttcatg 46620

cccatatttt taccgaattt tctaaaccag atgtggagta tgaaacagac agatttagag 46680

aacaaatgga agtcttacaa caggtatttg acttagctag ggagaataag gcagatattg 46740

tttttggagg agacttattc cataaacgag gagctgtaga tactagagta ctaaaccatg 46800

tttttcgtgt atttgaaaga aacacggata ttgtagttca cctactgaga ggaaatcatg 46860

atagctttac aaactcgatg aacacagaga gtagtttgga gacacttaga ggtttaccta 46920

atgtggaagt ttacagcaac ccttctattg tagagactga aaaatataag ttatatatgc 46980

taccttatgg ggatgaagtg caagagatga aagactggat taacagttta gacattgaag 47040

caggtaaaag taatatctta gttgctcata taggggtaga gggagcgtta acaggtaaag 47100

gttcccacag actagaaggt gcatttacaa caggagattt aagaacaagc gactttgatg 47160

ttattgctct tgggcattat cacaagagac agctactact agatgttcct aatatatttt 47220

atggggggaa caccattcag aacacttttt ctgatgaagg gcaagagaag ggtgtatggc 47280

taattgactg ttcagatgtt aaaaatacaa caagtacttt tattcctatt agtagccgta 47340

agtttattac tattgacgga gataacattc ctgatgatat agaagaactt attgaacaag 47400

gcaactttat taggtttatt ggaagtaaag aacaggctaa aaaaattgac ttatatggag 47460

aagaaagtac aggtattaga gtagagctac gaaaagagta tacaactgat gttcgactag 47520

ggattgatga aaagtcctct cccgaagcta tttctaaagc ctatgcagag aaatttagtc 47580

cctctgcaac aagtgaaata gtagagtgta taaaagaggc acttgtgaga gaaaaataag 47640

cctaaagctg aggatattcc tcagcttttc ttattgactt ttttagtaaa gtatagtata 47700

ctaaagttac aaataaaaag aaggaggaaa agaatgttca cttttaaaaa agtaaagata 47760

aaaaacttct tatctatcaa agacatggag cttaatttag acaaacaagg tctagttctg 47820

atagaaggaa agaataagac aaatgaagcc tttaagtcta atggtgcagg taagacaagt 47880

atgattgatg ctattactta cgctatcttt ggtaagacag taggtgggct taaatcggat 47940

tcagttgtta ataacaaaga gaagaagaac actgctgtaa ttttagactt cgaagttgac 48000

aagaataaat accgaattga acgctataga aaagacaaaa aagagggtaa tatagtaaag 48060

ctctaccaag gtaaaaacaa tattacaaag agtacagtgg acaacacaga taaagagatt 48120

ttatctctat tcggtattga ttttaatacg tatgtaaatg ctattatgta cggtcaagga 48180

gacatgccta ttttctccca agcaacggat aaaggtaaaa aggagatact agagagaatt 48240

actagtattg acatctatca gaaggcacaa gaaatcgcta aagagaaagt taaagagaag 48300

caagctgagc aacaagagat agaaaatgaa cttgtaaaat atgactatga gcttgtagct 48360

attgacaact tagaagagca agaaaaagaa aactataaag cagtccagca aacaatagaa 48420

ggtatccaag caaccctatc agtagctagt acagagctag aaaactttat gacagaggct 48480

taccctaaga aggcagaatt agagaatagt attgcacagt ttatagtccc tcaagtagat 48540

aatacaaaag ttaacgagct aagagctgac ttaacggagg cagaacgtaa attattacaa 48600

attaacagta ttaaagaagg ttttgacaga gacttacgaa agctagctaa agactatgaa 48660

aacctttcta gtagcaagat ttgtcctaca tgtggaacag acctcggggt ggatagtgaa 48720

gcacacattg cagaaacaaa agcagatatt gaaaaacaaa tgagtgttct tggtgcaaaa 48780

acccataaaa ttttagtagc gttaccttct gtaaatacaa ctattgagag caaaaaggag 48840

ctcgtacaac aagagatagc taaaaatgag gagatacaaa gaaattacaa tcagctagtg 48900

tcaacagtca ggggatatga acaagaggta caacaactag aaaacacaaa aaatagttta 48960

gaagagaaag tggcttcaca acaaacatat ttatcacagg tacaaagtat gcctaagcca 49020

aaaagtagag acaaggatag agaagaagta cacaaaaaaa tagaagcaac taagttgcaa 49080

gtgctagaca aacaaaaaga gactgagaag tatgaggaag cagtaaaagc cttctcaaat 49140

gctggtattc gttctatggt tcttgactta gtaactcctt ttttaaatga gaaagctaat 49200

aaatatttaa cagtgttgtc aggttcagat attgaaatta attttactac acaagttaaa 49260

aactctaaag gggaactaaa agataagttt gatgtagaaa taatcaatgg tagtggaggc 49320

tctaactata aagctaactc tgctggtgag aagaaaagaa tcgaccttgc aatttctttt 49380

gcaatccaag atttagttat gagtaaggca gaactatcta caaatattgc attgtatgac 49440

gaatgttttg acggtctgga cagcataggt tgtgagaacg ttattacttt gttaaaagaa 49500

agattagaaa cagtaagcag tatatttgtt ataacgcaca atgagaactt aaaaccttta 49560

tttgagaagg taatcactgt gcagaaagag gatggggtta ctaccctaat aaactaagga 49620

tttggagagc tataaaatga aagtaaaaat tatgacagaa gaaaaagggg tactcacctc 49680

agaagtaagc aagaggtcaa ctaagaaaga ggctatctac tccgcaaatg tttttcctat 49740

tagtagcttg tacaagtggt acccctttaa tactaactac gcctatgtag tagacaagca 49800

tggggtatac agactcactg aactagatga ctgtattcat acatcgtata acttggtaag 49860

tgaagaaaaa gctaagttag acccgacaaa gtggcacaat aaaaccgtaa cacaaagcaa 49920

cttattactt tgtagactag caaataagct agggataaaa agcattaaag atgattttaa 49980

ccaacaagat gtagaaaacg gatttgaggt tatcagtaca gagttagcct atatcaaaga 50040

acaagcgaca gcacgttgct acggggataa agatgtcttt gaaagaagtg aactactcaa 50100

gaaaagtaaa gaagaaagag aacaagcata ctttgagtac ttaactgtag aggaggtaaa 50160

cttccaatat aagttaggag agctatacgc tatgcttgta cttatgcgga aggtggtaaa 50220

ctactaatgt ttttagacta cattacaagt gagctaggaa cacataaaga tatagggcat 50280

gaattacggt tctgttgccc tttttgtgga gaagacgact acaaatttta tgtgaaggta 50340

tcaggggaca aaacagaagg tctttggcat tgtaaaaaat gtaatgaaaa aggtaaccct 50400

gttacttttg ttatgaagta tcttaacatt aattttaaag atgccttaga tatactagag 50460

gtgtacgact atgagataga cgaaaacagc ctatctagca gagacgtagg ggtaacagag 50520

gaagagtttc tgcttatact attaaagaaa tctagtgagc ctgaggaagt agaaattagt 50580

gaggaagtga ctctcacgcc acctaggcta cctaccaatt ttaaaagact agcagacaac 50640

atgaataacc ccgaggcatt accttttcta aactacgcac actcccgagg tataacttta 50700

gaccaactat tcctacacaa cataggatat gttatagagg ggcaagtaga aaaagtagat 50760

ggtaatatga tgacacttag aaatcacctt gtttttctaa cacatgggaa tgatggacaa 50820

taccaatatt ggaacacccg agccataaca cctatgggga taaaagcttt taatgctcct 50880

agcaaacagg acgagtactc taaaagaaca actgttttta acctaaatat tgctaagaat 50940

acgccttatg ttattgttca cgaaggggta attgatgcct taacaatggg agagtcggga 51000

gtagctactt ttggtaaaca gataacaaga gaacagatag agttactatt aaataatgtt 51060

agagaagacc agcctattta tttattccta gatacagagg cttatgaagt tactactaag 51120

atagctagca gtctttatga aaagcataaa gaaacctaca tcgttatcca tggagatgag 51180

gatgccaatg atatgggata tgaaaaatct tgggactgtt taagaaataa ttcagtaaga 51240

gctaatgagg aaggtataag tattttttta gtatcttgca ttcagtaata aagtatgata 51300

taatagagca gacaagtaag aaggaggaat tttattgagt aataaagtaa atgtaaaagt 51360

taagttagag ggaggagcta ctatgccctc acaagggtat gataatgatg tagccttcga 51420

tttatacaca tcagaggatg ctattatttg ccctgatgca gtgggagcta cactaattcc 51480

tacaggatta agaacagagt ttgaccccac taagtttggg ctattcatca gtcctcgttc 51540

aagtattatg aacttacctt taaccctagc taacaacaca ggaattgtag aaggaacata 51600

tcgtggaggt attggtattc ctttaaaaaa tacgttgtct atgacaggtt tttctgattt 51660

tgcactaaca tggaacccag acaagaaggt agtagaacgt atccctgtag agaagttacc 51720

tacaggtaaa cttagaagtg cggaaaaaat gttttacaca gaccaagcat tattgtatga 51780

taacacacta ccagaagaac tagcagacca attgtttgtt actttggtgc cgactgggac 51840

attcttttta ccaaaaggct tccgtattat gcaagcttac cttctaccta aatatcatct 51900

agacctagaa aaaacagaca aactatcaga cagccctaga ggggataaag ggtttggcag 51960

ttcaggacat ggaggaaagt gattccttta gaccctatta tccttattgc cgactctata 52020

ggcataggtt attcaaaagg aaatataaca ctgtctttaa aactacggga taagtatacg 52080

ggtaccgagg tagaaaaaat agctttatta gccgttggta gcttctcaga aaaagtaacg 52140

cttgttagta gtgagctagt agataatgtg tataaaataa aagttaagat ggaggaatta 52200

tgaaagattt tactcagtat tttaaagaag tacaagatgc acaaaaaaga gaggactctg 52260

aaactgtaga aagacttgca gtagaatatg gagataaaac tattacgtat aaagaactta 52320

gacatacgtt aggttccgtc ttgttgccac taattattgg aacaatggaa atcaataagc 52380

agttggcaga cacctcttta gacatcgtta tcaatatgtt agaaaaagag aatgtattat 52440

cagaggatac cttaattgat ttaaggcaac taattactag catgaaacaa tcagtagaag 52500

gaggagaagg agaaaatgag taaagaacaa aagcagttaa gtaaagagga tttaacttta 52560

tgggttaact ttgtagtaac tggtaaggtt gaaaaaggga cggagaaaca gctaaatcgt 52620

atttctaaac gagaggtatc tgtaggagat gtaaccaccc taatccgtgc tcttacaagt 52680

catcatgatg cgtacattag ttctcttatt gaagctaatt cagttcttag agtggtaatg 52740

cgagatgagt taaatatctc agaagctcaa atggacaagg cgttaaatac ctataagcaa 52800

caagtaggta cagcactcca aaagacacat gatgctatca tggaaaaagc acaagaaaat 52860

gtggagttca cagaagaaga actagaggta cagaaaaaac tagaagaata tgatactaaa 52920

aataaaaaag aggaataaga atggctagta caggaagagg aagtcaagta aaaggttcta 52980

aatttgaaag agagaccgct aaagctttat ctgtttggtg gggagctacc tttaatagaa 53040

caccgggttc tggcgggcta aaatgggcgg aagaaaacaa tgtaggtggc gatatagtag 53100

cccctccctc cgcaggtttc cctttcattg ttgaatgtaa aaaacatgaa gggtggacac 53160

tagacaatgt attccttaat acaggagagc ctaagtcttg gtggaaacaa gtagtatcag 53220

atgccacaag aacgaataag atacccttgt taatgttctc tagaaacagg gctaaaagct 53280

ttgttatgct accttatgat gacttgctat ataacgtatt acgtgataaa agaagtacag 53340

tagctagaac tacagtgtca tttaagaata tcagggaaga aagtgtgttc tatgatgtaa 53400

tggtgttcac tatagaagag ttaactagct tctctcctga ttttttaaga gaacactata 53460

gtaagtatag ttgggatttc ttttacaaag aagtggacga gaactctgta gaagatttag 53520

aaacaggtat agataaaatg ttagacaacc tagaaggtta aaataaagaa aaacactaag 53580

taggtctttt gtggtgacat atacataagc ctacttttag tgtattgtgc tatactagag 53640

tagaacggaa aaacaaaata tgtttggagg aatatcgttg acaataatag taaaagacaa 53700

cggaagtaga aaactacctt ttgaggaaga cagactaaac aggtacctag ataacataaa 53760

aaaagagttt cctaagttaa atatagagag ttatcgaaaa aaggcaatac actatatttt 53820

aagcaaggac gagtattctg tagacgaaat ggtagactac ctaataagag aagcagaatc 53880

tcgggtagac gttaccacac ctgaatggga gttttttgct actagactat atttacaaag 53940

attgtataag aaagctagta aaaacaggtt ttacgatgta attatgaagt atggttctta 54000

cgtgggtttg catgagagtc tagcacaaag aggaatatat tccgtagata ttttagagaa 54060

gtattcaaga gaagagctta tggaagctga aacgtttatt gacagtagca aagaccttct 54120

ctttacttac aatggtttgt acctactagc tactcgctat ctagcaacag atgaggataa 54180

gaaagtattt gagttaccac aagaacgatg gttaactatt gctttatttt tgatgcaaga 54240

tgaaaaagaa aatagaatga agttagtaga agaagcttat tgggcgttaa gtaacttgta 54300

tatgacagta gcaaccccca cattagcaaa tgcaggtaaa gtaggcgggc aactatccag 54360

ttgttttata gatacggtag atgatagttt gcaaggtatt tacgatagca atacagatgt 54420

tgctagagtt tctaagcaag gcggaggcgt aggagtctat ataggcaatg taaggtcgtc 54480

aggagcacct attagaggag ttctcggtgc tagtggggga acggtacctt ggattaaaca 54540

attaaacaat acggctgtta gtgtagacca gctaggagca agaaaaggcg ctataactgt 54600

aacgttagat gttttccaca aagatataga agcctttctg gatttaagac taaacaatgg 54660

agaccaacgt ttacgggctc acgacatttt tacttctgtt tctattcctg atttatttat 54720

ggaagctgta gagaatagag gggaatggta tttatttgac ccttacgagg ttaagcagaa 54780

aaaaggttgg tacctacaag actactatga taaagaaaaa ggtagtggtt tattccgaga 54840

aaaatatgaa gaactcattc aagatggcac gattagtaag aagagagtga aagctcttga 54900

tatgatgaaa cgtatcatga ttagtcagct agagacagga acaccattca tgttttatcg 54960

agatactgta aatagagcta accccaactc ccatgctgga atgatttatg ctagtaattt 55020

gtgttaattc tatggcacct ttgggtagca atactcatcg aaaacttttc ttaaacgggc 55080

atagctgaat acgatgaata agctggtaag agagcctaag tccttgagtg aggatagagg 55140

taatcccgtg ctaaattata ataaaatctt tattgtataa atgcctaacg actatcgaaa 55200

agctactttt tagtagaact tagtagagta cactcaagtg agtggaaaca gaaaagactc 55260

ccatgtggag tatgatatag tcttttcttt acagaaatgt aaagcagtta gtacactaac 55320

gggtagggaa ttaacgaccc ttactgaaag caagcaagaa atcgcacaaa atatgtcagc 55380

tacggtagtt acagaggaaa ttatagatgg agataaaatt gttgtaacaa aagaagcagg 55440

agacttcgta gtttgtaact tgtcttctat taacttaggt tctgtatttt ccgaagaaga 55500

cgttatcgac cgactagtaa ccattcaagt taggatgcta gacaatgtaa ttgagttaaa 55560

caaactacct gtaccacaag ctctaattac taaccagaag tatagggcag tgggattagg 55620

aacttttggg tggcaccacc tactagctaa aaaaggtatt cattgggaca gtgaagaggc 55680

tactaatttt gcagatagcc tatatgaaag aattgctttt cacactatcg aagcaagtat 55740

gaaactagcc gaggaaaaag gagcttaccc atactttaaa gacagttcgt gggatacagg 55800

ggagtacttc tctagtagaa actatactag tgatacttgg gttgacctag ctaaaaaagt 55860

gaaagagaaa ggtattagaa atggttactt aatggcagta gcacctaata tgtccacagc 55920

gcagatagca ggctctacag cctctattga ccctatctat gactctttct attatgaaga 55980

gaaaaaagac tacagacgac ctgtgatagc ccctgagttg aacttgaaca cttacgcttt 56040

ctataaaagt gcttacaaag taaaccagat tcaaagtgtt aagcagaatg gtaaaagaca 56100

aagacacata gaccaagcta tcagttttaa cttctatgta cctagtggca ttaaagcaag 56160

taaactactt gagttgcaca ctacagcgtg gagtgaaggt ctaaaaacaa cttactatgt 56220

tcgttctaat gacattgatg tggaagaatg tgagtggtgc tctagctaac agctattgac 56280

aacatgaccc ttttatgata tgcttatagc ataaagtaag agggtttttc cttgttgcag 56340

agaggtgtga gaaataattc aaggtatttg acaaacgatt taatgtgtgg tatacttgcc 56400

ctaaatgtac aggaaaacta ctaaaaatag aggaggagaa ctaattgtta aaaaaaataa 56460

aaatattaga accactaaac cctaacaaat caacagcgat tattaatgga gaggctagtg 56520

gtatcgtaaa ctggaacgat attcggtatc cgtctttcta tcgagcttac aaagagttaa 56580

ctacaaatta ctggttgcct gatgaggtag atatgaagag cgatgccaaa cagtacaacc 56640

acttatccga agaagagaag tatgcgtttg actccattat cggactttta gctacacttg 56700

actcacccca aacacgtttt atttataaca ttgcagagta tgtcagtgat gcctccgttc 56760

atgctattac agctatcatt gcacaacaag aagctattca taatgagagt tattcttatg 56820

tacttgcttc tattactaac ttacaagagc agaatcgagt atttgaggaa gctagaacac 56880

accctacgat tattaaacgt aatgcaccta ttatgaagtc ttatgacgat ttcatgaata 56940

acaaaacacc tgagaatatg gtaaaagctt tagtacagtc atctatctta gagggtatta 57000

atttctacag cggttttgct tacttctaca acctcgttcg tcaaaacaag atgacgggga 57060

caggtaaaat cattagcttc attaatcgtg atgaattagc ccattcgaaa tttatttctg 57120

agctaattag agctattgta ggggaaaacc ctgaactaca aacagatgaa ctaaatgact 57180

acgtgtacgc ttcttttaga catgccgtag agctagaaca agcgtggtct agtgaggtac 57240

tacaaggcat tgaaggaatt gatgtggaag aaatgttaga ctatgtaaaa tatcgtgcta 57300

ataaaatgtt agggatgtta ggtcttcaac caatgtatca aggtcacaca gaaaattcta 57360

tgacatggat taaagcttac gccgataact ttacagaaac aaaaaccgac tttttcgaaa 57420

tgcgtaattc tagttacaag aaaacaaatt tagacaatgg gttcgatgac ttatgagggt 57480

agggatttta tattcttcat tgagtggtaa cactagtgaa ctagctcaag gaattaaaga 57540

taacttggag gaactagcag tagaggtgaa atcttttcat attgaaagag aagaccaaat 57600

gactaatgaa gtaatccctt atctatctaa ttgtgacctg attatggtag gtacatggac 57660

aacagattac ggagtcatcc ctgaggagat ggaagaggct ttagaaaagt atgactatac 57720

ttgggtagat aagcctgttg ctgtatttgg aacaggagaa acccagtggg gtatggaata 57780

cttctgtgga gctaatgaca aaatagccaa aaagtaccac tctccttttg atacacttaa 57840

aatagagcag tttattatca cagaagagca agaaaataag ctattaagtt gggttactga 57900

tatagttaag cactggaggg agtagctatg tacgagctag aaaaacttct aggtaaagat 57960

aaacatggaa atcagataag aattggagat agactaagtt tacaactaga tgatggggag 58020

taccgagagt gcgtagtagc ggttagaaaa gttaatacta tcgtgcttaa ccctgtagct 58080

ttaactagta aaagtgaaac tgtaagtgta acaggtgtat tctttgtagt tactgaccca 58140

catacaggag aagaactacc tttactacct aaagtagata gtaacggtgt ggtagacact 58200

caaaaagcag aaataatctt ataattttat agaaagaagg aaaacaagtg cattcagtag 58260

aagtagaaaa gaaagaacag ttagcggaca ttgtatcaca agacaaggtt gtattaaagt 58320

tttataagga taactgtagc ccttgtaaga tagtaggtat gtctattgag gaagctagta 58380

gtaatataaa agaggatatt actatcgcta atgttaagtt agaaacttta ggagaggaag 58440

tcttctttga tttcggcatc aaaggcacac ctacactgtt gttagtagaa aaaggaatag 58500

agaaaaagag acatagtggg tttatttccc ctgatgactt ccaagagttt gttaaataag 58560

agaggcttag gcttctcttt ttttgttgac atttacaagt ttatatggta tagtaacagt 58620

agtaacagag gggaggtaaa aaaaatgaca aaaaagaata ttaaaataac gagtaagaca 58680

gaggtaaaaa atagctctgt tgaggcagga gacatcttct acattgaagg ggtgtctaag 58740

gggcttacta atttgtctgg tttgtatata gtgacatttg attttcatgc aacagaagag 58800

tcgctgtcta ggtacgctct agtttctttg gagacaggga tgttaaacac catttccatc 58860

aaccagtttt ctctagaaga aaaaataggg tgtatagtga aggtgaataa tatagaaata 58920

gtagcaaata ctaaggaggt ataactatga aagtaggaga aaaagtagta attaaaggtg 58980

aagtagttca cgtaaatggt tctttggtaa aagtagagac aaaaaatagt tctttttggg 59040

taaacaaaga agacctaaag aaagataaga gtagtggtaa gtaggtatgg cacacttaat 59100

tgaaaagcta agactatata aaaaaccaga agaggtcgta agaggaacat cagtaaaagc 59160

acagcatatt tatgttgcaa atgatgaagc aaagtcattt tcaggagccg aaaaatgggc 59220

gggaacgtct gcctatacct tgacaagtaa gaacgaattt acaaatgtaa ctattcactc 59280

tctagacatt agaggagagg gaggcagagc ttaccaagtt acactaaatc acgataataa 59340

tacttattta tgtgacttaa gggaaactca attaatggaa actattcgta acgtaggaat 59400

tgaggctggc ggaaagttaa ttggaactta cagctttatt gctgacggct cacagaaagt 59460

tctagtaagg ataggttcag acgcgtatgc aaaagcaagt gaacaagcgg agactaaaaa 59520

taaggcaaag agaattccca agaaggagct agaagtagga ggagtatacg cacctaaggc 59580

tacttattta ggaacaaatg actccctaga cttatatata ggggacttgt atagaaatga 59640

aattgtagac gataaccctt ggtaccgagg gtactacgaa cgaagaatag aattagggaa 59700

gcgtaagaag ttctctgtaa gcttaaatgt agcattacat gagggacagg tatgtgttcc 59760

aacatacaga gaagctgaaa tcatacctgt atttctaaca gacgatgggg ttacttcact 59820

aaatattagc aaccagctag ggcgtttatc cccaagaatg gacttaaaat caagccctaa 59880

tttctttgga aaggtagggg aggctcctga aattaaaaca acagcagagt tcttagaatg 59940

tttaagacgt ggcttagaag ctcgcattga agctttacct agtaaaatag agggcaatat 60000

tttaaaggga aacttaaggt tacaaaacgt tatccctgaa atggagacta tactcaagtt 60060

aataggactc aatactacga ttgaaaagcc taaatactcc gctagttttg ttaacaaaat 60120

aaaaaatata aaatatatag ttatagcaga acgtaaccaa aaagggacgt taaccgcatc 60180

ggaaagtaca gacttccatc agtttagtcg tgcttttgat gacttaatgt ccaatatttt 60240

agaggttatt atagtagagg aggaaaataa ttgacgttat atatgaatgg taaaaaagta 60300

catgagagta ctttccccaa tggggagatt aatatcatgc ctgatgaaaa tttatgggta 60360

cttgaggagg agaaagcaga gattacttgg cagtactcag gaaacggtgc agaagacttg 60420

ctagctgtca gactactagt gaacagtata agagagcaca acaagaaaca taatagcatt 60480

aatttaacag ttctttatat gccatacagc agaatggata gacttcaagg aaatcagccc 60540
tttagcttac tagagtattt taaaatgttc cctcaagttg acaaactaga aatagtggaa 60600
cctcattcag acgttacagg gagtattgct agaaaactta ttgacagtgg aacttactac 60660
tcagagcggt ttgctacagt agagatgcta ccagaagtgt tagcagaaga gttgtctaat 60720
acttggtggg aacctgtatt agtctatcct gataaaggag caaaagacag atatgacaaa 60780
ttagcaactg attctgatta ttcattcaga agagctactg aaaacagacg agtactattt 60840
ggaaataaag tacgggactt tgatacaggt agaattaaaa gtttagagct agtaggggaa 60900
ccacctacta gaaaaggatg gacagctatt attgtcgatg atttaacttc ttatggtgga 60960
acttttgtaa aaacagctac agaggctata aagttaggag caagccgagt agtactacta 61020
gtagctcata tggaaggtag cgccactaag ggagaattat ttgaatctat tgataaggtg 61080
tatactacga acagtatgga agctagctac acaccagaag agctaaaaga agtagaaaga 61140
tataaaaaac tagggttact agacgtaact aatattataa aataaggagg aaaatatgcg 61200
taaattaaat gaaatttcta tgttaatgag tgattgctat aaatttggtc acatgtcttt 61260
atacccgaaa ggtatcacac aggtgtatag tacacttgtt cctcgggata atacttactt 61320
ttttggaggt cgtacagata aaatggttgt ctttggatac cagatgtttg tacaacgttt 61380
cctgttagaa cattttaata aaaacttttt cgagttacct attgagactg taaaagagga 61440
ctaccactat gtagttagta acgctttagg ggagaagaat gctcggacag aacatatcga 61500
agcactgcac aagctaggat acttacctat aaaagtaaga gcccttccag agggtacctt 61560
agttcctatg cgtgtacctg tgttaaccat tgaaaataca caccctgact ttgcttggtt 61620
aactaatttc ttagaaacag tattactatc tcaaaccttc ttacctgcaa cagtagccac 61680
tactgcttta gaagctaaga aaattatgac taagtatgga gaagaaactt gtgataactt 61740
agaccatatt atgttccaag gtcatgattt ttcggagaga gggcaacact ctaatgaggc 61800
ttccttgtta gcaggtatgg gacatttagt aaactttgca ggttcagata ctattcaagc 61860
tagtgtgatg gctcacaact actacagtgc tgatttagag aaggaactcg ttcttaagtc 61920
ggtgacagca tcagaacact ccgttatgca aagctacggt acagatgaag tagagacatt 61980
ctcaacacta atagacaata accctgatgg tatcttgtct ctagttagtg atacgtatga 62040
ctattacggt gtacttacag aagttcttcc aaaacttaaa gataaaatta tgaatcgaaa 62100
tggtaaacta gtaattagac ctgatagtgg aaaccctgta gacattattg ctggacgtga 62160
ctatatagag tttggaacta attggtttaa ctatcatata actaaagaga acttagtaaa 62220
agcagtaacc actcgatacg agcagtcatg ctcaaacaaa ccctcaatag gtcaaagagt 62280
cgatattgta gactacctaa atctatttgt agatagcgac tatatttata ataatgatat 62340

tgttgataaa gagaaaaaat tagtatcagg gtattataaa gtaacattaa gtgccgtttt 62400

ccttggtgaa gacctgtgta acccttataa agtagacaaa cttgaactag aagattacca 62460

accaacccct gaggataaag gttctctaca actactatgg gaaacctttg gtggaagtgt 62520

aaatgagaaa gggtacaaag ttttagacga gcatattggt ttactgtacg gggagggtat 62580

tgacttagaa atgttagaga agatttttac tcgtatgggt gaaaaagggt ttgcttctag 62640

taacgtagta tttggtcttg gtgcttatat gtactccttc tctctttcca gagacagttt 62700

tgcccaagca ataaagagcc aaatggtagt tattaatggg gaagaaaaat taatctttaa 62760

gaatcctaag acagatacta atcatattaa gaaatctctt aaaggaagag tgtgtgttac 62820

aaaaaaagaa ggaacatatg aagttacaga tgctttaaca gctaaagaag aagccaacct 62880

tgaaaactta aacgagctga atgtaatttt tgaagatggt aagttagcct atcaagactc 62940

cttccatact attagagagc gtttagacag agaagcaaaa aaatattaga aaaacgaaag 63000

agcctttagt gggctctttt ttgttgacaa ttaagtaaaa gtgtggtaag atactagtat 63060

aaatcaataa aggagatgtt ttatttgcta gttaaagaaa gaaaattacg tacctcaaaa 63120

aatagtatgc tagatttttt aaaggagggg gagtttgtaa gagccttcgg tgacttatgg 63180

tatagaaaag ataagaaact atataatgtt acacttaaag aaatatttga catttatctg 63240

gagcttgacg tttctatgct agaggctact atgctagctt ttagcactaa agaaggattc 63300

tcacctaaaa agtatgcgtt agaagattta aaggaagaat atggtactaa tatagtaatc 63360

gactttactc ttcctgagaa taaagaggac tttgtagtag ataatacaaa aagtctgcct 63420

ctattatggg acacttacag aagttatatg cctaaaaccc aagcagagtt tcactacaaa 63480

aaacctattt ctaagaaagt gttgaaagaa acttgggggc acgtcctaga cacaggctca 63540

ctaagtgaag ttagaaagaa aatggtttta ggtacaactg ctagcattcg aggtctacgt 63600

aattcggtaa gttcgtcaag aataaaaact agcggagaac taaccgttac ctctgctaaa 63660

atggatttta aaacaggagt acttacttcc gttgttaagc gtgcaatggt agacgaatac 63720

gaactatctg cgaacgaata tacagtaaac tttaatcttt ataatgctat ggagcttatt 63780

aatgacatat cggactataa agtattagac aaaggacaca gagaccatta cgattctaac 63840

atgatgtggg agattgttac aaagagtata acccatatat acaaaaatat tgaggataag 63900

tacaccctct taaggctagt atcctctctt acctcaggtg tgcaaggaag atactgtaga 63960

agtgttcgtt atgagataga aggctcagag ttggaacact cagaagtacc tatgccttta 64020

gtacgtgaag tagtagctaa tctaaattta ggtgaaaata tacttgacag agcaggaaac 64080

tggttttacc aagacgtagt aggcagagaa agtgttgcat ctacattaaa aacaaacaat 64140

tatcaagagt tagtagagcg tgcaggttct ttagtagcag tttttcctct tttcgacagg 64200

attgcttctt atatgaacaa ccaaactaaa aaagagcaga agatttttga gagagctatt 64260

gaacgacatt ggctaggtgg atacaactac cttgagcgca gtaatttaaa aattattggt 64320

aatgcactag tacccaatga agtattaaga gacctactca cttataatgc agtacaactc 64380

gtcattgaga agtttacacg ctaccaacac aatgtggttg cttacgatta cttaccagat 64440

aaagggtatg aacaggagat tgaaaaaata cttccaagcc ttcaaaaaga aagtaagcgg 64500

attggagaaa aaattcctaa gtacattgag gatacattcc agaacataaa ggaaaatatg 64560

gtagaggatg taaaaaatgt acaaaataag attacacctc taaagaaacc atgggaatat 64620

attatccatg ttaaccaagg tatctggaaa aaagtaatgg aattagggaa ttttgagaaa 64680

gagtcctata ttattatctg gaaagctttg tacgaaaata cggaagagaa tgtaccattt 64740

cttaaatata aatccaagat agaaaacaag ttactattcg aaagctctgt tttagcaaac 64800

cctgctatac aatcatcatt tactgaggca tttcgagcta ttggcgggta tcttgagaga 64860

ctaggaagaa cacctgatga gtttatcaaa gtcattaact acttgtctac agactgtaca 64920

ttaagacaga gaatgagtct tagaaacgca acagtccaat atagagacta tattaattcc 64980

tatgtagcat taaactcatt agacttgaac gaggctaaaa gattcgattt gtgtcctttc 65040

tccttgaagt tggctcatga tattgcaaca tctgattacc aaactatgca ggattctaga 65100

gcggatgagt ttatggtagc acgatacaaa gttatgcctg acgagttcag cttaaacaac 65160

agaaaattct ctattgataa agaggaatat gttttagtac aacctaaagg agtagacgac 65220

ctaaaagcgg aaggaagttc tttaaaccac tgtgtcggaa catatgcagc tagaattatt 65280

aaaggggata acttgattta tatgttacgt aagaaagaca acatagaaga gagctatctt 65340

acaattgaaa tccttgtcca cgatgactcc cgagtatcat taggtcatct acagggagag 65400

tcttatcgca gtaagattcc aacaaaaata acagataaat taaaggaatt tgtggttaaa 65460

gtaaatgagg ctatgttagc tcaaagagaa ggcagaaatt ttactaaaga cactaaatcc 65520

gaaactttag ttagagccag ttaacttgta tttaaactct taaattatgg tataataaag 65580

gtagaggcta gctaataata tagctagcct tataattata ctgaaagagg aaattaaatg 65640

aaaaaagata atactcggaa aaaaaggtta aacatttata acaccgaccg atattttaat 65700

atgaacctta tgaaaagaag tgctattgag aaaagtatta aagtaacacg gttgaacgag 65760

aaagaaatcg aagaagaaat ggacaagcta gcagaaggtt ctacccgatt taacaagaag 65820

aataaaaaga gctacttact ctatcaagag aaatatacaa atgatagact aattcaaaag 65880

ctctttgctc atggaggtag tgttacatat tacactaact ctatcatacc ttactatgta 65940

ataaaacata tggcacagtc cactacaagt aaagccgtat actctgtaaa gaaagagttc 66000

acagaagaac aggtaaaaaa tatagagcta accaaaatgg ctactacagt gataattgac 66060

gttcctgttg ttgttcctga tgttagcccc tatgatgttc tgttctcact acacccttta 66120

aaaacagatg tagacggtgt acagttttcc tttccaagat taggtaaaag tgaaatacaa 66180

aaaaggcacc taccgttcta tgagttgaaa gggaaagtat acgaattacg acccgaaatt 66240

aaatatgaat atgtaaaata cactcaagta tctttatcta tttgggggat gtatctctgg 66300

ttagtctgta atagtgaaga agacttccaa gaaatagaca agctactaca aaaagaccta 66360

aaaagaagag acagtggcag acgacctagt gcaagtcagc aactaggagg gaaaacccat 66420

gcctaagaag atgataaaca gaacagatat agcccgtgaa gtagcacaaa gaacaggttt 66480

ttatatcaaa gatgtagaac agatactagc agaaagagac accgttatta tagatgccct 66540

tcaaggcgga gtcactaaga ttaaagagca caaactgtat cagattgaag tagaaacaaa 66600

accagcaaag aaaaatgctt ggaatgggtt agacaaagta tactttagta taccagagaa 66660

gaaaataatt aagtttaaac ccttagtagc agtagaagag gtaatagata gcttaaataa 66720

aaaagagacg taatagtctt tttttttatt gtacttgaca ggataatcaa ctcatgctat 66780

actaaggtat aagaaaataa gaggagaggt gccatgtgaa gatacttttt ttacaagagt 66840

acattagggt aaaccacgtc cttgtacaag atacaggtac agtcaatatt tttcttaaaa 66900

ctgacttagg gaagaagcta gtacaaaagc ttaaatcagt aggtataacc aaacaagact 66960

tttatattga ctatgcttat gctaagatac ctgaaccaaa aaaacaagta aacggtaaaa 67020

tactaacgta caaggagcca acagcacaag agaaaaagga accacaaaca ttactaacag 67080

agagaattat acaagaaaaa cctgatttaa tcgtacctat gggtaacatt gggtgtaaat 67140

accttcttaa tgttgcatcc attaggtctt taagaggtat accagaaaag gtgaccttac 67200

aatcagagca aacaggggaa aagtttactc catgggtaat gcctatgtat tctatggaat 67260

atctaatatc aagtcctaac gtagagggta tgtttgatgc ggatttaaca acactcagtc 67320

ggtacaaaac ccaaggagac gaggcgttta ctcctgcgga agtatcttat gagctagtta 67380

cttccttaga aagagtacag gatatttttg aaaatgaagt taaaaactat cctgttattg 67440

catgggattt agagactaac accttactac ctgagatgtt aggagccaag cctctagtca 67500

tgtcagttag ttggaaagaa gggcaaggtg ttactattcc gctagaacac cgagagagtc 67560

attgggaagc ctatgaactt gaagagattt ataactatat taaagaattt gtagcagata 67620

aaaatattgt taaagtaggt cacaacattt aacacaatgg gtgttgtaaa actcctctcc 67680

atcggtgaac gctaagtgca aagcatatgc caataccgag cctacattac tggtgacagt 67740

agatggggtg tagagactga accgagccat tgtagggcaa ggtgagaagt ggtacatatc 67800

acttaaacga ggagcattgt tccatatagg taactacaaa taaaacctat tggtaagggg 67860

cataaaggta tagtccacgc ttatatgaaa atataagact acgtgcagta tgatatacgt 67920

tttcttatgt cgtctaaagg gtttactaag tttaataata atcgtgatac gaagataggg 67980

tactacttgt ctgtaacaca acagatagag gactccttca aattatcaga tttagcctat 68040

gagatgacag atatgggtgg ttacgacaaa ccacttgccg atttcaagaa aaaatatatt 68100

aaagactaca acgataaaaa aacagcagaa caggaagaaa gaaaaaagaa agtagtagag 68160

gaaataaaca aacagtttgt agaagaaaca ctaaaattta aagaagatac taagaaatac 68220

aacgaagaaa aggcaaataa taagtattct gtactagtta agcctgtaaa acctaaaaaa 68280

ggtaaggcac ctacaaaggc agaactagct tacgagcctt tagctaacga agtagatgga 68340

ggaaacttta actatgactg gattcctcta gagatactac atccctatgc tagtggagac 68400

actgactgtt gcttacgtat tcataatgta cttacggaga taattgcaaa aaaccaaaaa 68460

atgtggttcc agtttgctaa tttttaccct agtgctattg tggcattagc tagaattgag 68520

tctaacggta tctatgctca gacggactat atggaagata ttaatgagaa gtacactgag 68580

gaggaagaac gcctcgtaga agaaatgcgt aagatagcag aggtatccga gttagaagag 68640

caacacatgg agttgtacga acgcggggta gaggagatgg caatacctcc tgcaaaaaga 68700

gataaggata tagctaagtt aagagacaaa tacaagaaga agctaaaatt taatcccaac 68760

tctgcaacag acaaaggaaa agtcctttat caaattatgg gtattacact tccctacgat 68820

aaagagcata ttaaagagtc tgcttttgat gtagggaaac ctgagaataa gctaacatgg 68880

gaggactaca aaacagataa gaaagcacta aactatattg tagaccattt tcccgaccaa 68940

aaggcgtttg cttctctcct actagagcac tccttagtaa aaacaagaaa aaataacttc 69000

acagccaagc ttctaaactt cattagtaat aaggacggaa gggtacatgg gtcgtttaac 69060

gctacaggaa cagagacaac ccgtttaagc tctaataatc ctaatatgca acagctacct 69120

gcaaaaactt ccgaccctaa aaggttcgac tatcagtacc cgataaaacg tatgttccag 69180

actgcatttg agaatggagc actactacaa ctggattact cttcactaga aatgcgtatc 69240

ttagctttag cagcaggtga cgaagctatg acacaagcat tctttgatgg ggcagacttg 69300

cacaaagaaa ctgcttctat tgtatgggga atgcctgtag acaaagttcc agcagaattt 69360

cgtaaaaagg ctaaagcagt aaacttcggt attgcctacg gagaaacacc tttcagtttt 69420

gcaccaaaac atgatatgag tgtagaagaa gcagaagaca tcttccaacg ttattttgca 69480

aataaaccta atatcaagac atttattgat gaaacacaca gagaagctct acgtgacggt 69540

tttgttgata cattacaagg tcacagaaga cttatccgtg atgccttttc taaagataag 69600

cagatacaaa atggtgcttt acgtaaatca gttaatacca agattcaagg aacaggtgct 69660

tactgtacaa atatgtctct aatttatatc gaccagtata tacaggataa caatatgaaa 69720

tctaaattag tattaactgt acatgatagt attgtagtag actgcccacc agaagaggtt 69780

gagattatgg ctcgggtgtg taaaacaatt atggaaaatc taccaattga cttcttgagt 69840

attgaatgga aaggtgaaaa aactaagtat cctattgaag cagacatcga gattggtact 69900

aactataacg atatggtaga atacaatcct agtgagttaa aagagttcaa ctctttaacg 69960

ggttatgtga agtactacaa agatttaggg aaaatttccg attacatgga ggctggtata 70020

attacagagg agagtatgga tttaggacaa actagaatta aagaatcaaa agaaatttac 70080

aaaaaaattg cctaaaaggt tgcttttgtt ttaaaaatag tgtataatga tacagaaagc 70140

tatttctaaa aacagaggag ggtaaaatat gacttaagga gggatttgtt tggacataca 70200

tgtagattca ttagaattta gccaattacg aattatagat ggtaacggac aagttacaga 70260

gcttgattta gagagagagt tgagagtaaa tgaatactcg atgcaggagg agtttctcaa 70320

ccaagcacca aaatatatct attgggcttc tattctagag aaagtacgta tgtatcagga 70380

gtctgcatca ctagctttag aggtagtggg agcacagatg aatgagaagg caagagagta 70440

ttacaagact gtagacacta agccaacaaa agaccaagta gaagcctata tacttcaaca 70500

agaggaatat caagtggctt acaaacagtt gttaacctat aatcacttgg ttaaacaact 70560

acagtacgta gtaaaagcgt ttgaacaaag aaaagacatg ctaattcaat acggagcaga 70620

tttaagaaaa aataaagata acggtataag cccaaacttc gggtaaaaaa aaataattat 70680

aaacgatagg agacaataac agtatggtaa actttaaaga atttttaaaa caggagaacc 70740

aagaaaacga aagtaataat aatgaagagg tagcatttcc aaaaggaaaa catcaacgct 70800

tattcttgtc taaaaaatca aataccgtgg tagttcgtat tttaccacca gctaatttaa 70860

cagataacta tgcacaaaag tatcgtaaaa ttatgctgaa caacgttatg acagatacag 70920

gaaaagcatt aaatccgtca tttacgttac catgggagcc taacgaggat tccccactag 70980

aacaggcttt agctgattgg tataaccgtg gagtagttcc taactactat ggaggaaaat 71040

cacgaccaag caaagctact gtagtaaacg ctgtggcatt agtaacagat gcaaacggga 71100

actgggtaca tgaggttgat gaatacggtc aactagtagt tcgagtgttg gacttacctt 71160

attcagcata tacagctatc aatcgtaaat tagcagaccc aatgctaaac actacaggct 71220

ctgactatag ctttatctct gatgtaggag ctaacccaat ccgtattact aaacctacat 71280

caaatacaga gaaagcatat caagttgaag tatatacagg tattacatta ccacctcttc 71340

ctgagaattg gagagaccaa ttggaggact tagcttatca agttagcccg tcagaagact 71400

ataacagcaa atttgttaac tttgttattg aacgagttaa tgctgtaact agtggaggag 71460

ctcaacagca acaagctaac ccttacggag gacagcaagc accacagcaa caaggtggtt 71520

atcaacaaca agttcctcaa caacaagctt accaacaacc tgcccaacaa gcacagacgt 71580

atcaacaacc agtacagcaa cagcaagcac aaacatacca acaaccaaca caggcgtacc 71640

aagccccaac acaacaagta cctcaacaac aagcgtacca agcacctgtg caacaaccaa 71700

ttcaacaagc tcctgtagcc cagccacaac aggctcctgt acagcaacaa acgtatcagg 71760

ctcctgtaca accacaggca caacaaccta ttacgcaagg aatggtggac tcagcaatga 71820

gcgggttagc atctcctaag gaacctgtac aacaacagaa accagtaacc ccaccaccat 71880

ctccacaagc acagcaacca gtaagcaatg attcaagctt acctgatgta gatgagttac 71940

tacgcagtat gcaagaaggt tgataagaac taacatgagg tctaggattt attttctaga 72000

ccttaataag aattttagag agggttgttt tcatggcaag agctaaacag agtgaaaaag 72060

ataacaagac agaaattgat ttaggtaaaa aaattgggtt aactactttg caggatagta 72120

aatgggcgga gatttcagat aagttaccta catttatccc tcagttagac tatattctag 72180

ggggagggat tccttttggt cgtttgacgg agattatggg taaaaacgca agtggtaagt 72240

ctactcttgc agtccactta actaaggtag ccttacagct agattgtaaa gttatttgga 72300

ttgacactga gggaacggca gaccctagta ggcttagtca gctaggcgta gatgtaaaca 72360

aaatattttt tgttcaacca aaaaaatcta agaatgctga ggtaaacaaa gaggattcta 72420

ttacagtaga atttgtagca gaacgagtag aagctatttt aaatgccttc tctagctctg 72480

aacaacgaat tgtgataatt tgggattcta ttggacatac accttctatc aaagagattg 72540

aacaaggggt agcaggtaaa caaccgggta ttaaagctaa ggcaatggct cagtttgcta 72600

ccattattgc tcctatggta actaaatctc attgtgcctt tattggtatc aaccaagcta 72660

gagacgaaat gggaagcatg tttggtgcag tagacagccc cggaggaaat gcactacacc 72720

attgggcttc tctaagacta gaggttaaac aggcatccaa aatagaagaa ccgatgcaaa 72780

atgcttttgg tactatggat aatgtatatg taggacataa attacgtgtt attactaaaa 72840

agtctaaggt atctacacct agacaaagtg cagaaatgtt ccttttatcc gcatcaggtt 72900

tgcaatatga agagaatatt tatcgttctt gtgtggacag taaaatgtac ggtcttatca 72960

aaggactaca atacaagtct tatattgcag atgatggaaa agaatacaaa atgtatggta 73020

aggactgggt tcctttctta cagtcaaaag acgggaaaaa gatacgaaaa gagttattcc 73080

agaaaatgat gttaattagt ttccctactg gatatagtcc acttgataat gaggtagtgg 73140

atgttaactt atgggaagat atgaaggggt taacagaact gtacaaaaag aataaagtaa 73200

agcaacctaa ggtgaacgag ggagcaaaag aagagtctat tgaggaagta ctagcaggaa 73260

ttgactcgga taaataactt tataggggag ggaaacttcc ccttttttct tttagtacta 73320

ttgaggagga agtaaaatat gagagctaat agaaatattg atttaaccga gcatgtggaa 73380

gtttggtctg aaaaaacaag aagtattcca ccctctaagt taaatccatt tctagatatt 73440

aaaaaaagat ttctatatgc aaaagagcat aatatcccaa ttattctaga aaacagcgag 73500

ctaaataact ctcaatctgt ggttctagac ctaagctttg tgggagataa atgggcaatg 73560

ggatacgtaa cacattattc agctattcgt acagagtcta ctccatacac tatccattac 73620

agtgatttgt attgtgcagg taaacaaagt aaaaagaata aaagagtaaa agtaatattt 73680

aaaggggata accettatac tatggaggaa gcctgatgcg taatattgat aaagaaaagg 73740

aatatattag aaacggcaat cgttatgtac aagagaacag tgggagaggg gtatttgcta 73800

gagatagtga ccgactattc catcagtaca gtaatctacg gtggagtgtc tacaataagc 73860

ataaagactc cttcaatgac gaaactacta gagcagaatt acaaagctat attgacgagc 73920

agtttattaa actagtgaag gagtacgaaa ttaacggtgc agtagacttc ccggggtaca 73980

ttaaaacaaa gttaaaccag agagtaaagc atacttttgt taagaatgta ttccgagaca 74040

aagctagaga acgtctaggt gctgatgagt ttgaagtagc taatctagta gaccgacagc 74100

atactgatga agcactacta gaagaccaga tgttacaaga gtatgtattt ggtaagacag 74160

agttcacacc tattcaaaaa gagttgcttg tctatttact agaaaaaggg atggtttctg 74220

atgaacgagt aattatcaga aatgtggcat ctaaattcaa agtgcctaga gaaaaaataa 74280

atgaggaatt atcggaacta cgcttttttg tttcttctaa gatagctaag tatgagtcag 74340

aaaaaggcta ttaatattga aataacgtct gctatattag agttagaaat tcagtatagg 74400

aggctttaaa caatgacaga aattcataca ccgcaagacg aagaagtaaa agatgaagta 74460

gttcaagaag ttattgtaga agaacaaccc gagaacgtag aagaagtagt cgttcctagt 74520

gacgatgaag taaaagcaaa atttatgtct tatgttccgt tatttgttac tttagttctt 74580

ttcttgaaca ccttattagc atcgttgggt tgggaaccat tagcagtttc tcaagaccaa 74640

ttgtatctag taggttcttc tgttgcaaca gttattgcaa ctgtatggtc ttggtaccgt 74700

aataacaacg ttactaaaaa aggcttgaag agagaaaaca tcgcaaacca agtattacct 74760

aaaaaagagg acaaataaga aaggatggaa tattaatggc tattaagaac attaaagatt 74820

tgcttaaaga gtctacagtc gttttagatg ggaagtatct acaagctaga acggtaaccg 74880

atggttttac aggaacctat caaggtaacc actcgtttaa agtagaaaaa acagggagta 74940

actactctgt ataccctatg tattttgaca aagatacaaa acaaatggag aagagaaaag 75000

acccgattat ctatacagat gacggagaaa ttttctttgt agttcgtact acagtagacc 75060

cttacaatta cccagttatt gagcaagact ccttaacagg aaaaaataca gagaaacaga 75120

ttctacaagc atttattgct ttctctgctg acgagtatag ttttgctaac tataacttgt 75180

ttatcaatga cgatttaaag ttctatgacg gttctgctat aaaagaagtt caaagtatta 75240

agttccctaa tgcgacagaa acaataaatt cttttggaga agataaaaca ttaactgtta 75300

tctttacacc tgcggatgct agcgataaaa cagtaacatt cacaagttct gacgagagtg 75360

ttattaaagt gagcaacaca ggtaagttaa ctggctctgt caactccact gaaaagtctg 75420

ctgtggttac tgctgaaact tctaatggaa aaaaagctac tgttactgta ataatgaaag 75480

catcagagct accagaaccg tgataaactc atagaaaaga aaggactttt cgagtctctt 75540

ttttttattg actttacagg taaactgtgt tatagttaca tagaagatac actaagaaag 75600

gagtgacaag atggaagaca attttgtaac agcctataat aaaggagaac agacatggga 75660

tgacttagca ttttgggagg agtcttggaa caacgaccct aatcatattg ccagtctagc 75720

ggagtatcta gggatgacag atagagacta ctggaattat gtagtaatgg acaaagaagt 75780

acctaaagag ctcctacctc gaagtgagca accccaacta gaaactgaat acatctacca 75840

catttgccat aaaaagaatg aagtacaaat ttttaaagaa ggttctttag tctctcataa 75900

tggaaaagtg gtacaagtag ataaaataat ctctgtaatc atcacagaga ataactcagt 75960

agccattcat cttaagatta aggagggaac taaatgattc agctaattac aaaaaataac 76020

atgcagggat actatgcaga ctatctgtta cgaaaagtat ctacacatga agtgagaacg 76080

acttacttag agaagttcag actgcaagac atcactagtt atagtgcaga tgctactatt 76140

attatcgtac taggatttag ctacagtgac agagaggcgg gagctactgt tagtaacttc 76200

gaagagtata gtgcaacatt taataaccca ttcactttct tttaccactt tgctagtttt 76260

ggtgataagt ttaaataccc aaatattgac tctctagttg acgaaaaagt aagtcctgtt 76320

atggtaatgt acaacacctt gaaggaaaaa aatcctgatt tcttaatagc agagaaagac 76380

aaagaagcta tgttggttgc tactgctgta gacgactacc actgctaccg ttttgcagaa 76440

acagggaatg ggctgtctct acctttaaaa gagctaggag acctctatgg acgttattta 76500

gctgaattgt acggaaacat gtctttggtg gagctaattc agtcaaacaa gaccattatc 76560

aaagtgagac aagagtatcg gaattattac atcaagaaaa aaatagagca gtctaaacga 76620

caacttgtta aagatgttat gctttatttt gtctatgcag aggaatacga gaatgagatt 76680

gcacaccata ttattgataa ggctaaagaa gttaacgata gggtgcttgt cttagtagga 76740

aagcatacaa caaataatga tatgttccat gtacgctccc ataatattca cgcaggagag 76800

gtagctaagc taataaataa aggttcaggt aaagaaagta cagcggtggt attcttaggg 76860

gattccaaaa cacctacatt taatgctgtt gtgaaagttt tgctagatgc tagtgatatt 76920

taaccactaa atgtggtata atagaagaga agtacacact aattagttag gagaaaataa 76980

tttgaaaaat ttaacgaagg cagaaagaaa agctattgag aaaggacact taggaaaaac 77040

agccgttatt ctaggagtgc taaatgcttc tcacggcaaa attgttatgt ctcagtataa 77100

taaaatagcg gaaagtttag gaatggaaaa ggtagctaaa ccagacttct tagcactaaa 77160

tgaaaccatt caagaagata aggttttact tggactgtac caaaaatcgt ctgtaggaaa 77220

gattacacta gatgatttaa caggaggaag taacgaagac aagtatagcc agtttaatgc 77280

tataacagct tatacagtag ataatgacaa agtattagca gacttacgag aatttagaaa 77340

aattcaacga gatggagctt atacacaaca tctttttaac ggagtgaaag cagatttaaa 77400

gaaagagctc tcttctattg ctaaacctaa atttattatt aaagagaaca agaaaaaaac 77460

aggtaaagaa atgattgtag ccttatctga ttggcacatc ggagctttag taatcaactc 77520

tgatacagga ggatacgact acaaaaagct agtaagctat ctgacatatt tactaggtga 77580

ggttgttcag acagtagaag agcatgatat tgacgtatgt catgtatatc atattggaga 77640

ttttattgag catattaata tgagaagtgt caaccaagca tatgaagccg agtttaatgc 77700

aacagagcag atttctaaat ctattcgtac aatgcttgac ttcttaaacg tattagccga 77760

ccatgtacat gtaaaatttg gtatggtagg cggtaaccac gataggttcc aaggaaataa 77820

aaatgaaaaa atttataatg ataatgtagc ttacattatt ctagatacat tgtttttact 77880

acaagattcc aaagcgctac acagtaacat tacgctaatt gataatcgga aagatgttta 77940

cagctttaaa gataatgttg caggtaagaa tattaaagta gtgcacgggg acacagaagg 78000

taagaaagta gatgtaaaaa taccaaaaca cattaagagt gaagtagtag actatctatt 78060

catggggcat atccatacaa ctagaattgt acaagaagat tatagcagat tccacgtata 78120

tgtaggtagc ccgatgggag ctaattccta ttctgcggaa aataacctac cgacaacaca 78180

aggttcccaa ttattagtta ttctatctaa aggtagtaac aatccaatgt ttttacctat 78240

ctttactaaa taaaggagga ggaaaacaaa atatggcatt tttaatcaca gtagtgctag 78300

taacgtacca agttattgta cttttgcagg cggttagtgg aactatccgt aaagaacgaa 78360

ttatggtaga agaaggcgga gaccccctac caacatcctc ctatgttttc ttaatcatta 78420

atcaaggagc tattgcagga ttcctattct tgtggggggc agtactaagc tttattctag 78480

ctatggttac taatgttatc ctagctgtaa tactatttac gctagcagta accatcttat 78540

caattgtaag tgcaacgatt ctaagtacaa tcgttcagac aattgcagtt attattgtaa 78600

gacgaaagca gatagcacat acgaaaaaag ttattgctga aaaagaaaga gagtagataa 78660

aatatgtctg agtatgaagc aagcagagca gacttaagta gagagttcct acaaggtgat 78720

ttacagccta gtattatgag acgagggatt ctctacatca gaaagaaacc tgaatatatt 78780

gttgagtatg tagaagaggc tagtagcgat acattaatgg taagtgtgtt tgcagttcaa 78840

ccaaagtcta aaccgccttt aaaggcttgg acattagttg ataaaacatt ccagtataac 78900

acaggaacca ctctaggttt actagctaag gagctgaaac atgtacctaa aaaaacagag 78960

aaaagagaaa atcccccttt gtttatcgca cctgcactag atggtgtgga cacctttaca 79020

ggaagagcag gtaagggttt ctatgaccga gaacctgata gagccacaag cgacggtaaa 79080

atcgttcaag ggaagcacac aggtgtattt gttgggtttg actctattac ttgggagaaa 79140

gctattgtgc taactgagga gatagctagt aggtatttaa ctgcccgagc ccaatggtat 79200

aacatttagt aatgctatat taaatagcat gttaacaatt gttaacagga gaggatggaa 79260

gatatgacct ttacagttgt tttattccta gctgtaatac tcttgttttt atccgcatta 79320

tgttttacca gtcataaaca gagttggaac gtagtaggcg gtgtactatt agcctttggt 79380

ctaatcgcat tgttctatgt gcttataatg gcaagtgctg tttcaggtat aaatggagaa 79440

tggtttggga tgaggttata atacacacat tgggggctct gtaaggggct ccctttttta 79500

tggaggttgg aatgaaaaaa ttttatgaag atgatattaa gcaggtacta ctaaaaaaag 79560

gaaagatgtt tggtgatata ggaaagagca ctattgtatt tgagaaaggt attactatct 79620

cctctacaat cgcagactgt cttatattta cagagaaaaa aggagtcatt ggtgtggaga 79680

taaagacaga gagagactcc actgcaagat taaataagca actaaaaaac tactccttag 79740

tgtgtgatta tgtatatgta atgtgtcatg ataaccatat taaaaaggta gagcagataa 79800

tgaaaaggta ccatcatgaa catgtaggca ttatatctta tattgagttt atgggagagc 79860

ctgctattgg tgtctataaa cctgcaagaa agtcaccgtt gaaagatgta taccacacga 79920

ttaatattct atggaaggaa gaaatactaa aaatattagg tgcctttaga cattatggtg 79980

caagagctcc taaaagctcc tacacagtag cagatagaag cggaggacta aatgggttat 80040

ccataaaatc cagtttctct gctcgtatga gtaagccaga actaataaga aatttgattg 80100

ctagggttgg acgaccagag gctaatcgga ttgtttgtga tattttcatt gaacatagaa 80160

acaatcctgc caaggctata aagtaccacc acttctttcc tagcaaacta gaaaggtaag 80220

tgaagaatag tggcaaacaa tactagaggt aatctaccta gggggtatac tgcactagga 80280

gacgggagtg agtctcacaa aacagtagaa agagcgctaa gaggttacta tacagtagac 80340

tttagtaata tatacgaaca catgacagaa agagacttag aagtacgaca ggcatacgct 80400

aaagacctca tatctcaaag aacaggcatc cctttagaca tgattttatt tagaaagaag 80460

acagagaagc agacattaac agctatagat gaagtctttt atgtaaaaca aggggtaaaa 80520

acaataggaa aagtatctat caggtcacaa agactactaa gcagtaatat gctgattatt 80580

atttacatgt ctaagagcca tatgcctcgt gtagttagaa acacaggaat gaaaacttat 80640

acaaaacgta gtaagtcaga catacgtgta gcaaaaagac aaaggaaaac aacaaaaaga 80700

cagagctatt gacaacagag tctacttatg ctatgctaat attagaaaaa taaacgaaat 80760

gaggtacaag tatgcaaata gtagaaaagg agagttcctt aaacagcttt ttaagcagta 80820

tgagaaatga aaaaggtaca gtggtagggg tctattataa aaatgcaatg atagacgaaa 80880

gtatcttagc agaagccagt cttcaattag aggtgcctaa taagacggag aaggtcttac 80940

aagccacggt agcactaaaa cctatgccaa gtcaggtaaa ccagttaacg accttagaga 81000

gcctcctaga ggactctgga atgggatggt tttttgatag agaacgacct atcacaccaa 81060

caatgagtac ccccttgttt aacgacttag ctattagtca aagtgcacta tcaaatatac 81120

aaaattatag actagtagtt aacatggctg accctaaagt aacaaaacaa acctttactt 81180

tttataaaaa tacctataca ttgtttaaag tagaggacag acagttggat agtaaggttg 81240

caggtaacat tattcacagt atccatgtct tatataagga agagcagtat gacggtgaaa 81300

ccttattcgg tagattgttt gtagaaagtg gtagtaaaga agctaaactt atgaagagag 81360

atactaaaat cttgttggac ttgttatgtg atgaaaacat cactagtctt tttattggag 81420

gaatagagtt atctttagaa gaccgtttat ccaatgctaa gcttttccct ctgtacacag 81480

cagttacagc tacaagaaca aactatgatg cttatagatt cctaacagat tcaggagaga 81540

ctactattgt ttcggatatg attctagaga gtaagttagt taaagtagct gactataagt 81600

ttacctttgt agtaaaatta aaaggaaatg cacaattaac tatatattta ggatagaagt 81660

aaaagaagtc agcatttaat tgttgacttt tttctttata tatgttatga tgtagttaat 81720

aaaaaagaaa aggggttgac agaagtgacg aattttttaa cattagtatt tattctagct 81780

attattgtag ctgttcctac gttattagct aaagcgatga gaaaccacta taccaacaag 81840

gagacaggtg tttggtggat gccatataca cactggtttg tgtacaactt tttatataca 81900

cagaagctag gatggctaat tttaggctcc ggtattatta tctccattta tattccattt 81960

attggtggat ttttaggaac agtcattgga tttgcagggg gactacttat gttatttggt 82020

gggctacctt atgtatttgg tttactaggg ggagcagtct ttggtgtttt tgtagacaac 82080

attgtgtatc atctaccaga atggtatcga gcttggttta gaaaccgatt tgggattgag 82140

tttccttacc tagaagacaa ggaagatgaa aacgaggagt gaaagtaatg aaaaatgaag 82200

atacagcttg gagtttacta ggtttaattt cagctattgc cttggtagtt atttcttttc 82260

tattcttagc agggtgttca caagagacat cacaggttaa agaaacagag caagagaggt 82320

atgagaagtt cactcctatg attgaatata tttactctca gaagttaatt aaggtgtata 82380

ggaatgaatc agacgagctt gtagagtaca cctactatgc ccgcacagaa gaggaagaag 82440

aagaagggct catccacgaa gaggagtata attattacaa aagtcaagga gttactgata 82500

gaggtgttat taaaacaaca ataaataggt atggtaaggg taagtaccca ttaaaactat 82560

gggatgtgca agagtacgaa ggaaatgtca gcttagatag aatagataat gaagtctact 82620

ttgagggtga ttaatataga aactacaagt acagtaggat actggaacat agataccttt 82680

tcgtatgaat atttgtactg tgctacagag ggagagcctt ggagcatagg gactatgcta 82740

acggagtact acactgatta taaacaagta aggcaactaa tgcaggacaa agagtttggt 82800

aataagaagt ttagcttcac gtcttcaaag gtaagcaagg caactaataa aaagaagcta 82860

aaggacaaag agacctattt tcagtatata tttgaaaaag ggcagtggtt ctgtgtgtat 82920

actaaagggt tcatggctaa cgtagagata cctgctatag aaaacccata ccgatttaat 82980

aagtggttag acaagaatgc taacaagtta agtaacaaaa gagtaaaact gttacgagaa 83040

ctagttgtaa gtagcagtga ggttaaagag gaaacagaga gagtagttcc tgctatggtt 83100

aaattagaac ctccgttacg attattgcta gacgacactc atagtgtcag ttctcataat 83160

atactggaat ttttaaagtc agggaatttg tactctttag ctaagagaga gttctatgtt 83220

gatgactatg gaaccgaagt agactgcttt actataaatg agttaacaaa accaagttcc 83280

caagtaccta gtcaaataac ggttagtaaa gagcatacat ctaattggta tcctctatta 83340

gacctactta ttgactgtgg ttatatatca gactatgtag ttgaggatac agggtctaat 83400

agtaaagaag taaaaataga gataaaagaa ggaggataac gggatgtcta tatatgagtc 83460

tatcgagggg ctagaacaaa gtattataga gggattacgt acaactttag aaacagaagg 83520

taagaatata gccctagacg ttatcagaaa agaattagaa gacagcccgt tcttacggtt 83580

ttttgagttg tctcaaggta gccctgcggg agatgtatac caagtagata ctactaagat 83640

agctagtaac cctacacagc attacgaaac tgcccgtgta gctagtatcg tgtctgctaa 83700

cgttcctgtg tggttgtggg ggcaggctgg aacaggaaaa acttctattg ctcaaagtat 83760

tgccaaaatg ctagatttag aattttaccc tatgcagaaa ataatggacg agttcgaact 83820

taaaggtttt gtggatgcca actctagatt cgtaccaact ccactgtatg aggcaatgaa 83880

aaatggtggg ttgttattta ttgacgaaat agatgcttcc gtaccagaag cactaatact 83940

tattaacact gctttagcta ataattttat gcgatttgct gacgagactg tatatgcaca 84000

taaagacttt agggttattg ttgcagggaa cactctaggt aaaggaggta acaactccta 84060

cgcacgtaat aaaatagact cagctacttt agacagattt gtacctattg agataggata 84120

cgatgaaaaa attgagttat taattacaaa tggagaccga gaattagtat attttgttgc 84180

actagttcga atagagatgg ctagaacaaa gttagaccat gttatctctt acagagcgtt 84240

aagtcattat aaaaaatttg agtcttttgg tgcggatttc ttgattgaaa atgttttgtt 84300

taaaacacta tctttagagg atattactag tgtagctcgt tctcttagca gaataatcaa 84360

aaaaacagat aaggattcta agtggaagaa caacaaagtg tggaagcagt tacagacata 84420

cctttcaaac aaaggagtag ttgtagatga cacgaactaa tcatacgtat tcatctaaat 84480

ttatagcaga aaaccaagga aatgtctata ggttgcacta tgcaggagag gactatacaa 84540

agataataga tgatatgtca gctatccccc actatgatat agataatatc aaaaaccctc 84600

aaatgaaact ctcatggtct acaggagggg aacctattcc agaatttata cacaagttcc 84660

aaacaaatta tatggaacct gagaaggcac aagagcttaa tgtaatgctc tctaaagtat 84720

ctgaattagt aagtagccaa ccacctattt atcattttta cggtaatact atatcagttc 84780

ctagagccat tcaagggcac cctaaaagtt ttaaacatcg gaaaaaagaa gtaactcctt 84840

atattagtat atttgtagct atcaatgttc tttatgacgt tagtgtagag aaccataata 84900

agtttataaa aaatatgttg aaagttattt tgatactgtc aaaagaccat actgtaaaag 84960

taacactagg taaagttagt gcctctaggg gaagaatagc tgtggagagc tttgtactaa 85020

aagaggctaa tcagagtttt aacttatcta gactttggta ccctttatct agtatagcca 85080

catatagaat tgttcttttt gagatgctag aaaggcatat gacactagag cctaaactat 85140

cctcttacaa aggtaattat gggtttacaa tacacagtac ggaaggaata gcagaagcag 85200

taaacttgtt tgagaaatac gactatgtag tcggttatta cgaaacaata ggcatgacag 85260

aaaaagaaat tatagaaaga tattttgtgt agttgacttt atctttcata tatggtatag 85320

tagctatgta aagtacaaag aggaggtaat actatggaag tagcagagtt agtaggtaaa 85380

ctggtaggta aagaagttat gattagtaca gaaaggcatg agcgccaaca ggtttatagt 85440

tttagaacac acttaataga acgtgttgag ttgaagcctg cccctaggac gattgacaat 85500

aaatcagtta ttgggaatgc cttttttgta gtctatttaa aaggactacc taatgaatat 85560

cgactagaac agttaagaat tagaatggat atactagaca gaggatgctt attacctgtt 85620

gagattgaca cagaaacagg agaggtaaca cttgttgtaa gcgactctct agctaaatat 85680

ttctttgcta gtcttttaga agttgaagca cgtactgacg aggacaagaa aaaggagcta 85740

gtacaggagt ttctagcagt gtctaaatta tttcacggtg gcgggtggac taacggtaac 85800

tattatgcag aggaggatta aaactccgta tataagaaat gaggtgaata tgtatgatta 85860

aagtagataa agtagaatgc aaggatggaa gagtttacta tgtctataat agtaataatg 85920

gatataataa aaagaacata tgtattaatg gaaaggtgca aacagtacct aaagataaat 85980

atgagaagta tttacctaaa ctttcagaga taaaaaaact agatacattg tatatgagta 86040

acaaaaacca actgtacaaa gaagttgtat gtgccagcag tggaaataca agtaataaag 86100

acaaaaaggt acgaatcaat ataaacaatg aagtagtcag ctacctgccg aagaaggcaa 86160

ataacttggc gatacttcta acgcctgaca attgccatct ggttagtagg gtagagccat 86220

ctatactagt agaacttctt tctaaaagaa cagtcattat ctctagagat ggcggtgggg 86280

ggaatgtaag aagaataggt tctgcttgtt gtggggacgt actaacgtta gcatataaat 86340

atataaatgg taaacagcag gtaagtatta atgtactatc tgttaaggat ttttaccaat 86400

atgaactagg agggttattt ttatgattaa agtagagcat attagatata agcctgtaga 86460

ttttggcttg tccagtacaa ctcgtacatc agaacactac gtatttaaaa gtaagagagg 86520

tcaaaaggtg gttacaatga aaattgtggg taatggtatt cgggcaatgc cttttgctaa 86580

gcttaaaaag caccatttag acaaggttac tgtattgaaa acattttatg tacctaatcg 86640

aaaacagcta gctaagcaac taggtttaag ttactacaat agtgggaaag cagtacaaaa 86700

aacagtaaaa cgtttagatg atattcagga ctcgattgca tttagtaatg ctattcaggc 86760

actacctgac cactcagcat tgagatttaa gttagtaggg gaggagaaaa cccctaaaac 86820

taaacttaac acagatggca gtacaaacta ccttattaga acttccgcta agctattggg 86880

agaggatgta aaccaaatta acttacttaa cgtatctggt gtgctaaata aagcggaggc 86940

agaagcagag aagttcttac gagtactaaa tctgtataag gaggctacca aagatgatta 87000

aagtagaaaa atatacagct agtaccacaa aaaaagcagg ttctattaga gggacttatg 87060

tagtaggtgg tactagaaac cagaacgaat cagatttaca cttaacaggg actacacact 87120

acaagtataa aaacaaaaaa ggtaatacga tgattacatt tgtcaattat tatacaaaag 87180

aattagtctc tattcctgaa aaggacttaa gccctcaaga cctagatacc ttttatagta 87240

cagtaaataa cagactaact actgtgttct atgtccctaa ctggaagcaa atgaataaaa 87300

atgtactaaa aaagggtaca aaatagcaaa attataggta gttcataaag attttatgat 87360

actacctagt actgttttct aatctgtttc acttatcttg tttcatttac ttcttttata 87420

ctctagattg tttcaaatgc ttataagtag ttatgcctag taaaccctac tctctagtac 87480

actttatggg tgtttcactt acaaatgaga taatacaaag tgtatgctat attatatatt 87540

aagaacactg ggaggcaaca gataaccagt aggtcataaa atagtataaa tgtagaggag 87600

ttacatgtga aatggcagat aacaaagata aaaacaagga tttagaaaat aaaaaagagg 87660

cacctacaac atcggtttta cttagactgc atgagatgcc tatcttagct tctaaggtaa 87720

atgatgcttt agatgaggac aagtcgtatg actatattgt aaacttatgt agtcagtata 87780

actttgtaat ctctaaatca gcaatcagta gatacaaagt aaaaagagaa gaatctatag 87840

aaacaggaat tgatttaggt gctttattag ataaaaggcg taaaaacgaa acaacagcta 87900

ctgtgtctat gattgcccct aaagagaaaa agtcatctcc cgaagataag agatacgagg 87960

aaacttttag tactgtagac aaggtataca acgatgtaat ggttttggaa gagattatcc 88020

aaaaaggaat ggaaggctta caatacacag agactataga ggcacctatt gcgatgaaag 88080

ctattgaaat aaaagctaaa atcacaggaa accaacttca aggactaagt attataggac 88140

tacgtgaatt gaaacttaga agtgtagcta aagagacagc aatcacagaa attttgatgc 88200

agtatatccc cgaagataaa caaggagaag tgcttactaa gatgagagaa gtagaaaagg 88260

aattctataa gaacttagac ttatcagatg aggatagaag aataactgag gcgcttaaag 88320

tcgcaggtat aaacagctag aaggaggttt ttttatggag atagtagtgc cagaaggtta 88380

tacaaaaata agcatgacta agtttgataa cctagaagat aagttactag cactattaac 88440

aggagagaca ctgttagtgc gtggtttaga aaaaagtacg aacgcagacg tactagttaa 88500

gctagatgac agaaagtacc ctgtgacaca gataagctat gaaattagta ttacagacaa 88560

tgtgtttcga gaacgttatt ggactatata tgacatatct attaatgcac taagtttgta 88620

tgatgttttc ctttttgagg aagacaaccc tgcatatgag ctaaagatac aagtatcaga 88680

caatgtgcag tatactagta tcagcggtgt taaagacaca gctaaagtaa tagctattta 88740

tgaagatgaa gaaaaaaact tgttttgcga gttatcaagg gaagacacat tgtacccatt 88800

agtagaactt acaaaaattt agcaaaaggt aaaatccctt gaaagctcta agtagccgac 88860

tagggatttt tttgttgaca ttatatagaa agtatgctat actacattta taacatagaa 88920

ggaggagatt taaattatga acaacaaaaa agaatttgat aaaaaggtga ctgaattaaa 88980

gaacgcgaca gagattatta aagagcgggt agcaaaagta acacacggtc taaaccaagc 89040

aacaaacctg ttgaacttaa atgtaaaaga agtaaaacga gttgcttcaa cactgcaacc 89100

tctagaggaa gttgtttatg agttgcagaa agaggttgac ggattagcac agtatagcaa 89160

agcggaggtt gtttactacg atggaagaga ggttaacctt gaggagattg acttagaaga 89220

ggacgactat gaagaggata ctacagaaga ggaaatccga gataccgtct ctactgatgg 89280

agggggagca cggattagag tatatcctcc tgaggaaact ttagtatata tggcagagca 89340

tattggacaa ggagtagaga ctgatgacct tgctgtttat gaagctgttc gagacacaaa 89400

cggtcacaca ggtttccgac aagaaggcta ttatgtagac cctacggaaa catatgctaa 89460

ccgagataaa cgattaaaag aaacctcaaa agtagaagag gttgaggaag tagaactaga 89520

agttactatt attagaccga aaagtaaatc taaatctaaa tctaaaccta aagctaagaa 89580

aaagcaaaat agtaaaacaa agtctaccac aactgagttt aaaaatgata cagaaaaagg 89640

tattgtaact gactatatta atggagcagg aacaagtgct attgcacgaa cttacaatgt 89700

tagtatgggt actatctaca cagcgcttaa tcgtcataag attcctaagc gtaacatcgc 89760

taataacagc gtagaggttc aaatggttcc tgtaatcaat aataagaaac tactaaatgc 89820

aattattgac agctacaaca aaggagtacc agttaaagac atctacgaaa aatacagtat 89880

tcataaaaac gggctatact acttgctaga ttatgtaggt gtagaccgta aagtacacca 89940

taaagggaaa aaaggagacg tagaacttaa gatggactaa cctccatctt agttttataa 90000

ttgtagttac cgaaggaggt tagttagttg aaagtccatt tcaagaggtc tgtgataata 90060

gttgctttag tagtaactag ttttattcta ggagtagctt taggttatac tatccatgta 90120

gataatgctg ttcaagaaca aatagcatta caaaataaaa aagattcttc tactagtaaa 90180

aaggaaccta gcaccaataa agacattgca gacacaaaag ctgtagacta tgagactgac 90240

gtagctgtaa ggttgaacct cctacgagta acagtaaaag aattttctga gaagtttaac 90300

gaggtagagg ataatgtaca agtacttaat gatatagaat actataatag ttatattgca 90360

ctaattggag acgtgaaacg ccaagttagt gatatacaaa gaatgaagta tcgtagtagt 90420

gagtctgctt tagctagttc tgtaaaccaa gcagtagaca acttctatca agcctcctcc 90480

ctagaagaaa ataacgttca gtcaagacag acggacattt ctactatggt tgttaacaag 90540

ctaaatacag gtaatgatga attagaaaaa gtctttagaa taataggagg taactaaatt 90600

tgggaattga agcaatagta tgcgccttat atggtgtttg ggtagcacgg aacctgatga 90660

ccgtgacaag tgttgatgaa acactgacgg aaaagattat tctagaaagc agtaacacac 90720

aagtagccca cttagcaata agtgaagcca aaaaacttct agagaaagaa acaaaagaga 90780

aaaatagaaa aattattatt gattacattg aaaaatggca tcgggcacta agtgacgata 90840

ctttaataga taccctagta gagaatgaaa gaaaaggggc taggaatgct agaattattg 90900

ctaatatatt tgtaaaaatt gggacattac ttggaatcct agtattaaat attccgttct 90960

ttcttgcatt agctaacttc acagtagcag gttgtgtggc ttttgttata ttaggcgtaa 91020

tatatggttg tcagacggaa gcaaagcaag aaggatacaa agcaagtaag gactatattg 91080

tagtctctta tgtgctaaag caggctttat tcttgctggt agtgttaatc tagtaggagg 91140

ttttattatg agagaatatg atgaggaaaa agaacgagag ctagctaaga agaaccatag 91200

gataaaaaga gaacgagctg aggcacttga aagaaaacga gagataatag agtatgtaag 91260

agaactagat aataaggaga aaaagttagt agaggaacag gcaaaagaaa aaataaacgg 91320

taagaaaagt agtactgtag aaaagtatgg taacgcactg gatatggtaa agaacacaga 91380

ctttgaagct ttgtgtcgaa tacagacagc ttaccaccta cagagagaat atcctatggt 91440

tttaagctct ctaaataaca aaggttctta tattcatcta ggagtagcta agcaagttgc 91500

agataagaaa accttgtcag gagatgctac ttacgagtgt gagctattat tttcagttca 91560

agtacctaaa gcaggtcaaa agaaacaaaa agatttacta ctagagctag aaaagagaaa 91620

aacacttgag ggtaactata ctaacgtaga aggtagtttt actagtcacc aatacattcc 91680

ttttctaact ttcttaaaca gaagaaagat tgatgcaaag attgattcta tgctagatga 91740

ctactctcta gctaaaacga aggcacagaa gaagaaaatc caaggagaaa taaatacttg 91800

ggtaggatac caaaaagtag taaaaaataa aggatacgtt ctttatagct atgcaagtgc 91860

caaagtggta gcagtttatc acagagaaat aagtgtatac agttaaggag gggttaaatt 91920

gagcggacta accgtatctg ataagcaaag aaaagaaatg ctttctatga tagagactct 91980

atggttaaag agaaatccca gccaaaggtt taatcaattt atgtttaact tagaaagaga 92040

gtttgcttcc tataaaaaga atgaaatttc aaaacgaaat agagttttct accaaatgag 92100

ccacttaggg tacaacaaag gatacgtcta tgtagcccac caagtccctg atttatttga 92160

tacatctgaa caagacttct attcttattt gaaggatagg ataggatagg ataggatagg 92220

ataggatagg ataggatagg ataggatagg ataggatagg ataggatagg ataggataga 92280

ccttcctatc ttttcttttg ttctaataaa gtagtgggtt acagctacac tttcagttgt 92340

ttacaagttg taggactctc taagtagcca gctatagttt tatctgtaaa aagagataac 92400

ccagacccca ttatagagca tatataccct aagtagcagg ctacacaaaa taaccaatct 92460

atagataact aatgtcattc agagactata gatgacacag catccttttg taaatttata 92520

tgttcgggct agattaagag gcgatacccc acccctcctc ttagagtact tgtagagggg 92580

gtatacagag tttcgttttc ctacctagta tttatataga atatataagt atcttcctta 92640

aaaagcctgt ggtggcttgt gagagcatac aggaaggtat tgtaccttgt atttatacgg 92700

tagcaggtac cttaacccct catacaagag gaagctttct accccatata aacgataaaa 92760

cactaagtag caggctatac tttctgatat aggacagtat gacttatcaa tatgtaaaag 92820

ggacaaagct ctaagtagca ggttacagaa aaaaggaaaa agctctaagt agccggctac 92880

agaatttcct aagtagcagg ttatagatat taaaaccatt tatggggtag gtgaatgcag 92940

gtagatggcg gggtggtgag gactttgaaa taccttgtga acgttggtgt tatgccgttt 93000

actgctagat gataaatagt ttcttctata taatacaacg aaagtaagct aaaaagaatt 93060

taaaaatgat gtataaaaac tgttgacact ttttatattg tatgctatta taaaagtagt 93120

taaagaaagc aaccaaataa ataaaaaaaa actttataat aactgttgac ttttaaaagt 93180

aagcatgtta ttataagagt atagaaaaga gcaacaaact aaaaaagtta aggtggtaat 93240

ctcaatgaaa aaattaaaag caaaaagtaa actaagcttt atcctcccaa aattaacaaa 93300

cgacaacaaa aaaattgact actctaaaat tcttacatca gcttgcaacg ttttcggagg 93360

ctacacatta agagagtgtt caggcggttg ggttgacaat aatacattat atactgacga 93420

aagcaacgaa ctagaaattt tacacgaagg cttaaacgaa gacatgaaaa acacagtaga 93480

ggaaattctt gatttctttt tcagtgaaga agaagcggga cagcttgcag tatacgcaaa 93540

caacaataac gaagtttaca ttttagaaaa cgaagatgta aaagaattta aaaatatttt 93600

ataataactg ttgactttta gaaaagaaca tgatataata aatgtacagt aaaaaacaaa 93660

taaaaaaaaa ttaaaaggtg gtaatattaa tgaatattga aatttgggcg tacgcaacga 93720

aagaagacga aatttgtagt gcagagcaat tcatttgttc agtaagtaat gaagaagagg 93780

cagaacaaga attaagctac gcagagatgg aaaactacca cgcttggaaa acttactaat 93840

aaatttatat agcaaaatta taataagtgt tgactttaat aaataatttt gctataataa 93900

gtttatcagt tagataggta aacaaactaa acaaaggtgg tagttaaaat gaatgtagta 93960

actaaaatca gtacaacgat gaagctagac gactatagca acttgacagg acaagaattt 94020

acaacatctt tctatcagtt agcaaaattg cataaaaacg aaattaaaga cggagtaatg 94080

aaagaactat ctaattatcc tgaggagtta cagaaaaaaa tacaggattg ccagaaagaa 94140

gcagaaggac ttaaaaaagt agtacaattt gttagtgaag acgtgaagaa caataaagta 94200

aaattacaaa aaaattctta tggtgacctt aacactgttt ctttgaaagc aagtgtgcgg 94260

agagctttag cgcaatacgg tgacaatatt aataattggg aaaaaaatta cttactagag 94320

gattgtgcta tacgccttca atacaatgtt tctaatgtgt atctttcgga actagctaac 94380

ttgcggtcag atgcaagtga aaatatgatt gcatggagct ataaaaaaga ttattcatta 94440

gaagcagagt ttgaaaaaat tgatgactac atggcggtaa aacttataga cttagttaca 94500

aaatcacagc atgagcatag aaacacgcat gacgatataa gaatcaaact gttagcaatt 94560

ctagaatttc aaaaaaaata cgaggttatt agctgggact tatacaacgg taaaaagctt 94620

tctgatgaag aaatcatagc aattcatagt aaactaccag aatttatttc aaagtttcat 94680

gctgtttaca ctgatattga ggagttaaga aattatttag aaattgagta aaaaacgctt 94740

gacttagtga tgatacaatg ctataatata agtatagtaa aggttatcac taagtcaagt 94800

acctacataa aattaaaaaa aagttataaa aggtgttgac tttagatact aaacatgata 94860

taataaatgt atagtaaaag ttaaggacgt gttaattatg aaaaaaccta ttcaggaact 94920

aagacaatta attgaggagg aagaaagagt attggtagac tacatcagtc tagaagacga 94980

caacggcaga aacatcattt ttattgaaaa cctaaacgca gaaaactcag atgacagcac 95040

tatgctagaa acttactccc cttatgaagc attagagttt ttagaaaatg aggggttgct 95100

tgcttaataa atttatataa cacagttgta aaaggtgttg actttaaata gtaactgtgt 95160

tataataagt ttatcaagta aaggtaaaca aattaaaaaa ttaaaaggtg gtaatattaa 95220

tgaaaaatca aattgttggg tataaaaaat taacggttca aaaaatttgg agagcaacaa 95280

aagcgcaatt agtatttgaa ggtgctagaa tggactacgc tagataccta aaaatgaaaa 95340

aacaaggaaa agaaatgggt attacagtac caaaattcac gttgaaagag atgtaccaaa 95400

atcgtgctag catgaaacgt gatgaactaa gaaaagacat gttcctgtgc cttgatattt 95460

taaatgaccg agtaggaaaa taaaatgtgg tttgtaatta ttggtatcag ctttggacta 95520

attttagggc tacgaaaata caataagggg aaaggagcgg aaaaaggtga gcaaaaaaaa 95580

ccttagtatt agtaagatga aagaactctg tcaaaaagaa gggagtacct tctttgaacc 95640

tagcgccatg aaattcttcg ggacaaaaat tgtaaccaaa ccaaaaaata acttatttat 95700

tacggaagat ttagcaaaag accacagaac taaaaagttt acggtaagaa aatttgagaa 95760

tggtgaagtg attagtgtta acggtttttt gaagtacagt tccttggcag aagctaaaga 95820

agttctaaaa actattttat aaatttatat agcatagctg ttgactttaa ataataacta 95880

tgctataata agtatataaa ggaagagggt aatttaaatg gcagacgtat ttggatataa 95940

tgacttttta acagaaatgg gaataaagtc agtaagagga aatgcaatta cagagcaatt 96000

aaaacaagat tcaagctata ttgtagagga tattaaaaga gcatttaact ttaaacttta 96060

caataatgag ctaacagggg aaaacaacat ggtgaacgta actaacaaac ctctgtcttt 96120

agtagtattc ttagcagaca ataaaagtaa agtattattt tacgatagtg tgaactttac 96180

tagtaaaaaa gctcgggact ggaaaccatt tcacgctatc tttaaaaatg gtaaaacgta 96240

tattgtacaa gataattcaa attttgcctt gacattcgtt attaaaaatg gtactattat 96300

taaagaaagg aaagcaccta aaaactcagg tttgattttc ttagaaagac tagcggaagg 96360

ggatttataa gatgaaaact gttaagaatt gggttgaaag caacatcttt gttattctaa 96420

cttttgtaat gggtttactc ttactactag ctttattaca ctattaagtg ttgactttac 96480

agaacaagca tgatataata aaagtataga aagagaggca cacaatgaaa gattacgaag 96540

taaaacgaat tgagacaaag gaagctaaac cttttatttt aggcttacac tatgcaaagc 96600

gtatgcctag cgtgtcgtat gcgttcggta tattccttga tgatgagtta cttgggattt 96660

gtacaatcgg gaagcctgca agtcacagct tatgtaaggg tgtatgcgga gaagagtacg 96720

cttataaagt atttgaacta aataggctgt gtatgaaaga aaagttagaa gctaatgttt 96780

taagttattt tgttagtaaa gtgcttaaag aattaaagaa ggaaaatcta atcttagtat 96840

cttatgcaga ttcaggtatg aaccatgcag gttatattta ccaagctacg aactggatgt 96900

acacaggtat cacaaacgaa agaacagaca aatatacacc tgaaaacaag cattcaagac 96960

attataataa cgagtatagt cacttacgaa aagtaagaac agctaagcac aggtatattt 97020

acttagcagg ggataaacgt tttgtaaaag aggtggcaaa caaaattaac tacaaaaaac 97080

aagtatttcc aaaatctgaa aacaaaaact acgagttagg aacaagacag aaaacaaaag 97140

taatcaatac aaaggataac actatttttt atcaatagag gagtgtcaca aatgaaaaat 97200

aatacaataa atataaaaga tgtaaagcta acaatcagaa aagatactat aaaaatgaaa 97260

aagctagcta aaaagatagc agagtattac acagtaaaaa aaccttcttt agcggatagc 97320

ttgcgtgtgc aacttattga aagtgaagct aaaaaactag aaagcactat tgcaaaaaat 97380

gaggagcttt tatatttgat gaataacttt actaaataaa ttagttttta ctgttgactt 97440

tagaggttaa acatgctata ctaaatatat aataaaaaaa ggtggtaata ttaatggaaa 97500

tgacagaact cattaaaact aaattcgaaa aacacggctt tgaaaatcta aaagaggaaa 97560

tggaagactt agcagagggt taccaaattt ttatagatac tacaccagaa gcatttgaac 97620

tagcttatga actagaccgt gagggcttca caacatggga agaagcggaa agtctttata 97680

ttgagttgga gagtgcgtaa cacatgtata ctactattcc acattacatt attaaaaaag 97740

tatctgataa ggaaaaggta aactaccaag caatttggaa ggtactacct gtagaggtgc 97800

aaaccatttt tgtatcaaag aaaacagcta gcttctatat agttgataga tacgctaata 97860

gccaattttt cagaaacttg ctaacaatgg cagggaaggc aaattttggg gtatcaatca 97920

gtagttccag caaatcccat ttagagataa ggctcacctt tctaaataca catttttagt 97980

tttatatagt gtagctagtc ctttattgct acactaaaat aagattaaaa aaattaatat 98040

aaagtgttga ctttaaatag taactgtgtt atactaagta tataataaaa gataaggatg 98100

tgtgccaagt ggaaaaattt aagtcactag gagaggctac aggtgaagaa ctagcctttg 98160

ttttagaaag taactcccaa atatttgatg gtgtgctaga cattgcagaa aaaaatcttg 98220

gaaaagaaga ctttgacaaa catgttagac ataacggtca ctatgatgtt gcgctatgtt 98280

accttgaaat atatgcagag gataatccta acatcttatt cgagaaaaat acctttcaac 98340

taaaaaatta aaaaaaaagt tatataaggt gttgacttta aaagttaaac gtggtatact 98400

aagtatatag taaagaacaa caaaaaaatt aaaaggtggt aatacaaatg gcaattaata 98460

aaaataagca cagtgtagca gttgttaaaa ctcaaacggg gaaaattgtt ggttatcttt 98520

caactagaga cgaagaaaat ggcgtggata ttcaaatgga catgcaaggt tatactgtag 98580

aagcgctata aaaataattt ataaatttat atagcatagc tgttgacttt aaataataac 98640

tatgctataa taagtttata agttaaacac aaaggcggag atagtaataa aaaaactagt 98700

ataaagtgtt gacttttaga aataagaatg gtataataaa tgtatagtaa gagttaagaa 98760

aaaaactaaa aaaaaactaa aaggtggtaa ttaagatgac agttaaaaca gtttatagcc 98820

caaaattagg gtacacttat ggaaatttag aagtagaata cgcagatgtt aaaagttttt 98880

atggtaaagc ttattttaga aatatgcaca cagacgacca cacgcatgta taccacttat 98940

actcttacga tactttaatt cttgaggtgg tagacggagt tgtaacagca atgacagatg 99000

acacagacca ctatagcatg acaactaata gacacattaa tgaattttta gcacagtatg 99060

aagatgacta cacaggtaaa ctaagcaaaa aagaactact agaaaaagct aaaaacctag 99120

gaactgcttt agtatagaca taaaaggggc gggttaacgt gactacattc aagaagaacc 99180

gcacagttaa ctgatactag accaagtaaa aaagagttta taattataaa aagtgttgac 99240

ttttagaaaa gaacatggta tactaaatgt atagtaagag ttaagaaaaa aaaactagga 99300

gcgtgttaaa aatgagcgta aaaattatca gtgaaggttt agaaagctat atcccgtgga 99360

gtggagcagt tgtgaactac gaaattattc aagaaaaggg actattagat gtttttgagt 99420

cattgttagc agaatggtat ccggaaggat taacagaaac acaagtcaac gatattttgt 99480

ggtttgatga agacttattc agctttttag gaattgaaga ggaggaggaa taataaaaat 99540

gagtataata gcaattaaaa aagaaatcca tgcaaaaggt tacaaagtga caggaaccca 99600

tcaaggctat atagcacaaa tcaattttga cggtacagga aacgaatatc ctttaccagc 99660

tacatgggat gagttcattg aaacatttaa agatggttgg aatggtactt atgaagacga 99720

acaagcgttc ttcaacgata tgcaggaagt tgcattaaaa gaaatcttag acgaactaat 99780

agacgtatta tataatctag acattactac atataatttt actatagatg atgttaaaaa 99840

gaaagttatt acattaaaca aacctacaga cgaagaagat gcagaagact tagtaataga 99900

atttgattct acttgctttt gggatgctac agtagaaaat gataaaataa aggttactgt 99960

aagaaacaaa agtaaataaa taaataaata aataaataaa taaataaata aataagggtt 100020

atcactaata tatttatata gcgtatctct acctttcact tgcaatacta gcggaggtat 100080

gctacaataa acatattaga cctaaataaa aaggagaaac ttatatgatg acaattgaag 100140

agttagcagg tctacaaaaa gtaaaaaata agctagaatg tgcatataaa caactaaacg 100200

atgctacaga atcactagaa accactctaa ttaatgaacc tctcgctata ataaataccg 100260

attactcttt tctagctaat aacatagagg atgccagaga ggtattaaac aaaacaatca 100320

ctaagttaga caggtataac gaagcactag caactaataa agaagagata gacaagtacc 100380

ttcatcttac taagtctaaa actaaataat aaatagtata atagacaaaa aaaaaataat 100440

aaaaccaaaa cctgtaaatc acctcagcat ttttttcaca cggtgtcaac tcaaaaggct 100500

tatatatgcg cagtctttct acctattttt tagaggggat aatagtacta tataaataac 100560

ctctataggt ataacttctt atatttagga ttatttcaaa ctaattaggt ctaaactcac 100620

attattataa tgtcaatagg gtataactcc taataatata taaaactatg tacctattga 100680

ctaataatgg tgtgtaaggt ataatagagg ggggaggtgt aggcatctta tgaaaaagaa 100740

gaaattcagt ttagttattt ctttattact acttcttcga ttatattagt gagggtacgc 100800

ttgtagattt aaaagaaaca aaagtaaaat aaaactgttg acttattagg ttaactatgc 100860

tatacttaag gtataaacag ttacaagtgg gataagaggg tataggagct cgtatatagg 100920

cttttacttt attagatagg taaatagcag taaataaact aatcccgctc agaaaggctt 100980

gtaggaggtt agagaagatg tttgtattaa atggtttact atgtacttta ctagtattga 101040

tgattctagt gggggctatt gctttaaacg cagataagca gactaagctt attgtaggaa 101100

ttatacaggt ggtatttatg actatgtgcg tagtcctcta tatcactatt atagggatta 101160

gcattactat tattcctttt ggtgtattat ttgcgatttg tggggctgtt gctttgttca 101220

atctacttac ttttaataag gaggagatat gatgacaaaa tacaagttaa atacggttag 101280

taataataat ttaaaagcag gggacgtggt acttgtcgag aacttagaat gctacgaatt 101340

aaaatcaggt gtttacaaga ttattagtga gtgcgacggt ctatataatt ttgtgggtat 101400

tgatgtagac gtgtacgaac ctgtttctgt agaaaacatt aaccgactgc ttgggagctg 101460

tgtgttacgt gtatccgaat taactttgga tgtagtcgtt caaaactaag attagaaaag 101520

gaggaaggga ctatgttgtt atttatcatg aatgttatca gtgtagctat attaatgagt 101580

actttttact atatacgcaa tatatcccct ttctattacg agcggacgac tagactagtt 101640

aatcacttag tagatataga gctgatttta ggattattta tctggttagt gtttattatc 101700

tctattactt ttgtataggg aaaggagtag aactatatgc ttattataaa gtctttaatt 101760

gtagctatta tatttactat cttactagtt tatactatag acgggttttc tcgtattcgt 101820

aaaggggcta aaagtaatgg tattttagct gttgtttgcg ggattatagg gataattacc 101880

ttttctattc tagtagttat tatcttttag aggtatacca attttttaag ggttcattta 101940

gctaaaaata gctttggggt gtgccaacga gtttcgcgtt ttggagtgaa aatatcccta 102000

ggggggaatt caaaaagttc acctatacca atttgaaatg agggggtttt attgtttatt 102060

gtactgatgg gcgttttggg aatagtgtta gtcggcttgg gtaactatta tacaatgaaa 102120

ggtctagcga tgtcacataa gcttagtaaa gacgtagcta gtgaagaatg ggtaaatgaa 102180

cagtttgaag attctataga aggtcgtata aagctaaata actatgctag agctactatg 102240

aatcgttcag actatgtatt taacaaaggg atgttattac tatttatagg tgccttatta 102300

cttatcccgc tagtaacttt aatttaaaag gaggtagaaa atgggtttaa cattagtaaa 102360

tgacgatgat aagtatctac gtgcgtttaa actagattct tttcaatcag taaaaggaat 102420

tatctatgta tatattgtta ttactcccga agtattaaca gagtacctct cacagagctc 102480

agatggacag tattttaaga gtagtaaaga ttatcaagta aatgtacagg aattagctag 102540

atggcttaaa acgtctaata gaggctttat agagacacta tctcataatg agaacattat 102600

ggcaagtgta ggagaaaata atatataaaa gagttgactt ctttcttttg atgtgctata 102660

gttagtgtat caagtaaagg agttcttttt atatacttaa aaaaaagtta taaaaggtgt 102720

tgactttata aaagaactat gctatactga atatataata aaaacaaagg tggtaatatt 102780

aatgaacaac aatcttaatt actggacgga tacactagca atcaacttgg cgggattaac 102840

attagtagaa cgggaagagg taactacaca ggaagcactc atatcagaga tgtatacaag 102900

agacttacaa gctaacaaac ctatgtttaa taacttatta gataacctag aagagttaaa 102960

cgacaaagca cgagaagctc actcagaggc tttctatcat gcagtagatg ctattatatc 103020

gctgtgggac ggaataagca aaaacacttt ataaaggagg aatagttata atgacaaata 103080

aaaagaaacc aactgtacag atgacagggg aggacggaaa tgcctttctt atcttaggaa 103140

gagttactag tgcgctgaaa cgggcgtgat acaccgaaga agaaagggaa cagtactata 103200

ccgaagccac atcaaaggac tacgaccatc tactggcggt tacggctgaa tgggtgaatg 103260

tagagtaagc atataaacca atactattag aaaattatta gataaaaggg ttgacaagtt 103320

caacccctta tggtatactt agtatatagt aaagagaggg gacgagttac atgctaacac 103380

taaagaaact attgaaagct aacaaaggac aaaagaaaga gattgagaaa gtattatccg 103440

aaagagaaca agctatcgca aacttagaaa agctagttaa actacaacaa tctgcttaag 103500

cattcagtta cgtcattagt ggtaggcata tatacccacc ccgtggttct gtgaccgtac 103560

tttggggcta tgctacacag ggggctcctc tcggggcacg tttttcggga acttttttta 103620

aattttttta ttcttaaagt caacaaggaa tggctctata gagggcttta caagaatctt 103680

tataaaaagg gcaccccgtt gttttttctt aggtaatttt aaggagaggc ttctataaat 103740

aaagtgaaaa aattccggta aaaattttta aaatagggaa taaggaggtg ttcctataga 103800

tatgaataaa gataaagaca acgcattaga gtttcgagct tggtgtaaga taaaacagat 103860

gatggcagtg gttatctcta ttaattttta taaaggagat atacaagggg aggatgtatt 103920

tctctcttac cctgcacttg ggtcatttaa cttaagaggc ttttccgagg ttatccttat 103980

gaggtacaca gggttacgag ataagctagg gacaaagata ttcgaagggg atattgtaga 104040

ggttccttta gaggcagacc atggatatgc aggctctgtg ggggttgtca cgtatcgtaa 104100

ctatggttta ggggttatga cacctcaagg gttcttttat ttagaggaac ctgagttgct 104160

tcttgttaca gaagataaat atacgaataa agtagaggag ggttaagaat gaaagcacct 104220

atttcagaat gtccttattg tggttctaca gaagggtaca cacttaggtc acaagcaagt 104280

ggtactatct actggcggat gaaatttaat gaaaaagagg cagataattc agagtcacat 104340

gataatttac agtatagtcc ccataagtac gcttattgta ccgtatgtga gagtaaactt 104400

ttcaaagcag aggaggtgaa tgggtaagat ggagcttcct aagcacatta tagtaaaaat 104460

taaacgagca gggcaccata ataaaaaagc aaatgactta caaacggaag tacgcaagtg 104520

gttagagagt agattaacag atggggaatc tctccctgac tatataggag accaactgat 104580

agatgcgttg gagcttggtg tagatggtag tgaggcgtta cttaaattct tagctgaata 104640

tgaggagtga atcttaatgg aaaaattgaa tgagaaccag cagattgtac taaagtggtt 104700

aaagaattac ttattagatg attctgattt ttataacacg gtatatgctt ctaaacaatt 104760

atacgtgaac ggctttccta gtgataaaag taatattgct tttatgaaac taacagacaa 104820

ggagttcgct agcgtactac tagcgttctc tacatggtac ttactagaat tagaaaagga 104880

ggatttaaca tgaaagagaa atgtagtata gagatttctt actataacgg tgaagacaag 104940

tttacgggaa aagtagtgtg ggttgtacta gacaaagaag aaggtatcca atttgtactt 105000

actgagaaac cagaggacga atactttgtt tttgacagtg aggagtcggc aggtattgta 105060

ggtggtctta ttagactaag actagcatgt gacaacatag aagatgactc ccctgtaaca 105120

attaattacc ttaaggagga ggagaataat gaatagctgg gagtctattt tagggctagg 105180

tagcacgcac aaaatggtaa tagcacaaga gttggtaagt tttctttata aggaaaattg 105240

tgatactact acgctagggg agaatgagct tttcatacta aagtttctcc tcgataacaa 105300

agtggagttt aacagaaaag aaacttatta tggaacaacg ttctacagag gttatactgt 105360

aaccgtaaat gttagggact ctgtgttagc aactataggt aaggatatag cttccgtaga 105420

taaagacgaa gataaggcta ctccagaagc agtaagttgg ttagaagtgg aatgttgtga 105480

tatactgtat gagttcctgt ctactaaact aactgctgag aagggtagtg gtagacttag 105540

tgacacgctt aaaataagtc gttcttatga agaaaaggta aaaggactag aaataactat 105600

aacaaaaaca aaagttttta acaaattagg aggagagtaa taatgaaaaa gagtttatgg 105660

agtacaatac tagaagacac agtactagag gataataaca actcaatagc atacgcaagt 105720

gttgttaaca cacatcgcaa gtttagacga ttactagaga atgaggacta tgaggctact 105780

attttacgta tgcttcttga aaggaaacaa gaaattgacg gggtgtttca aagcaacgtt 105840

gagtttagtc ccctaagtgt acgcccatca gcgccacctg taaaagaacg agggtacgaa 105900

atagttattg atgtgaagag ctttgtagcc gaagcattag atatgcctaa cgacctagat 105960

attaacctag gtattaatgg ctttgagcct gtctttgcta atatgctaca aaattattta 106020

aatgaaaaga tttttgaaga aaacgaggta atatatgagt ttgagggtag tacatctgtg 106080

gagcaatcta taaaagtaga gggtgacggt tgcatcgtag ttgtgaaaaa cataactcgt 106140

acttggtaag gaggaactta tgaatagaaa aacgtttaga cctagttggc ttggagaagc 106200

gtataagggg tatataccac caaaaaatga ttttcctgtg atacgtataa aaggagacat 106260

ggtttcttgg tttactatcc ttttagaaca aaatgaagaa tttgtacggg agtggatgtt 106320

aaagtataaa agaacaacac gtacaaaaga aacaggtgct cattatatag gggatagtga 106380

ctactccact tattgcgtga caacctacct tgttaccaca cgtacagcat cattcaatcc 106440

caaaaggctt gttatagctc tatttactaa agaactagct cttttttcta gggaatcgac 106500

atcagtaaca ctaacagaag aaaatctaaa tttaaatgag cgtattactg aatatgttga 106560

ggcattgagt gagtatctag agtctgtagg tatttttccg ttctctcttc ctagttggtg 106620

tcatcccgac tatcattaca catacgatgg ctgtcgtttt actatctcca agagagaaag 106680

ggaagtaata tctagcacta aaatgaactt agaagaatat aaccaacatt tactaaaaag 106740

caaaggagag aaactaatga aagtaactca attagaaaca tttaacgatt ttattgacta 106800

catggttaaa aagaatgaag aaggctggaa gtgggaaaac aaagaccctc tagtacctgt 106860

aagtttcgaa aggtggcaaa tagatagaga ccaaacactt atagctaaaa actttagaaa 106920

taaggttatt tatacagtta gggtggaaga ccttgaccaa acacctacaa ttgtatcata 106980

taagacagat gcggactggg tcggtagctt agacccaaaa gaatatgaat gggagacgat 107040

gaaacctaaa aaagtaccta ttgtccaccc gtgtacatta actgttgaag aactcgaaag 107100

ggattggaag actaatacaa aagagtatga cgaagttaat aatccttctc attatacgtc 107160

tgggggcatt gagcctatta aatatatcca gagtcacaaa atgaactacg aaaaaggtaa 107220

tgttattaag tatattactc gggcaggtaa aaagaaaggt cagagcgaga ttaaagacct 107280

taagaaagca atgaaatata ttgggttact gattgaagat atagagaaat atgagaatag 107340

ctaatttaat aacaaaagta atagggttgt ggattctgtg tagactagta agtgtctaag 107400

ggttcacaac cctaatttaa aatgtgaaaa caaacgaagt ataactgttg actttttact 107460

tatttcatgg tatagtaagt atatattaaa gaacaggagt gattacatga gtagagcagg 107520

ctacaaaatt cgttatgtaa accagagagg taagatggtt tgggttaaaa gtattgggtt 107580

tggaacaaag gcattagaag tcgatactac taccaacaaa gagaaggtta aagtattttt 107640

ggataagaaa tgggcacttc aagtatgtaa actcattgat ggcgacctag atgttaattg 107700

tatgatagac gacattactt ataagtattt tatcttctat gaagcagaag ataagaagta 107760

ctttctaaag tcgctaactg tagttcaaga taagattgag cctgaatgga cagaaaacga 107820

gccagaacga gcggttattc ataaatacaa tgatttagat agaattttag ggctagttaa 107880

cgatagaatt gataaggaag caagagcacg tcacgatatg tttttacttt aaacgaggag 107940

gtaataaaaa tgattttata tggagtgtct ctttatgggg aggtaactga gtggactaca 108000

gataaagcta ctgcaaataa atgggtagat aatgcagtac aatctataga ggataatgat 108060

atagatgagg actgttatgt tattttgtta agactagact tagaaaaact tctatatggc 108120

acattcgacc gtgaagtaga tttaaacgac caactgcatg atgaagctac agtactaaaa 108180

gaattacgtt ggcaggtgga cgaggaaggg ctatataata ttgtggaggt ggcggaatga 108240

ataataaaaa gacagattat aaagtataca aaataaccta caagcaacgt tttatggggg 108300

aggttcttat tgactcgtat gaaagagtag tgagggatga taacgaacta cagtctgcaa 108360

ttaacgcttt atatgatgac ccacatgtgt tctcggttag tagtgaggag gtgacagaat 108420

aatggagttt aaatcagtaa aagaaataaa tacagagatt agtttgttag ctgacaaagg 108480

aggtatctca gacggctcac acacatttga ggagctttac tttcatagaa tggttttatt 108540

tgcaacaata tgtaatgcta atcactctaa aagttggaag tctaagaaac atgaggacgg 108600

ttctatgttt gataactatt ttatcgttgg tatttctaca tcgaaaggta tgttttctta 108660

ccattatcac ttagacaatt gggactattt tagtgttcca gaactagaat ttgcccctac 108720

atgggatgga catacagcta atgatgttac tcgactttta gatatataag aaaatgtaag 108780

taaacaaagg aggtactact atggatttaa ataaattttt aactgaggta gataaagtta 108840

cttctgctta cggggaggat gcaaactacg agatttcttt tattcgtgag aaatatgcgg 108900

acttagacga aggtgccaaa caggactgtg aggaaacgga cttctttgag gaggtagaat 108960

acatcagtca gatgcaaaca ggagaagact gttttgaggg ctatattttt agaaaaataa 109020

aaaacacaga ttggtgtctt tgtgtcacgt atgctatctg aggtcttgga attactacta 109080

actttttagg ttggcttaaa atgtagtcaa ttagtaaaca tattaaggag gtaaacataa 109140

atgatacaag cttatagagt tcgttcacaa gaagattttg ttaacctaat ggttaactta 109200

gaaaaaaagg attttaaatg gtatagtggg ttacctcctc tagacggtgt atactacaga 109260

caaaacccag ataaagactt agctagtgat tctcttcttg ttgttctaaa ctatgaacat 109320

cgtactatta gatactatac agttgctaat ttaagggaag cttacccact agcaaccatc 109380

tatacgtaca cccctagtag aacatctaaa acaaactcag tagacggttt tactcctact 109440

cagttagtct atgacgaaga actttaccta taaatgtact ctacacaact aaaggcatta 109500

tacatgtctt tttttgttga ctttatttat cttatatggt atactagtat tataaataaa 109560

ggaggtgtct ttatgaggct atccctaaga aatatattgg aaggttttct tctattctgc 109620

ctagctttac cacctatcct agcagttatg ggatttttat taggtattat atggattaca 109680

ttttactttc ctatgtttgg tctagctttt tggagtattt taagtgcatg tttagtagag 109740

aacttgctag aaaaatggga taacagaaag actcttagaa taaggaactt tagtatgtgg 109800

tatgttatag ccttacttat tgctcttact attactggta tagctatact agtgctgttt 109860

ttattctaga acataaagag ttatgtctta agctagtttg gtaataaaat aaacataaga 109920

aggagaggtt attttgaaag ctttgttttt tgtaataggc attctagtat gccttttagg 109980

tgttgctatc ttagctacag caatactgtg gattactact agtcttcctg caatcggtgt 110040

cccttttcta actattactt tacttgtagg aatcctacta gttctgctta tagaaggaaa 110100

taagaaatac taaagaagta atggaggttg ttaaaatgag aaagatagac ataaaccaag 110160

caagtgtgga tttacaacta ctaatcgaat ttgagaatga ggactataca gtagcctata 110220

gaggagaaga gagtaggcat acagaagaaa tgaaggaagc aatcctttca gctcacaaag 110280

atacaataga ccgtttagag gtagctaacg ataaactact agagacttat gaagacctga 110340

tagaattgaa aaaagaatat cacaccacgt ataagaaaaa tgagaagtta gctgtaaaaa 110400

ataaagaact aaaaaaagag ttacgagata ttacaaatga aaaagaaaag ttagaaaaag 110460

agctactccg cataaaagtg tctaaggggg aactttaatg ctagtatccc ttactttttt 110520

agaggtactt ttatgggtac tgactttgtt atactttggt atggtaatct acgtactctt 110580

atcccaccta gttactagcg tagcttaccc cgagtctgat ggagttgcta gttcttttat 110640

agactatagt tatttatggg gggaatctga tgagacgagc aaggctagta actataaatg 110700

ctacgaccat ataaactaca gaaaaacgag ctattttaac cccacttata aaagcggtga 110760

atttctaact agcacagaaa aggtgtctaa gcataaaaga aaagtcttta aaaataaaca 110820

aaaagttaac ttagatgaag aagaagagtt ataaaagaca cataagaaaa ggtacctgct 110880

aagggtacct tttactttta tactattttt tgttttactg gctctactat gttaaattcc 110940

cctttatttg ttatagtgat agcattaagt tgcccttcaa aacagcaacc gccatctatt 111000

cctataagtt tttttctgtc gtgaacccac acgtcaaaat tagaataatt acggtctcta 111060

aacggtaagt aacaggtagg tgtgtgaccg aatacaatta cttttcctgt gaggttctct 111120

tgcttatacc agtccctaat ccacataaac tcgtggtctg ttgtcttctt ccagtcttcc 111180

ccgtataaag attgaatacc tgcatgagta aacagaacgt caccaaactc gtaatacggc 111240

ttagaagctc tcaggagctc tatatgatgt ctatatttct tttgtatgca aatacttgta 111300

aagtgataaa aagacgatag cctatgccta gaacgctcct cctgtggcat attattgaag 111360

tagctagaca aggtgtttcc tcctccacta gcaagccatc tgttaatagt gtactcattc 111420

tgctcttctt ctataaaatc aagcaacatt tggtcgtggt ttcctcttat aacaaccact 111480

tggtcaccgt actccatctt taaatcacaa ataatatcta aaacctctct agactcgtcc 111540

cctctatcta tatagtcccc acacaagaca agcaattgtt tatccctatc ccaattgttt 111600

aacatttcta aaaactctaa gctacaacca tggatgtcac taattgcaaa agcttctttg 111660

aatttagtca ccgaggcacc tccttaatca aaattaattt cttctttcca accttttata 111720

taagatgtta ggtctccttc gtaccccata agacgtattt ccattaccaa ctgacctatc 111780

gtagcatttg aagtgctcat accctctaaa acctctataa acggtcttag tcttctgtcc 111840

ataatctcgt ttgcagtttc tatagcactc tctttgtttc taccgttctc tatgagtaaa 111900

tttacgtaat cttcataagt catgttaaca tgggttacac cgtccaccac actagaaaac 111960

ctacagtatc taccatttgg ttgttgtgag ataaagctac tcatccttga tattcatctc 112020

ctctttaaca gtacttttta gccttttgtt ttccgctttt accttttcgt ataaccagag 112080

agctgtagac aagtcctttc gtgttacctc gctgttgttt cttacttttg ctagtagctc 112140

ctcatacgtt ttatctttaa agtccatagt agctccttct tctgcattgt cccaagcgac 112200

ctcgtaagca accattcctg tcattccttg catcacaccc acaatagtac ctatagatgt 112260

gttataacga tgttgtacca gttcaaactt tttaaactta ttcattctta tctatcccct 112320

agcaattatt tgtaaaagta attccaaaag attccatcct accatatcta acagaaacac 112380

aagctgttag gtagtcactt actcgtatct ttactttaac aatgtcacca tctataggag 112440

acttaaattt tatttctatg tcaactgcaa aatgattgtt gtgattaatc caagatttta 112500

aatggaatat tccttcacgc atctttgtta cagttgtgta tggatactct agtgaatgtt 112560

tgtgtatatt tcttggaaat tctccaaaca ttgcaattac ttcttttggg tttgttggtg 112620

ctaacatgtg atgtctattt tgatttgata ataactctct gccttttttg tcaagctcta 112680

aaaagtcttc tcctctattt ttcagtaaat caaataataa tttttctgca attacctttc 112740

cttctttttg tctttctttt ctctgctgtt tatcatattt cttcttttct ttaattatcc 112800

tattccttgc atgactaaat ttttccataa ttactgtcac ctcacatctt tatttactta 112860

taaaatctac taatattgcc aaaataataa gaaataatat cactatccaa acaaacacca 112920

ctattactac ccaaataatt attggaataa acactgtcca ccaaacaata ttaataaaac 112980

cagttaattg aagtattaat aaaactagga aagctacccc gcatactgta cttaatctca 113040

tacttattat tcaccttcca cactggcttc ttttaataat tttattgtaa cgttttcatc 113100

atctgttgct gttcccgcaa gaacctgctt aaccatctct tcaaaattct ctcttttggg 113160

ctcttcaaaa tcttcttccg tatacacctc aacttctact tcctttttag gaaccaaaag 113220

tagtccacca aattcatcat tttcgactcc ataagtcatc ttacctgctt ctacatcttc 113280

gtttgtttta attgccatta ttgcaattac atctttaata gtatccacca tattcactct 113340

cctattagtt tagtaagctt agccgggtac cacagttttt cattattatt aatatcccat 113400

tctattaaat aggcaaaatc ttcaccttgt actctagtgt atcattataa acttcctcta 113460

ctgtacctaa ctgtttttta ctctgtaaaa tgaactctac ttggtctcct actttaaact 113520

tcatgcttct tcctccattt ctaatatttc gtgagggtct acctttacaa aatcagataa 113580

agagtaccta aatacaaccc tgtctttttg agatacaagg gcttcttgtt tttctttttt 113640

atgaccccta caatgtttgc aaacaagaag tacatcacga gtgtaaatat tcgtgttctc 113700

tatagatttc cacttagaag ccgtaccctc ccatatgtag acgtgactgc aagtaggctt 113760

acttctaact aaactagcta gtttactagt gtatattttc atagtgtaaa cacctccaac 113820

tacattatat cataaaaaaa gaagagtgtc aataaacact cttactttag atgttcccac 113880

ccgtaggcac taaatatagc aattaccgct aatggtaaaa acgtaataaa ggacacacgt 113940

aagataaatc ttgagatagg atttctgtag taattccata gatatgttac tttaacatca 114000

tggctatcat ctttagactc taaataaata agtaacagga acagtgggta tattgctaca 114060

taaatttgaa ggattagtaa caccatctat ttagtatcct ccttaagaga caaattatcg 114120

aggaaggcta ctaagtcttt tttagctaag tttcctttgt gattgcctac aaagtccttt 114180

atagcagcca ccagttcttc gtgaagtctt ctgttctctt cgtaaacctc attctttacc 114240

gtccgctcta ggttacttct tgcactctct tctcttttaa gtaaaacgca agcttccccc 114300

tttagtctag tgttgtcttc ctttagagtc tctatctctt tttccttttc ttccatctgc 114360

ccgacaaccc tttgtaactc tcttagttgc cgttctttct cacccaaaat aacttcaaga 114420

gcgctctttt ctcctacaga tttccctagt ttgctggtta cctcttctag ttcagtagtc 114480

atatttttaa tagcttcctt atctttcttt tgttcttcat aagcatctac aatatattct 114540

tttaaattct tatatgggat ttctttctgt tctttttcct ttagtttgct attttttctt 114600

ttagaactaa ttacctctaa aaacttcatt cggattcctc cttcattgct tcaatctctc 114660

tatgcacttc ttcatagtaa ctagctgtaa aagttactct tgaaaactcc tcgtaagcct 114720

ttagctcccg ctcgtccctc tcagttatct gctcccatgt aaaagttttg tcacactcct 114780

tgcagtataa atcgtaatct aagtcataat tacattgtaa acactctact gattcgtctc 114840

cttcgtagtg catatctatt taccaccttt agagatacta tcgaactttt ccgctaaatc 114900

tgcatacata tctgataggg agtcatacca gactgttcta gagaaagcaa cagagtatac 114960

aatggctttt tggtagtaaa gtctgtcaac tcttaccgca agacgtctaa aactatctct 115020

actctcttct aggaaatatg tgttactagg gtcaactgct tcttttatca cagggtaaat 115080

ctctttaaca aaataagctt cttctttgga gtcaattccc ttttgagtca gtatacctaa 115140

aagaatacct gcacgttgtt ttacctcctc tggtgtatct cctgatgcta ctcgtttaac 115200

taattcttta atatcttctt ctgtacctac gcctataagg tgacaataaa cttctaagta 115260

gaatttattt actcctaggt gatattcttc gtcttcaaca tttttccaga taaactcttt 115320

aaggctttca gcaggtaagt ctagcatttt taaaacacag ctaacgttaa gggctctgta 115380

cttgtctaat aactcctctt ctttttttct atcataagca agggaagctg tatgtctgct 115440

aaatgtatct acgaaacatt ggtctttgaa aagatagttc agcttttctt caccagattg 115500

ttttacgcat accttaaaaa gtttatacag acggtcataa tcagcagagg ataagtaagt 115560

aaaccaatca gctcctactt taggtgtgaa ccctataacc ttataactgt tctcattaca 115620

aatacgaaac tcttgtcttt taattatact agcgtaaacg aaaaatttac ctttcttttc 115680

cacaccaaaa atatttttgt ctttaacagg ttctgtagtg accttaatat cccaacagga 115740

cttcaactcc acccctaaac cacctaagat actgcccgct acattttcta tcttaatact 115800

atcatttaat ttgaactgac tgtacttaag ccccaattcc tcagtaacta cttctatcac 115860

caaaggcatt atatcctgta tacgttcctc tgggttctca cttgacttgc attcaactat 115920

aataagaggt agccatatag tcccattacc cccttcactt gtactacccg catctctttt 115980

ttcataatta tccttaatac gtttttattt tagttcttct actatacctc tgtagtaaat 116040

gttcatcatt cgagtgttat ctttgatgtc tactaacgga atgttcttat actccttatg 116100

cttagccata ctattcctcc tcactaccta ttatttcttt gtagaagtcc tttacaaagt 116160

ctgcttgctc ctctttagga acaatactat taatttcctc ctcaaagcaa aatagaataa 116220

agcccactgt tttgatagag ccctcattat atagcttact gcttagaacc atgtagtcca 116280

acaattcttc tttcttcgta ttgtctctat gaagcttgtc aaaggtatta acagaaaaac 116340

caaaaggact cagctccttt ataactaacg gctttaactc accaaactct actttagggt 116400

tagttttact tccgaaagaa agctccaact ctttagtgta ctttttaaca agttcttcta 116460

cttctgtgta cgttcctacg ctacagttgt ttttctcctc ctgtgagcta tattttacgt 116520

aatactcttt actcatacta tcaactcctt attctaattg tatcacaaaa actctattaa 116580

gtcaacaaaa aaagaagatg ttactcttct tttaatggca gtctagtttt tagttttcgt 116640

gttcttcctt tgtatctgag attgctagta tcagtgtgta ctgaccttac ttttacaata 116700

ctctctttga cactatcata tagaaaatcc cctacagcta ccttacctaa caagctgtca 116760

tctaaactaa ataaatatgt tttagcatag gtacagctta ggtcatcctg aacatagctt 116820

atgtaaacgt acttgttctc ataatctgtc gctccccaag actcgtctac ttcccatcca 116880

ccaagtatag tttcaatatt ggcatttagt atctctgcaa tgcttctagc ttgcgttaag 116940

cttgtcagcg gtcttacctg attaggttct cgaacttttt cccagtttaa tacaaaagta 117000

cctcttttgt tcttaataaa tgatgttgtg tagtatgcgt atggaaaccc cttctggcgt 117060

agaacataca tatttctttt ttttaccata taaatcaacc tcctagtatt tttggtaagt 117120

cctctgataa gtttacagat acgtcttttc ctagtaaatt aagtaactct ttgatagctt 117180

cttctagttc tacaaagtac tgtggtgacc tatttcgact atcttgttgc aacttaatca 117240

cgcaatccat attcaaatca gggcatgaac cctctcttat tcttataata ttcagtttat 117300

tatttaacgc atgttcgcac ttctgtctat ctttttcagc attttggtgc caacgacctc 117360

catcatattc taccactagt ttcacagaag gtacgtagat gtcaaatctt tttcctgcta 117420

tatttttttc taatacaatt cgagggaacg ctttttttaa atagtaggca attatcttat 117480

ttggtttaga aacagtagag gaacacttag ggcaagattt aaaccctaca aacttagact 117540

tcacactctg ctgataagag ccatgctcct tacatttcca ccaaaccttt atgtcgcttc 117600

ctcgacttac gttcttaggt tttataccta tagcattgtt tttttcatag tcccatagtt 117660

ttcttagaac agagctatgg gactcactaa taggtataca ccctctctta agagctttag 117720

tagccatagt tatctcctcc gctctctact atatcttagg ttgcaaaggt ctacttctgc 117780

aaagaacatg tagtctacct ccgcatgaga catccctaag tctcttaact catatacttt 117840

atcttttaca gtttccctta cacgttccag ttctttaata ccttcggaag ttaggttacc 117900

ttccgcatct cttaatttaa tagccataga attttctctt attggagtta gttgctctcc 117960

taaaattgtc tgaatctgat aatatagcgg ttgcctttaa taactctcct tttgtaagcc 118020

agttgaatag gtcatcaaca gataccttca taagaacagc tagttgctcc ataatgtcat 118080

cttggtctgt atgacatgac ctgcttgctt ggtctagtag tccgtaacaa gcttttttaa 118140

gtatcttcat gggtttatct gagatagtgc tagaaggctc acgagaggat aaagaaacaa 118200

caccagagct tgctctacaa ctagtgcaca tgtagcacat tccattaccg tactccttgc 118260

catataaatc agcattagat gtgtacttta tattaccttg acataacggg cactttgtag 118320

gaatttcctt cttcatacgt gccaaccccc aatcgtcagt ccatcgaata acataccagc 118380

cgttattgct acagagacaa tcaaaatcat taaaattatt gacccccata aggtaagccc 118440

tctggtgaag aactccttaa ccgtaactaa cagccaaata aatgaaataa taaaaattat 118500

ccaagtagtt aatcccataa ctttgtctcc tctcggtatt tcttaatctt caatcttgca 118560

tctgatataa ctgttgacaa aggtactagt agccttcctg ccatgtaatc actatctaca 118620

ccaatgttaa tttggtcttt acttggacta ggtttactat gaatgtgtcc atgaatgttg 118680

aatctacttt tattacatcc aagttccata ggataatggg aaaaatgaaa ctctactgag 118740

tatgcgtgaa taatcatacc aatatcgtgt acttctacta cactagggca taggtcacta 118800

ttgtataagt ttactttttt tcgtgtatca tggttccctc ttataataat ctttttacct 118860

cgtagagcat tcataatttt aaccgctttt tcttcgtcac ctaatgcaaa gtctcccatg 118920

aagataacga catcattatc attaacagta tcgttccact ccattataaa ctgcgagttc 118980

atgttgtcaa taaaaacgta tttatctcct ctataatgtt caaacttaag tatgttatcg 119040

tgattaaaat gtgtatctga tgctaagaaa acattgttag aacttgaaat gttatattta 119100

accaaaaaat cagctagact aatcaattaa aaacacctgc ctctagaggt acttctttat 119160

aacctcctgt ataaaaactt aagttgtact ccgtcataga cttatcttta ctatatactt 119220

ttactacacg attactggtt actagaatac tagctacctc ttttttacct actacatgtt 119280

tatgctttct tccgccacca tctgtgattt ctacttctag taattcacga ataccgaaca 119340

taccaaacaa ctcctatcta ttctataata atattttttc tgatttctaa ccagtctacc 119400

tctttgccaa ctgcttttaa agccttatca tcaatgtata agtcagcgcc aatttttcta 119460

gtatcatttt tccatattgc tatcttatct gggttgtttt catttacaaa gtggtacggt 119520

atactgttat tggcaagaaa atccaaacac aaatccatac caagctcttc tctacatgtc 119580

caaatagtga taactgcccc aagttcaaca agttcttcta ttgtctcttt agcataaggt 119640

aggagctccc ctactaaggg gtagctctcc tcctgctcac tagcaatagt tccatcaaag 119700

tctatagcta tatacttata gcgtaaactt ctcatctcta cgcctttggt ttgctatctt 119760

taaattttaa ctgctctttt gttgtagatg gtaaacgata agactcatac aagtgcgggt 119820

ggtctttctc taacttctta ccgttaaatc gtaaaccttc cacctccgct tgataagtta 119880

gttcaaaatc tcctaaaatc tctaatgatt ttacgtttgc cttcaccatc ataggcatta 119940

attcatcttt aagtgcatct aactgttttt ttaatatctc gtagttacgt tttgttgttg 120000

cataattttt agctttgtcc tgcaaggttt tagtaataga cacccctgca ccctctttaa 120060

gttctaccgc ttgtaatgca ccttgttttg ccatttataa tctcctcctg tcttatttat 120120

aaattaacta taacacatat tttattataa gtcaacaaaa atgtatattt tttatttact 120180

atcctctact tttaggatta tttctctgag atgtactgga acactacgtc caagagactt 120240

tttagctgta tcctcatcgt aaacaaaagc taagtcatct ggtgattcct cactcataat 120300

atcatcatac acaattgagg gtactacctt cattagttgg gaaaagttag aaaattctaa 120360

gttaggcaac ttattttcct cttctagtct ctgtagagct ttcctaaccc tagtctcagt 120420

gacgaattgt cgaatccact cggtactttc aggtaaatct ttaacttccc tttttctaga 120480

ttggactctt tctgcgtgtt ttggggaaac acttttaaat agtttctggc taccatcgat 120540

gtctacttcg tctaaaaatt taacaataat accttctcct tcattagttt ccttagtaaa 120600

tctggactga cctacaaatt cgttaactgt atcaaagtca gtaaataccc ctaagtaaaa 120660

catatctggc ataggtagat ttagtgtaaa agcaatacta cgtacctcat ctaaaggtaa 120720

gtatcgccct tctacgttac tgtacacgtc aaataagtag aagttatttc taaattgctc 120780

cttatacggt gtatttaact tcttagaacc tagccactca ccaaagaaag ttaggtttac 120840

tagacctgaa tggacaacat caggaatact taccttgtct ttaatgtaag tagtgaaacc 120900

tgatagtgtg tttacctctc ctacttcatt aggaatgtct aactctatat ttcttgagaa 120960

tttacgcata gcatagccac catcaggaag tttctctaca aaaaaactag cgttgctacc 121020

atctaacttc tctagaatga taatttcacc tgtatatgtt tttgccattt gttgagctct 121080

tccaattctt tgaatttttc tatatctatt catattaact ctcctcctaa atttttaagt 121140

ctttgaacga aatattatct agattgctgg gttcaagctc tcctgtacga atataagtag 121200

tcatgagctt gctgtctaac tcagtatcct cttttaataa ttcttcatta actttattta 121260

taagaatgcc aatatctttt cctttagcgg gaataggtat atttaatgat ttagctatct 121320

tgtctacttc taacttaatt gcctcaggga gagtttcctg ataaggcaag aactcatata 121380

gtaagccaat ctccataccc aagtcatcag cttcactctc tgttctagca aaatcaattt 121440

gttgacctat ttgttttacc tcctgcttta ctgcttggcg tacctgctca tcagtaagtt 121500

ctgatacact tttaacttta acttctactc gcatgttatc aagtctagca attaaatttt 121560

ggtatacatt gatgaaatca cttttacttt ttctggcatg tataagactt tctttcactc 121620

tttctaccaa actagttcct ttgtcttttt cttctaactt cataaatttt ccctactttc 121680

tctatattct ctttttgtat catcttttat aagttttctc tcatatctgt ttcttagttt 121740

tttgtctctt ccttggcagt gtggaaacat cccatcctta gtgaagagta gctcctcgta 121800

cactctatta gccttattct taaaatattt atttgtagat tttttactca tttagttatc 121860

tctcacacgt tgtaaaattt tacctaacaa gtttgaacct ctccataaat ctttgtttaa 121920

aatacgaggg tcactttctc gtagtcctac gccccatata gtgtcataag ggcttgcctc 121980

tactaaagta gttcccttag tagcttctag tttatctttt aattccttat tttgagagaa 122040

cttagccatt aagataataa aaacaatagc ctctttgtta tcatcccacg tagcctcgtt 122100

aaaacctcta acttttctac ccagagattt aactgtttta gggtctccac taggaacgga 122160

tagaatttgt gaagccacta actcatcatt gaatagacgt gccttctcat acatgaatgc 122220

ctgttctgca ttatgaaaca taatagactt gttagcttct ttcccttctt gcttgtactc 122280

taaagaaaaa tttgatgggt gccaattact aaagggcctt tcctttcccc aaaaaaatgt 122340

gtatctatct ctcattttat aactcccctc cttgacttag taaataagta ggcgtttctg 122400

tgttagccat tctgcttact cgggtagcta atgtctcaat aaatttttta ttatcaccat 122460

aaatattaat agtctgcgct tcttttttag ttagtgtagt gaactttgta gttttgtcaa 122520

actgtacaaa acggtaataa aatttttgtg cttcttccac agaactagct aatacccaag 122580

ttttttcttt atttcgttct gaaatttcta ctactaattg tttcacgtga aacaacctcc 122640

ttatttattt tagtttgtgt tctcttattt aattaatgcg ccttaaaatt atagattggt 122700

ttaacaatga agtcaatgtc aaccgtatct gtaatattct ctatgatact ctctttcttt 122760

ttataggcaa atggtgcctc atctagtgta gactccaaca cagaggaaga ccaaacatct 122820

ttcattgtat tcttaaaatc ttccatttct agtgtttctt tagctttaga acgggacagt 122880

accctacctg ccccatgagg tgctgaattg ttccagtctt cgtttccctt tcctgtagca 122940

ataatgctac catcacgcat atttagtggg ataactagtt gttttccttt agaggcatct 123000

gtcgcacctt tacgaataac cttattaggg atgtcaatat aattatgaat agagtcaaac 123060

tcagaaacta ccaagttttc ccaacccatt ttagacataa ttactttatg aatagctttt 123120

cggttatcct ccgcaaattt ttgcgcaata gccatatcat ttagatagtc ctctactaat 123180

ttaccgtcta gataagcaag gtctttatta aaaggtgctg tacttagttt taagcgcaat 123240

aactcttcct gtatgtcttt ttccctacct tgctctttta gagctctaat gagctcctgt 123300

ggcttctcag tgttagaaca atacttaaca gctagttctt ggtgtgtttt agctacttga 123360

acgcctaagt tacgggaacc tgtgtgtgtc ataaggaaga ttcgaccatc gtaggcacct 123420

agttcaatat agtggttccc tcctcctagt gtaccttctg ataaagcgat tctatttttt 123480

acctcttctt ttaagtttcc atagatagtc agattcttaa gtgacttact taccttctcc 123540

tgatttagtg ctactcctct tacggagttt cccgaaggaa caaactcttt aataacacta 123600

tctaattttt ctaatgtagt attgtttacc tctaaatcta attcggttac tcgtattccg 123660

caaccaatat ctactccgat aaggttaggt actactttgt tagtataagt catagtggtt 123720

cctacaacac accctttgcc tgcatgaata tcaggcataa atctaacacg ctctccttta 123780

acaaattcct gacttaacaa ttctattact tgacccacag cggtttcttc tacctcacta 123840

gtaaaaacct tagctgtatt ataccttcca agtagttcta tcattctgct cacgctcctt 123900

ttattatact tcttactaaa aaccattata ccatatgtca gtttataagt caacactaag 123960

ttaaaaaaag aggaacacgt atgcccctct attttagttc tttaaagttc ttagagaaga 124020

actcctcata atattggtct tctgtagtaa ctttcctacc tgagtctgat actactgtta 124080

tatctcctac tagaacaccg tcttctgtga tactatttac agtaaacgtt ctgttcttgt 124140

tacctttttc atgaaatgtc ttatttaaat atttttttcc ttttttagtc atacataaca 124200

tcctcttttg ttaattgtgc tagtgcttta caatattttt ctttttgtga ggcaaaagca 124260

atagtgttat ttgaaaaata gtctagaatc tctgctagtt ttactttttt agctagatta 124320

tcctgcttca atctctggat atagcaagta tacgcctcat tttgaatgcg tgttaggtga 124380

actaaacttc caagtaaata atccttgtcc actagagata aaaaagtttg gacatcacta 124440

aatctgacgg cggtatcctc aaatatatca tgcagtaaac ttaaataata agcatcttca 124500

tctttgtctt tcctaaagtt ttctgctact cggtacacgt gcgctagctg attttcctca 124560

taattagcta aaacagtctt taaatgatta cttacgttgt ctttaattgt aaacaatacg 124620

tagcctcctt taaaaaataa ttggtagaac caatagtaca catattccta gagaaacaag 124680

taataacata atagtatctc ttactgaaag taggaactta agtttaggta catatggcat 124740

agatacccct gctctgccta aaagattaga agcaataggc acaaggttat actcaataag 124800

aaaagctaaa tataaataag agaatattaa aataaaagtg attataatcc ccatagatgt 124860

ctcctctcta ctcccctaag gagctatttt catgcttatc tattttaaaa agctcactag 124920

aaggaacacc aaaatatttt tctagtttca ctcgatttct ctctctagga aacttcttac 124980

ctgtctccca ataagaaatt agggagtagt taacatctaa atcctgcgct aattgtcgaa 125040

tagacaactg ctgattctct ctaattcttc ttaaatttct tactttccca atatcttctt 125100

tatttgtcat tttttttttc accccttact ttttatatat ttattataac ataaaagtca 125160

acgtctgtca agcgtacaaa aaaaagagca cgtattaggt gctctctagg ttactataga 125220

atgctgtcaa taaagctgta ggcttcactt cttaaaacag ataccataaa atcttttggt 125280

gtattttttt ctaaagacat tacatgttta aagacagtag gtgtaattcc agatactaaa 125340

gcaactccgc tttcagaaaa ccgaactggt gagttatcat tgcgggcatt tacattccaa 125400

aaagttactg ttggaagtaa gtaacctgct tttgaaaact tacgtctcat actttcgtag 125460

aaagagctgt cgttctcttt ttgtgaacta cactggtcga actccatgtc agagataatg 125520

acaatatcag taggtagctc actttgtggc agtttgctct ttgttgcggt attcagaatt 125580

aagttaaaaa cagcctctag gttagtggac atgccccagt cgctactaat agcagtagaa 125640

acccgctccc gtaagcttaa ctcactagga acatgaacta aagctggttt agaactgaat 125700

gtaataaagt ggtctttgaa cactccattt agtctctctg cgcagtatag ccctaaagaa 125760

atagaaacgt ccataggtga cacttctttt gccccaccat acatagaacc tgatacgtca 125820

gaaactacaa tagcatttct agtcatattt tctacgtagt taggtagtga tttccaagtt 125880

gcctctactg tgttgtctac agagtcgtcc cagtcaccac ttgcggtgta cttcttaact 125940

aactcataag gcataatgtt gctggcattt agttttttat ctgtggagtt tactctgtta 126000

ataaacattt cataggatgt gggcaggtgt tttctaaaag cccctctata tttaaacagg 126060

gcacgagaag gtaacttgtc aaaattgata gtatcgtaat ccttgtcggc taagctagtc 126120

tccactaagt ttaactttgc tcgtaaagcg gaaagtaatt tacgatactc tcgactggtg 126180

tagcctaggg aagcagacaa gtctctagct gtatgtttct tctccttgtt tggtgaagac 126240

tccgaaggta accacttagc taataaagat atagattcat ctgattttaa gttcttttta 126300

tctagttcta gttgttcaga aattagttcc actagcacca ttgttaagac tttgttttta 126360

gtttcacaac tatagcgata tagagataca acatcgtccc aacgaccaat ttcactcata 126420

aactctgtaa acttaattag agtgtctaag tcaagctcag ctagcccctt aatggactcg 126480

ttatacactg ctcgttcacc taagccacta agtcggatat tacgaagatg tactaagttc 126540

ttgagtgcta gctctcggtc ttctatatat gccttcttaa ataacagctt agcatcattt 126600

gttctatcac gcattgctcc tgctcgtgag aaaaaatcta agttatagtc taaagtagtg 126660

ctaaatgtgt tagccccctt ttctgtttta cttaaagtag tctcttcttt taatttattt 126720

aaaaattctg acataataat ctaccccttt tcttttttat aatcttatag ctgacctaag 126780

ctatattcag gtcagctatc gtatgtacag gaagcgtttt tttctcgtac attttttttt 126840

tacatggtta gacaccatgt cgtactaact gctaatatgc gctgttttag tttttagtat 126900

ttgctgtgtg cttcctaatt aatttttaca gagagcgttt ttactttttc aaatagtata 126960

ggttagtaat tgctgttagc tctctatttt atattctgta gattattttt ttatcacagg 127020

aatcattttt atattttatc caataatata gtagtaattg ctgttagatt cctaagttta 127080

ttttatctta aatttatgat aacatatttt gtctagtatg tcaacatctt tttacatttt 127140

gtttttgttg tcattctgcc agttttccca aaattgctct tttcactttt ttcggctctc 127200

gacaaaacct tgtgtcttct tacaatatgg gcaatacata tgtttaatat gaccgtcctt 127260

cttttgctta gctgaccttc tgaatataga gactttttcc ccacactcag ggcatgttaa 127320

cgtggtttgt actgtcttta ccatagttat aaccagattg ccccgttggg aatcgaaccc 127380

aaaccgagga gttatgagct ccttgctcga ccttctagct acgggacaat gagggaaact 127440

tctcttagtt agagaaggct ccaaaatgtt ttagttttcc accgagtgcc tttgtcttgt 127500

ctgatagttc tatttttgat gttagacgag acttaccttc tgccacgtac agaaaatctt 127560

tctcaacagt aactaacttc atttcttttt tgccagtctc tttgttgtat cgttcctgta 127620

attctttaac aggttcggaa attacgccgt aaaatttttt cattgtaaaa caactcctaa 127680

ttttttattt tttaactaac tttgtaattt aataatacaa taaaagaaat ttggagagat 127740

tccactctca actacttcgt gcatcaatat attcagtcac ggctaaacta gattcttggt 127800

aaggagctac cttgtcctcc atccagtctg tcaattaaga ctaagtttaa acacatcacg 127860

ttggaagtga ttaaaaattt accgacagga ctattttacc tgtacatttc accaagcatg 127920

gaagagtata tttttattaa taccaatttt aaagatgtag attttggcta ctttgactcc 127980

catcatccct tatactcggc tttcaccgaa tcaactacat aaatgacttt tagtaagtag 128040

ttttaaaaga gtacttacat tcccttctaa aacgcttcat cacgggcagg taggatactt 128100

ttatcttata acacacgtag cagtaccttt gtctcacact aaccataact atggctcagt 128160

ccccaacgca tactgatatt taccacatat actaatcgag gcggaaaatt taagtacatt 128220

agaagagcta ccttcttgtt atgttacaaa tttcctttat tctattatca atgttcttag 128280

taacactata ccatagattt catatagtgt caagtaataa atcaaggtgg taggattcga 128340

acctacgact tcccgtctcc aaagcgggta ctctaaccaa actgagctac accctgtcgg 128400

ttccgtacac aaacctttta attgcacggc atattttttt gactagctac ggacgtctag 128460

aacgccagcc gacatctcct gtatcagaga agatgaagca gatatatagt attatctgta 128520

gtttaaggta tgaggatact agaaccagaa agaagcacct ctattagttt tcctgttcct 128580
atagtatcgg aagcaaaacc gaagttatag gtggatttcc tatctttttt actcgttgta 128640
actcacttta gttaccgttt gcttttagcg gtgtgagtgg taatctcttt actgtacacc 128700
tagtgtacca cactttgcgt ttattgtcaa cacttatttt ctaaaatttt tcctgtacca 128760
attttctatc atactattta tatagttatt catgtagtag tcagtaacta taataccctt 128820
gtctgctgct aatgtatact catgaacccg taatatatag taagtactta taggaagtac 128880
aacgttacct gaataaggtt tagtagttct ctctacatgt tttataaatg actctatttc 128940
tttcatttct tgttggaaag ataggtattt tccatgtcta gtcctaaaaa ctagtttgcg 129000
gtaaaaccca ccatcaggga tatgctcttc tctagatatt ctagaccttt gaagtttatt 129060
caaccttatt ttgtctagtc tcctctcctt tgaatgccag tccgctttac tcttgggcgt 129120
cttcttataa cttctgctca taggacacct cctaatgatt atctacctag tatttctctc 129180
tgatagtcat tttaccattc ttttcttctg ctttacgaaa attctctcta actttagcta 129240
ctctagcttg ctcttctttt ttctcttcgg gtgtcagctc cattaaaccg agaactttca 129300
tagcatggtc atactcttct tgtgagttac acactactgt atgtttaaaa ttaatctctg 129360
tcaatattga agcctccctt gttgataatt atgtacctat ggctgttaac tcctattcat 129420
ttaacttcca gtctataaga agcaccacag tttgaaacag atttataagg ttcctcaacc 129480
tacatataag ttaactttat tatgtattta caaagtcctc agcaggattc gaacccgcgc 129540
taagggagta gaaatccctt gtgcatccgc tacaccataa ggacacgatt ctcctataca 129600
cacagccccg ctcttaacca gatgagacgt aacggtcacc gaagcaaccg taagggagtc 129660
gaaccctttc cgagctgtgc aaaggaaagt aacataggtc tcttattatg ttacttagtc 129720
tagtgtccgt ccacataggt tgagactacc tcgggacacg tccgcattct gttaattcag 129780
atttatttag tattgtgttg caacacaacg ctgggatgac taatccctaa agcgggtagg 129840
gggattcgaa cccccactac atggttggaa accttgggta ctacccttat actatacccg 129900
cagaggcaac ctacttaggg caagtaagtt accaatacaa aaggagtaaa aattaattaa 129960
aacaaaaaag cgtatcctgc tggatttgaa ccagcgactt ccgacttaac aggacggcac 130020
tctgacctct gagttaagga cacaaaatac tcggcaagga ttttcacctt gcaataacct 130080
tcatcattcg tttcagctat caattcaagc atcggatatg ctctcagctt acttctatta 130140
taggttatac cagtcctact gtgcgatatt ttttccgcca cgagtataaa aaatagtaat 130200
ttaagtacaa taggtagcaa gtttttgtaa gtgctagcaa gcgcaatact gtaccactca 130260
gtattaaaaa aggtgggata tacaattgta atttcatttt actgtgtttg taattgttat 130320
cgtacatggt ttcatacaca ttgctattga gttaaataac ttgctaccga ggtaactgta 130380

ctatttttgc ctgctattcc taaagctctt acaattgaca ggtaaggagt cgaaccttaa 130440

taactaccac tagcttgtct ttaatactta tcggtgcacc ttatagtaga cttagggtta 130500

ctagacaaac taaagtatac tacatacttg acttactacc cgacatttga agggaggtta 130560

gaaattgatt ttagcctctt tttatttgct gataatagtt ttttctacta tagcagtaag 130620

gtgcaccgtt tgtttgtctc accacatagt aatcctaatt ttaaggaagg taagggattt 130680

gaacccttgt accctgtatt ccaagggcaa actagttttc aagactagcg tattaccaag 130740

ctctaccaac cttccatttt gttgctatgg agtccgcagg tatcgaacct gcctctcctg 130800

cttttcaagc aagcgctaat ccatctcagc taggactacg ttaatggagc cactaggatt 130860

cgaacctaga ataacggggt aaaaacccgc agtgatagcc atttcaccat aactcctttg 130920

tatagaggtt agagggattt gaaccctccg taactagcta tatagtctag tatgtcgtca 130980

tttcatctta acctctataa taacattata acatacttta agatgtttgt caactgtttt 131040

acagggatag taggacttga acccacctca ttggagttgg aaaccaatat tttaccagat 131100

aaactatacc cctaagctgg tctagttgga ttcgaaccaa cgtatcacgg aatcaaaatc 131160

cgttgcctta ccgcttggct acagaccaat agtaccaagt aagctatgct tgtagtagga 131220

cttgaaccca cgtctgaggc ttacaaaacc actgttctac caattgaact atacaagctt 131280

atttttttat taagtcagct ctctaagtct ccgctcttta cgttagtaat tattagctga 131340

ctcgaagagc taacctatta tgcgccatca aggactcgaa ccttggaacc ccgaagggag 131400

gggagttaca gtcccctgca attgcctcta tgcgaatggc acgttgtaaa gctagagtac 131460

atacttcttg cttattaaga taccctagct aatttttact acttgttata tacttcccag 131520

tcattagcaa atacatctaa gttgctaata caccacatta catgacttcc agtagctgtt 131580

ctcatttgca agtaaggttc tactttaaat agtgcacctt cctctaactc ataagcatct 131640

gctgtttgtt tattagcagg gataccctct ggatagcctt tctgagctac tacaaacatg 131700

tctttaccat tccaacctgt tctgctagct ttctttcctt ttttaattaa aggtaaaatc 131760

tcttcgaatg tcataatttt ctcctccttc tatgtcttat taataccgag agtgggattc 131820

gaacccacat atccttggga cagcagattc taagtctacc gtgtatacca gttccaccat 131880

ctcggcaagt gtttctacta gtctttgatt acattataat agttagcata atgagtagag 131940

ataacaaacc cacctcgaat gtttaagtat ggagggattc tttctaagtc ctgtaaatca 132000

ataccatcta ctaagtaacc gtcattaggg gcatctgtta atggggatac agcaagagag 132060

ttgtctaccg aaatagtttt cacgttagga tattttttat caaaaatagt tttctgaaac 132120

tttgtgctta caataacaga gtatccaaat tcctcagcta tctccattat agcctgtgtt 132180

tttccttttc tcctatactt aacagtactg ataaagaata gaggctccat tcgatacata 132240

gccttcttaa atcgccttac ttctttactc tctttaaact tagacttttt tactttctta 132300

tgagtgttct tttggttcag catattcatc agtccctttc atttttattc aagcgcacgt 132360

aaggggaatc gaacccctat ttctcgatag acagtcgagt ataatagcca ttatatcata 132420

cgcacagtac tgaggtactt ctagctaaaa agttacctca gagtttaact tggcaataga 132480

ctggtcggct gtgtttcttt agctaaggaa gccttctacg cactattagt accaacactt 132540

ggaattgaac caaggactac cgattatcgg tcggttgctt tgcctcctaa gctatgctgg 132600

tatagtatac tctctcgttg actactatac gggggaatgt attaatagga acgagagaat 132660

acctattttt aattatacca cactctaaca gctttctgag attttaatct cttgggtttt 132720

cctttttcaa ggtcaaggtt tctggtttag tgtgtctact acaaaccttt tttcgaaagg 132780

agagtaaggg attcgaaccc ttggtactat tactagtaca acgagttagc aacccgtatc 132840

attaagcctc tctgacaact ctccaagaaa caaaatttaa acttatattt atattataac 132900

ataaattagt acgtttgtct actaataact tatttttttt cataaagcgg gtaatgggaa 132960

ttgaacccat atacgtagat ttgcagtcta ctacctttcc agttggttat acccgcaagt 133020

tctagaagac taataagact cctagagttt ttaaatattt agtgaaggta aagatataaa 133080

cctgatgata agaaaaacgt gttactaaca atcgtctaac ttacctgtat atacaatata 133140

gcatacttac tgataaaaag caacagtttt ctataatatt tttcatactg agctaagaaa 133200

atgtatagca accctaaaac tatataatta tctgacaact gtcctattta attttattcg 133260

tcccctatat tatattctaa tatattatat aattatttaa agatatgtaa ttagacttag 133320

aatataaaag gaatataata taggagaacc ttaaaattaa ataggacaat tatctgaata 133380

ttttataacg actactatta cttgagtaac aatagaaact tgatactagt atcaaataaa 133440

aagtaattta atattgatag taacccacct caggtaccta aatatattca gaaaatttat 133500

actactgtcc tatttaattt tatctacctc ctatattata ttctaatata ttatataaag 133560

actataataa attattatta ttattattat ttataatata gaagagtcct aaaattaaat 133620

aggacaatta tctgaatata aagtttaatt aacataaaaa gacacctgta aaggcatctt 133680

tattaaacat ctaaaaattt cttttcgtag gatactacaa gtgaggcatt tacataaact 133740

tcttctccat ctaatgcttt agtagttcta accttaataa atggtaactc agaacctctt 133800

acagcagagt ttaacttagt actagggtca gtaacagtat caaatacatc ttcactgaaa 133860

agataaaata catctcctgc aatagtcttt agttctaaaa cagtttgtct tctagccatt 133920

tttattgttc ctcctctgtt atgtggtata attaaagtat atcacacatt acttcttaac 133980

taaactgaaa acataggtaa ctaccgcacc tagtataatc ataaatactt tttctactaa 134040

gtctttttta ccttcatctt ctttttggtt cgtttgtctt agcgcttcca cacctttctc 134100

taattgacct atctgataga agatgtgtga ctgcttttca gattgtaggg cagaatcttt 134160

atctagagcc actacaatat ctcgtaattc cttgatagct tttgttagct cctcattgtc 134220

agtcttacgt gttagttcac ccgtttgtac tttatcttca aggtttttta ttttatctag 134280

cagactttga atatttgtat ctttttccac tcacatcctc ctcctctcag ttctttattt 134340

ttggtagggt ttacgaatgt gttctgatac atccgagctt tcagggtagt tatctaagaa 134400

gttcctaatt atatcttcat aatcaggtgt tacaattctc ccgtaatgtg taacaaagtt 134460

taggtgaaag tcaattctac cgtaggaacc taggtcaagg tacttaccaa ccatgtcgtc 134520

ttcatcaact ttaaaataac caaagtcaaa acctataagg gttcccattg atagagccct 134580

actaataaag tagatatgat attcttcacg attttccgag aggaagttac gtaatatatc 134640

tctaaattct acttttagca tatccctgta ttctttcatt gcagactcct ttctttacat 134700

actccatgca accactacta taaccataaa agatacaact attacaaaat atataataaa 134760

ggtgtaaact aacgacataa gtagtacaat gttagccaca cctgctataa acgttgctat 134820

aattagcgta aagttttgcg taataccttg tatatctcct agagaaacat ggcgttgcaa 134880

gtcacttata acgtggtatg aagtccaaaa aatacccact tgaaacaaga cagaggctag 134940

tacagctata aacattatac tcatagtaag acaacctcct taaagctagt tctaatatag 135000

tatacgacat agaataattt aaagttag 135028

<210> 3
<211> 135029
<212> DNA
<213> Unknown

<220>
<223> Bacteriophage P200

<400> 3
aaggttgtgc taaattaata cagtagaact atgtggaatg gaggaaatat tatgaacaag 60

ccagacaaaa atttctttat gaaaatgatt atcgttattc tatcaggatt acttgtgtta 120

tttgttataa acctagcatc tatctacttt gggtactctc ctcacgttgt agctacgtac 180

tttatgacag ggggctttat tgcgtatatc gtgataaact tatacgaccg aattgttttc 240

caaaaaggtt ctcctaggga agaacgagag aaacaagaga gacaaaagat agcatctaga 300

caaaaagctt tagaaagcgt agtaactcaa cgactaggta tccctataga ggagctagat 360

ttagaagtac tcattcagat ggaaaaggat ttagcagaca aaactgctaa aggtaataaa 420

aatgaaaaaa ctgagtagag acgagataaa gaaaagacga aaatatttca ttaacgaagc 480

aaaaagagtt acacatatac gagcaggaga ccgtagaata gcaacaggtt caagaatatg 540

taccttatgt ggtagaagtc tatcatctca gaaacggtct acaggagaga cctttgcctc 600

agtaaaccac atccattgtt actttaatga acttcttatc gttaacttat gtgaagatgc 660

aacttcttgt tatagaagac atgaaaggag ttagatagta tgaatttaga caacatagaa 720

agaaacttaa gcaagaaaaa aactacagga gatgccgtaa aggatgagtt caatgacatg 780

gctatgaagc tactagcagg ctttaaagca gatgtttcgg caggagataa aaaattagaa 840

gatgtagcag acatggctag gctgttacaa acctttatac aagttaacaa agaagatagt 900

ggtagcggag gaacatccac ttctttacca gagcttagta gcttgcaaca agaagcaata 960

accaatcaga taaagacaga gagcgtagtt tcaggtgata aagagcaaga actagtagac 1020

ctacaggact tagctaacat gacagaagag gacgtacaac ggatgataat ggataaagaa 1080

aaagcaatga acatggaaaa tgaggagacg ttttaatgga tggcaacact cttttaaaga 1140

tggcgcaaga aacttttcaa acagaaaatg taactacaga gcagttaaac tatgttctgg 1200

atatgctaac tccatcaaca tatttactaa gaaaccacaa agtaagaaat cataacatta 1260

cgttctctat tgcaaacaag tctactgaaa aggcgcaggc acatcgacct tggcaaccta 1320

agattataaa tgactcccac ccgaatgtag ctgttattaa gtcaagacaa ttagggttaa 1380

aattagcccc tctataaagt aatttataaa gaaaactctg ttaaacgggc ataggaaaat 1440

aaccaatatc ctgataagag accctaaatc ctaagtagga cagtgggaat cccgtgctaa 1500

atcaggcgca agcttgtaaa tgcctaacga ctaaatttct aggtagctaa ccaaatgggg 1560

ttagtgagaa ctagataaga aaatatattc tcttacgata ccaactgaaa aactctatca 1620

gatgttaaaa aattcttaga agaaaatata aagtaaagca gagcaaccta aaccaaaaaa 1680

ttaggttgat gatatagtct agtcccctaa taaatatcgg gaaaccgagg gtataacgta 1740

agtgagatgg gtgtagggag tatgattcat tttgcagata cacacagcta tgcagctgtt 1800

aaatgtttat acacgtttcc tacaggtgaa cagatgcgta aatttgtaca gtcacggtta 1860

gaccctgtac ttgagtctag ttactatagt tctattctag acaaagataa caacagtctg 1920

aactacaaaa gaatacgaaa tagtcacctt tactttagaa catctagtaa gcccggagct 1980

gtagagggtg tagatattga ctacttatcc atggatgaat atgacagagt tcctgttttc 2040

tcagaggctt ccgctttaga gtctatggct agttcaccct ttaaaattgt tagaagatgg 2100

tctacaccat cagctcccgg tatgggaata caccgattat tcgaaaactc agaccaacat 2160

tggtacttgc ataaatgtga aaaatgtaac cactggaacc aactagacta cgaggactac 2220

gacagtagct ctgtagaagc aggaggaaat atactatgtg taaatccaca aggtgttgat 2280

accttagcta aaacagttgt agaaggttcg ttccaattcg tatgtaaaaa atgtggagcc 2340

ccattagacc gttggtataa tggtgaatgg gtagcaaagc accctgatag aacaaaaaat 2400

ggagacggta ttagaggata tatgatttct cagatgaatg ctgtttggat aagtgctgat 2460

gacttaaaac gtaaagaact aaattcactt tcaaaacaag cattttttaa ctatacacta 2520

gggtacccat tcgaagatgc taaactagca gtttatagtg aagatattat agaaaatcca 2580

agcccaagag taaaggttcc aatgcacagc agaggagatt acaagtacat ttctgttggt 2640

atagactggg gaaatacaca ctgggtttct gttcatggta tgacggaaag aggagaaata 2700

gacttgataa gactgtttag tgttgagaag agtcgaggag taggtaatat tgagtctgat 2760

ttagataaga ttatcctaga agtatctatg tacaaccctg atatgattgt agctgacgta 2820

ggggatagcg ggaactacgt ggacaaatta gtaaaacatt tcggagaaga ccgagtattt 2880

ggttgtattt ataagtccag ccctaaatct acaggtcagt tgaaaccgca gtggaatgaa 2940

gcagggaatg tagttactgt agacaagtta atgcagaaca aaagatatat tatagaaatg 3000

aagacaaaga aagttaactt ctatagcttt atagacccta tgctaaaact attggtagac 3060

cattggagaa acgttgtaat acaagacgaa gaggacgaaa aagatggaac tttctaccaa 3120

gttattggca gaagaggaga cgaccattta gcgcaggcat cagtgtacag ctttattggt 3180

ttagacagac tagctgatat gtatttgaga ggctctaagt atgagttcaa ctctactttt 3240

gtaacaagca actaccaaga tgtttcgtca acaccaccag atatattcag taaatattaa 3300

attaatttag ttagggggct atgctccctt tcttatttgt gttataatat agacaaggag 3360

gttttactgt gacaaatgaa gagaagtttg acaaagtaat gtctatcgtt aaagaagaca 3420

actctaccgt cctacctgac tccgcttcta ttagtaaagg aattttaaga gagttgacac 3480

aggaaagttt acaagactac ggagaggtca ttgaaaagaa tgatttttta agcagggagt 3540

ttaaacatat tgtttccctc tacggttttg acgactttta ctcactatat gcctatgctg 3600

atttttgcga tactatgagt aatctagcta ttaaaggcgg gaacaaagat ttatctaagt 3660

taaaaaaggt aaaaagaacc gttatgagaa acggaaaacc tacagagatg actttttatg 3720

agtcaactac cgaagaagag aataaagaca agaaagaaga aagtaatacc aagcgccaag 3780

gagtaagcac agtatctgca aaagacttac ttgcagagct actaaagtct aaaggggaac 3840

cagaaaaagt aactcagtta ataaaaataa ttcttaaatt ccctgataca cctgctaacc 3900

ccaaagtatc tgattatttt taccagttaa aagacgactc gggagaagta gtcgctgtgg 3960

caggttacag tataggagcc caatacgtct ctcttgattt ttataccagt aatggctcta 4020

ttagcggagt agcatcaaga gcgttctttg aattactacg gatagcaata gaaaaagact 4080

taggagctga attaaatgac gaggataacg agctagctca agtactattt tcaaaattta 4140

attttcagct agatgaggga aaacagagag tgtctgctag aaacttaaaa aaacacttta 4200

aggggtaact atatgttgcc attctattca atatatgcta taataatagc atgtgttata 4260

tacacagtta tctttatttt agtagtacgc agaatgtatc gtaaagcaga caaacgagag 4320

aaggaaaaag aagacattct taacactatg acagaaataa cagaggaaat aaaaacattt 4380

attggaggag aaaataacaa tgacaattga gaacacgaag actttagaac aaacactaaa 4440

tactgtagcc gaggaaaaag attatgactt attcctatca acgttggcgg gggtagttaa 4500

atcactatac aaagagtacc aatttcttag acaagaccca gcacccgagg ataaaacatc 4560

aggaaacatt ttgattttcc aaattttccg aggagaccct gacggttatg ctaaagattt 4620

tgatacactg tttaaaattg cagtagacag aaatttagag cacagaaaaa tatcccaaaa 4680

attctttgaa tgggtagaga aaggtaagtt cttagagttg ggtgataaca tcgttattag 4740

cagggatggt acaggagtaa gctctattgt tgagtctgtt gaacttaaag tttttattac 4800

ttttatgact aaaagttatg ccgaagaacg agaagatttt ttagctaaag agaaggaaaa 4860

gcaagaagag gctaaaaaga aagaagaaga gcgactagct ctaaaagagg aatcgtataa 4920

atctttagga gaggctacag aaatgctaaa tactctcatg gaagagcaag gtgtatctcc 4980

tgaggagctt gctttagctt tagaacaggg gaaggagagt tatcgtaatg cacgataatg 5040

aatttgaagt acctgagagt gttattaatg cttctgctac accttttgag gcgcctgata 5100

gaacaaaaga tacctaccaa agttcattaa agaactggtt ggacagagta aaatcagtcg 5160

gagatgcagg aatgtcacga gagtttgtta ttcgtattcc agcaaaggca ctagaggaga 5220

acaacccaga agctacctta tttttaaaac agtgtaacga aataaaaaga aaccctaagt 5280

gcaaccttcc tattaaaatt gaaaaaacag tagagaagta caaagacaag aaacacttaa 5340

tgttagactt ggtgttcact tatagtaata aaacagatat tgatgcagta aatagtgtaa 5400

taaaataaca aacaggttcc tctttacgtc tgctatatta gatagtagaa aacgtaagga 5460

ggaattttta tatggtaaaa tataccgtag agaacaaaat tattgcagga ttacctaaag 5520

gtaaactaaa aggggctaac tttgttattg ctcatgaaac tgcaaatagc aagtctacta 5580

ttgacaatga agtaagctac atgactagga actggaagaa cgcatttgta actcactttg 5640

taggtggcgg aggtagagtc gttcaggttg ctaatgtaaa ctatgtttct tggggagcag 5700

gtcagtatgc taactcttat tcctatgcgc aggtagagtt gtgccgtaca agtaatgcaa 5760

ctacatttaa gaaagactat gaagtgtact gtcaattact agtagaccta gctaaaaaag 5820

caggtatccc tattacactt gactctggta gtaaaactag tgataaaggt attaaatccc 5880

ataaatgggt tgctgataag ctaggaggaa caacacacca agacccatat gcttacttaa 5940

gctcatgggg tattagtaaa gcacaatttg ctagtgactt ggctaaagta tctggcggag 6000

gaaacacagg aacagcgcca gctaaaccaa gcacaccagc acctaaacca agcacaccat 6060

ctactaacct agacaaactt ggcttagtag actacatgaa cgctaagaaa atggactcta 6120

gctacagtaa cagagctaag ttagctaaac agtatggtat tgctaactat tcaggaacag 6180

ctagccagaa cactacactc cttagtaaaa ttaaaggagg agcacctaaa ccaagcacac 6240

cagcacctaa acctagtaca tctacagcta agaaaattta tttcccacca aataaaggaa 6300

actggtctgt gtatccaaca aataaagcac ccgttaaggc taatgctatt ggtgctatta 6360

accctactaa attcggagga ttgacttaca ctatccaaaa agatagagga aacggtgtat 6420

acgaaatcca aacagaccaa ttcggcagag ttcaagtcta tggtgcacct agtacaggag 6480

cagttatcaa aaaataaata atggtaaact agaggagact tttatagtct cttcttttct 6540

tttgtaacag tagtgaaata aaacttaacc cctttgtaat attatggaat tacttctgta 6600

acacatctat gttatattta gttggtaatt taaaaacgca tatgcggaat atagaaagga 6660

aaccagctaa tgaaaaaagg aagtaagatt gcaattgcta ctgtagtagc tgtacttatt 6720

gtcgttacag ttagcctagg gtaccataca gttaagttga atgagacaaa tgaggcaaag 6780

acagaagagg tagttaagct aaaggagaat atctctagtg tatctaaaga tttagataat 6840

ttaggtaaag aacatgaaaa gaaagtctca gaatccgaag ctcttaagaa agaccttgac 6900

aataaaaaga aagagttaga ggataagaac aagaaagaag agcaacttaa aaaagaaaaa 6960

gaagaactac aaaaacaagt aagctataaa aagagcttaa agaaaaaggc agtagcagaa 7020

aacattccaa atgcaactag tagtaataaa agtgctaaaa cggcagaaag caaaggaaat 7080

ggaagaagtt ttaacgtaga agttacttac tatgctattg gtgacggttt tactcccggt 7140

gtacataccg caaatggtac taacatcaaa aacacaataa caacatcaga aggacatcga 7200

ataattgctg tagacccatc agtaatccct atgaactcta ttgttaaagt aactacaggt 7260

aacggagaga gttttgtagc taaagcttgt gatacaggtg gagctatcaa aggtaacatt 7320

attgatattt tagtagaacc tagcgcagat atttatcaac tagggagaac aacaggtact 7380

gtagaaatta ttggataatt taaagaaata agagtctgca aggtataaca accttgtggg 7440

cttttttatt atgctatatt atatatgacg gattatgaag tttgaggagg tatattaacg 7500

tggttataac acagatagtg atgattatag cgattattat tactacattt cttattattt 7560

atggtatata ctcttctagg agaaaaacgt ctaagtcgtt tagactagta ctcagatatg 7620

ctataatggt agtaggtctt cttattgtct actatctatt tcaagtttcc tctattgaca 7680

ttcaacagga ttttttacag cacttaaaaa caggaggtaa cgggatagaa gaagtttttt 7740

tcacaaattt ctttcttatc atggtaggag cagtaggtac acttgtagta caactattcc 7800

aaaatagaaa gaaggattaa tgtgtcatat acaaaagact ccttgtggtt ggagacaaaa 7860

aaggttgcta gagataatgg gtttttaggt aactggatag agatggttga ctactatgag 7920

agtataggag gaagccatgt attcgtttat gcggttatgg aaggtcagaa gtaccgaatt 7980

ttaggcgttc ttagcacgga tgaggtatta ctactaaaaa gagatttaac gctctcaaaa 8040

gccctatatg aagatgttat gaatagcaaa aagatgtttt tctataaaga ggacagtgaa 8100

aagaaaaaaa taaaattgac agatgaaata agtaagttag aagataaaga tagtatcata 8160

gtatatatgt aggtaggtga aaaataaatt gggactattt gaaagtataa gattagcggg 8220

ggtaaataag agcgatgcag taaaacacat tgaggttgat gataactact ccattgcaat 8280

tcaacaaagg gaacaggaac aaatatctaa agcaatgaat aacaaagaag tagcttattc 8340

tcaaccagta attggctcta tgtctgctaa cccggggttt aaaacaaaac ctagtattcg 8400

taataaccaa gatttacacg gtgttcttaa aaagtttggt ggaaacatca tcctgaacgc 8460

aataatcaat acaaggtcaa accaagtttc tatgtattgt aaaccagcta gacactctga 8520

gaagggtgtt ggttttgagg ttagattaaa agatttagat aagaagccta cttctcatga 8580

tgaagcaaca attaaaagaa tagaaagctt tatcgaaaaa acaggagtag ataacgatat 8640

taaccgagat agcttttctt cctttgttaa aaaaatagta cgagacactt atatgtacga 8700

ccaagtaaac tttgaaaaag tatttaacag gaaccaaagc atggttcggt ttgtagctaa 8760

agacccaaca actatatttt ttgcgacaac agcagatggg aaaattcctg ataacggtaa 8820

tcggtttgta caggttatag accagaaaat agtagctacg tttaacgcaa gagagatggc 8880

atttgccgta aggaacccac ggtctgatat ttatgctaca ggatacgggt accctgagtt 8940

agagatagca cttaagcagt ttatagcaca tgagaataca gaagcgttca acgatagatt 9000

ttttagtcac ggtgggacaa cacgaggtat tctacaaata aaagcagcac aacaacagtc 9060

gcaacatgct ttagagatat ttaaaagaga atggaagaac tcgttaagtg gtatcaacgg 9120

ttcatggcaa atccctgtag tatctgccga agacgttaag tttgtaaata tgacaccttc 9180

tgcaagagat atggagtttg aaaagtggtt aaattatctc attaatgtta tctccgcact 9240

gtatggcatc gacccagcag aaataaacat acctaacaac ggaggagcca caggttcaaa 9300

aggtggtagc ctaaacgaag gaaactctgc cgaaaaaaat caggcatcca agaacaaggg 9360

cttacaacca ttattaggat ttattgaaga ctttataaat aaacatattg tagctgaatt 9420

tggagacaag tacacattcc aatttgtagg cggagacata aaatcagaac tagagtcggt 9480

taagatacta gcagaaaaag caaaagttgc tatgacagta aatgaagttc gtaaagaact 9540

aaacttaccg ggagatgtaa ttggagggga cattccatta aacggagtaa tcgttcagcg 9600

tataggacaa cttatgcaac aagagcagtt tgagcatgag aaacaacagt ctaatttaca 9660

aatgttacaa gaacagacag gtaacagagt aagtactgat gtagaagaca ttcctgatgg 9720

gaaagataca acaggagaca taggaaaaga cggacaaaga aaagataaag ataacgcaaa 9780

cgctggtaaa caaggcatga aaggtgataa acctaacgat tggcagacct aatattttgt 9840

agttctgcct tttaggtaac taactagtag aggtatatgc tatattaata acagaatgtg 9900

ttataataga tatgagaggt gatttcttaa ttgaataacg tcgtagagaa agagatgaaa 9960

atctttgtac ctattgatat tgaaggttct ttagaaaaat cccataaggg agatgacaag 10020

gactggtatg ttaaaggtta cgcatctaca cctgctctag acttacaggg agaaatggtt 10080

aaccctgctg gaatagacat tggttatttt gtagataatg gttacattaa ctttgagcat 10140

agacaagaac cagactatgt agttggagtt cctacaagta actgttatgt agacttccaa 10200

aaaggtctgt tcgttgaagc aagactgttt aaagacaacg aacacgccca atctatgtgg 10260

aagttagcta acagcattag taagacaggt attgatagac aacttggttt ttctatcgaa 10320

ggtgcaataa gaaagagaaa cacagatgac agccgaatta ttgaagaggt tgtaatcaaa 10380

aatgtagcag tcacaatgaa cccagcaaac ccagaagcta cgtgggaaac atttatgaaa 10440

tcatggacta ctggacatgg aacaacacct gatacgcaag taaatgcagg agctttaaga 10500

agagagagct tagctaacag catatcagta ctagcatcag catacaaaat ttctgaccct 10560

attgaatttg acaacttatg gaaagatgta agtaagtttt tagatgaaaa aggagagtat 10620

aacgagaatg aggcaatagt tatgttacaa ttagctaaag gcgtttctcg tgtaagtgca 10680

tgtgagtctt taatgaatag acgaaagggg aaacaaaatg aataacagtg aagaaaagtt 10740

cgaagatgtt ttaaacgaat ttggtaatac agtagaaaaa gataaagaag aaatttctaa 10800

gtctaccgaa gaaaaagagg aggttacaga tattaataaa gataaaaaat cagaagaaga 10860

aactgctgag gaagaagtaa ctgaggctcc cgaagtagac gttgcagaag acaaagaagt 10920

gtccactgag actcccgaag aagagtctac tagtaagtca gctaaggaag aaaagaaaga 10980

aaaagatagt gaagaagaca aggacgaaga cgagaacaaa aaaggtaaga aagacaaaaa 11040

agacaaaaaa gaagaagaca gcaaagaaga aaaaacggag aagtctgatg aagaagattc 11100

cgaagaggct gtaaaaaata ctaactctga tgcagatatt gcaggagcat tgaatgctgt 11160

tgctaagtct tatgagaatg ttaaagaaag ccaaagagag caaaatacaa ctgtagaacg 11220

cttagagaag aatatcggta gcttagtcag taaattagac agcttggtag atttaatgag 11280

tgaacgtgta tctaagagtg tagtagaaga tattgaaact cttgaaggag ctagtgaaaa 11340

agctgttggc tatgtagcaa aaaacgtaga tagcagtgta gtagaagtta ttgaaggaga 11400

agaaacttct aagagtgtag atgcagaagt gagtcctaga gatgagtttc tagctaataa 11460

gagtgctttc ctagagtctt ttgaatatgc aagtcgtaac ggtgctgagc gtagagattt 11520

aactgatgtt cgtcacgctc atgggaacat tacaaatgat attgctagta aagcagatta 11580

taaccgagtt aacgacttta tgaaaaaatg gagtaagtaa taaaggtaac tgtatagagt 11640

attttttgta aacagtatgc tatattatag gaacatggga aacatgttac gttatgagag 11700

agcttgcccg ttttaggact cctcccctag ggcaggttat ctcactaaca aaaaaataaa 11760

actatgaaac tgaaaggtga taaatataaa tgccaaaaaa taacaaagaa gaagaagtta 11820

aagaagtaaa ccttaattca gtacaagagg acgcgttaaa gtcctttaca actggttatg 11880

gtatcacacc tgatacacaa acagatgcag gagcattaag acgtgagttc ctagacgacc 11940

aaatctcaat gcttacttgg acagagaatg atttaacatt ctataaagac atcgctaaaa 12000

aaccagctac atctacagta gcaaaatacg atgtatacat gcaacatggt aaggtaggtc 12060

atactagatt tactcgtgag attggggtag caccagtaag tgaccctaac atccgtcaaa 12120

aaacagtaaa tatgaaattt gcttccgata ctaaaaacat cagtatcgca gcaggtctag 12180

taaacaacat tcaagaccca atgcaaattt tgactgacga tgctatcgta aatattgcta 12240

aaacaattga gtgggcttca ttctttggag attctgactt atcagatagc ccagaaccac 12300

aagcaggact agaatttgac ggcttggcta aacttattaa ccaagataac gttcatgatg 12360

ctcgtggagc tagcttgact gaaagcttgt taaaccaagc agcagtaatg attagtaaag 12420

gttatggtac acctacagat gcttacatgc cagtaggggt tcaagcagac tttgttaacc 12480

aacaactttc taaacaaaca cagcttgttc gtgataacgg aaacaacgta agcgttggtt 12540

tcaacatcca aggtttccat tcagctcgtg gatttatcaa acttcacggt tctacagtaa 12600

tggaaaacga acaaatctta gatgaacgta ttcttgcttt accaacagct ccacaaccag 12660

ctaaggtaac tgcaacacaa gaagcaggta aaaaaggaca atttagagca gaagacttag 12720

cagcacacga atacaaagtt gttgtaagtt ctgacgatgc agagtctatt gcaagtgaag 12780

tggctacagc tacagttact gcaaaagatg acggcgttaa actagaaatc gagttagctc 12840

caatgtacag ctcccgtcca caattcgttt caatctatag aaaaggtgca gaaacaggtt 12900

tattctacct aatcgctcgt gtacctgcta gcaaagcaga gaacaacgta atcactttct 12960

atgacttaaa cgactctatt cctgaaacag tagacgtatt cgttggtgaa atgtctgcta 13020

acgtagtaca cttgtttgaa ttactaccaa tgatgagatt acctctagct caaattaacg 13080

catctgttac atttgcagtt ttatggtatg gagcattagc tctaagagca cctaagaaat 13140

gggtacgtat tagaaacgtt aaatatattc ctgtaaaaaa cgttcatagc aactaataat 13200

tataggataa ttgaataaaa acagtataga gagcagataa atactgctct ctattttact 13260

aataaggagg atttaaattg ctaaaaaata caaacttagc taattataaa aaagtgaata 13320

cacgatttgg aaatcttagt tttgatgata aaggtatttc taatgaccta acggaagagc 13380

agcaaaaaga attaggtaag cttagaggat tcgaatatat taagacagaa cagaaaacga 13440

aagaagaacc taagaaagaa gaacctaaga aagaaagtac agaaaatgaa ttagacagct 13500

tcttagctaa agaaccttca atcaaagaat taaaagaatt tgcgagtaaa aaaggcatta 13560

aaattgaaaa aactaagaaa aatgatataa ttgaagaact aaagagaggg taatgtacaa 13620

tgtatggagg ttatgaagga caagattctt acgaataccc ttactcacac gggaacccta 13680

agcatgtaga gccagaaaaa gttgacgaat atgttctttc tgattatggc tggactgcgg 13740

aaacaattaa agcatacatg tatggtgttc gtgtagtaga ccctgaaaca ggagaggaaa 13800

tgggagacac cttctacaat catattatag aggttgccgt tgataaggca gagaaagaac 13860

tagatatagc tattctccca agacgggagt cagaacacca cgattataac caaacagaat 13920

ttagtagtta tatgtatacc catgttcata aaaaacctat cttacaagtg gatgacttaa 13980

aactagaaat gaatggtagg gcaatatata gataccctgc taactggtgg aaagtttatt 14040

gcttagctgg acatatagaa ctgtacccga cagccttgat gcagacttct caaagtatga 14100

catatgactc tgtatttgga ggatacccgc agttggcagg aacagcccct tctataggta 14160

gaacatatgc ccctcaaatg attcatgtag aatatatagc aggtatgcta ccaaggaaaa 14220

gagcaggtgt tagtcgtgac tgggaagtac ctgctgcatt agaacagtta gtaactaaat 14280

atgctttagt agaaatattc caagtttggg gtagacttat catcggagca ggtattgcta 14340

gtagaacact agtttacgac ggagtttcag agtctattga tacaactcag tctgccatgt 14400

atacaggttc agcagcagat attgagctta taaataaaga catagacagt ttagtagctc 14460

aactgcgttc tcactttgga ataaacatga ttggactata agagaggaga atgtttaaat 14520

ggcagagcct actagatttt caagtgatac tcctattaaa aacctaacaa cacaaaaagt 14580

agagtacaac atatcacagc atagaaagtt tattaaagac cgtagtatcc atacgttatg 14640

ggaaagagct tatttatgcc cttgtatgaa caagtcctcg ggttctgctg actcttcttg 14700

tcaaagatgc catggtaggg gatgggcgta tttaccagct aagcccgcaa atatgattat 14760

tcaaggacag gaaaaagggg ttactaacac agacctagga ctatatgact caggaacggc 14820

tatcggtaca aatgattttg atgctagaat ggcttttagg gacagaataa cagtacctga 14880

tgttaaaata ggtcaatccc ttatctactc tataaccgaa agcagacaaa aacgaggtat 14940

atttttaaca tatgacgttg atactataga ctttataaca agtatggaaa gagaactaat 15000

agaaggtaaa gactacacct ttgataaaca cagtaataaa ctattccctg atattagttt 15060

gataggtgaa gttatatctt taaacgtatc tgttactcta aggtatattg ttattgactt 15120

actaaaagaa agtcgctatc agtacaccac attaaataga acagacccta agtttgagca 15180

gttgcctcag aagttattac taaaaagaga agacactatt gtattacctg aaccatttgt 15240

acttgatgct gatacagaga ctagagtaga gttagttaat gaccctaaaa ggaaaccaag 15300

acaacaaggc gggttctacg aaggatgttt tgactagtga aacagagtac acagagacca 15360

tctctattca ataatccaaa gcaggtaatg agaaatatgg caagagctat agaatcagat 15420

gttattgcaa caggagctca agaggcatct cgtataccca atgtagagat taaaattatg 15480

cctaagtatt tagaagtaac tgaaaagaga atggaaaaaa atggtgtaat tgaccttaaa 15540

ccttattttg ctaaaagtaa taaaaagaaa tggaatcaag aaggtaactg gtacttagta 15600

atccctataa gattaaagac aagagatatg tctcgaaaat tatatgatga cttaaggaaa 15660

cagtcttccc caccagttgg aagttcaaca acaattgtat cagatttcct atatgaccgc 15720

agaagaacga gccctagtgt cccttctata aactataatc caaagtcaaa taatgttaca 15780

aaaaaacgta ttagtaacag tagaacaaca tatacagtat accgtacagt aactgctaag 15840

tcacctgcta atagttggat tgttaacagg gacagggtta atgaagagga tatgagtaaa 15900

acgatgttag acagaataga caggctaatg aaatggaagc ttaaaaactt gtagaggagg 15960

agtacaatgc ctattccaag ccttgattct tatgtagcaa gaagagtaag agagagcctt 16020

aaaattatgc tttcagagtg ctatattata gaggaagctt tgagtgacat ggatgtcgaa 16080

gcaaaaagta actttatgaa gacatattgt ggagataatc ctaaaaaaga aatagaagtg 16140

atatacgagt tccctcaggc aaaaacatca gtagatgcta cttatgtaat tcagctagga 16200

ggaggttccg aagatactaa aagcatcggc ggtattgaag gtacttacga gtataaagag 16260

gattctttaa gaaaagagac agtagttgct actagagaag gaaccgagtt atttctagaa 16320

acaaaagagc taattggaag tatagaggga atacctaatt tatccttttc agaaagtgat 16380

aataaacgaa tagaaggtaa acgattatac ttctcttatg aaggtaatgc agatattgag 16440

ggagtttcct tcgatattat ctatacaagt aaagtagacc tagagagcga ccccgttgga 16500

ctaaaaaaag ggtttacttc tagagacaat atcactgttg taggtttaag cactaacatg 16560

gatactgcaa ggtgcctaga tgctatctta aaagttatcc ttattatcat gagagaaaac 16620

ttagaagaga agagttacta ctccctacaa acaactcagt tttccgatat gtctcctgta 16680

attatgggag aggaaaaacc tgtatttggt agacctgtaa caatgagtta cactatatca 16740

tatactgtag actttaatct agtcaaccaa ataaataaaa ttatagtaca ggagcgtaat 16800

aagtgatggc agaaaaaaaa gaggtaacta aagaacctat tgtagacgaa gtaaaagtaa 16860

caaagaagaa ccttaaggaa cctacaacag aagaaaataa agtagtgaag aaaaaactta 16920

aagaagaaca aacagatact gtagagaaag ttactacagg atatgttcat atagatgtat 16980

tccttggaac agccaagccg tggtacggtt tatcagaaat gcaagtacaa gggtttaaat 17040

cttttatgca agggaaacaa taccagttcg gtgaacaggc ttttgttcct tatttagaaa 17100

aatatctagg aagggaagta aaataaaata tggcaatcga aatttaccca agaaaaccag 17160

tttctagacc tcatacagaa atttctgtag atacaagcgg tattggtggt tctagctctg 17220

gaagtgaaaa aatactttgt ttagtaggtt cagctacagg aggaaaacct aatgctgttt 17280

acaaagtaag aaactattct caagcaaaat ctgttttccg ttcaggagaa cttttagatg 17340

caattgaaag agcatggaac cccggtgaag gaacaggtgc aggagacatt ttagctatgc 17400

gtgttgagga agcaaaagaa gctacctttg aagctgaggg ggttaaggtt agctctacta 17460

tttatggagc tgatgccaac gacatccaag tggcactcga agataacacc atcacaggta 17520

caaaacgctt aagcattgta tttgcgaaag aaagagtcaa ccaagtttat gacaacttag 17580

gtagcatctt ctctatcaag tataaaggta ccgaagcttc tgcaacattt acagtagctg 17640

tagacccagt aacatttaaa gctacaaagc taactcttaa agcaggggat aaaacagtta 17700

aggaatacga tttaggttca ggagcgtatg cagaaactaa tgtacttatt agtgatatta 17760

acaacctacc tgactttgaa gctaagtttt tcccaatcgg agataaaaac ttaacaacag 17820

acaacttcga tgcacaaatc gatgtagata ttaaaacaaa agaagcttat gtaaaagctg 17880

ttggcggaga cattgagaaa caaacagcat ataatggtta cgtagacttt gaatttgacc 17940

gctcaaaaga aattgctaat ttcccactaa ctaagctaac tggtggagac aacggtacaa 18000

ttcctgagtc ttgggctgat aaattcagtt actttgcaaa cgagggtggc tactacctag 18060

ttccattaac atctaaacaa gctgttcatg cagaggcact tcaatttgtt agagattgct 18120

cttataacgg taatccaatg cgggtattcg taggtggagg aatcggagag tcaatggagc 18180

agttgttcac aagagctatc ggcttacaga atgaacgagc aggtctaatc gggttctcag 18240

gaacagtgaa gatggacgat ggacgttctc tgaaaatgcc gggatatatg tttgcagcac 18300

aagttgcagg acttacatgt ggactagaga taggagaagc aatcactttc aaaaacattg 18360

cgattgagac attagataca atttacgaag gttctcaact agaccaatta aacgaaagtg 18420

gtattattac cgcagaattt gtacgtaata gagcagttac caatttccgt attgtagacg 18480

atgtaactac atttaatgat aaaacagacc ctgttaagtc agaaattggt gtaggagaag 18540

ctaatgactt cttagtttct gagttgaaga taagcctaga caacgagtat attggaacta 18600

aaattattga cacctctgct tctttagtta agaacttcgt tcaatctttc ttagatagaa 18660

agaaattagc taaagaaatt caagactaca gcccagagga agttcaagtt gtaattgagg 18720

gcgacgttgc tcgtatctca ctaacagtat tcccaatccg cagtatgaag aaaattgagg 18780

tatctctagt ataccgtcaa caaatcctta ctgcctaatc taatacaggg gagggcttag 18840

ctctccctat gtaaataaaa aataaaacag gagttgaaac ataaaatggc aagtgttact 18900

aatcagacgg ttcatacagg taatacggtc taccttatga taaaaggtaa aatcgtagga 18960

agatgtcagt ctgcatcagg tgaacgttct tatggaacaa ctggtgtata cgaaatcggt 19020

tctatcatgc cacaagaaca cgtttactta aaatacgaag gtagtttaac agtagacaga 19080

ctgcgcatga gaaaagaaga tttagctaag ctaggtatta cagcattagg agaagacatt 19140

cttaaaagag atgttatcga cattgttatg atggataata ctactaaaga agtaatcatt 19200

gcttatcgcg gatgttctgc tgagacatac aatgaagaaa tcaaagctaa tgagattgtt 19260

tctgaaagtg caagattctt gtacctaagt gcagcaaacg taaaataata ctaataaaag 19320

gctaccttaa tctggtagtc ctttttttat agtttactaa ccttagcaaa tatggtataa 19380

taaagtaaga gaacgaaaaa gtattactag tttgttatat tagataatga gagagtaaac 19440

aaggaggaga caaattaaca tgtcagaaga acaaaaagta aatgaagaac aaatagaaga 19500

aagtaaagag aagatgttga cgaaagaaga catcgaccgt atcaactatg aacaacaaac 19560

gaagcaaaag aaaacacttg accgtattat taaaggggta aatgatgttt ggacacatga 19620

gtatgacttt aaggagttag atttaaagtt caccattaga ttacgagcac ctaatgcttt 19680

agaacaagca acaattcaag ctaacagaga agcttattta aaaggaacag gtgcattagt 19740

tagtgactac atttatacaa tctatcatac attagccact ataagaacag ttggtgtaga 19800

tattccaaag gaactagcag aagacgataa catttacaat actgctattt tataccaaat 19860

tggacgtgat tttgccgaat ggctagacac gtttcgctac tgagataaaa gatttaggtg 19920

gaataaaagc tttagtaaaa actccctatg caagaaacat gtggggaata atgaaagagt 19980

tcaatgtttt acctacagac gagaggttca gagcgcttac tgaggagcaa ataaacttca 20040

tctactataa caaagagaga gactacaaag aggcagagag agcttctaga ggcgtgtcta 20100

tggagtctga atttgaagac tataactcgg atgactggtg ggaagcttct catgaggact 20160

tccaagcaat tcaggaaggt catgatgaag atgacatcgc tcaacaagta gagaacatga 20220

ctagtatcga agaccaaaaa cgcctaagag acagattcca gtctcatgag gagtacgaag 20280

agtttataag aaatggtggt aaagatacta gacaggaaat gattgatgaa cacatcaata 20340

acaaactgaa agaactgttt gcagatgccg aagagttaaa taaacaaggc ttgagtaagt 20400

ggggagaagt atcagaaatg acaactatcc aagaagctgt agcagacgag ttagaagagc 20460

taacatcaga tagtgttaag gaagcaatca gtatgttcga agatgacgaa gttcaggtaa 20520

taccgggtca agatgaagac gatgattttg aaatttaata ctaaaaagaa agggtagagc 20580

tgtgtactct actctttttt actaaggaag tgaaagtaat tggaaaacac taactatcgc 20640

ttttcggtag aagccttaac ggcagatgca gagaaaaagt tagagcgtat cgctagaata 20700

atggacgaca ttgagggaac aaataggaaa ggtgcaagta atttcttcac cagctctcaa 20760

aaagatatag acaacaacat aagagctatg agagaatatg tttcattagt taatgaagtt 20820

gagaaggaaa tgaaaaacgc ccagtcttct atgtctcgtt ctggaaataa cgaaggtgtt 20880

caaaaattaa taagagacca gcaaagacta agggaagaag cggaaaaaac acggaaaaca 20940

ttccaagata ctacacaaaa taaaacaggt agtaacgctt atcttaaaga cctgttaaga 21000

gaccaaaaag catacaacga tgaaatagac gaaggtactc gtagattaaa agagttgaaa 21060

gacttacaaa gagatgtagc cagaacagat agcagactgg ctgccagaac aaggcaagct 21120

acttcatcag gtaatatgaa ctcacaacaa gcaggaacag ctaggtctga cttagcaaat 21180

aaaaccgcat atatgagtca attacaagcc aaccaacaac gaagagatga gttaagaaaa 21240

gggctagagt ccgaccaagc aaaaaaaaga aacttaaatg ctaactttag aggtaacgac 21300

caagaaagac agaatgagtt gtcagcgtta aaccataata ttgagactcg tagaaaagaa 21360

atagaagctt taaaagagct cgatagtcaa ataaataaga cagtaagtaa tatggaaagc 21420

tacagtaacc aattaaataa tgtaaatgta aaaccagacc gtaactcgtg gcaaggggtc 21480

gcttatgaaa gagcaccatc tatcgctatg gctgcattag gtggagcagg tgcaatattt 21540

ggcggtctat atgctaaagg tagctctata aaagaccaga ttagacctga cactatctct 21600

atagggcaac gaacaggtaa ctatgatttc cgttctatca gaaaacagaa cgaagaagta 21660

ggtatggata ggtctcttgg ttacaacgca gtagacatga ttgatttcca aaacacctat 21720

ttgtcttctg ctgggtttaa aaaccaaaaa gacttagatg caggaactag acaaatggca 21780

gaagggtcta gagctgtacc cgtagataaa gattctctaa atcagtttat gggtgggcta 21840

atgacgaatg gagccgtatc aggagcagac caagtaaaag cggtacaaca aggttttatt 21900

ggagcagtaa aagcttctgg aatggaaggt agggaaaaag accaactaaa agctttagaa 21960

gctatgagta cccaaacctt tgcaggaaga aatggttcta atgctgagct aaataacctg 22020

ttagctatcc agtcactgtt ttcttcatca ggtgaccgtt ctatgcaagg aacagcagga 22080

gcagaagcct tatctagtct agatgcagga atgaaacaat ctgcatataa cccacaagca 22140

agattactat ttggtatggg gagtgagtac caaggagtag gtggtatgta tcaagttcaa 22200

ggacaaatgg aaaaaggtct cggagataca gacaatttaa atagactact taaattctcc 22260

gcaggtgcag gaagaggtaa agaagaacaa aaaggttact tcaagaacat ggctgagaat 22320

gtatttggta ctacattaac aaatgaccaa gtcgatgcaa tgtataaaca agtaggctca 22380

gatggaacaa tagataaaga caaaatgaaa aacatccaaa gtattattga tggaggaaaa 22440

gacggatacg ataagaacaa aaaagggtat gaagactcta aggaagctac agaaaacgcc 22500

tctaaagcag taactgataa gcaggctaca gcgatgaacg acatggggga catagttaga 22560

aaactaaaca gtgttctcgg tggtatgccc gcaccattgt atggtttggc agccgcagca 22620

gttgcagcag caggagcctt agctatgtct ggcggaatga gtattctatc tggtggtatt 22680

aaaggaaaac taggtaagaa atttggaggc ggaataggta aagcaggtag cagtacagta 22740

gcttccgcag gagcaggcgc aggagcctct tctacagcta gtaaagtatt taccaataaa 22800

acatggggag caggagcaga aggagctaca aaggcttcct cggggctctc atggggtagt 22860

aaagcaatgg aaggcggaat ttctgcaact aaaggattag gtggagcagg aaaactagcc 22920

cagtatggaa aaggagcctt aaaaggtctc gggccgcttg gtcttctaat aggcggagga 22980

gcctcaatcg ccaatattgc aggtgcagaa aacaaaaccg aagccatcgg agaggaagcg 23040

ggtggctggg gaggcgccat gggtggtgca gcaggcggtg cagccatcgg agccatgttt 23100

ggtggagtag gtgctatacc gggagccctt attggagggg tactgggagg tattggagga 23160

agcaaagtag gtaagtgggc tggcggaaaa ataaagagtg gctgggattc tctatggggc 23220

ggagacaaag tagatgctgc tgagataaac ggcacaggag gagctaaaaa agctcaaaca 23280

gagaaaaaaa ccaacatgaa cggtgcttct atggctaact ctggtgtagg aactatgcta 23340

ggaaatacag taatgcccgg tgtaggagga attatcggag gagctatagg aggttcaggt 23400

atcctagaac aactttctaa gctaactggt attggaagaa acgcacaaga agaaacaagt 23460

tcaggaaaac aagatgaaaa agacacagta ggtaagaaag ctacagcaga gaaagagaga 23520

caaagtaata caagagaaga agcagcaaac ctttcttttt acagtaaact tcttgacaga 23580

gccgaagcat tattgatgca agctagaaat caaaatggat tattcggaaa agaaggctta 23640

ggaactactg ctacaggtag cggtggagga gatgctctca attcaggtct taacttctta 23700

acagatggta aaaagtggac taacaaagat ttaacaaagc atgacttagg ttcaactatt 23760

caaggtatta ctgctgatga gttagacgaa tggattaact cgaaagcacc tgagggttct 23820

gcaatgagag ggatgggttc aactttccta aaagcagcac aagaatcagg attagaccca 23880

agatatttag tatctcatgc agccacagaa actggttggg gaacatcgaa catcctaaaa 23940

gaaaaaggta actggtttgg tattggggca tatgataact cacctatggc aagtgcctac 24000

aaatacgggg acaaagaaac tggtgtagta gaaggagcta aatggattgc tgaaaactac 24060

tacaataaag gtcaaacaac actagagagt atgcgacata acggaggaca acacgagtat 24120

gctaccgacc ctaactggga taaaacgata gctagcataa tggggggctc agagaaatac 24180

gtgcaaggta aaggtgcttc tttaacaact tctgctaata taactgttaa catgaatgga 24240

gactcatcga aagataagaa gacagcagga aaagcagtag gagatgccgt tgctaaatcg 24300

tataaagaac aatcagagtt cttctctaaa caatacaaga gaacataatg ttctcttgtt 24360

attacatata agataaggag gataaaatta tatatgacta cagttaaaag aatgcctgaa 24420

tttgatttaa agtttgtaac agaaaagaat gactatctta taagatatga tgcaaggaac 24480

ccttcctcag atacattagc tgagaaagta ataagtgtaa ctactaaaaa tgcaatgtct 24540

gacgacagcg ccgttttttc tattgtagta gctggtgata tggaatggga taaaatatta 24600

gactctaatg atgtagtaat actaaaaata tacccaaact taagagtaat gatgcctgac 24660

aacgttgttg ttttagtggg tctcatatcc gaagttcgaa gagaaggcga ctactctaat 24720

aactctatta tttatcgtat aacagggcaa tcttttgcta aatccttcat gcagttccaa 24780

ttaggggtaa ttcaagaggt atctgtagta attaccgata ttgggtggtt gcctgactct 24840

aaggcagatg gtgttgagtt tacaggaaaa acagcagcag agataggtaa gtctatcaca 24900

gataggttta aaaaatatat gaaatacaat tttaacagag aatatacaat ggaaaacttc 24960

ttagactact ctttcagcag ttggaaagat tacgaaaaat tagcagaccc taccccattt 25020

attaactatg agggtagcct aaaacaactt ctagatgatg ttacagcaaa gccatttaat 25080

gagttattct ttgagtccac aagtgatgag aagtgtaaaa tgattatgag aagaacacct 25140

tttaataaag aagactggga caagttacca tcttataaaa tatctacaga agcagtaata 25200

tcagattctt tagctaaagg ggacacagaa gcctactcta tttttaacgt cactagtgga 25260

aatatggcag gagcaacatc agtagactta aattcattcc ctcaatatca ccaagcttta 25320

gtagataaat atggatacaa aaagttagag gtagacaaca ggtatttatt tgagtcctca 25380

acagatggct ctagtaccac agaaaaggca gatgtaggaa gtaaggaaaa aacaaaaaca 25440

gtaattactt acagcaagtt taactctttt atgcgtagtt atacttccga ccaagttcgt 25500

atgaatcaaa gtagtattgc aaaaagttta gtggacagct acgacaaatt gacaacttct 25560

caagccaacc aactgcttgc taaatattct gctgtaggtg cgataagtga ggcagatttt 25620

aaaaaaattg taggagacat agcggaggga gacaacacag gaacagctac tttagatttt 25680

gatagtgtaa actcttggtt ctctctaaat tatagttctt tgtctgaggt ttctacaaat 25740

agagatgcta caataaaagc ttttgtaaaa aactttgcta acacagatga agaccaagca 25800

actaagatag tagcactata tatatcaagt caaggagtca tgacaaaaga aaaatttgat 25860

gcaatcataa aggagtctac tagttctagc actaaagacc cagataacac aacaggcaac 25920

tctagttcag cactacagta cttctctaaa acgatatata actggtacag tgaaaacgct 25980

aatttttatg caggggatat aaaggttatt ggtagtcctg tatataggtt aggaagtaaa 26040

ctccttgtag aagataaaca acaaggtgat gagtgggagt tttatattga aagtgtctct 26100

catgaatata gctatacagc aggttatact acaacactcg gagtcactag gggattgaac 26160

aataaaggga aagatagatt tactcattta tggggaaaaa gttcggactt taaaggcggg 26220

ttacttggag aaaaaacaag tgctgaactt atacaagaag caggttctac ctcgtcaggt 26280

tccgatggaa gtggcgatgt aagcgcacca gatgtacaag gtagtgatgt tgccgtagcc 26340

gctttaagat acgggctagc tcataaaaag ccagagaaaa aatcagtata ttcttttggt 26400

ggaggtagag gtagtagtaa cccaatggag ggtaaggaac cttacgcaat ggactgttct 26460

tcttttgtat ggtggtgtta taaagcctgt ggtgttactt tagcaggagc acaaacacaa 26520

gcgatattag gggacgatag gtttaacaca gtaagctcaa gaggttctaa gagtaaagaa 26580

atatttaaaa aaatgcaagt aggcgacctt gtttactttt acgataacaa cactcatatt 26640

ggtatgtatg caggagaagg aaagttctta ggatgtaatg gagatggaag ttgggacacc 26700

aacggaggag tacagttgaa accaatggac tctggttatt ggtggacaca gtttcaagga 26760

catgtaatac gttttgttta aaaaggagga atgactagat gagtgatgta gaatcaggag 26820

caccacgaac acaggctagt ttaggtagtg agtgggaacg agtatacaaa gcaggagaaa 26880

aagtagacac aatcgtatta gcccagataa taaaagtaaa ctataaatat aacaccgcag 26940

aggttatgat actaaaaaat aaagaagtga tgtctaagtc gtatatttct aaaggtaagt 27000

tctcagctcc actacctcaa gattttgctg gaagaactct agaaggactt ccatatggac 27060

aaattaaacc aattcaagaa ggagctattg ttcttatagg gttcctaaat agcaagaagg 27120

acaacccaat tattttagca gtctacgggg actcagatgt aaataataag ctagctagaa 27180

ctccttatga aagtgcagac ccattagacc ctaagctagc tagaataaat aaccagtttt 27240

ttaacttgta cccttccttg acgtatgata atatagacgg ggacggaaat agaatatcca 27300

cttaccccgg caagacattt tctattatga caagtttaat aaacgagtct tccaacattt 27360

cagacagcaa taatgggact gaatatgcag acttacccga atcatactac acagactaca 27420

aactgataga gccgagaagc cctttagcac catcaatgct atttaaacac caaggaataa 27480

gaacactaga cgggaaggaa gacaatcatt tagtaactgc ctttataaat acagatggaa 27540

caacaaggct gtctttccta aataaggtta tgaactggcg ttcctattat gaggctgata 27600

ctgagggagg tcaaaagttc agacttcaaa gagacagtaa ggtaccaaac caaggaagca 27660

tattttcaga gtttgggtta acaaaaaacg gagacttctt tattgtaaat ggaaaccata 27720

agtttgaagt aagagagaac ggtattttta ttaatgactc tgctttagca gacgacttag 27780

gggaagtttt agaagaaatt caaaatagaa tactagggat aaacgaggag attgctaata 27840

tcatccgtat gttggatgag attgatgttg agaacctagg aaagttattt gaccgtatga 27900

caaaagtaga aacagacatt attagcttaa ggaatgatta taaaggaata gacagtagag 27960

tgaacgatat gtcagaagac cttaacagtc tgattaaatc ctttgataac tttaaacaga 28020

caatgtctgc ctacaccgag gctaatacta taagggttac tgctattgag accgctactg 28080

caaataatac agtatctata ggaaacatta ataatagtat tactgcgatt gagcagaaga 28140

acacagccca agatacgaaa ataagtagcc tagagagtaa agttacgtca ctagagacaa 28200

gtaatgctag cttagtaaca aaagtagggg agttagaatc tgatgttact ttcctaaaag 28260

cagaaaatat agatttaact aatgcactag cccaattagc agaaagagtt actgctttag 28320

aaaatagctg atagtagtac tatattataa aggagacgag gtggaaaaat gccacaagca 28380

gacggaaaag gtggagtatt aagacgagtt gctttccaaa taggagagaa taaatggtat 28440

cgttttgcga taaaccccgg tagttactct tttacaggag gacaaagggt tgctgtgttt 28500

aaaacgaagt ccttaaacgt agtagaagac tttggtagag atattcctac tataactttc 28560

tcgggaacga caggtcttag aaaagactct aacggtaaaa caggagcaga taggttaaaa 28620

gagcttaaaa aatatattga agattatgca gactcatcag gaaacggaaa tagacctaat 28680

acagacctta cttttcataa ttttacagac gatgaaagct atattgtaca tttatcacct 28740

gaggcattta agatagaaag aagcgcagaa aaacctctgt tatatgacta cacaataact 28800

ttagtagttt taagggatgc ttctcaacca gaccaaggag acattgtaga cccagactta 28860

ggtaataaac aacctagtgt aggggacaag acaaaggata atgacgatgt ttatggggat 28920

gtaggaggcg cagacaagac accagaagct aatattactg gaagagtccc tctagaagta 28980

ggaggtaacg gggagggggc agtaaacccc cgaacaacag gtaaagcata ctcctacggt 29040

gtagatgaat taaaaaaaca aattggttat ggggagggtt aacaatatgg aggtatatca 29100

gtcatcagat ttaatgaggt ttattacagg gttaaaagtt aacccagata acactattgt 29160

agtaaacatc ttagaccagc aatcaccatt tatttctagg ttttacactc ctaagcttat 29220

tctaagttct ttatcagtaa agcttattcc tgaattgcaa ggagtaaaca ttatacaaac 29280

aaacaaagca ttaagtaaaa atacagttac agctaaaata tcagcaagta ggttaaaaat 29340

agaggctcct tacatgtatc taatcgcaca gtctatagtc atggaatgct ttgctctaat 29400

ctacagtata gaagaaaagc aagatatgct aaggtacatt gaagtaggag aagttaaaca 29460

agttagagat aacataaatt atttagctga ctacttagct gacaaaccag aatacagcag 29520

tattgttacg gacttacatg atatggaaat tgctgtaggg tatattgaga accaaatacc 29580

aattatacag agggagataa gattaaatgg ctcgttataa aaaacacgta gtacaatatg 29640

gcgatactat gcagtctatc tctcagctag agactggtag tgttggaaac tgggtagata 29700

tagtagaata taataagtta aagtatcctt atattgtaca aacaaatgag gaaagaaaag 29760

aaaatcttga gagtacagtt actattgggg atgttattat catacctgta gcatcagacc 29820

taacagaaat agacccttat gctctaacaa accaagataa gaacttaata atgagtttag 29880

ctctaggtag ggatttaagt atgacctcca atcaagatta ctataataaa agaggaacta 29940

gtgacgagat actagaagtt gtagctaacg gcaagggaga cttagcgact gttgacggag 30000

tagaaaacct aaagcaggca ctaatagcta gactattaac tcctaaagga gccttgatgc 30060

tacatccaga atatggctct aacttacaca agatgtttaa aaaagctaca catgaacaag 30120

catcactaat agatattgaa ataacaagaa cattgttact agacggtaga gtagaaacag 30180

tagaaagtct cggatacaaa gtagaagagg acgtatatgt aggaaaattc ctagttacta 30240

tagagtcttt acagacagcg tttgagttcg ttgttcaagg tgatactaca ggttctatta 30300

ttttgctaga taattgagga ggaaaacagg ttgagattaa aaagaatttc agaaatttta 30360

tctcgtttaa tagataagat tatgattaac acatcagagc taaatgattt ttctgtaggg 30420

tctactatct tgtctatact agaggcagtt tctatggagt tggagcagta ctacatccta 30480

tctagagaga acattgtgtg gggtattcag caaggggtat ttcaggcttt cgactttttt 30540

aagagagagc caaaaagagc ttatggagat gtcctaattg agtttcatag cgcagttcaa 30600

agtgatgtat atattccaaa aggaaccact tttagttcaa caattaacgg atacgaccag 30660

caattcgaga cagtcataga ctactacgtt cctaaagatt ccatattagc tcgtgtagaa 30720

gtgtattgta aagtagcggg ggaaataggt aatgtcccag caggaactat taatttgatt 30780

atgaatagtg taagtaacgt taagagtgtc ataaatgaga gtaacttcca aacaggaact 30840

gatgaagagt ccttagaatc agtaaaaaat aggtttcatg ctttcgtaga gagcagaggg 30900

agagctacag tccctgctat aaactatggt acccgtttag tagaggaagt atcaggcgta 30960

tatgtaaagg aatatgtagg gtatgtaatt gtctatgcac acgatagaaa cggaaacctt 31020

tcagctaaca tccaagataa cattaaaata gcattagaag actatagacc agcaggtata 31080

aaattagatg taaaacccgt aaacaaaagg ttgctacctg tcgaggttac tgtaactatt 31140

actaacaaaa acagaatagg ggatacacta caggcacaca ttcaaggagt gataacagac 31200

tacttaaata gtatgcaagt atctaacaac ttaattttat cagagcttat tcaaataatt 31260

atgagtatag acgactccct tatttatgac tgtaagattg aaaactttac agaaaatgtt 31320

ataataaata gtgaggatat tattagagca ggagaagtaa aggttaactt aaaataacat 31380

agttaacaaa tgagaggagc tgacaactac ccttgccaaa ctttttaaaa aatatacacc 31440

ctttattaaa aagaaaaaaa gatgacaacc aagtaagtag cgtaaactac gctgttttac 31500

aagcgctcga aacagagctg agtgaagtag aacaggaaac cttagagagt aaagtacagt 31560

catcccttgg aacagctaca ggagactacc tagatatttg gggagactgg tttaatgtat 31620

accgtagaga agatgaagga gacgaacgtt acagagatag gattattcgg catttgttat 31680

taaagagagg tacaatacca gctataatcg aggcaattcg tgattttcta gaagactata 31740

gcgcagatat tactatctat gagccgttta aaaatatctt ttacacaaat aaatcagact 31800

taaacggtga agactacttg atggggtact actatcgttt tgctatcatt gatgttacaa 31860

ttggtagacg gtttccttta gaagtaattg acattataaa cgcatttaag ccagcaggtg 31920

taaaattcta tctgacttat gacggtagtg gagaaattga tgaagaaaac agtatagttg 31980

aactaccacc ttatagtata gaaattgaaa catatcaaga ggtagatatt ctaaacgggt 32040

taaatacaac acttcacggg cacttcactt taggagagtc taatattgta gagggtgtta 32100

tccttgagtt gtttcgaaca aatagaagtc cactaaactc taatgatgta ctatctggct 32160

cttttaatgc aggtagacga gtatacaatc tagcaacaat tggagacaac tcctataaac 32220

ctacgatgag ttctcttatt ggggaaataa aaaattcgtt cgaggaatta cccgaagact 32280

tctacgtaga aactaaccgc ttagatgata ggtttgctag catagacatg gcaaataata 32340

cacaagatac tgcattctat ttcaacatgg atttaggaac ttatatcaat actaactacg 32400

caggatattt agctgagtta gcaggaagcc agattataaa tagagcacac tatgtatcct 32460

tagctaaaga gccaaagtta accgtggcgt ttaaagctaa aatatcccca gcaaacccag 32520

taaattacaa tatagaaggt tatgacttag tttctaaaaa atgggttgtt cttgagacag 32580

gagctatagc ggttgaaaag aaaatatcac agataccttt aggttcatta gaagactatc 32640

ttaatagtaa ctatatatgt tttttccgaa ttgtagtgaa acctaaacgg gaaacctact 32700

cctttgaatt agactatttc gaattaggat ttttaaacaa ctttaaagaa ggggagtata 32760

caattaagcc ttttacaggt attgtagaaa cttatgcaga gttgttcggt agagttatag 32820

aagttcctat gtatcggata ataaattcag gaacagaaaa tattatttct aaacaaggtc 32880

atatacctgt tagatatatt agagatactt tagagggtgg tagtactaca aacactgcta 32940

acctatggat agaaattcag gctaaaaata aagacaatat caatgttgcg cttaataagc 33000

ctgtatatag accacaagct gaacctacaa atggtaatat tcttctatat ccacagttcc 33060

ttaatattgg agctacttac atgaatggta agtggagagg aaaactaact agtgaggtaa 33120

acatggcata tactgtagac ggtgtaagaa cttacggagg aaacatgaac gcagatggaa 33180

cctatacagt ctatatcggg aacaaagtaa cctctactag ccaaaaagta gttgtggagg 33240

tatatgcaga cactgcaaga acgaagttaa tagacaaagc taatgttcct attacaaatg 33300

caggggacgg aagccctata gaggctatca cagacggagt aattagtggc tctaatatat 33360

tctatattgc accaacaagc gccaaatact cagtaactgt agacttagaa gctacctttg 33420

tagacttatc agaggtaagg atatggcact acccaacacg aacgtttaag tctgttttga 33480

cagaagtatc agcagatggt gtaaactgga ctactatata taactccgct gttgatggga 33540

cttatctaga aactagtgaa ggaaaagcgt ttcctgtacc agagatggac ttatatggat 33600

tgaaagctac tcctctaaca agtgtactag caggttctga aagacaaatt attacattcc 33660

tagactacaa aatagaggac tttgagaacc cagctaatac tattgaaaac ctaaacaatg 33720

attactttaa cgcagtgtgg caagagattg accgacttga agagtttgtt ctaggcaagt 33780

tcactatttt agctgatgtt ggctctgacg gagtactagt aaatgactgg ggtcttattg 33840

aggctaaaat acctacaggt gctacagcat accaagaagc ggacagtaga agtgaattgt 33900

cttccgtgta cactgtggag ctcacagcgg gctctcacga ggttattatg gatgttgcta 33960

ataaagttgt tactacagag gcagtaaccc taaaaacgga ttacagggtg tctaacgagc 34020

ttaccttgga agggctacct acaaaaatac ctgtgagata cattcgagac tccttgtggg 34080

gaggaaacag tataaataca gcaaaccact gggtagagct acaggctaga gacttgaaag 34140

gggacaatgt tgcccttaat aaaaaagtgg tagagaacaa cggagttagc ttactatctc 34200

ctgaaaatct aatgttagac tcgaagacag agattactag tacaacaaac aatggttata 34260

cttttgcttc tgatatagct tatatatttg ataattatgg agtagataaa gagtatacta 34320

ttagttttga catcaagtcc actacggtag gcacagttac tgtttatagt aggtacaaag 34380

atgaagctaa atattctata ggaaacaaaa ctgttcctgt aacgactgct tacacaagac 34440

agcaagttgt agtaacacct actagaaaca cagcagaaca agctaaatat gcagtcagct 34500

ctattctagg ttttcttaat agtacaggag caatatctac agttaaggat ataaaaatat 34560

cagtaggaaa catctcgaat atgatttgga cacctgcacc taaagactac agtattctta 34620

ctgatggagt aacaacaagt gctaacggat tcttgagtgg agttaactcc ttaactattg 34680

acttaggtag ccctagatat gatattgaca acatcactat gtggcactat tggcaagggg 34740

ctgaacctag gtactacaaa tatgtaaaac tagaagtatc agcagatggt gtgaactgga 34800

ctacactgtt tgatagtgat gtaagtggaa cttattacga gactagtgga ggtaagcagg 34860

ttagcgtacc tgctataaaa gaatacctta agagtactaa tgctatattt gcacctagcg 34920

tagcaggaac aggtactttt gagagagtaa ccgaagcaaa cggagtaagt tctataaaag 34980

tgacacctga tactgataag acagctagaa tgtacaagtt tttcggaact tctacagacc 35040

ctaacgctaa ttcgagagac cttctagaat cagggctaac tacaacgaag aaatacacag 35100

tagcagtatc cctaaaacct caaaataata gttcggttat cttagcggta aaagatggaa 35160

gtaacactaa catagagtcg ggctccttaa atattgcaaa agatgtgtgg acaagagttt 35220

cttacaatgt aacgttacct gcaacagcta ttgataggct agctttatgt ttgtacaatg 35280

ctaacacagg agaatatatt aattataaag attggagcat taaagaaacg atgtttccat 35340

aaagtaaaaa ttatggtata atagagatag ggcacaaagt agtgccttat ctttgctata 35400

ttagtaatga gagtacgaat acaataccga ttttaaaata aggaaatgag gtaaataaat 35460

ggcaatagca acatataatg cgcatgtata tgcagcacta aacctaaaaa gtaaggtcga 35520

tacaacattt atggcaattg gtaaaacaac agcgtggact gatgagacta accctccaga 35580

accagaccca aatgctacag ggttaacaga agttatcggt tacaagaagc taaaaactat 35640

gtctctatgt agaccacaaa gaacaggtga aacaccaaca ctacctacag tttcttacgg 35700

aaataaaaca tgggttcttg tacctgatgc acaagcttat acagaaggag ctaaatggct 35760

ttattgtgag gcagagttcg taggggatga actaccagta ggaacataca gacaagtagg 35820

tgtatttaca gacttagcgc caaaatctgg tgtaacaaaa ccgaatttat taccaagtga 35880

agtagctaat gtaggtgtac tacagttttt tgagaacaag cagttccaaa acagaacacc 35940

acaagttaca gctagagaaa gattcgtagc ggaattataa gaaaggtgaa ataagtttgg 36000

cagatttcaa ttttagttca gcaccatatc ataataggtt caatgccttg aatgattgga 36060

cattagcact tttccaacca gacagacccc ttcaacagtc cgagttaaac gaaatgcaat 36120

ctattttgca ttattaccta aaatctgttg gggatgctgt tttttctgat gggaacatgc 36180

aaacaggtat ggagtttgtc ttggataact caggaaactt aactgttggt gatggtcaag 36240

tttatgttga gggcaaagta agaaacttca aaaaacaaac tataaaatta aataagatag 36300

gtagagagtt tgtaggagta aaagtagaac agtctattat taccgcagac caagacagta 36360

gtcttctaga ccaaacagca ggagttccta gtgctttttc taaaggagct gacagactaa 36420

aagaaactgt tgttttaacg ttagacgacc ctcagtcagc aactatctac cagtttgata 36480

acggaaaact ttttatctcc tctgataacc cagagatgac aaaaataaac gaaattctag 36540

ctgaaagaac atacgatgaa tcaggctctt atcgtgttaa cggttttaac atgtggacag 36600

aacctcatcc gacagacact agtaaaattc agttagttat tgacacaggt agagcttatg 36660

tgttaggata caaggtagat aaaccaacat ctacacgtat acctttagat aaagctagag 36720

atactcgtag tgtagccaat gaagggcaat actacgatac agcagttaac cgtattaagc 36780

taggaaacag tcctgtagaa acagtaacac gagttacagg tcaagttcaa gtacctaagg 36840

agcaagtgag cagaggttct caatcaggag gcaccgactt cttgagtaga acttccgtat 36900

tccagataga ccgtgtgtgg acagaaaacc caacatcagg agaagttatt aaggaataca 36960

ctcaagggac agacttccag ttaataaatg gacaagcggt ttcatgggca cctaacggag 37020

cagaacctac ggcaggagca acttactacg tgtcttaccg ttataacaag tctatggtac 37080

tgaatacaga ctacaagata actattgaag gtacagggga tacaaaaact tggtatattg 37140

attttagtga tatgacagga agtaaaccta tccaacaaag tttagtaaac gttgattaca 37200

actactactt ggctcgtaaa gacctaatag tacttgatag atacggagaa gttacagtac 37260

acaaaggaca accagataac ttaagattag cggaagcacc tgcacaggta gacccctcaa 37320

cacttcaaat tggggtagtt actatttacc ctgaaagtga gaccgctagt gctatgtcat 37380

tctctgttac tcgtctttct atggctgatt tacaaaaaat gagaacacgt attgataata 37440

ttgagtataa cgaagcagta aatgctttag ataatcctgc aatggcagga gaaaacccag 37500

tgacattacg ttctgtattc tctgacggtt ttatttcact agacaaatac gatagcacgc 37560

atccagatgc tattatagct tttagttttg aggatgcaga aattacccta ccttaccaag 37620

aagttaacaa ggtagttcca aaaatgttag agaacgcatc agaagcttat gtttggggac 37680

gtttagtatc agcaccattt acagaagaga aaacaatttc tcaacctttt gcatcagaag 37740

ctatgaacgt aaacccatat aatgtgttta ataaacaagg ggttattact ttaacacctt 37800

ctgcggataa ctggattgag gaagaacgag taactgtaac tcaggacgaa gttaagtctt 37860

atacggtaaa ccgttggtgg ctacatggag gtaactacta ttctgatgaa gctagatact 37920

tgcataacaa cctagcacta gatgcagggc agtcatggag tgggactcct tctcagaaca 37980

aacaagtaac aggaacaaca ttaacagcag gtggacaaca aacacttgag tctatgatag 38040

agtttatgcg acaaattgat atagtggtag aaggtaaaaa ctacttgcct aacgctaaca 38100

acttatacgc actattcgat ggtgttcggg tacctattac accagcttct ggttaccgaa 38160

aaggtacaga cacaggaaca ttaatggcta atgcagatgg gacagtaaaa ggaactatca 38220

ctattccagc aggtatccgt tgtggtgtga gagaagtaac tttacggaat gcagataact 38280

taggtatttc tacattcact gcacaaggta gaaagaaaac cgtgcaagac atcattatca 38340

gaactcacgt aacagctaac ttgtatgacc cgttagctca gtcattccaa ttcacagatg 38400

accgagttat aactagctta ggattacatt ttgctagtaa agatgcagga gaaaacaacg 38460

ttatcgttca gattagaggt atgagtgacg gaggacaacc aaataaaact atttatgcag 38520

aaagggtatt aaccccagct caagtaaatg tgtctgctga cagtagtaag gaaactaaag 38580

tcacttttga tgacccaatt atgtgtaaag caggtcaaga gtactgtatc gttattgtaa 38640

cagactctga tagttacact atgtggttag gtactagagg taaacctaga atagactcac 38700

ctagcgactt aattacgtca aacccttatg tacaaggtgt actatttagt tcttctaatg 38760

cttctacatg gacagcacac caagattctg atttgaagtt taatgtatat actgctaagt 38820

ttaacgaaca agctgtacta gaatttgaca caatgaaaaa tgttaaagtt gatagagtag 38880

tactaatgag tacgtaccta actcctaata acacaggatg tatctgggat atgaagattg 38940

ttctagacaa tgaacctaca ggagttacag tagcagacaa agaatggcaa ccaattggta 39000

actaccaaga tttggatgta gagcaattag ctagagaagt caagttaaga gctacttttg 39060

ttgctaacaa ctacatttca ccattgttat cattggacga catcatgttt gcaggtttct 39120

taactgcttt aaaaggtagt tatgttagta gaaatattaa tatgacagac tctccttata 39180

atacagttaa gattagttac gaagctttcc taccagctaa cactacagta aaagcacagt 39240

ttagtactga tgcaggtatt acatggaaag acttgacgtc tattcctgta gtaacacaac 39300

aaacagccga tttctacaag tatgaatacg aagagaaagt catccaatca ggcacatata 39360

aaggatttaa ggttcggctg aacttagctt cacaaaacag tttcttaaga cctcgtgctc 39420

ggagactaat gtgtttaatg aaagatgaat aggggaactt ttgttccctt atttttaact 39480

aaaaaggggc ggtaaacatg ccagagtcat atagagaccc ccattcaggg gctttaaagt 39540

ttattcctac gaaacaggag aaaataatta aagatgtgac tgaaaaacta agagaagacg 39600

aagaaaaaat tcgaaagaaa atagaagagt tagatattaa actagcacag gcagacagca 39660

ggttgcaaga aataactaac ttgtatgaag aattaaaaga actaaaggag ggtaactaaa 39720

tttgtctaga tacgaccata gtactgtaga gtacacagac aaaattaagg acttaacaga 39780

ttcggtaaac cgagtaggaa actacttatc aggagacaat agcccttatg acgatgttca 39840

gaaattaaaa gcaattacac aaaatattaa gctaactaaa gatacaggtt tagctaaaag 39900

cattacagca ggaacaactg cgttgaggtc agtagtcgaa gtaggggttt actatattaa 39960

tagcacagaa gctctagccc taactgataa acccccagag ctaacaggag cctttatact 40020

agtaaactac ccgacaacag cctctacttc tgttaagcaa gaagtacaca tgtttgctac 40080

aggaacaaca ggtagctacg tagggtaccg atggatttct gcttctagtg tcagctcatg 40140

gtggacttat gaaaacacac taggttcaca agctaaagca gataaagcat tagcggacgg 40200

gaaaacatat actgatagct cagtaaactc cgctttgcaa gcaattcaaa actcagcaca 40260

aatgtacaag ttaacagcgg atgatggtaa gcctattgat gcttctgcta tggctactcc 40320

accaactagt gtagcttctc taacgaagac aggaatatat tactttactg ccgcttttgg 40380

aaatacaatg ccagataccc cttgcacagg gcaaccattc tggttggttg ttttacagca 40440

tgtaacagat aacagtatat cacaatctgt gacagccaac actgtagcag tagagagggt 40500

agtcgcagat aggattatta ctactctagg ggtaccgagt aagtgggaat acagagctaa 40560

agcctctaat ttcttcttta gtgcttccaa ttcgtcacgt ataacactag tatcgaccca 40620

acagaatatt ataacaccta ataaatttat taataacccc ggtagtttac cactatctgc 40680

gataaacccc gcaataacta ttccagaaga tgggatgtac caagtgatgg cggccctaaa 40740

cattaacggt attttaaaaa aagtgtacct agtaagtgag ctaactctac taataaatga 40800

cgtagtacac ccagctacat ttggaatggt aaaaacagta gataatgcta acgggcagta 40860

ctctttagca gggaatggca tatatcaact aaaaaaaggg gacaagttaa aactaagaag 40920

ttactgtaac aatacaggga ataaccctta cttggatgta gacaaacttt atatatctgt 40980

tggtaagatt gcagacattt cattatttaa ataaacttag tatgctgaga aaggaagtca 41040

caataatggc agaagaaaaa ttttcattag acggaaggca gtatgtaaca gcagtcctat 41100

atatgtaccc taacctagac cctcagtacg actttggagt tgtatcatcc gaagggaagg 41160

tagaggcttt tgaatggaac gtaaaaaata taacaaagcc ttctattgca cagttagacc 41220

aagcatataa agactatgta aaatctatcg gaagtccaag taacccacca gccctagaac 41280

cagaggtctc aaaagaagcc tcaataggta agattctaac tgaaaaattt agtgcgttag 41340

aggaagttaa tgctaatatg ttattggaaa atttagagct tcaagctagc ctagataaaa 41400

caaaacaaaa aaattcagaa ctagtatttg agctagtaga caggggagtc ttatgagtgt 41460

aaattggtat gagttagttt tagattacta cacaaaaggt tattacacac caagtcaggt 41520

aaaaagattt gctgaggtag gaaaactaac tttcgagcaa gccgaagaaa ttatagctac 41580

aaaatcatag agtatgttta aactatatag gtaattaaga ctaggactaa tatcctagtc 41640

tttttatgtt ataataaaga tatagtaaat atatttggag gagtacatta atgagaattg 41700

ttgtagatat aatgaataca cgagttttat tcgataaaga agaagaggca attaaaaaaa 41760

gatttcttag ggatatgcac caaaaattag gggtaaaagt agatgggttc ttctttagcc 41820

cagcatataa atcaggtcat tgggacggta tccaagactt ctatgacttt aaaacaaata 41880

cgttccctac agggctgttg ccacaagtgc aagagatact aggagctatg caaaatgatg 41940

ccgtatttca atttgagatt attgatgaca gaccagaccc gtttttaaca gtagaggact 42000

tagatagtga gataactctt ataggagatg acggaggaga tatacagctt agagattatc 42060

agtatgactc tgtaaagtct atcattgaaa actatagtgg gattatcaat attgcaacta 42120

atggaggaaa aacggagatt gcttctggta tcattcagca gattttaccg catttagagt 42180

ctggggagag aatagccttt tttactcact cctcatctat atttaaccaa tcaattgacc 42240

gtatagaaaa aagactaggt attaaagtag gtaggctagg aaatggtaaa aaggacatca 42300

gacaagttac ttttgtaatg atacctacgt taacagcctc cctaaaagac cccgaagttg 42360

gtttgaaatt aacgcataag gaacgagtta ttaagaagtt agctaaagag gtagttcctc 42420

gtttcactat gcaaaaaaat catagaaagc ttcttaatat ttatattgag aacaacagac 42480

cgaaaaataa agtagaagaa gatataaaag ctcgactaga agacttgtta tattcctgtg 42540

gaacagactc cgaagttcta atgggactaa aaggttttaa cgcagaatat gaaaaaatta 42600

ttgagaaaaa aaataaaaaa ctatatgaca agaacaaaca agctagagaa tttctatcat 42660

ctgtatctgt tatgattgcg gatgaggcgc accataccag ttcagaaact tggtacaact 42720

ctttgatgtc ttgtgagaat gctatgtaca gggtggcttt gacaggttct atagacaaag 42780

aaaataaaat gttatggcag agaatgcaag gagtattcgg tcaaattacg gctagaacaa 42840

gtaatgagac cttaattaca cttgggcact ctgctaaacc aacgattaaa atattcccta 42900

tcaaggctcc tacagatatt gaaaatgtag ataactatgc tactgcttat gaaaaaggaa 42960

tagtagaaaa tgattatcgt aatttactta ttgcaaaatt agcaaagatg tggtatgata 43020

aagacaaagg tgttcttatt atagtaaata gaatagagca cggtaacaga ttatctgaac 43080

ttcttgagtc tctcgaagtg tcgcactact tcattcatgg agacttagac atagatttaa 43140

gagaacaaca actccaaaat atgagagatg ggaagcttaa agtgatgatt tctacatcta 43200

ttgtagatga gggagtagac atctcaggta ttgatgcgct tattttaggt gcaggaggga 43260

agtctttaag acagacactt caacgtgttg gcagggcttt acgtaagaag aagcaaggag 43320

aaaacacttg ccaagtattt gactttaaag atatgactca tagattttta ctaagtcact 43380

cagaccaacg aagacagatt tacgtagatg aaaactttga aattcaagac atttaggggg 43440

aagaacaaga atggatttag tatatgtagg atgtcagatg gaaataggaa tgaggaaagg 43500

tttacgagac tttctagtgg atgttgctaa aacagctcag caatggggag aaagcgtggc 43560

tatggttact caaaaacata gcctagctga gcaaacagga gtaaacatta gaacagtctc 43620

acgttatata gctgaacttg aaaacaaagg tattattaaa acagaagcaa aaagaggaag 43680

aaacggtgga ctagttatca tgtttaataa aaatatggtt acttttaaat taacagacaa 43740

ccctataacc tctgattcac ctgatgcggt agaacttaga gaccgtatgt tccctaaaaa 43800

gaatgaagac ccgaataaag agaaaagacc tcgtagaaca aaagaacaaa tggcagagga 43860

aaaagtacta aaaaatcgta agagaagtct taataatgag ttaaacgata tgttagacga 43920

tacattattc cctacagaag agatatttag taaaacagag gagcctgaga ggtactttaa 43980

agcttattta ctgtctcgaa tggttaatgc ttatgcgtat atttatccta cagagagaga 44040

gctagacatg ctaagcagag gggtgcaggt agaagcagag aaggcacgaa aaaatagagt 44100

acgagcttac tcttttaact cactacctgc tagatttttt gggacatctc agtttacttg 44160

ctacttaaaa ctacaagact tattagaaaa agagaacatt aacccattat cttatttaac 44220

agttcaattt gactacttag actacagatt agataacagt cttggaggaa ctccactagc 44280

atatagaggt atccctgagg caaactgctt aataggtgag actgccttta gtcgttacac 44340

atctgctaag gacttctaca ctgttatgcg aggtaaaaac atgttacgca ccagtacagc 44400

agatgttgcc tttaaaggtg gaaaataccc tattgtggag gcgttattac acgcttacaa 44460

tgtaggtaag caagatatta ctgcactaaa ccaacaaata gaggatatga agacacaagc 44520

tagtgtgttc cataaatccg aagccctagc agacttttat gaaacaacaa aactattagt 44580

tgaagaaagt gctttagaaa caaatgaaaa agacgagcta tacaaatttg ttactgaaca 44640

agttgtttta cactctcgta agaatgcaat gagtcttagc caacagttgc taagcttccc 44700

acttcaaatt caacgtgtaa gacagacagc cgaattaaaa ggattagaca aaaaggacta 44760

ctatactttt gtaggcaact tagagaaaca agtgaaagta agtagaaaca cgtttaactc 44820

gtttactgaa aaaggtaagc ttttacacga gtctctagag gcaaacagta ctttttatag 44880

taccattcgt atgctaggag actaccgagg actaaccgta aacatcccgt tactcggaga 44940

agctatcaag aaattaggag aggaacatat tcctctagat tcttatggaa tgttagatat 45000

aacacaggtt tacgacaagt atttaacaga cctgcaaatg caagaagatg aagagaacta 45060

tgaaacacct acaagtttta ttgttaagta aggaggagga gatatacgtt gagttccatt 45120

attcaaaagc aagttatata ccgagctatt agagaccctt tatttggtaa ggatgtgtta 45180

gcacagttac ctagtaatgt atttgacgag aatgagcact acaaaaatct agtaattatt 45240

attaaaaagc attaccagac aagtaacaag gcgcttgatg aaagtacgtt gctaactatt 45300

acggaggaac atttagttag acagaaaaag agcttagaag tacaggaaag ctttaatgag 45360

acaattcatg atttgtataa aattgatgaa ctaggtatag acgatgaggc tgtcggagaa 45420

agcattaaac gattcgtcaa aaaaaatctg gcagctcact tgataaagga gactttgtct 45480

aagggtgaag ggctagacaa cgaagaaaca ttagaaaaac tatacacagg tatcaaagaa 45540

atctctattt tagatattaa ccgtagtgaa aacactttat ttagtttctt tgatgatgta 45600

gaagccaaga aaaagatgct acaaagtatc caccaagata agttctctac aggattcaca 45660

gacttagatg ttattgctga gggtggtatt ggacgtggag agctcggtct agttattgcc 45720

cctacaggtg gtggtaaaac tatgtgggca gtaaaccaag caaaaaacta tgttgctcaa 45780

ggtttaaacg ttttgtatgt agtgttagag gaacgattag aacgtatgat tttacgtttt 45840

gaacaaaata tgatgggtat ccctaaatct cgacttatgc cagataatga acttgacgaa 45900

gaagctttcg acaaattaca aacgatttat gatattaaac gtaaacaagg attaggtaat 45960

ctttacattg aaaagagaat gcctcaggaa gttagtccta caatgttaga gcaaattatc 46020

gttgatgcta gtgttcgtaa agggaaacat ttagatgtgg ttattattga ctatcctgac 46080

ttaatgaaaa accctcatga aagagataat agtgagtccc gtgcaggagg aaaactatac 46140

gaagaaatac gtaagttagc tcaaacctat aagtttgctg gttgggtact ctctcaggtt 46200

aaccgcggag gatattcaga ggatgtactt actgctaaaa gtatcgaggg ttctaaacag 46260

aaactaaatg ctgtggaaat tgctatgacc ttaaaccaaa aacctattga gtttgaaaaa 46320

ggttttatta gagcatatgc cgacaagatt cgtaataata acggtaaatc ttataaccgt 46380

atgcttagct ttaaggttgt tccagaaaca atgactatca gaaatataac acctgatgaa 46440

agagtagaac acgatatgat tttaaataac ctcgaagaaa aagaaggtcg tgtatttaaa 46500

aatacgaaaa aggctacacc tgacccccaa gcagttgtag acgaagtaaa agcactaaat 46560

agtagattat aaggaggaga tagtttttgg ataaaattat attttttact gattttcatg 46620

cccatatttt taccgaattt tctaaaccag atgtggagta tgaaacagac agatttagag 46680

aacaaatgga agtcttacaa caggtatttg acttagctag ggagaataag gcagatattg 46740

tttttggagg agacttattc cataaacgag gagctgtaga tactagagta ctaaaccatg 46800

tttttcgtgt atttgaaaga aacacggata ttgtagttca cctactgaga ggaaatcatg 46860

atagctttac aaactcgatg aacacagaga gtagtttgga gacacttaga ggtttaccta 46920

atgtggaagt ttacagcaac ccttctattg tagagactga aaaatataag ttatatatgc 46980

taccttatgg ggatgaagtg caagagatga aagactggat taacagttta gacattgaag 47040

caggtaaaag taatatctta gttgctcata taggggtaga gggagcgtta acaggtaaag 47100

gttcccacag actagaaggt gcatttacaa caggagattt aagaacaagc gactttgatg 47160

ttattgctct tgggcattat cacaagagac agctactact agatgttcct aatatatttt 47220

atggggggaa caccattcag aacacttttt ctgatgaagg gcaagagaag ggtgtatggc 47280

taattgactg ttcagatgtt aaaaatacaa caagtacttt tattcctatt agtagccgta 47340

agtttattac tattgacgga gataacattc ctgatgatat agaagaactt attgaacaag 47400

gcaactttat taggtttatt ggaagtaaag aacaggctaa aaaaattgac ttatatggag 47460

aagaaagtac aggtattaga gtagagctac gaaaagagta tacaactgat gttcgactag 47520

ggattgatga aaagtcctct cccgaagcta tttctaaagc ctatgcagag aaatttagtc 47580

cctctgcaac aagtgaaata gtagagtgta taaaagaggc acttgtgaga gaaaaataag 47640

cctaaagctg aggatattcc tcagcttttc ttattgactt ttttagtaaa gtatagtata 47700

ctaaagttac aaataaaaag aaggaggaaa agaatgttca cttttaaaaa agtaaagata 47760

aaaaacttct tatctatcaa agacatggag cttaatttag acaaacaagg tctagttctg 47820

atagaaggaa agaataagac aaatgaagcc tttaagtcta atggtgcagg taagacaagt 47880

atgattgatg ctattactta cgctatcttt ggtaagacag taggtgggct taaatcggat 47940

tcagttgtta ataacaaaga gaagaagaac actgctgtaa ttttagactt cgaagttgac 48000

aagaataaat accgaattga acgctataga aaagacaaaa aagagggtaa tatagtaaag 48060

ctctaccaag gtaaaaacaa tattacaaag agtacagtgg acaacacaga taaagagatt 48120

ttatctctat tcggtattga ttttaatacg tatgtaaatg ctattatgta cggtcaagga 48180

gacatgccta ttttctccca agcaacggat aaaggtaaaa aggagatact agagagaatt 48240

actagtattg acatctatca gaaggcacaa gaaatcgcta aagagaaagt taaagagaag 48300

caagctgagc aacaagagat agaaaatgaa cttgtaaaat atgactatga gcttgtagct 48360

attgacaact tagaagagca agaaaaagaa aactataaag cagtccagca aacaatagaa 48420

ggtatccaag caaccctatc agtagctagt acagagctag aaaactttat gacagaggct 48480

taccctaaga aggcagaatt agagaatagt attgcacagt ttatagtccc tcaagtagat 48540

aatacaaaag ttaacgagct aagagctgac ttaacggagg cagaacgtaa attattacaa 48600

attaacagta ttaaagaagg ttttgacaga gacttacgaa agctagctaa agactatgaa 48660

aacctttcta gtagcaagat ttgtcctaca tgtggaacag acctcggggt ggatagtgaa 48720

gcacacattg cagaaacaaa agcagatatt gaaaaacaaa tgagtgttct tggtgcaaaa 48780

acccataaaa ttttagtagc gttaccttct gtaaatacaa ctattgagag caaaaaggag 48840

ctcgtacaac aagagatagc taaaaatgag gagatacaaa gaaattacaa tcagctagtg 48900

tcaacagtca ggggatatga acaagaggta caacaactag aaaacacaaa aatagtttta 48960

gaagagaaag tggcttcaca acaaacatat ttatcacagg tacaaagtat gcctaagcca 49020

aaaagtagag acaaggatag agaagaagta cacaaaaaaa tagaagcaac taagttgcaa 49080

gtgctagaca aacaaaaaga gactgagaag tatgaggaag cagtaaaagc cttctcaaat 49140

gctggtattc gttctatggt tcttgactta gtaactcctt ttttaaatga gaaagctaat 49200

aaatatttaa cagtgttgtc aggttcagat attgaaatta attttactac acaagttaaa 49260

aactctaaag gggaactaaa agataagttt gatgtagaaa taatcaatgg tagtggaggc 49320

tctaactata aagctaactc tgctggtgag aagaaaagaa tcgaccttgc aatttctttt 49380

gcaatccaag atttagttat gagtaaggca gaactatcta caaatattgc attgtatgac 49440

gaatgttttg acggtctgga cagcataggt tgtgagaacg ttattacttt gttaaaagaa 49500

agattagaaa cagtaagcag tatatttgtt ataacgcaca atgagaactt aaaaccttta 49560

tttgagaagg taatcactgt gcagaaagag gatggggtta ctaccctaat aaactaagga 49620

tttggagagc tataaaatga aagtaaaaat tatgacagaa gaaaaagggg tactcacctc 49680

agaagtaagc aagaggtcaa ctaagaaaga ggctatctac tccgcaaatg tttttcctat 49740

tagtagcttg tacaagtggt acccctttaa tactaactac gcctatgtag tagacaagca 49800

tggggtatac agactcactg aactagatga ctgtattcat acatcgtata acttggtaag 49860

tgaagaaaaa gctaagttag acccgacaaa gtggcacaat aaaaccgtaa cacaaagcaa 49920

cttattactt tgtagactag caaataagct agggataaaa agcattaaag atgattttaa 49980

ccaacaagat gtagaaaacg gatttgaggt tatcagtaca gagttagcct atatcaaaga 50040

acaagcgaca gcacgttgct acggggataa agatgtcttt gaaagaagtg aactactcaa 50100

gaaaagtaaa gaagaaagag aacaagcata ctttgagtac ttaactgtag aggaggtaaa 50160

cttccaatat aagttaggag agctatacgc tatgcttgta cttatgcgga aggtggtaaa 50220

ctactaatgt ttttagacta cattacaagt gagctaggaa cacataaaga tatagggcat 50280

gaattacggt tctgttgccc tttttgtgga gaagacgact acaaatttta tgtgaaggta 50340

tcaggggaca aaacagaagg tctttggcat tgtaaaaaat gtaatgaaaa aggtaaccct 50400

gttacttttg ttatgaagta tcttaacatt aattttaaag atgccttaga tatactagag 50460

gtgtacgact atgagataga cgaaaacagc ctatctagca gagacgtagg ggtaacagag 50520

gaagagtttc tgcttatact attaaagaaa tctagtgagc ctgaggaagt agaaattagt 50580

gaggaagtga ctctcacgcc acctaggcta cctaccaatt ttaaaagact agcagacaac 50640

atgaataacc ccgaggcatt accttttcta aactacgcac actcccgagg tataacttta 50700

gaccaactat tcctacacaa cataggatat gttatagagg ggcaagtaga aaaagtagat 50760

ggtaatatga tgacacttag aaatcacctt gtttttctaa cacatgggaa tgatggacaa 50820

taccaatatt ggaacacccg agccataaca cctatgggga taaaagcttt taatgctcct 50880

agcaaacagg acgagtactc taaaagaaca actgttttta acctaaatat tgctaagaat 50940

acgccttatg ttattgttca cgaaggggta attgatgcct taacaatggg agagtcggga 51000

gtagctactt ttggtaaaca gataacaaga gaacagatag agttactatt aaataatgtt 51060

agagaagacc agcctattta tttattccta gatacagagg cttatgaagt tactactaag 51120

atagctagca gtctttatga aaagcataaa gaaacctaca tcgttatcca tggagatgag 51180

gatgccaatg atatgggata tgaaaaatct tgggactgtt taagaaataa ttcagtaaga 51240

gctaatgagg aaggtataag tattttttta gtatcttgca ttcagtaata aagtatgata 51300

taatagagca gacaagtaag aaggaggaat tttattgagt aataaagtaa atgtaaaagt 51360

taagttagag ggaggagcta ctatgccctc acaagggtat gataatgatg tagccttcga 51420

tttatacaca tcagaggatg ctattatttg ccctgatgca gtgggagcta cactaattcc 51480

tacaggatta agaacagagt ttgaccccac taagtttggg ctattcatca gtcctcgttc 51540

aagtattatg aacttacctt taaccctagc taacaacaca ggaattgtag aaggaacata 51600

tcgtggaggt attggtattc ctttaaaaaa tacgttgtct atgacaggtt tttctgattt 51660

tgcactaaca tggaacccag acaagaaggt agtagaacgt atccctgtag agaagttacc 51720

tacaggtaaa cttagaagtg cggaaaaaat gttttacaca gaccaagcat tattgtatga 51780

taacacacta ccagaagaac tagcagacca attgtttgtt actttggtgc cgactgggac 51840

attcttttta ccaaaaggct tccgtattat gcaagcttac cttctaccta aatatcatct 51900

agacctagaa aaaacagaca aactatcaga cagccctaga ggggataaag ggtttggcag 51960

ttcaggacat ggaggaaagt gattccttta gaccctatta tccttattgc cgactctata 52020

ggcataggtt attcaaaagg aaatataaca ctgtctttaa aactacggga taagtatacg 52080

ggtaccgagg tagaaaaaat agctttatta gccgttggta gcttctcaga aaaagtaacg 52140

cttgttagta gtgagctagt agataatgtg tataaaataa aagttaagat ggaggaatta 52200

tgaaagattt tactcagtat tttaaagaag tacaagatgc acaaaaaaga gaggactctg 52260

aaactgtaga aagacttgca gtagaatatg gagataaaac tattacgtat aaagaactta 52320

gacatacgtt aggttccgtc ttgttgccac taattattgg aacaatggaa atcaataagc 52380

agttggcaga cacctcttta gacatcgtta tcaatatgtt agaaaaagag aatgtattat 52440

cagaggatac cttaattgat ttaaggcaac taattactag catgaaacaa tcagtagaag 52500

gaggagaagg agaaaatgag taaagaacaa aagcagttaa gtaaagagga tttaacttta 52560

tgggttaact ttgtagtaac tggtaaggtt gaaaaaggga cggagaaaca gctaaatcgt 52620

atttctaaac gagaggtatc tgtaggagat gtaaccaccc taatccgtgc tcttacaagt 52680

catcatgatg cgtacattag ttctcttatt gaagctaatt cagttcttag agtggtaatg 52740

cgagatgagt taaatatctc agaagctcaa atggacaagg cgttaaatac ctataagcaa 52800

caagtaggta cagcactcca aaagacacat gatgctatca tggaaaaagc acaagaaaat 52860

gtggagttca cagaagaaga actagaggta cagaaaaaac tagaagaata tgatactaaa 52920

aataaaaaag aggaataaga atggctagta caggaagagg aagtcaagta aaaggttcta 52980

aatttgaaag agagaccgct aaagctttat ctgtttggtg gggagctacc tttaatagaa 53040

caccgggttc tggcgggcta aaatgggcgg aagaaaacaa tgtaggtggc gatatagtag 53100

cccctccctc cgcaggtttc cctttcattg ttgaatgtaa aaaacatgaa gggtggacac 53160

tagacaatgt attccttaat acaggagagc ctaagtcttg gtggaaacaa gtagtatcag 53220

atgccacaag aacgaataag atacccttgt taatgttctc tagaaacagg gctaaaagct 53280

ttgttatgct accttatgat gacttgctat ataacgtatt acgtgataaa agaagtacag 53340

tagctagaac tacagtgtca tttaagaata tcagggaaga aagtgtgttc tatgatgtaa 53400

tggtgttcac tatagaagag ttaactagct tctctcctga ttttttaaga gaacactata 53460

gtaagtatag ttgggatttc ttttacaaag aagtggacga gaactctgta gaagatttag 53520

aaacaggtat agataaaatg ttagacaacc tagaaggtta aaataaagaa aaacactaag 53580

taggtctttt gtggtgacat atacataagc ctacttttag tgtattgtgc tatactagag 53640

tagaacggaa aaacaaaata tgtttggagg aatatcgttg acaataatag taaaagacaa 53700

cggaagtaga aaactacctt ttgaggaaga cagactaaac aggtacctag ataacataaa 53760

aaaagagttt cctaagttaa atatagagag ttatcgaaaa aaggcaatac actatatttt 53820

aagcaaggac gagtattctg tagacgaaat ggtagactac ctaataagag aagcagaatc 53880

tcgggtagac gttaccacac ctgaatggga gttttttgct actagactat atttacaaag 53940

attgtataag aaagctagta aaaacaggtt ttacgatgta attatgaagt atggttctta 54000

cgtgggtttg catgagagtc tagcacaaag aggaatatat tccgtagata ttttagagaa 54060

gtattcaaga gaagagctta tggaagctga aacgtttatt gacagtagca aagaccttct 54120

ctttacttac aatggtttgt acctactagc tactcgctat ctagcaacag atgaggataa 54180

gaaagtattt gagttaccac aagaacgatg gttaactatt gctttatttt tgatgcaaga 54240

tgaaaaagaa aatagaatga agttagtaga agaagcttat tgggcgttaa gtaacttgta 54300

tatgacagta gcaaccccca cattagcaaa tgcaggtaaa gtaggcgggc aactatccag 54360

ttgttttata gatacggtag atgatagttt gcaaggtatt tacgatagca atacagatgt 54420

tgctagagtt tctaagcaag gcggaggcgt aggagtctat ataggcaatg taaggtcgtc 54480

aggagcacct attagaggag ttctcggtgc tagtggggga acggtacctt ggattaaaca 54540

attaaacaat acggctgtta gtgtagacca gctaggagca agaaaaggcg ctataactgt 54600

aacgttagat gttttccaca aagatataga agcctttctg gatttaagac taaacaatgg 54660

agaccaacgt ttacgggctc acgacatttt tacttctgtt tctattcctg atttatttat 54720

ggaagctgta gagaatagag gggaatggta tttatttgac ccttacgagg ttaagcagaa 54780

aaaaggttgg tacctacaag actactatga taaagaaaaa ggtagtggtt tattccgaga 54840

aaaatatgaa gaactcattc aagatggcac gattagtaag aagagagtga aagctcttga 54900

tatgatgaaa cgtatcatga ttagtcagct agagacagga acaccattca tgttttatcg 54960

agatactgta aatagagcta accccaactc ccatgctgga atgatttatg ctagtaattt 55020

gtgttaattc tatggcacct ttgggtagca atactcatcg aaaacttttc ttaaacgggc 55080

atagctgaat acgatgaata agctggtaag agagcctaag tccttgagtg aggatagagg 55140

taatcccgtg ctaaattata ataaaatctt tattgtataa atgcctaacg actatcgaaa 55200

agctactttt tagtagaact tagtagagta cactcaagtg agtggaaaca gaaaagactc 55260

ccatgtggag tatgatatag tcttttcttt acagaaatgt aaagcagtta gtacactaac 55320

gggtagggaa ttaacgaccc ttactgaaag caagcaagaa atcgcacaaa atatgtcagc 55380

tacggtagtt acagaggaaa ttatagatgg agataaaatt gttgtaacaa aagaagcagg 55440

agacttcgta gtttgtaact tgtcttctat taacttaggt tctgtatttt ccgaagaaga 55500

cgttatcgac cgactagtaa ccattcaagt taggatgcta gacaatgtaa ttgagttaaa 55560
caaactacct gtaccacaag ctctaattac taaccagaag tatagggcag tgggattagg 55620
aacttttggg tggcaccacc tactagctaa aaaaggtatt cattgggaca gtgaagaggc 55680
tactaatttt gcagatagcc tatatgaaag aattgctttt cacactatcg aagcaagtat 55740
gaaactagcc gaggaaaaag gagcttaccc atactttaaa gacagttcgt gggatacagg 55800
ggagtacttc tctagtagaa actatactag tgatacttgg gttgacctag ctaaaaaagt 55860
gaaagagaaa ggtattagaa atggttactt aatggcagta gcacctaata tgtccacagc 55920
gcagatagca ggctctacag cctctattga ccctatctat gactctttct attatgaaga 55980
gaaaaaagac tacagacgac ctgtgatagc ccctgagttg aacttgaaca cttacgcttt 56040
ctataaaagt gcttacaaag taaaccagat tcaaagtgtt aagcagaatg gtaaaagaca 56100
aagacacata gaccaagcta tcagttttaa cttctatgta cctagtggca ttaaagcaag 56160
taaactactt gagttgcaca ctacagcgtg gagtgaaggt ctaaaaacaa cttactatgt 56220
tcgttctaat gacattgatg tggaagaatg tgagtggtgc tctagctaac agctattgac 56280
aacatgaccc ttttatgata tgcttatagc ataaagtaag agggtttttc cttgttgcag 56340
agaggtgtga gaaataattc aaggtatttg acaaacgatt taatgtgtgg tatacttgcc 56400
ctaaatgtac aggaaaacta ctaaaaatag aggaggagaa ctaattgtta aaaaaaataa 56460
aaatattaga accactaaac cctaacaaat caacagcgat tattaatgga gaggctagtg 56520
gtatcgtaaa ctggaacgat attcggtatc cgtctttcta tcgagcttac aaagagttaa 56580
ctacaaatta ctggttgcct gatgaggtag atatgaagag cgatgccaaa cagtacaacc 56640
acttatccga agaagagaag tatgcgtttg actccattat cggactttta gctacacttg 56700
actcacccca aacacgtttt atttataaca ttgcagagta tgtcagtgat gcctccgttc 56760
atgctattac agctatcatt gcacaacaag aagctattca taatgagagt tattcttatg 56820
tacttgcttc tattactaac ttacaagagc agaatcgagt atttgaggaa gctagaacac 56880
accctacgat tattaaacgt aatgcaccta ttatgaagtc ttatgacgat ttcatgaata 56940
acaaaacacc tgagaatatg gtaaaagctt tagtacagtc atctatctta gagggtatta 57000
atttctacag cggttttgct tacttctaca acctcgttcg tcaaaacaag atgacgggga 57060
caggtaaaat cattagcttc attaatcgtg atgaattagc ccattcgaaa tttatttctg 57120
agctaattag agctattgta ggggaaaacc ctgaactaca aacagatgaa ctaaatgact 57180
acgtgtacgc ttcttttaga catgccgtag agctagaaca agcgtggtct agtgaggtac 57240
tacaaggcat tgaaggaatt gatgtggaag aaatgttaga ctatgtaaaa tatcgtgcta 57300
ataaaatgtt agggatgtta ggtcttcaac caatgtatca aggtcacaca gaaaattcta 57360
tgacatggat taaagcttac gccgataact ttacagaaac aaaaaccgac tttttcgaaa 57420

tgcgtaattc tagttacaag aaaacaaatt tagacaatgg gttcgatgac ttatgagggt 57480

agggatttta tattcttcat tgagtggtaa cactagtgaa ctagctcaag gaattaaaga 57540

taacttggag gaactagcag tagaggtgaa atcttttcat attgaaagag aagaccaaat 57600

gactaatgaa gtaatccctt atctatctaa ttgtgacctg attatggtag gtacatggac 57660

aacagattac ggagtcatcc ctgaggagat ggaagaggct ttagaaaagt atgactatac 57720

ttgggtagat aagcctgttg ctgtatttgg aacaggagaa acccagtggg gtatggaata 57780

cttctgtgga gctaatgaca aaatagccaa aaagtaccac tctccttttg atacacttaa 57840

aatagagcag tttattatca cagaagagca agaaaataag ctattaagtt gggttactga 57900

tatagttaag cactggaggg agtagctatg tacgagctag aaaaacttct aggtaaagat 57960

aaacatggaa atcagataag aattggagat agactaagtt tacaactaga tgatggggag 58020

taccgagagt gcgtagtagc ggttagaaaa gttaatacta tcgtgcttaa ccctgtagct 58080

ttaactagta aaagtgaaac tgtaagtgta acaggtgtat tctttgtagt tactgaccca 58140

catacaggag aagaactacc tttactacct aaagtagata gtaacggtgt ggtagacact 58200

caaaaagcag aaataatctt ataattttat agaaagaagg aaaacaagtg cattcagtag 58260

aagtagaaaa gaaagaacag ttagcggaca ttgtatcaca agacaaggtt gtattaaagt 58320

tttataagga taactgtagc ccttgtaaga tagtaggtat gtctattgag gaagctagta 58380

gtaatataaa agaggatatt actatcgcta atgttaagtt agaaacttta ggagaggaag 58440

tcttctttga tttcggcatc aaaggcacac ctacactgtt gttagtagaa aaaggaatag 58500

agaaaaagag acatagtggg tttatttccc ctgatgactt ccaagagttt gttaaataag 58560

agaggcttag gcttctcttt ttttgttgac atttacaagt ttatatggta tagtaacagt 58620

agtaacagag gggaggtaaa aaaaatgaca aaaaagaata ttaaaataac gagtaagaca 58680

gaggtaaaaa atagctctgt tgaggcagga gacatcttct acattgaagg ggtgtctaag 58740

gggcttacta atttgtctgg tttgtatata gtgacatttg attttcatgc aacagaagag 58800

tcgctgtcta ggtacgctct agtttctttg gagacaggga tgttaaacac catttccatc 58860

aaccagtttt ctctagaaga aaaaataggg tgtatagtga aggtgaataa tatagaaata 58920

gtagcaaata ctaaggaggt ataactatga aagtaggaga aaaagtagta attaaaggtg 58980

aagtagttca cgtaaatggt tctttggtaa aagtagagac aaaaaatagt tctttttggg 59040

taaacaaaga agacctaaag aaagataaga gtagtggtaa gtaggtatgg cacacttaat 59100

tgaaaagcta agactatata aaaaaccaga agaggtcgta agaggaacat cagtaaaagc 59160

acagcatatt tatgttgcaa atgatgaagc aaagtcattt tcaggagccg aaaaatgggc 59220

gggaacgtct gcctatacct tgacaagtaa gaacgaattt acaaatgtaa ctattcactc 59280

tctagacatt agaggagagg gaggcagagc ttaccaagtt acactaaatc acgataataa 59340

tacttattta tgtgacttaa gggaaactca attaatggaa actattcgta acgtaggaat 59400

tgaggctggc ggaaagttaa ttggaactta cagctttatt gctgacggct cacagaaagt 59460

tctagtaagg ataggttcag acgcgtatgc aaaagcaagt gaacaagcgg agactaaaaa 59520

taaggcaaag agaattccca agaaggagct agaagtagga ggagtatacg cacctaaggc 59580

tacttattta ggaacaaatg actccctaga cttatatata ggggacttgt atagaaatga 59640

aattgtagac gataaccctt ggtaccgagg gtactacgaa cgaagaatag aattagggaa 59700

gcgtaagaag ttctctgtaa gcttaaatgt agcattacat gagggacagg tatgtgttcc 59760

aacatacaga gaagctgaaa tcatacctgt atttctaaca gacgatgggg ttacttcact 59820

aaatattagc aaccagctag ggcgtttatc cccaagaatg gacttaaaat caagccctaa 59880

tttctttgga aaggtagggg aggctcctga aattaaaaca acagcagagt tcttagaatg 59940

tttaagacgt ggcttagaag ctcgcattga agctttacct agtaaaatag agggcaatat 60000

tttaaaggga aacttaaggt tacaaaacgt tatccctgaa atggagacta tactcaagtt 60060

aataggactc aatactacga ttgaaaagcc taaatactcc gctagttttg ttaacaaaat 60120

aaaaaatata aaatatatag ttatagcaga acgtaaccaa aaagggacgt taaccgcatc 60180

ggaaagtaca gacttccatc agtttagtcg tgcttttgat gacttaatgt ccaatatttt 60240

agaggttatt atagtagagg aggaaaataa ttgacgttat atatgaatgg taaaaaagta 60300

catgagagta ctttccccaa tggggagatt aatatcatgc ctgatgaaaa tttatgggta 60360

cttgaggagg agaaagcaga gattacttgg cagtactcag gaaacggtgc agaagacttg 60420

ctagctgtca gactactagt gaacagtata agagagcaca acaagaaaca taatagcatt 60480

aatttaacag ttctttatat gccatacagc agaatggata gacttcaagg aaatcagccc 60540

tttagcttac tagagtattt taaaatgttc cctcaagttg acaaactaga aatagtggaa 60600

cctcattcag acgttacagg gagtattgct agaaaactta ttgacagtgg aacttactac 60660

tcagagcggt ttgctacagt agagatgcta ccagaagtgt tagcagaaga gttgtctaat 60720

acttggtggg aacctgtatt agtctatcct gataaaggag caaaagacag atatgacaaa 60780

ttagcaactg attctgatta ttcattcaga agagctactg aaaacagacg agtactattt 60840

ggaaataaag tacgggactt tgatacaggt agaattaaaa gtttagagct agtaggggaa 60900

ccacctacta gaaaaggatg gacagctatt attgtcgatg atttaacttc ttatggtgga 60960

acttttgtaa aaacagctac agaggctata aagttaggag caagccgagt agtactacta 61020

gtagctcata tggaaggtag cgccactaag ggagaattat ttgaatctat tgataaggtg 61080

tatactacga acagtatgga agctagctac acaccagaag agctaaaaga agtagaaaga 61140

tataaaaaac tagggttact agacgtaact aatattataa aataaggagg aaaatatgcg 61200

taaattaaat gaaatttcta tgttaatgag tgattgctat aaatttggtc acatgtcttt 61260

atacccgaaa ggtatcacac aggtgtatag tacacttgtt cctcgggata atacttactt 61320

ttttggaggt cgtacagata aaatggttgt ctttggatac cagatgtttg tacaacgttt 61380

cctgttagaa cattttaata aaaacttttt cgagttacct attgagactg taaaagagga 61440

ctaccactat gtagttagta acgctttagg ggagaagaat gctcggacag aacatatcga 61500

agcactgcac aagctaggat acttacctat aaaagtaaga gcccttccag agggtacctt 61560

agttcctatg cgtgtacctg tgttaaccat tgaaaataca caccctgact ttgcttggtt 61620

aactaatttc ttagaaacag tattactatc tcaaaccttc ttacctgcaa cagtagccac 61680

tactgcttta gaagctaaga aaattatgac taagtatgga gaagaaactt gtgataactt 61740

agaccatatt atgttccaag gtcatgattt ttcggagaga gggcaacact ctaatgaggc 61800

ttccttgtta gcaggtatgg gacatttagt aaactttgca ggttcagata ctattcaagc 61860

tagtgtgatg gctcacaact actacagtgc tgatttagag aaggaactcg ttcttaagtc 61920

ggtgacagca tcagaacact ccgttatgca aagctacggt acagatgaag tagagacatt 61980

ctcaacacta atagacaata accctgatgg tatcttgtct ctagttagtg atacgtatga 62040

ctattacggt gtacttacag aagttcttcc aaaacttaaa gataaaatta tgaatcgaaa 62100

tggtaaacta gtaattagac ctgatagtgg aaaccctgta gacattattg ctggacgtga 62160

ctatatagag tttggaacta attggtttaa ctatcatata actaaagaga acttagtaaa 62220

agcagtaacc actcgatacg agcagtcatg ctcaaacaaa ccctcaatag gtcaaagagt 62280

cgatattgta gactacctaa atctatttgt agatagcgac tatatttata ataatgatat 62340

tgttgataaa gagaaaaaat tagtatcagg gtattataaa gtaacattaa gtgccgtttt 62400

ccttggtgaa gacctgtgta acccttataa agtagacaaa cttgaactag aagattacca 62460

accaacccct gaggataaag gttctctaca actactatgg gaaacctttg gtggaagtgt 62520

aaatgagaaa gggtacaaag ttttagacga gcatattggt ttactgtacg gggagggtat 62580

tgacttagaa atgttagaga agatttttac tcgtatgggt gaaaaagggt ttgcttctag 62640

taacgtagta tttggtcttg gtgcttatat gtactccttc tctctttcca gagacagttt 62700

tgcccaagca ataaagagcc aaatggtagt tattaatggg gaagaaaaat taatctttaa 62760

gaatcctaag acagatacta atcatattaa gaaatctctt aaaggaagag tgtgtgttac 62820

aaaaaaagaa ggaacatatg aagttacaga tgctttaaca gctaaagaag aagccaacct 62880

tgaaaactta aacgagctga atgtaatttt tgaagatggt aagttagcct atcaagactc 62940

cttccatact attagagagc gtttagacag agaagcaaaa aaatattaga aaaacgaaag 63000

agcctttagt gggctctttt ttgttgacaa ttaagtaaaa gtgtggtaag atactagtat 63060

aaatcaataa aggagatgtt ttatttgcta gttaaagaaa gaaaattacg tacctcaaaa 63120

aatagtatgc tagatttttt aaaggagggg gagtttgtaa gagccttcgg tgacttatgg 63180

tatagaaaag ataagaaact atataatgtt acacttaaag aaatatttga catttatctg 63240

gagcttgacg tttctatgct agaggctact atgctagctt ttagcactaa agaaggattc 63300

tcacctaaaa agtatgcgtt agaagattta aaggaagaat atggtactaa tatagtaatc 63360

gactttactc ttcctgagaa taaagaggac tttgtagtag ataatacaaa aagtctgcct 63420

ctattatggg acacttacag aagttatatg cctaaaaccc aagcagagtt tcactacaaa 63480

aaacctattt ctaagaaagt gttgaaagaa acttgggggc acgtcctaga cacaggctca 63540

ctaagtgaag ttagaaagaa aatggtttta ggtacaactg ctagcattcg aggtctacgt 63600

aattcggtaa gttcgtcaag aataaaaact agcggagaac taaccgttac ctctgctaaa 63660

atggatttta aaacaggagt acttacttcc gttgttaagc gtgcaatggt agacgaatac 63720

gaactatctg cgaacgaata tacagtaaac tttaatcttt ataatgctat ggagcttatt 63780

aatgacatat cggactataa agtattagac aaaggacaca gagaccatta cgattctaac 63840

atgatgtggg agattgttac aaagagtata acccatatat acaaaaatat tgaggataag 63900

tacaccctct taaggctagt atcctctctt acctcaggtg tgcaaggaag atactgtaga 63960

agtgttcgtt atgagataga aggctcagag ttggaacact cagaagtacc tatgccttta 64020

gtacgtgaag tagtagctaa tctaaattta ggtgaaaata tacttgacag agcaggaaac 64080

tggttttacc aagacgtagt aggcagagaa agtgttgcat ctacattaaa aacaaacaat 64140

tatcaagagt tagtagagcg tgcaggttct ttagtagcag tttttcctct tttcgacagg 64200

attgcttctt atatgaacaa ccaaactaaa aaagagcaga agatttttga gagagctatt 64260

gaacgacatt ggctaggtgg atacaactac cttgagcgca gtaatttaaa aattattggt 64320

aatgcactag tacccaatga agtattaaga gacctactca cttataatgc agtacaactc 64380

gtcattgaga agtttacacg ctaccaacac aatgtggttg cttacgatta cttaccagat 64440

aaagggtatg aacaggagat tgaaaaaata cttccaagcc ttcaaaaaga aagtaagcgg 64500

attggagaaa aaattcctaa gtacattgag gatacattcc agaacataaa ggaaaatatg 64560

gtagaggatg taaaaaatgt acaaaataag attacacctc taaagaaacc atgggaatat 64620

attatccatg ttaaccaagg tatctggaaa aaagtaatgg aattagggaa ttttgagaaa 64680

gagtcctata ttattatctg gaaagctttg tacgaaaata cggaagagaa tgtaccattt 64740

cttaaatata aatccaagat agaaaacaag ttactattcg aaagctctgt tttagcaaac 64800

cctgctatac aatcatcatt tactgaggca tttcgagcta ttggcgggta tcttgagaga 64860

ctaggaagaa cacctgatga gtttatcaaa gtcattaact acttgtctac agactgtaca 64920

ttaagacaga gaatgagtct tagaaacgca acagtccaat atagagacta tattaattcc 64980

tatgtagcat taaactcatt agacttgaac gaggctaaaa gattcgattt gtgtcctttc 65040

tccttgaagt tggctcatga tattgcaaca tctgattacc aaactatgca ggattctaga 65100

gcggatgagt ttatggtagc atgatacaaa gttatgcctg acgagttcag cttaaacaac 65160

agaaaattct ctattgataa agaggaatat gttttagtac aacctaaagg agtagacgac 65220

ctaaaagcgg aaggaagttc tttaaaccac tgtgtcggaa catatgcagc tagaattatt 65280

aaaggggata acttgattta tatgttacgt aagaaagaca acatagaaga gagctatctt 65340

acaattgaaa tccttgtcca cgatgactcc cgagtatcat taggtcatct acagggagag 65400

tcttatcgca gtaagattcc aacaaaaata acagataaat taaaggaatt tgtggttaaa 65460

gtaaatgagg ctatgttagc tcaaagagaa ggcagaaatt ttactaaaga cactaaatcc 65520

gaaactttag ttagagccag ttaacttgta tttaaactct taaattatgg tataataaag 65580

gtagaggcta gctaataata tagctagcct tataattata ctgaaagagg aaattaaatg 65640

aaaaaagata atactcggaa aaaaaggtta aacatttata acaccgaccg atattttaat 65700

atgaacctta tgaaaagaag tgctattgag aaaagtatta aagtaacacg gttgaacgag 65760

aaagaaatcg aagaagaaat ggacaagcta gcagaaggtt ctacccgatt taacaagaag 65820

aataaaaaga gctacttact ctatcaagag aaatatacaa atgatagact aattcaaaag 65880

ctctttgctc atggaggtag tgttacatat tacactaact ctatcatacc ttactatgta 65940

ataaaacata tggcacagtc cactacaagt aaagccgtat actctgtaaa gaaagagttc 66000

acagaagaac aggtaaaaaa tatagagcta accaaaatgg ctactacagt gataattgac 66060

gttcctgttg ttgttcctga tgttagcccc tatgatgttc tgttctcact acacccttta 66120

aaaacagatg tagacggtgt acagttttcc tttccaagat taggtaaaag tgaaatacaa 66180

aaaaggcacc taccgttcta tgagttgaaa gggaaagtat acgaattacg acccgaaatt 66240

aaatatgaat atgtaaaata cactcaagta tctttatcta tttgggggat gtatctctgg 66300

ttagtctgta atagtgaaga agacttccaa gaaatagaca agctactaca aaaagaccta 66360

aaaagaagag acagtggcag acgacctagt gcaagtcagc aactaggagg gaaaacccat 66420

gcctaagaag atgataaaca gaacagatat agcccgtgaa gtagcacaaa gaacaggttt 66480

ttatatcaaa gatgtagaac agatactagc agaaagagac accgttatta tagatgccct 66540

tcaaggcgga gtcactaaga ttaaagagca caaactgtat cagattgaag tagaaacaaa 66600

accagcaaag aaaaatgctt ggaatgggtt agacaaagta tactttagta taccagagaa 66660

gaaaataatt aagtttaaac ccttagtagc agtagaagag gtaatagata gcttaaataa 66720

aaaagagacg taatagtctt tttttttatt gtacttgaca ggataatcaa ctcatgctat 66780

actaaggtat aagaaaataa gaggagaggt gccatgtgaa gatacttttt ttacaagagt 66840

acattagggt aaaccacgtc cttgtacaag atacaggtac agtcaatatt tttcttaaaa 66900

ctgacttagg gaagaagcta gtacaaaagc ttaaatcagt aggtataacc aaacaagact 66960

tttatattga ctatgcttat gctaagatac ctgaaccaaa aaaacaagta aacggtaaaa 67020

tactaacgta caaggagcca acagcacaag agaaaaagga accacaaaca ttactaacag 67080

agagaattat acaagaaaaa cctgatttaa tcgtacctat gggtaacatt gggtgtaaat 67140

accttcttaa tgttgcatcc attaggtctt taagaggtat accagaaaag gtgaccttac 67200

aatcagagca aacaggggaa aagtttactc catgggtaat gcctatgtat tctatggaat 67260

atctaatatc aagtcctaac gtagagggta tgtttgatgc ggatttaaca acactcagtc 67320

ggtacaaaac ccaaggagac gaggcgttta ctcctgcgga agtatcttat gagctagtta 67380

cttccttaga aagagtacag gatatttttg aaaatgaagt taaaaactat cctgttattg 67440

catgggattt agagactaac accttactac ctgagatgtt aggagccaag cctctagtca 67500

tgtcagttag ttggaaagaa gggcaaggtg ttactattcc gctagaacac cgagagagtc 67560

attgggaagc ctatgaactt gaagagattt ataactatat taaagaattt gtagcagata 67620

aaaatattgt taaagtaggt cacaacattt aacacaatgg gtgttgtaaa actcctctcc 67680

atcggtgaac gctaagtgca aagcatatgc caataccgag cctacattac tggtgacagt 67740

agatggggtg tagagactga accgagccat tgtagggcaa ggtgagaagt ggtacatatc 67800

acttaaacga ggagcattgt tccatatagg taactacaaa taaaacctat tggtaagggg 67860

cataaaggta tagtccacgc ttatatgaaa atataagact acgtgcagta tgatatacgt 67920

tttcttatgt cgtctaaagg gtttactaag tttaataata atcgtgatac gaagataggg 67980

tactacttgt ctgtaacaca acagatagag gactccttca aattatcaga tttagcctat 68040

gagatgacag atatgggtgg ttacgacaaa ccacttgccg atttcaagaa aaaatatatt 68100

aaagactaca acgataaaaa aacagcagaa caggaagaaa gaaaaaagaa agtagtagag 68160

gaaataaaca aacagtttgt agaagaaaca ctaaaattta aagaagatac taagaaatac 68220

aacgaagaaa aggcaaataa taagtattct gtactagtta agcctgtaaa acctaaaaaa 68280

ggtaaggcac ctacaaaggc agaactagct tacgagcctt tagctaacga agtagatgga 68340

ggaaacttta actatgactg gattcctcta gagatactac atccctatgc tagtggagac 68400

actgactgtt gcttacgtat tcataatgta cttacggaga taattgcaaa aaaccaaaaa 68460

atgtggttcc agtttgctaa tttttaccct agtgctattg tggcattagc tagaattgag 68520

tctaacggta tctatgctca gacggactat atggaagata ttaatgagaa gtacactgag 68580

gaggaagaac gcctcgtaga agaaatgcgt aagatagcag aggtatccga gttagaagag 68640

caacacatgg agttgtacga acgcggggta gaggagatgg caatacctcc tgcaaaaaga 68700

gataaggata tagctaagtt aagagacaaa tacaagaaga agctaaaatt taatcccaac 68760

tctgcaacag acaaagggaa agtcctttat caaattatgg gtattacact tccctacgat 68820

aaagagcata ttaaagagtc tgcttttgat gtagggaaac ctgagaataa gctaacatgg 68880

gaggactaca aaacagataa gaaagcacta aactatattg tagaccattt tcccgaccaa 68940

aaggcgtttg cttctctcct actagagcac tccttagtaa aaacaagaaa aaataacttc 69000

acagccaagc ttctaaactt cattagtaat aaggacggaa gggtacatgg gtcgtttaac 69060

gctacaggaa cagagacaac ccgtttaagc tctaataatc ctaatatgca acagctacct 69120

gcaaaaactt ccgaccctaa aaggttcgac tatcagtacc cgataaaacg tatgttccag 69180

actgcatttg agaatggagc actactacaa ctggattact cttcactaga aatgcgtatc 69240

ttagctttag cagcaggtga cgaagctatg acacaagcat tctttgatgg ggcagacttg 69300

cacaaagaaa ctgcttctat tgtatgggga atgcctgtag acaaagttcc agcagaattt 69360

cgtaaaaagg ctaaagcagt aaacttcggt attgcctacg gagaaacacc tttcagtttt 69420

gcaccaaaac atgatatgag tgtagaagaa gcagaagaca tcttccaacg ttattttgca 69480

aataaaccta atatcaagac atttattgat gaaacacaca gagaagctct acgtgacggt 69540

tttgttgata cattacaagg tcacagaaga cttatccgtg atgccttttc taaagataag 69600

cagatacaaa atggtgcttt acgtaaatca gttaatacca agattcaagg aacaggtgct 69660

tactgtacaa atatgtctct aatttatatc gaccagtata tacaggataa caatatgaaa 69720

tctaaattag tattaactgt acatgatagt attgtagtag actgcccacc agaagaggtt 69780

gagattatgg ctcgggtgtg taaaacaatt atggaaaatc taccaattga cttcttgagt 69840

attgaatgga aaggtgaaaa aactaagtat cctattgaag cagacatcga gattggtact 69900

aactataacg atatggtaga atacaatcct agtgagttaa aagagttcaa ctctttaacg 69960

ggttatgtga agtactacaa agatttaggg aaaatttccg attacatgga ggctggtata 70020

attacagagg agagtatgga tttaggacaa actagaatta aagaatcaaa agaaatttac 70080

aaaaaaattg cctaaaaggt tgcttttgtt ttaaaaatag tgtataatga tacagaaagc 70140

tatttctaaa aacagaggag ggtaaaatat gacttaagga gggatttgtt tggacataca 70200

tgtagattca ttagaattta gccaattacg aattatagat ggtaacggac aagttacaga 70260

gcttgattta gagagagagt tgagagtaaa tgaatactcg atgcaggagg agtttctcaa 70320

ccaagcacca aaatatatct attgggcttc tattctagag aaagtacgta tgtatcagga 70380

gtctgcatca ctagctttag aggtagtggg agcacagatg aatgagaagg caagagagta 70440

ttacaagact gtagacacta agccaacaaa agaccaagta gaagcctata tacttcaaca 70500

agaggaatat caagtggctt acaaacagtt gttaacctat aatcacttgg ttaaacaact 70560

acagtacgta gtaaaagcgt ttgaacaaag aaaagacatg ctaattcaat acggagcaga 70620

tttaagaaaa aataaagata acggtataag cccaaacttc gggtaaaaaa aaataattat 70680

aaacgatagg agacaataac agtatggtaa actttaaaga atttttaaaa caggagaacc 70740

aagaaaacga aagtaataat aatgaagagg tagcatttcc aaaaggaaaa catcaacgct 70800

tattcttgtc taaaaaatca aataccgtgg tagttcgtat tttaccacca gctaatttaa 70860

cagataacta tgcacaaaag tatcgtaaaa ttatgctgaa caacgttatg acagatacag 70920

gaaaagcatt aaatccgtca tttacgttac catgggagcc taacgaggat tccccactag 70980

aacaggcttt agctgattgg tataaccgtg gagtagttcc taactactat ggaggaaaat 71040

cacgaccaag caaagctact gtagtaaacg ctgtggcatt agtaacagat gcaaacggga 71100

actgggtaca tgaggttgat gaatacggtc aactagtagt tcgagtgttg gacttacctt 71160

attcagcata tacagctatc aatcgtaaat tagcagaccc aatgctaaac actacaggct 71220

ctgactatag ctttatctct gatgtaggag ctaacccaat ccgtattact aaacctacat 71280

caaatacaga gaaagcatat caagttgaag tatatacagg tattacatta ccacctcttc 71340

ctgagaattg gagagaccaa ttggaggact tagcttatca agttagcccg tcagaagact 71400

ataacagcaa atttgttaac tttgttattg aacgagttaa tgctgtaact agtggaggag 71460

ctcaacagca acaagctaac ccttacggag gacagcaagc accacagcaa caaggtggtt 71520

atcaacaaca agttcctcaa caacaagctt accaacaacc tgcccaacaa gcacagacgt 71580

atcaacaacc agtacagcaa cagcaagcac aaacatacca acaaccaaca caggcgtacc 71640

aagccccaac acaacaagta cctcaacaac aagcgtacca agcacctgtg caacaaccaa 71700

ttcaacaagc tcctgtagcc cagccacaac aggctcctgt acagcaacaa acgtatcagg 71760

ctcctgtaca accacaggca caacaaccta ttacgcaagg aatggtggac tcagcaatga 71820

gcgggttagc atctcctaag gaacctgtac aaacaacaga aaccagtaac cccaccacca 71880

tctccacaag cacagcaacc agtaagcaat gattcaagct tacctgatgt agatgagtta 71940

ctacgcagta tgcaagaagg ttgataagaa ctaacatgag gtctaggatt tattttctag 72000

accttaataa gaattttaga gagggttgtt ttcatggcaa gagctaaaca gagtgaaaaa 72060

gataacaaga cagaaattga tttaggtaaa aaaattgggt taactacttt gcaggatagt 72120

aaatgggcgg agatttcaga taagttacct acatttatcc ctcagttaga ctatattcta 72180

gggggaggga ttccttttgg tcgtttgacg gagattatgg gtaaaaacgc aagtggtaag 72240

tctactcttg cagtccactt aactaaggta gccttacagc tagattgtaa agttatttgg 72300

attgacactg agggaacggc agaccctagt aggcttagtc agctaggcgt agatgtaaac 72360

aaaatatttt ttgttcaacc aaaaaaatct aagaatgctg aggtaaacaa agaggattct 72420

attacagtag aatttgtagc agaacgagta gaagctattt taaatgcctt ctctagctct 72480

gaacaacgaa ttgtgataat ttgggattct attggacata caccttctat caaagagatt 72540

gaacaagggg tagcaggtaa acaaccgggt attaaagcta aggcaatggc tcagtttgct 72600

accattattg ctcctatggt aactaaatct cattgtgcct ttattggtat caaccaagct 72660

agagacgaaa tgggaagcat gtttggtgca gtagacagcc ccggaggaaa tgcactacac 72720

cattgggctt ctctaagact agaggttaaa caggcatcca aaatagaaga accgatgcaa 72780

aatgcttttg gtactatgga taatgtatat gtaggacata aattacgtgt tattactaaa 72840

aagtctaagg tatctacacc tagacaaagt gcagaaatgt tccttttatc cgcatcaggt 72900

ttgcaatatg aagagaatat ttatcgttct tgtgtggaca gtaaaatgta cggtcttatc 72960

aaaggactac aatacaagtc ttatattgca gatgatggaa aagaatacaa aatgtatggt 73020

aaggactggg ttcctttctt acagtcaaaa gacggggaaa agatacgaaa agagttattc 73080

cagaaaatga tgttaattag tttccctact ggatatagtc cacttgataa tgaggtagtg 73140

gatgttaact tatgggaaga tatgaagggg ttaacagaac tgtacaaaaa gaataaagta 73200

aagcaaccta aggtgaacga gggagcaaaa gaagagtcta ttgaggaagt actagcagga 73260

attgactcgg ataaataact ttatagggga gggaaacttc cccttttttc ttttagtact 73320

attgaggagg aagtaaaata tgagagctaa tagaaatatt gatttaaccg agcatgtgga 73380

agtttggtct gaaaaaacaa gaagtattcc accctctaag ttaaatccat ttctagatat 73440

taaaaaaaga tttctatatg caaaagagca taatatccca attattctag aaaacagcga 73500

gctaaataac tctcaatctg tggttctaga cctaagcttt gtgggagata aatgggcaat 73560

gggatacgta acacattatt cagctattcg tacagagtct actccataca ctatccatta 73620

cagtgatttg tattgtgcag gtaaacaaag taaaaagaat aaaagagtaa aagtaatatt 73680

taaaggggat aacccttata ctatggagga agcctgatgc gtaatattga taaagaaaag 73740

gaatatatta gaaacggcaa tcgttatgta caagagaaca gtgggagagg ggtatttgct 73800

agagatagtg accgactatt ccatcagtac agtaatctac ggtggagtgt ctacaataag 73860

cataaagact ccttcaatga cgaaactact agagcagaat tacaaagcta tattgacgag 73920

cagtttatta aactagtgaa ggagtacgaa attaacggtg cagtagactt cccggggtac 73980

attaaaacaa agttaaacca gagagtaaag catacttttg ttaagaatgt attccgagac 74040

aaagctagag aacgtctagg tgctgatgag tttgaagtag ctaatctagt agaccgacag 74100

catactgatg aagcactact agaagaccag atgttacaag agtatgtatt tggtaagaca 74160

gagttcacac ctattcaaaa agagttgctt gtctatttac tagaaaaagg gatggtttct 74220

gatgaacgag taattatcag aaatgtggca tctaaattca aagtgcctag agaaaaaata 74280

aatgaggaat tatcggaact acgctttttt gtttcttcta agatagctaa gtatgagtca 74340

gaaaaaggct attaatattg aaataacgtc tgctatatta gagttagaaa ttcagtatag 74400

gaggctttaa acaatgacag aaattcatac accgcaagac gaagaagtaa aagatgaagt 74460

agttcaagaa gttattgtag aagaacaacc cgagaacgta gaagaagtag tcgttcctag 74520

tgacgatgaa gtaaaagcaa aatttatgtc ttatgttccg ttatttgtta ctttagttct 74580

tttcttgaac accttattag catcgttggg ttgggaacca ttagcagttt ctcaagacca 74640

attgtatcta gtaggttctt ctgttgcaac agttattgca actgtatggt cttggtaccg 74700

taataacaac gttactaaaa aaggcttgaa gagagaaaac atcgcaaacc aagtattacc 74760

taaaaaagag gacaaataag aaaggatgga atattaatgg ctattaagaa cattaaagat 74820

ttgcttaaag agtctacagt cgttttagat gggaagtatc tacaagctag aacggtaacc 74880

gatggtttta caggaaccta tcaaggtaac cactcgttta aagtagaaaa aacagggagt 74940

aactactctg tataccctat gtattttgac aaagatacaa aacaaatgga gaagagaaaa 75000

gacccgatta tctatacaga tgacggagaa attttctttg tagttcgtac tacagtagac 75060

ccttacaatt acccagttat tgagcaagac tccttaacag gaaaaaatac agagaaacag 75120

attctacaag catttattgc tttctctgct gacgagtata gttttgctaa ctataacttg 75180

tttatcaatg acgatttaaa gttctatgac ggttctgcta taaaagaagt tcaaagtatt 75240

aagttcccta atgcgacaga aacaataaat tcttttggag aagataaaac attaactgtt 75300

atctttacac ctgcggatgc tagcgataaa acagtaacat tcacaagttc tgacgagagt 75360

gttattaaag tgagcaacac aggtaagtta actggctctg tcaactccac tgaaaagtct 75420

gctgtggtta ctgctgaaac ttctaatgga aaaaaagcta ctgttactgt aataatgaaa 75480

gcatcagagc taccagaacc gtgataaact catagaaaag aaaggacttt tcgagtctct 75540

tttttttatt gactttacag gtaaactgtg ttatagttac atagaagata cactaagaaa 75600

ggagtgacaa gatggaagac aattttgtaa cagcctataa taaaggagaa cagacatggg 75660

atgacttagc attttgggag gagtcttgga acaacgaccc taatcatatt gccagtctag 75720

cggagtatct agggatgaca gatagagact actggaatta tgtagtaatg gacaaagaag 75780

tacctaaaga gctcctacct cgaagtgagc aaccccaact agaaactgaa tacatctacc 75840

acatttgcca taaaaagaat gaagtacaaa tttttaaaga aggttcttta gtctctcata 75900

atggaaaagt ggtacaagta gataaaataa tctctgtaat catcacagag aataactcag 75960

tagccattca tcttaagatt aaggagggaa ctaaatgatt cagctaatta caaaaaataa 76020

catgcaggga tactatgcag actatctgtt acgaaaagta tctacacatg aagtgagaac 76080

gacttactta gagaagttca gactgcaaga catcactagt tatagtgcag atgctactat 76140

tattatcgta ctaggattta gctacagtga cagagaggcg ggagctactg ttagtaactt 76200

cgaagagtat agtgcaacat ttaataaccc attcactttc ttttaccact ttgctagttt 76260

tggtgataag tttaaatacc caaatattga ctctctagtt gacgaaaaag taagtcctgt 76320

tatggtaatg tacaacacct tgaaggaaaa aaatcctgat ttcttaatag cagagaaaga 76380

caaagaagct atgttggttg ctactgctgt agacgactac cactgctacc gttttgcaga 76440

aacagggaat gggctgtctc tacctttaaa agagctagga gacctctatg gacgttattt 76500

agctgaattg tacggaaaca tgtctttggt ggagctaatt cagtcaaaca agaccattat 76560

caaagtgaga caagagtatc ggaattatta catcaagaaa aaaatagagc agtctaaacg 76620

acaacttgtt aaagatgtta tgctttattt tgtctatgca gaggaatacg agaatgagat 76680

tgcacaccat attattgata aggctaaaga agttaacgat agggtgcttg tcttagtagg 76740

aaagcataca acaaataatg atatgttcca tgtacgctcc cataatattc acgcaggaga 76800

ggtagctaag ctaataaata aaggttcagg taaagaaagt acagcggtgg tattcttagg 76860

ggattccaaa acacctacat ttaatgctgt tgtgaaagtt ttgctagatg ctagtgatat 76920

ttaaccacta aatgtggtat aatagaagag aagtacacac taattagtta ggagaaaata 76980

atttgaaaaa tttaacgaag gcagaaagaa aagctattga gaaaggacac ttaggaaaaa 77040

cagccgttat tctaggagtg ctaaatgctt ctcacggcaa aattgttatg tctcagtata 77100

ataaaatagc ggaaagttta ggaatggaaa aggtagctaa accagacttc ttagcactaa 77160

atgaaaccat tcaagaagat aaggttttac ttggactgta ccaaaaatcg tctgtaggaa 77220

agattacact agatgattta acaggaggaa gtaacgaaga caagtatagc cagtttaatg 77280

ctataacagc ttatacagta gataatgaca aagtattagc agacttacga gaatttagaa 77340

aaattcaacg agatggagct tatacacaac atctttttaa cggagtgaaa gcagatttaa 77400

agaaagagct ctcttctatt gctaaaccta aatttattat taaagagaac aagaaaaaaa 77460

caggtaaaga aatgattgta gccttatctg attggcacat cggagcttta gtaatcaact 77520

ctgatacagg aggatacgac tacaaaaagc tagtaagcta tctgacatat ttactaggtg 77580

aggttgttca gacagtagaa gagcatgata ttgacgtatg tcatgtatat catattggag 77640

attttattga gcatattaat atgagaagtg tcaaccaagc atatgaagcc gagtttaatg 77700

caacagagca gatttctaaa tctattcgta caatgcttga cttcttaaac gtattagccg 77760

accatgtaca tgtaaaattt ggtatggtag gcggtaacca cgataggttc caaggaaata 77820

aaaatgaaaa aatttataat gataatgtag cttacattat tctagataca ttgtttttac 77880

tacaagattc caaagcgcta cacagtaaca ttacgctaat tgataatcgg aaagatgttt 77940

acagctttaa agataatgtt gcaggtaaga atattaaagt agtgcacggg gacacagaag 78000

gtaagaaagt agatgtaaaa ataccaaaac acattaagag tgaagtagta gactatctat 78060

tcatggggca tatccataca actagaattg tacaagaaga ttatagcaga ttccacgtat 78120

atgtaggtag cccgatggga gctaattcct attctgcgga aaataaccta ccgacaacac 78180

aaggttccca attattagtt attctatcta aaggtagtaa caatccaatg ttttacctta 78240

tctttactaa ataaaggagg aggaaaacaa aatatggcat ttttaatcac agtagtgcta 78300

gtaacgtacc aagttattgt acttttgcag gcggttagtg gaactatccg taaagaacga 78360

attatggtag aagaaggcgg agacccccta ccaacatcct cctatgtttt cttaatcatt 78420

aatcaaggag ctattgcagg attcctattc ttgtgggggg cagtactaag ctttattcta 78480

gctatggtta ctaatgttat cctagctgta atactattta cgctagcagt aaccatctta 78540

tcaattgtaa gtgcaacgat tctaagtaca atcgttcaga caattgcagt tattattgta 78600

agacgaaagc agatagcaca tacgaaaaaa gttattgctg aaaaagaaag agagtagata 78660

aaatatgtct gagtatgaag caagcagagc agacttaagt agagagttcc tacaaggtga 78720

tttacagcct agtattatga gacgagggat tctctacatc agaaagaaac ctgaatatat 78780

tgttgagtat gtagaagagg ctagtagcga tacattaatg gtaagtgtgt ttgcagttca 78840

accaaagtct aaaccgcctt taaaggcttg gacattagtt gataaaacat tccagtataa 78900

cacaggaacc actctaggtt tactagctaa ggagctgaaa catgtaccta aaaaaacaga 78960

gaaaagagaa aatccccctt tgtttatcgc acctgcacta gatggtgtgg acacctttac 79020

aggaagagca ggtaagggtt tctatgaccg agaacctgat agagccacaa gcgacggtaa 79080

aatcgttcaa gggaagcaca caggtgtatt tgttgggttt gactctatta cttgggagaa 79140

agctattgtg ctaactgagg agatagctag taggtattta actgcccgag cccaatggta 79200

taacatttag taatgctata ttaaatagca tgttaacaat tgttaacagg agaggatgga 79260

agatatgacc tttacagttg ttttattcct agctgtaata ctcttgtttt tatccgcatt 79320

atgttttacc agtcataaac agagttggaa cgtagtaggc ggtgtactat tagcctttgg 79380

tctaatcgca ttgttctatg tgcttataat ggcaagtgct gtttcaggta taaatggaga 79440

atggtttggg atgaggttat aatacacaca ttgggggctc tgtaaggggc tccctttttt 79500

atggaggttg gaatgaaaaa attttatgaa gatgatatta agcaggtact actaaaaaaa 79560

ggaaagatgt ttggtgatat aggaaagagc actattgtat ttgagaaagg tattactatc 79620

tcctctacaa tcgcagactg tcttatattt acagagaaaa aaggagtcat tggtgtggag 79680

ataaagacag agagagactc cactgcaaga ttaaataagc aactaaaaaa ctactcctta 79740

gtgtgtgatt atgtatatgt aatgtgtcat gataaccata ttaaaaaggt agagcagata 79800

atgaaaaggt accatcatga acatgtaggc attatatctt atattgagtt tatgggagag 79860

cctgctattg gtgtctataa acctgcaaga aagtcaccgt tgaaagatgt ataccacacg 79920

attaatattc tatggaagga agaaatacta aaaatattag gtgcctttag acattatggt 79980

gcaagagctc ctaaaagctc ctacacagta gcagatagaa gcggaggact aaatgggtta 80040

tccataaaat ccagtttctc tgctcgtatg agtaagccag aactaataag aaatttgatt 80100

gctagggttg gacgaccaga ggctaatcgg attgtttgtg atattttcat tgaacataga 80160

aacaatcctg ccaaggctat aaagtaccac cacttctttc ctagcaaact agaaaggtaa 80220

gtgaagaata gtggcaaaca atactagagg taatctacct agggggtata ctgcactagg 80280

agacgggagt gagtctcaca aaacagtaga aagagcgcta agaggttact atacagtaga 80340

ctttagtaat atatacgaac acatgacaga aagagactta gaagtacgac aggcatacgc 80400

taaagacctc atatctcaaa gaacaggcat ccctttagac atgattttat ttagaaagaa 80460

gacagagaag cagacattaa cagctataga tgaagtcttt tatgtaaaac aaggggtaaa 80520

aacaatagga aaagtatcta tcaggtcaca aagactacta agcagtaata tgctgattat 80580

tatttacatg tctaagagcc atatgcctcg tgtagttaga aacacaggaa tgaaaactta 80640

tacaaaacgt agtaagtcag acatacgtgt agcaaaaaga caaaggaaaa caacaaaaag 80700

acagagctat tgacaacaga gtctacttat gctatgctaa tattagaaaa ataaacgaaa 80760

tgaggtacaa gtatgcaaat agtagaaaag gagagttcct taaacagctt tttaagcagt 80820

atgagaaatg aaaaaggtac agtggtaggg gtctattata aaaatgcaat gatagacgaa 80880

agtatcttag cagaagccag tcttcaatta gaggtgccta ataagacgga gaaggtctta 80940

caagccacgg tagcactaaa acctatgcca agtcaggtaa accagttaac gaccttagag 81000

agcctcctag aggactctgg aatgggatgg ttttttgata gagaacgacc tatcacacca 81060

acaatgagta cccccttgtt taacgactta gctattagtc aaagtgcact atcaaatata 81120

caaaattata gactagtagt taacatggct gaccctaaag taacaaaaca aacctttact 81180

ttttataaaa atacctatac attgtttaaa gtagaggaca gacagttgga tagtaaggtt 81240

gcaggtaaca ttattcacag tatccatgtc ttatataagg aagagcagta tgacggtgaa 81300

accttattcg gtagattgtt tgtagaaagt ggtagtaaag aagctaaact tatgaagaga 81360

gatactaaaa tcttgttgga cttgttatgt gatgaaaaca tcactagtct ttttattgga 81420

ggaatagagt tatctttaga agaccgttta tccaatgcta agcttttccc tctgtacaca 81480

gcagttacag ctacaagaac aaactatgat gcttatagat tcctaacaga ttcaggagag 81540

actactattg tttcggatat gattctagag agtaagttag ttaaagtagc tgactataag 81600

tttacctttg tagtaaaatt aaaaggaaat gcacaattaa ctatatattt aggatagaag 81660

taaaagaagt cagcatttaa ttgttgactt ttttctttat atatgttatg atgtagttaa 81720

taaaaaagaa aaggggttga cagaagtgac gaatttttta acattagtat ttattctagc 81780

tattattgta gctgttccta cgttattagc taaagcgatg agaaaccact ataccaacaa 81840

ggagacaggt gtttggtgga tgccatatac acactggttt gtgtacaact ttttatatac 81900

acagaagcta ggatggctaa ttttaggctc cggtattatt atctccattt atattccatt 81960

tattggtgga tttttaggaa cagtcattgg atttgcaggg ggactactta tgttatttgg 82020

tgggctacct tatgtatttg gtttactagg gggagcagtc tttggtgttt ttgtagacaa 82080

cattgtgtat catctaccag aatggtatcg agcttggttt agaaaccgat ttgggattga 82140

gtttccttac ctagaagaca aggaagatga aaacgaggag tgaaagtaat gaaaaatgaa 82200

gatacagctt ggagtttact aggtttaatt tcagctattg ccttggtagt tatttctttt 82260

ctattcttag cagggtgttc acaagagaca tcacaggtta aagaaacaga gcaagagagg 82320

tatgagaagt tcactcctat gattgaatat atttactctc agaagttaat taaggtgtat 82380

aggaatgaat cagacgagct tgtagagtac acctactatg cccgcacaga agaggaagaa 82440

gaagaagggc tcatccacga agaggagtat aattattaca aaagtcaagg agttactgat 82500

agaggtgtta ttaaaacaac aataaatagg tatggtaagg gtaagtaccc attaaaacta 82560

tgggatgtgc aagagtacga aggaaatgtc agcttagata gaatagataa tgaagtctac 82620

tttgagggtg attaatatag aaactacaag tacagtagga tactggaaca tagatacctt 82680

ttcgtatgaa tatttgtact gtgctacaga gggagagcct tggagcatag ggactatgct 82740

aacggagtac tacactgatt ataaacaagt aaggcaacta atgcaggaca aagagtttgg 82800

taataagaag tttagcttca cgtcttcaaa ggtaagcaag gcaactaata aaaagaagct 82860

aaaggacaaa gagacctatt ttcagtatat atttgaaaaa gggcagtggt tctgtgtgta 82920

tactaaaggg ttcatggcta acgtagagat acctgctata gaaaacccat accgatttaa 82980

taagtggtta gacaagaatg ctaacaagtt aagtaacaaa agagtaaaac tgttacgaga 83040

actagttgta agtagcagtg aggttaaaga ggaaacagag agagtagttc ctgctatggt 83100

taaattagaa cctccgttac gattattgct agacgacact catagtgtca gttctcataa 83160

tatactggaa tttttaaagt cagggaattt gtactcttta gctaagagag agttctatgt 83220

tgatgactat ggaaccgaag tagactgctt tactataaat gagttaacaa aaccaagttc 83280

ccaagtacct agtcaaataa cggttagtaa agagcataca tctaattggt atcctctatt 83340

agacctactt attgactgtg gttatatatc agactatgta gttgaggata cagggtctaa 83400

tagtaaagaa gtaaaaatag agataaaaga aggaggataa cgggatgtct atatatgagt 83460

ctatcgaggg gctagaacaa agtattatag agggattacg tacaacttta gaaacagaag 83520

gtaagaatat agccctagac gttatcagaa aagaattaga agacagcccg ttcttacggt 83580

tttttgagtt gtctcaaggt agccctgcgg gagatgtata ccaagtagat actactaaga 83640

tagctagtaa ccctacacag cattacgaaa ctgcccgtgt agctagtatc gtgtctgcta 83700

acgttcctgt gtggttgtgg gggcaggctg gaacaggaaa aacttctatt gctcaaagta 83760

ttgccaaaat gctagattta gaattttacc ctatgcagaa aataatggac gagttcgaac 83820

ttaaaggttt tgtggatgcc aactctagat tcgtaccaac tccactgtat gaggcaatga 83880

aaaatggtgg gttgttattt attgacgaaa tagatgcttc cgtaccagaa gcactaatac 83940

ttattaacac tgctttagct aataatttta tgcgatttgc tgacgagact gtatatgcac 84000

ataaagactt tagggttatt gttgcaggga acactctagg taaaggaggt aacaactcct 84060

acgcacgtaa taaaatagac tcagctactt tagacagatt tgtacctatt gagataggat 84120

acgatgaaaa aattgagtta ttaattacaa atggagaccg agaattagta tattttgttg 84180

cactagttcg aatagagatg gctagaacaa agttagacca tgttatctct tacagagcgt 84240

taagtcatta taaaaaattt gagtcttttg gtgcggattt cttgattgaa aatgttttgt 84300

ttaaaacact atctttagag gatattacta gtgtagctcg ttctcttagc agaataatca 84360

aaaaaacaga taaggattct aagtggaaga acaacaaagt gtggaagcag ttacagacat 84420

acctttcaaa caaaggagta gttgtagatg acacgaacta atcatacgta ttcatctaaa 84480

tttatagcag aaaaccaagg aaatgtctat aggttgcact atgcaggaga ggactataca 84540

aagataatag atgatatgtc agctatcccc cactatgata tagataatat caaaaaccct 84600

caaatgaaac tctcatggtc tacaggaggg gaacctattc cagaatttat acacaagttc 84660

caaacaaatt atatggaacc tgagaaggca caagagctta atgtaatgct ctctaaagta 84720

tctgaattag taagtagcca accacctatt tatcattttt acggtaatac tatatcagtt 84780

cctagagcca ttcaaggcca ccctaaaagt tttaaacatc ggaaaaaaga agtaactcct 84840

tatattagta tatttgtagc tatcaatgtt ctttatgacg ttagtgtaga gaaccataat 84900

aagtttataa aaaatatgtt gaaagttatt ttgatactgt caaaagacca tactgtaaaa 84960

gtaacactag gtaaagttag tgcctctagg ggaagaatag ctgtggagag ctttgtacta 85020

aaagaggcta atcagagttt taacttatct agactttggt accctttatc tagtatagcc 85080

acatatagaa ttgttctttt tgagatgcta gaaaggcata tgacactaga gcctaaacta 85140

tcctcttaca aaggtaatta tgggtttaca atacacagta cggaaggaat agcagaagca 85200

gtaaacttgt ttgagaaata cgactatgta gtcggttatt acgaaacaat aggcatgaca 85260

gaaaaagaaa ttatagaaag atattttgtg tagttgactt tatctttcat atatggtata 85320

gtagctatgt aaagtacaaa gaggaggtaa tactatggaa gtagcagagt tagtaggtaa 85380

actggtaggt aaagaagtta tgattagtac agaaaggcat gagcgccaac aggtttatag 85440

ttttagaaca cacttaatag aacgtgttga gttgaagcct gcccctagga cgattgacaa 85500

taaatcagtt attgggaatg ccttttttgt agtctattta aaaggactac ctaatgaata 85560

tcgactagaa cagttaagaa ttagaatgga tatactagac agaggatgct tattacctgt 85620

tgagattgac acagaaacag gagaggtaac acttgttgta agcgactctc tagctaaata 85680

tttctttgct agtcttttag aagttgaagc acgtactgac gaggacaaga aaaaggagct 85740

agtacaggag tttctagcag tgtctaaatt atttcacggt ggcgggtgga ctaacggtaa 85800

ctattatgca gaggaggatt aaaactccgt atataagaaa tgaggtgaat atgtatgatt 85860

aaagtagata aagtagaatg caaggatgga agagtttact atgtctataa tagtaataat 85920

ggatataata aaaagaacat atgtattaat ggaaaggtgc aaacagtacc taaagataaa 85980

tatgagaagt atttacctaa actttcagag ataaaaaaac tagatacatt gtatatgagt 86040

aacaaaaacc aactgtacaa agaagttgta tgtgccagca gtggaaatac aagtaataaa 86100

gacaaaaagg tacgaatcaa tataaacaat gaagtagtca gctacctgcc gaagaaggca 86160

aataacttgg cgatacttct aacgcctgac aattgccatc tggttagtag ggtagagcca 86220

tctatactag tagaacttct ttctaaaaga acagtcatta tctctagaga tggcggtggg 86280

gggaatgtaa gaagaatagg ttctgcttgt tgtggggacg tactaacgtt agcatataaa 86340

tatataaatg gtaaacagca ggtaagtatt aatgtactat ctgttaagga tttttaccaa 86400

tatgaactag gagggttatt tttatgatta aagtagagca tattagatat aagcctgtag 86460

attttggctt gtccagtaca actcgtacat cagaacacta cgtatttaaa agtaagagag 86520

gtcaaaaggt ggttacaatg aaaattgtgg gtaatggtat tcgggcaatg ccttttgcta 86580

agcttaaaaa gcaccattta gacaaggtta ctgtattgaa aacattttat gtacctaatc 86640

gaaaacagct agctaagcaa ctaggtttaa gttactacaa tagtgggaaa gcagtacaaa 86700

aaacagtaaa acgtttagat gatattcagg actcgattgc atttagtaat gctattcagg 86760

cactacctga ccactcagca ttgagattta agttagtagg ggaggagaaa acccctaaaa 86820

ctaaacttaa cacagatggc agtacaaact accttattag aacttccgct aagctattgg 86880

gagaggatgt aaaccaaatt aacttactta acgtatctgg tgtgctaaat aaagcggagg 86940

cagaagcaga gaagttctta cgagtactaa atctgtataa ggaggctacc aaagatgatt 87000

aaagtagaaa aatatacagc tagtaccaca aaaaaagcag gttctattag agggacttat 87060

gtagtaggtg gtactagaaa ccagaacgaa tcagatttac acttaacagg gactacacac 87120

tacaagtata aaaacaaaaa aggtaatacg atgattacat ttgtcaatta ttatacaaaa 87180

gaattagtct ctattcctga aaaggactta agccctcaag acctagatac cttttatagt 87240

acagtaaata acagactaac tactgtgttc tatgtcccta actggaagca aatgaataaa 87300

aatgtactaa aaaagggtac aaaatagcaa aattataggt agttcataaa gattttatga 87360

tactacctag tactgttttc taatctgttt cacttatctt gtttcattta cttcttttat 87420

actctagatt gtttcaaatg cttataagta gttatgccta gtaaacccta ctctctagta 87480

cactttatgg gtgtttcact tacaaatgag ataatacaaa gtgtatgcta tattatatat 87540

taagaacact gggaggcaac agataaccag taggtcataa aatagtataa atgtagagga 87600

gttacatgtg aaatggcaga taacaaagat aaaaacaagg atttagaaaa taaaaaagag 87660

gcacctacaa catcggtttt acttagactg catgagatgc ctatcttagc ttctaaggta 87720

aatgatgctt tagatgagga caagtcgtat gactatattg taaacttatg tagtcagtat 87780

aactttgtaa tctctaaatc agcaatcagt agatacaaag taaaaagaga agaatctata 87840

gaaacaggaa ttgatttagg tgctttatta gataaaaggc gtaaaaacga aacaacagct 87900

actgtgtcta tgattgcccc taaagagaaa aagtcatctc ccgaagataa gagatacgag 87960

gaaactttta gtactgtaga caaggtatac aacgatgtaa tggttttgga agagattatc 88020

caaaaaggaa tggaaggctt acaatacaca gagactatag aggcacctat tgcgatgaaa 88080

gctattgaaa taaaagctaa aatcacagga aaccaacttc aaggactaag tattatagga 88140

ctacgtgaat tgaaacttag aagtgtagct aaagagacag caatcacaga aattttgatg 88200

cagtatatcc ccgaagataa acaaggagaa gtgcttacta agatgagaga agtagaaaag 88260

gaattctata agaacttaga cttatcagat gaggatagaa gaataactga ggcgcttaaa 88320

gtcgcaggta taaacagcta gaaggaggtt tttttatgga gatagtagtg ccagaaggtt 88380

atacaaaaat aagcatgact aagtttgata acctagaaga taagttacta gcactattaa 88440

caggagagac actgttagtg cgtggtttag aaaaaagtac gaacgcagac gtactagtta 88500

agctagatga cagaaagtac cctgtgacac agataagcta tgaaattagt attacagaca 88560

atgtgtttcg agaacgttat tggactatat atgacatatc tattaatgca ctaagtttgt 88620

atgatgtttt cctttttgag gaagacaacc ctgcatatga gctaaagata caagtatcag 88680

acaatgtgca gtatactagt atcagcggtg ttaaagacac agctaaagta atagctattt 88740

atgaagatga agaaaaaaac ttgttttgcg agttatcaag ggaagacaca ttgtacccat 88800

tagtagaact tacaaaaatt tagcaaaagg taaaatccct tgaaagctct aagtagccga 88860

ctagggattt ttttgttgac attatataga aagtatgcta tactacattt ataacataga 88920

aggaggagat ttaaattatg aacaacaaaa aagaatttga taaaaaggtg actgaattaa 88980

agaacgcgac agagattatt aaagagcggg tagcaaaagt aacacacggt ctaaaccaag 89040

caacaaacct gttgaactta aatgtaaaag aagtaaaacg agttgcttca acactgcaac 89100

ctctagagga agttgtttat gagttgcaga aagaggttga cggattagca cagtatagca 89160

aagcggaggt tgtttactac gatggaagag aggttaacct tgaggagatt gacttagaag 89220

aggacgacta tgaagaggat actacagaag aggaaatccg agataccgtc tctactgatg 89280

gagggggagc acggattaga gtatatcctc ctgaggaaac tttagtatat atggcagagc 89340

atattggaca aggagtagag actgatgacc ttgctgttta tgaagctgtt cgagacacaa 89400

acggtcacac aggtttccga caagaaggct attatgtaga ccctacggaa acatatgcta 89460

accgagataa acgattaaaa gaaacctcaa aagtagaaga ggttgaggaa gtagaactag 89520

aagttactat tattagaccg aaaagtaaat ctaaatctaa atctaaacct aaagctaaga 89580

aaaagcaaaa tagtaaaaca aagtctacca caactgagtt taaaaatgat acagaaaaag 89640

gtattgtaac tgactatatt aatggagcag gaacaagtgc tattgcacga acttacaatg 89700

ttagtatggg tactatctac acagcgctta atcgtcataa gattcctaag cgtaacatcg 89760

ctaataacag cgtagaggtt caaatggttc ctgtaatcaa taataagaaa ctactaaatg 89820

caattattga cagctacaac aaaggagtac cagttaaaga catctacgaa aaatacagta 89880

ttcataaaaa cgggctatac tacttgctag attatgtagg tgtagaccgt aaagtacacc 89940

ataaagggaa aaaaggagac gtagaactta agatggacta acctccatct tagttttata 90000

attgtagtta ccgaaggagg ttagttagtt gaaagtccat ttcaagaggt ctgtgataat 90060

agttgcttta gtagtaacta gttttattct aggagtagct ttaggttata ctatccatgt 90120

agataatgct gttcaagaac aaatagcatt acaaaataaa aaagattctt ctactagtaa 90180

aaaggaacct agcaccaata aagacattgc agacacaaaa gctgtagact atgagactga 90240

cgtagctgta aggttgaacc tcctacgagt aacagtaaaa gaattttctg agaagtttaa 90300

cgaggtagag gataatgtac aagtacttaa tgatatagaa tactataata gttatattgc 90360

actaattgga gacgtgaaac gccaagttag tgatatacaa agaatgaagt atcgtagtag 90420

tgagtctgct ttagctagtt ctgtaaacca agcagtagac aacttctatc aagcctcctc 90480

cctagaagaa aataacgttc agtcaagaca gacggacatt tctactatgg ttgttaacaa 90540

gctaaataca ggtaatgatg aattagaaaa agtctttaga ataataggag gtaactaaat 90600

ttgggaattg aagcaatagt atgcgcctta tatggtgttt gggtagcacg gaacctgatg 90660

accgtgacaa gtgttgatga aacactgacg gaaaagatta ttctagaaag cagtaacaca 90720

caagtagccc acttagcaat aagtgaagcc aaaaaacttc tagagaaaga aacaaaagag 90780

aaaaatagaa aaattattat tgattacatt gaaaaatggc atcgggcact aagtgacgat 90840

actttaatag ataccctagt agagaatgaa agaaaagggg ctaggaatgc tagaattatt 90900

gctaatatat ttgtaaaaat tgggacatta cttggaatcc tagtattaaa tattccgttc 90960

tttcttgcat tagctaactt cacagtagca ggttgtgtgg cttttgttat attaggcgta 91020

atatatggtt gtcagacgga agcaaagcaa gaaggataca aagcaagtaa ggactatatt 91080

gtagtctctt atgtgctaaa gcaggcttta ttcttgctgg tagtgttaat ctagtaggag 91140

gttttattat gagagaatat gatgaggaaa aagaacgaga gctagctaag aagaaccata 91200

ggataaaaag agaacgagct gaggcacttg aaagaaaacg agagataata gagtatgtaa 91260

gagaactaga taataaggag aaaaagttag tagaggaaca ggcaaaagaa aaaataaacg 91320

gtaagaaaag tagtactgta gaaaagtatg gtaacgcact ggatatggta aagaacacag 91380

actttgaagc tttgtgtcga atacagacag cttaccacct acagagagaa tatcctatgg 91440

ttttaagctc tctaaataac aaaggttctt atattcatct aggagtagct aagcaagttg 91500

cagataagaa aaccttgtca ggagatgcta cttacgagtg tgagctatta ttttcagttc 91560

aagtacctaa agcaggtcaa aagaaacaaa aagatttact actagagcta gaaaagagaa 91620

aaacacttga gggtaactat actaacgtag aaggtagttt tactagtcac caatacattc 91680

cttttctaac tttcttaaac agaagaaaga ttgatgcaaa gattgattct atgctagatg 91740

actactctct agctaaaacg aaggcacaga agaagaaaat ccaaggagaa ataaatactt 91800

gggtaggata ccaaaaagta gtaaaaaata aaggatacgt tctttatagc tatgcaagtg 91860

ccaaagtggt agcagtttat cacagagaaa taagtgtata cagttaagga ggggttaaat 91920

tgagcggact aaccgtatct gataagcaaa gaaaagaaat gctttctatg atagagactc 91980

tatggttaaa gagaaatccc agccaaaggt ttaatcaatt tatgtttaac ttagaaagag 92040

agtttgcttc ctataaaaag aatgaaattt caaaacgaaa tagagttttc taccaaatga 92100

gccacttagg gtacaacaaa ggatacgtct atgtagccca ccaagtccct gatttatttg 92160

atacatctga acaagacttc tattcttatt tgaaggatag gataggatag gataggatag 92220

gataggatag gataggatag gataggatag gataggatag gataggatag gataggatag 92280

accttcctat cttttctttt gttctaataa agtagtgggt tacagctaca ctttcagttg 92340

tttacaagtt gtaggactct ctaagtagcc agctatagtt ttatctgtaa aaagagataa 92400

cccagacccc attatagagc atatataccc taagtagcag gctacacaaa ataaccaatc 92460

tatagataac taatgtcatt cagagactat agatgacaca gcatcctttt gtaaatttat 92520

atgttcgggc tagattaaga ggcgataccc cacccctcct cttagagtac ttgtagaggg 92580

ggtatacaga gtttcgtttt cctacctagt atttatatag aatatataag tatcttcctt 92640

aaaaagcctg tggtggcttg tgagagcata caggaaggta ttgtaccttg tatttatacg 92700

gtagcaggta ccttaacccc tcatacaaga ggaagctttc taccccatat aaacgataaa 92760

acactaagta gcaggctata ctttctgata taggacagta tgacttatca atatgtaaaa 92820

gggacaaagc tctaagtagc aggttacaga aaaaaggaaa aagctctaag tagccggcta 92880

cagaatttcc taagtagcag gttatagata ttaaaaccat ttatggggta ggtgaatgca 92940

ggtagatggc ggggtggtga ggactttgaa ataccttgtg aacgttggtg ttatgccgtt 93000

tactgctaga tgataaatag tttcttctat ataatacaac gaaagtaagc taaaaagaat 93060

ttaaaaatga tgtataaaaa ctgttgacac tttttatatt gtatgctatt ataaaagtag 93120

ttaaagaaag caaccaaata aataaaaaaa aactttataa taactgttga cttttaaaag 93180

taagcatgtt attataagag tatagaaaag agcaacaaac taaaaaagtt aaggtggtaa 93240

tctcaatgaa aaaattaaaa gcaaaaagta aactaagctt tatcctccca aaattaacaa 93300

acgacaacaa aaaaattgac tactctaaaa ttcttacatc agcttgcaac gttttcggag 93360

gctacacatt aagagagtgt tcaggcggtt gggttgacaa taatacatta tatactgacg 93420

aaagcaacga actagaaatt ttacacgaag gcttaaacga agacatgaaa aacacagtag 93480

aggaaattct tgattttctt ttcagtgaag aagaagcggg acagcttgca gtatacgcaa 93540

acaacaataa cgaagtttac attttagaaa acgaagatgt aaaagaattt aaaaatattt 93600

tataataact gttgactttt agaaaagaac atgatataat aaatgtacag taaaaaacaa 93660

ataaaaaaaa attaaaaggt ggtaatatta atgaatattg aaatttgggc gtacgcaacg 93720

aaagaagacg aaatttgtag tgcagagcaa ttcatttgtt cagtaagtaa tgaagaagag 93780

gcagaacaag aattaagcta cgcagagatg gaaaactacc acgcttggaa aacttactaa 93840

taaatttata tagcaaaatt ataataagtg ttgactttaa taaataattt tgctataata 93900

agtttatcag ttagataggt aaacaaacta aacaaaggtg gtagttaaaa tgaatgtagt 93960

aactaaaatc agtacaacga tgaagctaga cgactatagc aacttgacag gacaagaatt 94020

tacaacatct ttctatcagt tagcaaaatt gcataaaaac gaaattaaag acggagtaat 94080

gaaagaacta tctaattatc ctgaggagtt acagaaaaaa atacaggatt gccagaaaga 94140

agcagaagga cttaaaaaag tagtacaatt tgttagtgaa gacgtgaaga acaataaagt 94200

aaaattacaa aaaaattctt atggtgacct taacactgtt tctttgaaag caagtgtgcg 94260

gagagcttta gcgcaatacg gtgacaatat taataattgg gaaaaaaatt acttactaga 94320

ggattgtgct atacgccttc aatacaatgt ttctaatgtg tatctttcgg aactagctaa 94380

cttgcggtca gatgcaagtg aaaatatgat tgcatggagc tataaaaaag attattcatt 94440

agaagcagag tttgaaaaaa ttgatgacta catggcggta aaacttatag acttagttac 94500

aaaatcacag catgagcata gaaacacgca tgacgatata agaatcaaac tgttagcaat 94560

tctagaattt caaaaaaaat acgaggttat tagctgggac ttatacaacg gtaaaaagct 94620

ttctgatgaa gaaatcatag caattcatag taaactacca gaatttattt caaagtttca 94680

tgctgtttac actgatattg aggagttaag aaattattta gaaattgagt aaaaaacgct 94740

tgacttagtg atgatacaat gctataatat aagtatagta aaggttatca ctaagtcaag 94800

tacctacata aaattaaaaa aaagttataa aaggtgttga ctttagatac taaacatgat 94860

ataataaatg tatagtaaaa gttaaggacg tgttaattat gaaaaaacct attcaggaac 94920

taagacaatt aattgaggag gaagaaagag tattggtaga ctacatcagt ctagaagacg 94980

acaacggcag aaacatcatt tttattgaaa acctaaacgc agaaaactca gatgacagca 95040

ctatgctaga aacttactcc ccttatgaag cattagagtt tttagaaaat gaggggttgc 95100

ttgcttaata aatttatata acacagttgt aaaaggtgtt gactttaaat agtaactgtg 95160

ttataataag tttatcaagt aaaggtaaac aaattaaaaa attaaaaggt ggtaatatta 95220

atgaaaaatc aaattgttgg gtataaaaaa ttaacggttc aaaaaatttg gagagcaaca 95280

aaagcgcaat tagtatttga aggtgctaga atggactacg ctagatacct aaaaatgaaa 95340

aaacaaggga aagaaatggg tattacagta ccaaaattca cgttgaaaga gatgtaccaa 95400

aatcgtgcta gcatgaaacg tgatgaacta agaaaagaca tgttcctgtg ccttgatatt 95460

ttaaatgacc gagtaggaaa ataaaatgtg gtttgtaatt attggtatca gctttggact 95520

aattttaggg ctacgaaaat acaataaggg gaaaggagcg gaaaaaggtg agcaaaaaaa 95580

accttagtat tagtaagatg aaagaactct gtcaaaaaga agggagtacc ttctttgaac 95640

ctagcgccat gaaattcttc gggacaaaaa ttgtaaccaa accaaaaaat aacttattta 95700

ttacggaaga tttagcaaaa gaccacagaa ctaaaaagtt tacggtaaga aaatttgaga 95760

atggtgaagt gattagtgtt aacggttttt tgaagtacag ttccttggca gaagctaaag 95820

aagttctaaa aactatttta taaatttata tagcatagct gttgacttta aataataact 95880

atgctataat aagtatataa aggaagaggg taatttaaat ggcagacgta tttggatata 95940

atgacttttt aacagaaatg ggaataaagt cagtaagagg aaatgcaatt acagagcaat 96000

taaaacaaga ttcaagctat attgtagagg atattaaaag agcatttaac tttaaacttt 96060

acaataatga gctaacaggg gaaaacaaca tggtgaacgt aactaacaaa cctctgtctt 96120

tagtagtatt cttagcagac aataaaagta aagtattatt ttacgatagt gtgaacttta 96180

ctagtaaaaa agctcgggac tggaaaccat ttcacgctat ctttaaaaat ggtaaaacgt 96240

atattgtaca agataattca aattttgcct tgacattcgt tattaaaaat ggtactatta 96300

ttaaagaaag gaaagcacct aaaaactcag gtttgatttt cttagaaaga ctagcggaag 96360

gggatttata agatgaaaac tgttaagaat tgggttgaaa gcaacatctt tgttattcta 96420

acttttgtaa tgggtttact cttactacta gctttattac actattaagt gttgacttta 96480

cagaacaagc atgatataat aaaagtatag aaagagaggc acacaatgaa agattacgaa 96540

gtaaaacgaa ttgagacaaa ggaagctaaa ccttttattt taggcttaca ctatgcaaag 96600

cgtatgccta gcgtgtcgta tgcgttcggt atattccttg atgatgagtt acttgggatt 96660

tgtacaatcg ggaagcctgc aagtcacagc ttatgtaagg gtgtatgcgg agaagagtac 96720

gcttataaag tatttgaact aaataggctg tgtatgaaag aaaagttaga agctaatgtt 96780

ttaagttatt ttgttagtaa agtgcttaaa gaattaaaga aggaaaatct aatcttagta 96840

tcttatgcag attcaggtat gaaccatgca ggttatattt accaagctac gaactggatg 96900

tacacaggta tcacaaacga aagaacagac aaatatacac ctgaaaacaa gcattcaaga 96960

cattataata acgagtatag tcacttacga aaagtaagaa cagctaagca caggtatatt 97020

tacttagcag gggataaacg ttttgtaaaa gaggtggcaa acaaaattaa ctacaaaaaa 97080

caagtatttc caaaatctga aaacaaaaac tacgagttag gaacaagaca gaaaacaaaa 97140

gtaatcaata caaaggataa cactattttt tatcaataga ggagtgtcac aaatgaaaaa 97200

taatacaata aatataaaag atgtaaagct aacaatcaga aaagatacta taaaaatgaa 97260

aaagctagct aaaaagatag cagagtatta cacagtaaaa aaaccttctt tagcggatag 97320

cttgcgtgtg caacttattg aaagtgaagc taaaaaacta gaaagcacta ttgcaaaaaa 97380

tgaggagctt ttatatttga tgaataactt tactaaataa attagttttt actgttgact 97440

ttagaggtta aacatgctat actaaatata taataaaaaa aggtggtaat attaatggaa 97500

atgacagaac tcattaaaac taaattcgaa aaacacggct ttgaaaatct aaaagaggaa 97560

atggaagact tagcagaggg ttaccaaatt tttatagata ctacaccaga agcatttgaa 97620

ctagcttatg aactagaccg tgagggcttc acaacatggg aagaagcgga aagtctttat 97680

attgagttgg agagtgcgta acacatgtat actactattc cacattacat tattaaaaaa 97740

gtatctgata aggaaaaggt aaactaccaa gcaatttgga aggtactacc tgtagaggtg 97800

caaaccattt ttgtatcaaa gaaaacagct agcttctata tagttgatag atacgctaat 97860

agccaatttt tcagaaactt gctaacaatg gcagggaagg caaattttgg ggtatcaatc 97920

agtagttcca gcaaatccca tttagagata aggctcacct ttctaaatac acatttttag 97980

ttttatatag tgtagctagt cctttattgc tacactaaaa taagattaaa aaaattaata 98040

taaagtgttg actttaaata gtaactgtgt tatactaagt atataataaa agataaggat 98100

gtgtgccaag tggaaaaatt taagtcacta ggagaggcta caggtgaaga actagccttt 98160

gttttagaaa gtaactccca aatatttgat ggtgtgctag acattgcaga aaaaaatctt 98220

ggaaaagaag actttgacaa acatgttaga cataacggtc actatgatgt tgcgctatgt 98280

taccttgaaa tatatgcaga ggataatcct aacatcttat tcgagaaaaa tacctttcaa 98340

ctaaaaaatt aaaaaaaaag ttatataagg tgttgacttt aaaagttaaa cgtggtatac 98400

taagtatata gtaaagaaca acaaaaaaat taaaaggtgg taatacaaat ggcaattaat 98460

aaaaataagc acagtgtagc agttgttaaa actcaaacgg ggaaaattgt tggttatctt 98520

tcaactagag acgaagaaaa tggcgtggat attcaaatgg acatgcaagg ttatactgta 98580

gaagcgctat aaaaataatt tataaattta tatagcatag ctgttgactt taaataataa 98640

ctatgctata ataagtttat aagttaaaca caaaggcgga gatagtaata aaaaaactag 98700

tataaagtgt tgacttttag aaataagaat ggtataataa atgtatagta agagttaaga 98760

aaaaaactaa aaaaaaacta aaaggtggta attaagatga cagttaaaac agtttatagc 98820

ccaaaattag ggtacactta tggaaattta gaagtagaat acgcagatgt taaaagtttt 98880

tatggtaaag cttattttag aaatatgcac acagacgacc acacgcatgt ataccactta 98940

tactcttacg atactttaat tcttgaggtg gtagacggag ttgtaacagc aatgacagat 99000

gacacagacc actatagcat gacaactaat agacacatta atgaattttt agcacagtat 99060

gaagatgact acacaggtaa actaagcaaa aaagaactac tagaaaaagc taaaaaccta 99120

ggaactgctt tagtatagac ataaaagggg cgggttaacg tgactacatt caagaagaac 99180

cgcacagtta actgatacta gaccaagtaa aaaagagttt ataattataa aaagtgttga 99240

cttttagaaa agaacatggt atactaaatg tatagtaaga gttaagaaaa aaaaactagg 99300

agcgtgttaa aaatgagcgt aaaaattatc agtgaaggtt tagaaagcta tatcccgtgg 99360

agtggagcag ttgtgaacta cgaaattatt caagaaaagg gactattaga tgtttttgag 99420

tcattgttag cagaatggta tccggaagga ttaacagaaa cacaagtcaa cgatattttg 99480

tggtttgatg aagacttatt cagcttttta ggaattgaag aggaggagga ataataaaaa 99540

tgagtataat agcaattaaa aaagaaatcc atgcaaaagg ttacaaagtg acaggaaccc 99600

atcaaggcta tatagcacaa atcaattttg acggtacagg aaacgaatat cctttaccag 99660

ctacatggga tgagttcatt gaaacattta aagatggttg gaatggtact tatgaagacg 99720

aacaagcgtt cttcaacgat atgcaggaag ttgcattaaa agaaatctta gacgaactaa 99780

tagacgtatt atataatcta gacattacta catataattt tactatagat gatgttaaaa 99840

agaaagttat tacattaaac aaacctacag acgaagaaga tgcagaagac ttagtaatag 99900

aatttgattc tacttgcttt tgggatgcta cagtagaaaa tgataaaata aaggttactg 99960

taagaaacaa aagtaaataa ataaataaat aaataaataa ataaataaat aaataagggt 100020

tatcactaat atatttatat agcgtatctc tacctttcac ttgcaatact agcggaggta 100080

tgctacaata aacatattag acctaaataa aaaggagaaa cttatatgat gacaattgaa 100140

gagttagcag gtctacaaaa agtaaaaaat aagctagaat gtgcatataa acaactaaac 100200

gatgctacag aatcactaga aaccactcta attaatgaac ctctcgctat aataaatacc 100260

gattactctt ttctagctaa taacatagag gatgccagag aggtattaaa caaaacaatc 100320

actaagttag acaggtataa cgaagcacta gcaactaata aagaagagat agacaagtac 100380

cttcatctta ctaagtctaa aactaaataa taaatagtat aatagacaaa aaaaaaataa 100440

taaaaccaaa acctgtaaat cacctcagca tttttttcac acggtgtcaa ctcaaaaggc 100500

ttatatatgc gcagtctttc tacctatttt ttagagggga taatagtact atataaataa 100560

cctctatagg tataacttct tatatttagg attatttcaa actaattagg tctaaactca 100620

cattattata atgtcaatag ggtataactc ctaataatat ataaaactat gtacctattg 100680

actaataatg gtgtgtaagg tataatagag gggggaggtg taggcatctt atgaaaaaga 100740

agaaattcag tttagttatt tctttattac tacttcttcg attatattag tgagggtacg 100800

cttgtagatt taaaagaaac aaaagtaaaa taaaactgtt gacttattag gttaactatg 100860

ctatacttaa ggtataaaca gttacaagtg ggataagagg gtataggagc tcgtatatag 100920

gcttttactt tattagatag gtaaatagca gtaaataaac taatcccgct cagaaaggct 100980

tgtaggaggt tagagaagat gtttgtatta aatggtttac tatgtacttt actagtattg 101040

atgattctag tgggggctat tgctttaaac gcagataagc agactaagct tattgtagga 101100

attatacagg tggtatttat gactatgtgc gtagtcctct atatcactat tatagggatt 101160

agcattacta ttattccttt tggtgtatta tttgcgattt gtggggctgt tgctttgttc 101220

aatctactta cttttaataa ggaggagata tgatgacaaa atacaagtta aatacggtta 101280

gtaataataa tttaaaagca ggggacgtgg tacttgtcga gaacttagaa tgctacgaat 101340

taaaatcagg tgtttacaag attattagtg agtgcgacgg tctatataat tttgtgggta 101400

ttgatgtaga cgtgtacgaa cctgtttctg tagaaaacat taaccgactg cttgggagct 101460

gtgtgttacg tgtatccgaa ttaactttgg atgtagtcgt tcaaaactaa gattagaaaa 101520

ggaggaaggg actatgttgt tatttatcat gaatgttatc agtgtagcta tattaatgag 101580

tactttttac tatatacgca atatatcccc tttctattac gagcggacga ctagactagt 101640

taatcactta gtagatatag agctgatttt aggattattt atctggttag tgtttattat 101700

ctctattact tttgtatagg gaaaggagta gaactatatg cttattataa agtctttaat 101760

tgtagctatt atatttacta tcttactagt ttatactata gacgggtttt ctcgtattcg 101820

taaaggggct aaaagtaatg gtattttagc tgttgtttgc gggattatag ggataattac 101880

cttttctatt ctagtagtta ttatctttta gaggtatacc aattttttaa gggttcattt 101940

agctaaaaat agctttgggg tgtgccaacg agtttcgcgt tttggagtga aaatatccct 102000

aggggggaat tcaaaaagtt cacctatacc aatttgaaat gagggggttt tattgtttat 102060

tgtactgatg ggcgttttgg gaatagtgtt agtcggcttg ggtaactatt atacaatgaa 102120

aggtctagcg atgtcacata agcttagtaa agacgtagct agtgaagaat gggtaaatga 102180

acagtttgaa gattctatag aaggtcgtat aaagctaaat aactatgcta gagctactat 102240

gaatcgttca gactatgtat ttaacaaagg gatgttatta ctatttatag gtgccttatt 102300

acttatcccg ctagtaactt taatttaaaa ggaggtagaa aatgggttta acattagtaa 102360

atgacgatga taagtatcta cgtgcgttta aactagattc ttttcaatca gtaaaaggaa 102420

ttatctatgt atatattgtt attactcccg aagtattaac agagtacctc tcacagagct 102480

cagatggaca gtattttaag agtagtaaag attatcaagt aaatgtacag gaattagcta 102540

gatggcttaa aacgtctaat agaggcttta tagagacact atctcataat gagaacatta 102600

tggcaagtgt aggagaaaat aatatataaa agagttgact tctttctttt gatgtgctat 102660

agttagtgta tcaagtaaag gagttctttt tatatactta aaaaaaagtt ataaaaggtg 102720

ttgactttat aaaagaacta tgctatactg aatatataat aaaaacaaag gtggtaatat 102780

taatgaacaa caatcttaat tactggacgg atacactagc aatcaacttg gcgggattaa 102840

cattagtaga acgggaagag gtaactacac aggaagcact catatcagag atgtatacaa 102900

gagacttaca agctaacaaa cctatgttta ataacttatt agataaccta gaagagttaa 102960

acgacaaagc acgagaagct cactcagagg ctttctatca tgcagtagat gctattatat 103020

cgctgtggga cggaataagc aaaaacactt tataaaggag gaatagttat aatgacaaat 103080

aaaaagaaac caactgtaca gatgacaggg gaggacggaa atgcctttct tatcttagga 103140

agagttacta gtgcgctgaa acgggcgtga tacaccgaag aagaaaggga acagtactat 103200

accgaagcca catcaaagga ctacgaccat ctactggcgg ttacggctga atgggtgaat 103260

gtagagtaag catataaacc aatactatta gaaaattatt agataaaagg gttgacaagt 103320

tcaacccctt atggtatact tagtatatag taaagagagg ggacgagtta catgctaaca 103380

ctaaagaaac tattgaaagc taacaaagga caaaagaaag agattgagaa agtattatcc 103440

gaaagagaac aagctatcgc aaacttagaa aagctagtta aactacaaca atctgcttaa 103500

gcattcagtt acgtcattag tggtaggcat atatacccac cccgtggttc tgtgaccgta 103560

ctttggggct atgctacaca gggggctcct ctcggggcac gtttttcggg aacttttttt 103620

aaattttttt attcttaaag tcaacaagga atggctctat agagggcttt acaagaatct 103680

ttataaaaag ggcaccccgt tgtttttcct taggtaattt taaggagagg cttctataaa 103740

taaagtgaaa aaattccggt aaaaattttt aaaataggga ataaggaggt gttcctatag 103800

atatgaataa agataaagac aacgcattag agtttcgagc ttggtgtaag ataaaacaga 103860

tgatggcagt ggttatctct attaattttt ataaaggaga tatacaaggg gaggatgtat 103920

ttctctctta ccctgcactt gggtcattta acttaagagg cttttccgag gttatcctta 103980

tgaggtacac agggttacga gataagctag ggacaaagat attcgaaggg gatattgtag 104040

aggttccttt agaggcagac catggatatg caggctctgt gggggttgtc acgtatcgta 104100

actatggttt aggggttatg acacctcaag ggttctttta tttagaggaa cctgagttgc 104160

ttcttgttac agaagataaa tatacgaata aagtagagga gggttaagaa tgaaagcacc 104220

tatttcagaa tgtccttatt gtggttctac agaagggtac acacttaggt cacaagcaag 104280

tggtactatc tactggcgga tgaaatttaa tgaaaaagag gcagataatt cagagtcaca 104340

tgataattta cagtatagtc cccataagta cgcttattgt accgtatgtg agagtaaact 104400

tttcaaagca gaggaggtga atgggtaaga tggagcttcc taagcacatt atagtaaaaa 104460

ttaaacgagc agggcaccat aataaaaaag caaatgactt acaaacggaa gtacgcaagt 104520

ggttagagag tagattaaca gatggggaat ctctccctga ctatatagga gaccaactga 104580

tagatgcgtt ggagcttggt gtagatggta gtgaggcgtt acttaaattc ttagctgaat 104640

atgaggagtg aatcttaatg gaaaaattga atgagaacca gcagattgta ctaaagtggt 104700

taaagaatta cttattagat gattctgatt tttataacac ggtatatgct tctaaacaat 104760

tatacgtgaa cggctttcct agtgataaaa gtaatattgc ttttatgaaa ctaacagaca 104820

aggagttcgc tagcgtacta ctagcgttct ctacatggta cttactagaa ttagaaaagg 104880

aggatttaac atgaaagaga aatgtagtat agagatttct tactataacg gtgaagacaa 104940

gtttacggga aaagtagtgt gggttgtact agacaaagaa gaaggtatcc aatttgtact 105000

tactgagaaa ccagaggacg aatactttgt ttttgacagt gaggagtcgg caggtattgt 105060

aggtggtctt attagactaa gactagcatg tgacaacata gaagatgact cccctgtaac 105120

aattaattac cttaaggagg aggagaataa tgaatagctg ggagtctatt ttagggctag 105180

gtagcacgca caaaatggta atagcacaag agttggtaag ttttctttat aaggaaaatt 105240

gtgatactac tacgctaggg gagaatgagc ttttcatact aaagtttctc ctcgataaca 105300

aagtggagtt taacagaaaa gaaacttatt atggaacaac gttctacaga ggttatactg 105360

taaccgtaaa tgttagggac tctgtgttag caactatagg taaggatata gcttccgtag 105420

ataaagacga agataaggct actccagaag cagtaagttg gttagaagtg gaatgttgtg 105480

atatactgta tgagttcctg tctactaaac taactgctga gaagggtagt ggtagactta 105540

gtgacacgct taaaataagt cgttcttatg aagaaaaggt aaaaggacta gaaataacta 105600

taacaaaaac aaaagttttt aacaaattag gaggagagta ataatgaaaa agagtttatg 105660

gagtacaata ctagaagaca cagtactaga ggataataac aactcaatag catacgcaag 105720

tgttgttaac acacatcgca agtttagacg attactagag aatgaggact atgaggctac 105780

tattttacgt atgcttcttg aaaggaaaca agaaattgac ggggtgtttc aaagcaacgt 105840

tgagtttagt cccctaagtg tacgcccatc agcgccacct gtaaaagaac gagggtacga 105900

aatagttatt gatgtgaaga gctttgtagc cgaagcatta gatatgccta acgacctaga 105960

tattaaccta ggtattaatg gctttgagcc tgtctttgct aatatgctac aaaattattt 106020

aaatgaaaag atttttgaag aaaacgaggt aatatatgag tttgagggta gtacatctgt 106080

ggagcaatct ataaaagtag agggtgacgg ttgcatcgta gttgtgaaaa acataactcg 106140

tacttggtaa ggaggaactt atgaatagaa aaacgtttag acctagttgg cttggagaag 106200

cgtataaggg gtatatacca ccaaaaaatg attttcctgt gatacgtata aaaggagaca 106260

tggtttcttg gtttactatc cttttagaac aaaatgaaga atttgtacgg gagtggatgt 106320

taaagtataa aagaacaaca cgtacaaaag aaacaggtgc tcattatata ggggatagtg 106380

actactccac ttattgcgtg acaacctacc ttgttaccac acgtacagca tcattcaatc 106440

ccaaaaggct tgttatagct ctatttacta aagaactagc tcttttttct agggaatcga 106500

catcagtaac actaacagaa gaaaatctaa atttaaatga gcgtattact gaatatgttg 106560

aggcattgag tgagtatcta gagtctgtag gtatttttcc gttctctctt cctagttggt 106620

gtcatcccga ctatcattac acatacgatg gctgtcgttt tactatctcc aagagagaaa 106680

gggaagtaat atctagcact aaaatgaact tagaagaata taaccaacat ttactaaaaa 106740

gcaaaggaga gaaactaatg aaagtaactc aattagaaac atttaacgat tttattgact 106800

acatggttaa aaagaatgaa gaaggctgga agtgggaaaa caaagaccct ctagtacctg 106860

taagtttcga aaggtggcaa atagatagag accaaacact tatagctaaa aactttagaa 106920

ataaggttat ttatacagtt agggtggaag accttgacca aacacctaca attgtatcat 106980

ataagacaga tgcggactgg gtcggtagct tagacccaaa agaatatgaa tgggagacga 107040

tgaaacctaa aaaagtacct attgtccacc cgtgtacatt aactgttgaa gaactcgaaa 107100

gggattggaa gactaataca aaagagtatg acgaagttaa taatccttct cattatacgt 107160

ctgggggcat tgagcctatt aaatatatcc agagtcacaa aatgaactac gaaaaaggta 107220

atgttattaa gtatattact cgggcaggta aaaagaaagg tcagagcgag attaaagacc 107280

ttaagaaagc aatgaaatat attgggttac tgattgaaga tatagagaaa tatgagaata 107340

gctaatttaa taacaaaagt aatagggttg tggattctgt gtagactagt aagtgtctaa 107400

gggttcacaa ccctaattta aaatgtgaaa acaaacgaag tataactgtt gactttttac 107460

ttatttcatg gtatagtaag tatatattaa agaacaggag tgattacatg agtagagcag 107520

gctacaaaat tcgttatgta aaccagagag gtaagatggt ttgggttaaa agtattgggt 107580

ttggaacaaa ggcattagaa gtcgatacta ctaccaacaa agagaaggtt aaagtatttt 107640

tggataagaa atgggcactt caagtatgta aactcattga tggcgaccta gatgttaatt 107700

gtatgataga cgacattact tataagtatt ttatcttcta tgaagcagaa gataagaagt 107760

actttctaaa gtcgctaact gtagttcaag ataagattga gcctgaatgg acagaaaacg 107820

agccagaacg agcggttatt cataaataca atgatttaga tagaatttta gggctagtta 107880

acgatagaat tgataaggaa gcaagagcac gtcacgatat gtttttactt taaacgagga 107940

ggtaataaaa atgattttat atggagtgtc tctttatggg gaggtaactg agtggactac 108000

agataaagct actgcaaata aatgggtaga taatgcagta caatctatag aggataatga 108060

tatagatgag gactgttatg ttattttgtt aagactagac ttagaaaaac ttctatatgg 108120

cacattcgac cgtgaagtag atttaaacga ccaactgcat gatgaagcta cagtactaaa 108180

agaattacgt tggcaggtgg acgaggaagg gctatataat attgtggagg tggcggaatg 108240

aataataaaa agacagatta taaagtatac aaaataacct acaagcaacg ttttatgggg 108300

gaggttctta ttgactcgta tgaaagagta gtgagggatg ataacgaact acagtctgca 108360

attaacgctt tatatgatga cccacatgtg ttctcggtta gtagtgagga ggtgacagaa 108420

taatggagtt taaatcagta aaagaaataa atacagagat tagtttgtta gctgacaaag 108480

gaggtatctc agacggctca cacacatttg aggagcttta ctttcataga atggttttat 108540

ttgcaacaat atgtaatgct aatcactcta aaagttggaa gtctaagaaa catgaggacg 108600

gttctatgtt tgataactat tttatcgttg gtatttctac atcgaaaggt atgttttctt 108660

accattatca cttagacaat tgggactatt ttagtgttcc agaactagaa tttgccccta 108720

catgggatgg acatacagct aatgatgtta ctcgactttt agatatataa gaaaatgtaa 108780

gtaaacaaag gaggtactac tatggattta aataaatttt taactgaggt agataaagtt 108840

acttctgctt acggggagga tgcaaactac gagatttctt ttattcgtga gaaatatgcg 108900

gacttagacg aaggtgccaa acaggactgt gaggaaacgg acttctttga ggaggtagaa 108960

tacatcagtc agatgcaaac aggagaagac tgttttgagg gctatatttt tagaaaaata 109020

aaaaacacag attggtgtct ttgtgtcacg tatgctatct gaggtcttgg aattactact 109080

aactttttag gttggcttaa aatgtagtca attagtaaac atattaagga ggtaaacata 109140

aatgatacaa gcttatagag ttcgttcaca agaagatttt gttaacctaa tggttaactt 109200

agaaaaaaag gattttaaat ggtatagtgg gttacctcct ctagacggtg tatactacag 109260

acaaaaccca gataaagact tagctagtga ttctcttctt gttgttctaa actatgaaca 109320

tcgtactatt agatactata cagttgctaa tttaagggaa gcttacccac tagcaaccat 109380

ctatacgtac acccctagta gaacatctaa aacaaactca gtagacggtt ttactcctac 109440

tcagttagtc tatgacgaag aactttacct ataaatgtac tctacacaac taaaggcatt 109500

atacatgtct ttttttgttg actttattta tcttatatgg tatactagta ttataaataa 109560

aggaggtgtc tttatgaggc tatccctaag aaatatattg gaaggttttc ttctattctg 109620

cctagcttta ccacctatcc tagcagttat gggattttta ttaggtatta tatggattac 109680

attttacttt cctatgtttg gtctagcttt ttggagtatt ttaagtgcat gtttagtaga 109740

gaacttgcta gaaaaatggg ataacagaaa gactcttaga ataaggaact ttagtatgtg 109800

gtatgttata gccttactta ttgctcttac tattactggt atagctatac tagtgctgtt 109860

tttattctag aacataaaga gttatgtctt aagctagttt ggtaataaaa taaacataag 109920

aaggagaggt tattttgaaa gctttgtttt ttgtaatagg cattctagta tgccttttag 109980

gtgttgctat cttagctaca gcaatactgt ggattactac tagtcttcct gcaatcggtg 110040

tcccttttct aactattact ttacttgtag gaatcctact agttctgctt atagaaggaa 110100

ataagaaata ctaaagaagt aatggaggtt gttaaaatga gaaagataga cataaaccaa 110160

gcaagtgtgg atttacaact actaatcgaa tttgagaatg aggactatac agtagcctat 110220

agaggagaag agagtaggca tacagaagaa atgaaggaag caatcctttc agctcacaaa 110280

gatacaatag accgtttaga ggtagctaac gataaactac tagagactta tgaagacctg 110340

atagaattga aaaaagaata tcacaccacg tataagaaaa atgagaagtt agctgtaaaa 110400

aataaagaac taaaaaaaga gttacgagat attacaaatg aaaaagaaaa gttagaaaaa 110460

gagctactcc gcataaaagt gtctaagggg gaactttaat gctagtatcc cttacttttt 110520

tagaggtact tttatgggta ctgactttgt tatactttgg tatggtaatc tacgtactct 110580

tatcccacct agttactagc gtagcttacc ccgagtctga tggagttgct agttctttta 110640

tagactatag ttatttatgg ggggaatctg atgagacgag caaggctagt aactataaat 110700

gctacgacca tataaactac agaaaaacga gctattttaa ccccacttat aaaagcggtg 110760

aatttctaac tagcacagaa aaggtgtcta agcataaaag aaaagtcttt aaaaataaac 110820

aaaaagttaa cttagatgaa gaagaagagt tataaaagac acataagaaa aggtacctgc 110880

taagggtacc ttttactttt atactatttt ttgttttact ggctctacta tgttaaattc 110940

ccctttattt gttatagtga tagcattaag ttgcccttca aaacagcaac cgccatctat 111000

tcctataagt ttttttctgt cgtgaaccca cacgtcaaaa ttagaataat tacggtctct 111060

aaacggtaag taacaggtag gtgtgtgacc gaatacaatt acttttcctg tgaggttctc 111120

ttgcttatac cagtccctaa tccacataaa ctcgtggtct gttgtcttct tccagtcttc 111180

cccgtataaa gattgaatac ctgcatgagt aaacagaacg tcaccaaact cgtaatacgg 111240

cttagaagct ctcaggagct ctatatgatg tctatatttc ttttgtatgc aaatacttgt 111300

aaagtgataa aaagacgata gcctatgcct agaacgctcc tcctgtggca tattattgaa 111360

gtagctagac aaggtgtttc ctcctccact agcaagccat ctgttaatag tgtactcatt 111420

ctgctcttct tctataaaat caagcaacat ttggtcgtgg tttcctctta taacaaccac 111480

ttggtcaccg tactccatct ttaaatcaca aataatatct aaaacctctc tagactcgtc 111540

ccctctatct atatagtccc cacacaagac aagcaattgt ttatccctat cccaattgtt 111600

taacatttct aaaaactcta agctacaacc atggatgtca ctaattgcaa aagcttcttt 111660

gaatttagtc accgaggcac ctccttaatc aaaattaatt tcttctttcc aaccttttat 111720

ataagatgtt aggtctcctt cgtaccccat aagacgtatt tccattacca actgacctat 111780

cgtagcattt gaagtgctca taccctctaa aacctctata aacggtctta gtcttctgtc 111840

cataatctcg tttgcagttt ctatagcact ctctttgttt ctaccgttct ctatgagtaa 111900

atttacgtaa tcttcataag tcatgttaac atgggttaca ccgtccacca cactagaaaa 111960

cctacagtat ctaccatttg gttgttgtga gataaagcta ctcatccttg atattcatct 112020

cctctttaac agtacttttt agccttttgt tttccgcttt taccttttcg tataaccaga 112080

gagctgtaga caagtccttt cgtgttacct cgctgttgtt tcttactttt gctagtagct 112140

cctcatacgt tttatcttta aagtccatag tagctccttc ttctgcattg tcccaagcga 112200

cctcgtaagc aaccattcct gtcattcctt gcatcacacc cacaatagta cctatagatg 112260

tgttataacg atgttgtacc agttcaaact ttttaaactt attcattctt atctatcccc 112320

tagcaattat ttgtaaaagt aattccaaaa gattccatcc taccatatct aacagaaaca 112380

caagctgtta ggtagtcact tactcgtatc tttactttaa caatgtcacc atctatagga 112440

gacttaaatt ttatttctat gtcaactgca aaatgattgt tgtgattaat ccaagatttt 112500

aaatggaata ttccttcacg catctttgtt acagttgtgt atggatactc tagtgaatgt 112560

ttgtgtatat ttcttggaaa ttctccaaac attgcaatta cttcttttgg gtttgttggt 112620

gctaacatgt gatgtctatt ttgatttgat aataactctc tgcctttttt gtcaagctct 112680

aaaaagtctt ctcctctatt tttcagtaaa tcaaataata atttttctgc aattaccttt 112740

ccttcttttt gtctttcttt tctctgctgt ttatcatatt tcttcttttc tttaattatc 112800

ctattccttg catgactaaa tttttccata attactgtca cctcacatct ttatttactt 112860

ataaaatcta ctaatattgc caaaataata agaaataata tcactatcca aacaaacacc 112920

actattacta cccaaataat tattggaata aacactgtcc accaaacaat attaataaaa 112980

ccagttaatt gaagtattaa taaaactagg aaagctaccc cgcatactgt acttaatctc 113040

atacttatta ttcaccttcc acactggctt cttttaataa ttttattgta acgttttcat 113100

catctgttgc tgttcccgca agaacctgct taaccatctc ttcaaaattc tctctttttg 113160

gctcttcaaa atcttcttcc gtatacacct caacttctac ttccttttta ggaaccaaaa 113220

gtagtccacc aaattcatca ttttcgactc cataagtcat cttacctgct tctacatctt 113280

cgtttgtttt aattgccatt attgcaatta catctttaat agtatccacc atattcactc 113340

tcctattagt ttagtaagct tagccgggta ccacagtttt tcattattat taatatccca 113400

ttctattaaa taggcaaaat cttcaccttg tactctagtg tatcattata aacttcctct 113460

actgtaccta actgtttttt actctgtaaa atgaactcta cttggtctcc tactttaaac 113520

ttcatgcttc ttcctccatt tctaatattt cgtgagggtc tacctttaca aaatcagata 113580

aagagtacct aaatacaacc ctgtcttttt gagatacaag ggcttcttgt ttttcttttt 113640

tatgacccct acaatgtttg caaacaagaa gtacatcacg agtgtaaata ttcgtgttct 113700

ctatagattt ccacttagaa gccgtaccct cccatatgta gacgtgactg caagtaggct 113760

tacttctaac taaactagct agtttactag tgtatatttt catagtgtaa acacctccaa 113820

ctacattata tcataaaaaa agaagagtgt caataaacac tcttacttta gatgttccca 113880

cccgtaggca ctaaatatag caattaccgc taatggtaaa aacgtaataa aggacacacg 113940

taagataaat cttgagatag gatttctgta gtaattccat agatatgtta ctttaacatc 114000

atggctatca tctttagact ctaaataaat aagtaacagg aacagtgggt atattgctac 114060

ataaatttga aggattagta acaccatcta tttagtatcc tccttaagag acaaattatc 114120

gaggaaggct actaagtctt ttttagctaa gtttcctttg tgattgccta caaagtcctt 114180

tatagcagcc accagttctt cgtgaagtct tctgttctct tcgtaaacct cattctttac 114240

cgtccgctct aggttacttc ttgcactctc ttctctttta agtaaaacgc aagcttcccc 114300

ctttagtcta gtgttgtctt cctttagagt ctctatctct ttttcctttt cttccatctg 114360

cccgacaacc ctttgtaact ctcttagttg ccgttctttc tcacccaaaa taacttcaag 114420

agcgctcttt tctcctacag atttccctag tttgctggtt acctcttcta gttcagtagt 114480

catattttta atagcttcct tatctttctt ttgttcttca taagcatcta caatatattc 114540

ttttaaattc ttatatggga tttctttctg ttctttttcc tttagtttgc tattttttct 114600

tttagaacta attacctcta aaaacttcat tcggattcct ccttcattgc ttcaatctct 114660

ctatgcactt cttcatagta actagctgta aaagttactc ttgaaaactc ctcgtaagcc 114720

tttagctccc gctcgtccct ctcagttatc tgctcccatg taaaagtttt gtcacactcc 114780

ttgcagtata aatcgtaatc taagtcataa ttacattgta aacactctac tgattcgtct 114840

ccttcgtagt gcatatctat ttaccacctt tagagatact atcgaacttt tccgctaaat 114900

ctgcatacat atctgatagg gagtcatacc agactgttct agagaaagca acagagtata 114960

caatggcttt ttggtagtaa agtctgtcaa ctcttaccgc aagacgtcta aaactatctc 115020

tactctcttc taggaaatat gtgttactag ggtcaactgc ttcttttatc acagggtaaa 115080

tctctttaac aaaataagct tcttctttgg agtcaattcc cttttgagtc agtataccta 115140

aaagaatacc tgcacgttgt tttacctcct ctggtgtatc tcctgatgct actcgtttaa 115200

ctaattcttt aatatcttct tctgtaccta cgcctataag gtgacaataa acttctaagt 115260

agaatttatt tactcctagg tgatattctt cgtcttcaac atttttccag ataaactctt 115320

taaggctttc agcaggtaag tctagcattt ttaaaacaca gctaacgtta agggctctgt 115380

acttgtctaa taactcctct tctttttttc tatcataagc aagggaagct gtatgtctgc 115440

taaatgtatc tacgaaacat tggtctttga aaagatagtt cagcttttct tcaccagatt 115500

gttttacgca taccttaaaa agtttataca gacggtcata atcagcagag gataagtaag 115560

taaaccaatc agctcctact ttaggtgtga accctataac cttataactg ttctcattac 115620

aaatacgaaa ctcttgtctt ttaattatac tagcgtaaac gaaaaattta cctttctttt 115680

ccacaccaaa aatatttttg tctttaacag gttctgtagt gaccttaata tcccaacagg 115740

acttcaactc cacccctaaa ccacctaaga tactgcccgc tacattttct atcttaatac 115800

tatcatttaa tttgaactga ctgtacttaa gccccaattc ctcagtaact acttctatca 115860

ccaaaggcat tatatcctgt atacgttcct ctgggttctc acttgacttg cattcaacta 115920

taataagagg tagccatata gtcccattac ccccttcact tgtactaccc gcatctcttt 115980

tttcataatt atccttaata cgttttttat ttagttcttc tactatacct ctgtagtaaa 116040
tgttcatcat tcgagtgtta tctttgatgt ctactaacgg aatgttctta tactccttat 116100
gcttagccat actattcctc ctcactacct attatttctt tgtagaagtc ctttacaaag 116160
tctgcttgct cctctttagg aacaatacta ttaatttcct cctcaaagca aaatagaata 116220
aagcccactg ttttgataga gccctcatta tatagcttac tgcttagaac catgtagtcc 116280
aacaattctt ctttcttcgt attgtctcta tgaagcttgt caaaggtatt aacagaaaaa 116340
ccaaaaggac tcagctcctt tataactaac ggctttaact caccaaactc tactttaggg 116400
ttagttttac ttccgaaaga aagctccaac tctttagtgt actttttaac aagttcttct 116460
acttctgtgt acgttcctac gctacagttg tttttctcct cctgtgagct atattttacg 116520
taatactctt tactcatact atcaactcct tattctaatt gtatcacaaa aactctatta 116580
agtcaacaaa aaaagaagat gttactcttc ttttaatggc agtctagttt ttagttttcg 116640
tgttcttcct ttgtatctga gattgctagt atcagtgtgt actgacctta cttttacaat 116700
actctctttg acactatcat atagaaaatc ccctacagct accttaccta acaagctgtc 116760
atctaaacta aataaatatg ttttagcata ggtacagctt aggtcatcct gaacatagct 116820
tatgtaaacg tacttgttct cataatctgt cgctccccaa gactcgtcta cttcccatcc 116880
accaagtata gtttcaatat tggcatttag tatctctgca atgcttctag cttgcgttaa 116940
gcttgtcagc ggtcttacct gattaggttc tcgaactttt tcccagttta atacaaaagt 117000
acctcttttg ttcttaataa atgatgttgt gtagtatgcg tatggaaacc ccttctggcg 117060
tagaacatac atatttcttt tttttaccat ataaatcaac ctcctagtat ttttggtaag 117120
tcctctgata agtttacaga tacgtctttt cctagtaaat taagtaactc tttgatagct 117180
tcttctagtt ctacaaagta ctgtggtgac ctatttcgac tatcttgttg caacttaatc 117240
acgcaatcca tattcaaatc agggcatgaa ccctctctta ttcttataat attcagttta 117300
ttatttaacg catgttcgca cttctgtcta tctttttcag cattttggtg ccaacgacct 117360
ccatcatatt ctaccactag tttcacagaa ggtacgtaga tgtcaaatct ttttcctgct 117420
atattttttt ctaatacaat tcgagggaac gcttttttta aatagtaggc aattatctta 117480
tttggtttag aaacagtaga ggaacactta gggcaagatt taaaccctac aaacttagac 117540
ttcacactct gctgataaga gccatgctcc ttacatttcc accaaacctt tatgtcgctt 117600
cctcgactta cgttcttagg ttttatacct atagcattgt ttttttcata gtcccatagt 117660
tttcttagaa cagagctatg ggactcacta ataggtatac accctctctt aagagcttta 117720
gtagccatag ttatctcctc cgctctctac tatatcttag gttgcaaagg tctacttctg 117780
caaagaacat gtagtctacc tccgcatgag acatccctaa gtctcttaac tcatatactt 117840
tatcttttac agtttccctt acacgttcca gttctttaat accttcggaa gttaggttac 117900

cttccgcatc tcttaattta atagccatag aattttctct tattggagtt agttgctctc 117960

ctaaaattgt ctgaatctga taatatagcg gttgccttta ataactctcc ttttgtaagc 118020

cagttgaata ggtcatcaac agataccttc ataagaacag ctagttgctc cataatgtca 118080

tcttggtctg tatgacatga cctgcttgct tggtctagta gtccgtaaca agctttttta 118140

agtatcttca tgggtttatc tgagatagtg ctagaaggct cacgagagga taaagaaaca 118200

acaccagagc ttgctctaca actagtgcac atgtagcaca ttccattacc gtactccttg 118260

ccatataaat cagcattaga tgtgtacttt atattacctt gacataacgg gcactttgta 118320

ggaatttcct tcttcatacg tgccaacccc caatcgtcag tccatcgaat aacataccag 118380

ccgttattgc tacagagaca atcaaaatca ttaaaattat tgacccccat aaggtaagcc 118440

ctctggtgaa gaactcctta accgtaacta acagccaaat aaatgaaata ataaaaatta 118500

tccaagtagt taatcccata actttgtctc ctctcggtat ttcttaatct tcaatcttgc 118560

atctgatata actgttgaca aaggtactag tagccttcct gccatgtaat cactatctac 118620

accaatgtta atttggtctt tacttggact aggtttacta tgaatgtgtc catgaatgtt 118680

gaatctactt ttattacatc caagttccat aggataatgg gaaaaatgaa actctactga 118740

gtatgcgtga ataatcatac caatatcgtg tacttctact acactagggc ataggtcact 118800

attgtataag tttacttttt ttcgtgtatc atggttccct cttataataa tctttttacc 118860

tcgtagagca ttcataattt taaccgcttt ttcttcgtca cctaatgcaa agtctcccat 118920

gaagataacg acatcattat cattaacagt atcgttccac tccattataa actgcgagtt 118980

catgttgtca ataaaaacgt atttatctcc tctataatgt tcaaacttaa gtatgttatc 119040

gtgattaaaa tgtgtatctg atgctaagaa aacattgtta gaacttgaaa tgttatattt 119100

aaccaaaaaa tcagctagac taatcaatta aaaacacctg cctctagagg tacttcttta 119160

taacctcctg tataaaaact taagttgtac tccgtcatag acttatcttt actatatact 119220

tttactacac gattactggt tactagaata ctagctacct cttttttacc tactacatgt 119280

ttatgctttc ttccgccacc atctgtgatt tctacttcta gtaattcacg aataccgaac 119340

ataccaaaca actcctatct attctataat aatatttttt ctgatttcta accagtctac 119400

ctctttgcca actgctttta aagccttatc atcaatgtat aagtcagcgc caatttttct 119460

agtatcattt ttccatattg ctatcttatc tgggttgttt tcatttacaa agtggtacgg 119520

tatactgtta ttggcaagaa aatccaaaca caaatccata ccaagctctt ctctacatgt 119580

ccaaatagtg ataactgccc caagttcaac aagttcttct attgtctctt tagcataagg 119640

taggagctcc cctactaagg ggtagctctc ctcctgctca ctagcaatag ttccatcaaa 119700

gtctatagct atatacttat agcgtaaact tctcatctct acgcctttgg tttgctatct 119760

ttaaatttta actgctcttt tgttgtagat ggtaaacgat aagactcata caagtgcggg 119820

tggtctttct ctaacttctt accgttaaat cgtaaacctt ccacctccgc ttgataagtt 119880

agttcaaaat ctcctaaaat ctctaatgat tttacgtttg ccttcaccat cataggcatt 119940

aattcatctt taagtgcatc taactgtttt tttaatatct cgtagttacg ttttgttgtt 120000

gcataatttt tagctttgtc ctgcaaggtt ttagtaatag acacccctgc accctcttta 120060

agttctaccg cttgtaatgc accttgtttt gccatttata atctcctcct gtcttattta 120120

taaattaact ataacacata ttttattata agtcaacaaa aatgtatatt ttttatttac 120180

tatcctctac ttttaggatt atttctctga gatgtactgg aacactacgt ccaagagact 120240

ttttagctgt atcctcatcg taaacaaaag ctaagtcatc tggtgattcc tcactcataa 120300

tatcatcata cacaattgag ggtactacct tcattagttg ggaaaagtta gaaaattcta 120360

agttaggcaa cttattttcc tcttctagtc tctgtagagc tttcctaacc ctagtctcag 120420

tgacgaattg tcgaatccac tcggtacttt caggtaaatc tttaacttcc ctttttctag 120480

attggactct ttctgcgtgt tttggggaaa cacttttaaa tagtttctgg ctaccatcga 120540

tgtctacttc gtctaaaaat ttaacaataa taccttctcc ttcattagtt tccttagtaa 120600

atctggactg acctacaaat tcgttaactg tatcaaagtc agtaaatacc cctaagtaaa 120660

acatatctgg cataggtaga tttagtgtaa aagcaatact acgtacctca tctaaaggta 120720

agtatcgccc ttctacgtta ctgtacacgt caaataagta gaagttattt ctaaattgct 120780

ccttatacgg tgtatttaac ttcttagaac ctagccactc accaaagaaa gttaggttta 120840

ctagacctga atggacaaca tcaggaatac ttaccttgtc tttaatgtaa gtagtgaaac 120900

ctgatagtgt gtttacctct cctacttcat taggaatgtc taactctata tttcttgaga 120960

atttacgcat agcatagcca ccatcaggaa gtttctctac aaaaaaacta gcgttgctac 121020

catctaactt ctctagaatg ataatttcac ctgtatatgt ttttgccatt tgttgagctc 121080

ttccaattct ttgaattttt ctatatctat tcatattaac tctcctccta aatttttaag 121140

tctttgaacg aaatattatc tagattgctg ggttcaagct ctcctgtacg aatataagta 121200

gtcatgagct tgctgtctaa ctcagtatcc tcttttaata attcttcatt aactttattt 121260

ataagaatgc caatatcttt tcctttagcg ggaataggta tatttaatga tttagctatc 121320

ttgtctactt ctaacttaat tgcctcaggg agagtttcct gataaggcaa gaactcatat 121380

agtaagccaa tctccatacc caagtcatca gcttcactct ctgttctagc aaaatcaatt 121440

tgttgaccta tttgttttac ctcctgcttt actgcttggc gtacctgctc atcagtaagt 121500

tctgatacac ttttaacttt aacttctact cgcatgttat caagtctagc aattaaattt 121560

tggtatacat tgatgaaatc acttttactt tttctggcat gtataagact ttctttcact 121620

ctttctacca aactagttcc tttgtctttt tcttctaact tcataaattt tccctacttt 121680

ctctatattc tctttttgta tcatctttta taagttttct ctcatatctg tttcttagtt 121740

ttttgtctct tccttggcag tgtggaaaca tcccatcctt agtgaagagt agctcctcgt 121800

acactctatt agccttattc ttaaaatatt tatttgtaga ttttttactc atttagttat 121860

ctctcacacg ttgtaaaatt ttacctaaca agtttgaacc tctccataaa tctttgttta 121920

aaatacgagg gtcactttct cgtagtccta cgccccatat agtgtcataa gggcttgcct 121980

ctactaaagt agttccctta gtagcttcta gtttatcttt taattcctta ttttgagaga 122040

acttagccat taagataata aaaacaatag cctctttgtt atcatcccac gtagcctcgt 122100

taaaacctct aacttttcta cccagagatt taactgtttt agggtctcca ctaggaacgg 122160

atagaatttg tgaagccact aactcatcat tgaatagacg tgccttctca tacatgaatg 122220

cctgttctgc attatgaaac ataatagact tgttagcttc tttcccttct tgcttgtact 122280

ctaaagaaaa atttgatggg tgccaattac taaaggggct ttcctttccc caaaaaaatg 122340

tgtatctatc tctcatttta taactcccct ccttgactta gtaaataagt aggcgtttct 122400

gtgttagcca ttctgcttac tcgggtagct aatgtctcaa taaatttttt attatcacca 122460

taaatattaa tagtctgcgc ttctttttta gttagtgtag tgaactttgt agttttgtca 122520

aactgtacaa aacggtaata aaatttttgt gcttcttcca cagaactagc taatacccaa 122580

gtttttcttt tatttcgttc tgaaatttct actactaatt gtttcacgtg aaacaacctc 122640

cttatttatt ttagtttgtg ttctcttatt taattaatgc gccttaaaat tatagattgg 122700

tttaacaatg aagtcaatgt caaccgtatc tgtaatattc tctatgatac tctctttctt 122760

tttataggca aatggtgcct catctagtgt agactccaac acagaggaag accaaacatc 122820

tttcattgta ttcttaaaat cttccatttc tagtgtttct ttagctttag aacgggacag 122880

taccctacct gccccatgag gtgctgaatt gttccagtct tcgtttccct ttcctgtagc 122940

aataatgcta ccatcacgca tatttagtgg gataactagt tgttttcctt tagaggcatc 123000

tgtcgcacct ttacgaataa ccttattagg gatgtcaata taattatgaa tagagtcaaa 123060

ctcagaaact accaagtttt cccaacccat tttagacata attactttat gaatagcttt 123120

tcggttatcc tccgcaaatt tttgcgcaat agccatatca tttagatagt cctctactaa 123180

tttaccgtct agataagcaa ggtctttatt aaaaggtgct gtacttagtt ttaagcgcaa 123240

taactcttcc tgtatgtctt tttccctacc ttgctctttt agagctctaa tgagctcctg 123300

tggcttctca gtgttagaac aatacttaac agctagttct tggtgtgttt tagctacttg 123360

aacgcctaag ttacgggaac ctgtgtgtgt cataaggaag attcgaccat cgtaggcacc 123420

tagttcaata tagtggttcc ctcctcctag tgtaccttct gataaagcga ttctattttt 123480

tacctcttct tttaagtttc catagatagt cagattctta agtgacttac ttaccttctc 123540

ctgatttagt gctactcctc ttacggagtt tcccgaagga acaaactctt taataacact 123600

atctaatttt tctaatgtag tattgtttac ctctaaatct aattcggtta ctcgtattcc 123660

gcaaccaata tctactccga taaggttagg tactactttg ttagtataag tcatagtggt 123720

tcctacaaca caccctttgc ctgcatgaat atcaggcata aatctaacac gctctccttt 123780

aacaaattcc tgacttaaca attctattac ttgacccaca gcggtttctt ctacctcact 123840

agtaaaaacc ttagctgtat tataccttcc aagtagttct atcattctgc tcacgctcct 123900

tttattatac ttcttactaa aaaccattat accatatgtc agtttataag tcaacactaa 123960

gttaaaaaaa gaggaacacg tatgcccctc tattttagtt ctttaaagtt cttagagaag 124020

aactcctcat aatattggtc ttctgtagta actttcctac ctgagtctga tactactgtt 124080

atatctccta ctagaacacc gtcttctgtg atactattta cagtaaacgt tctgttcttg 124140

ttaccttttt catgaaatgt cttatttaaa tatttttttc cttttttagt catacataac 124200

atcctctttt gttaattgtg ctagtgcttt acaatatttt tctttttgtg aggcaaaagc 124260

aatagtgtta tttgaaaaat agtctagaat ctctgctagt tttacttttt tagctagatt 124320

atcctgcttc aatctctgga tatagcaagt atacgcctca ttttgaatgc gtgttaggtg 124380

aactaaactt ccaagtaaat aatccttgtc cactagagat aaaaaagttt ggacatcact 124440

aaatctgacg gcggtatcct caaatatatc atgcagtaaa cttaaataat aagcatcttc 124500

atctttgtct ttcctaaagt tttctgctac tcggtacacg tgcgctagct gattttcctc 124560

ataattagct aaaacagtct ttaaatgatt acttacgttg tctttaattg taaacaatac 124620

gtagcctcct ttaaaaaata attggtagaa ccaatagtac acatattcct agagaaacaa 124680

gtaataacat aatagtatct cttactgaaa gtaggaactt aagtttaggt acatatggca 124740

tagatacccc tgctctgcct aaaagattag aagcaatagg cacaaggtta tactcaataa 124800

gaaaagctaa atataaataa gagaatatta aaataaaagt gattataatc cccatagatg 124860

tctcctctct actcccctaa ggagctattt tcatgcttat ctattttaaa aagctcacta 124920

gaaggaacac caaaatattt ttctagtttc actcgatttc tctctctagg aaacttctta 124980

cctgtctccc aataagaaat tagggagtag ttaacatcta aatcctgcgc taattgtcga 125040

atagacaact gctgattctc tctaattctt cttaaatttc ttactttccc aatatcttct 125100

ttatttgtca tttttttttt caccccttac tttttatata tttattataa cataaaagtc 125160

aacgtctgtc aagcgtacaa aaaaaagagc acgtattagg tgctctctag gttactatag 125220

aatgctgtca ataaagctgt aggcttcact tcttaaaaca gataccataa aatcttttgg 125280

tgtatttttt tctaaagaca ttacatgttt aaagacagta ggtgtaattc cagatactaa 125340

agcaactccg ctttcagaaa accgaactgg tgagttatca ttgcgggcat ttacattcca 125400

aaaagttact gttggaagta agtaacctgc ttttgaaaac ttacgtctca tactttcgta 125460

gaaagagctg tcgttctctt tttgtgaact acactggtcg aactccatgt cagagataat 125520

gacaatatca gtaggtagct cactttgtgg cagtttgctc tttgttgcgg tattcagaat 125580

taagttaaaa acagcctcta ggttagtgga catgccccag tcgctactaa tagcagtaga 125640

aacccgctcc cgtaagctta actcactagg aacatgaact aaagctggtt tagaactgaa 125700

tgtaataaag tggtctttga acactccatt tagtctctct gcgcagtata gccctaaaga 125760

aatagaaacg tccataggtg acacttcttt tgccccacca tacatagaac ctgatacgtc 125820

agaaactaca atagcatttc tagtcatatt ttctacgtag ttaggtagtg atttccaagt 125880

tgcctctact gtgttgtcta cagagtcgtc ccagtcacca cttgcggtgt acttcttaac 125940

taactcataa ggcataatgt tgctggcatt tagtttttta tctgtggagt ttactctgtt 126000

aataaacatt tcataggatg tgggcaggtg ttttctaaaa gcccctctat atttaaacag 126060

ggcacgagaa ggtaacttgt caaaattgat agtatcgtaa tccttgtcgg ctaagctagt 126120

ctccactaag tttaactttg ctcgtaaagc ggaaagtaat ttacgatact ctcgactggt 126180

gtagcctagg gaagcagaca agtctctagc tgtatgtttc ttctccttgt ttggtgaaga 126240

ctccgaaggt aaccacttag ctaataaaga tatagattca tctgatttta agttcttttt 126300

atctagttct agttgttcag aaattagttc cactagcacc attgttaaga ctttgttttt 126360

agtttcacaa ctatagcgat atagagatac aacatcgtcc caacgaccaa tttcactcat 126420

aaactctgta aacttaatta gagtgtctaa gtcaagctca gctagcccct taatggactc 126480

gttatacact gctcgttcac ctaagccact aagtcggata ttacgaagat gtactaagtt 126540

cttgagtgct agctctcggt cttctatata tgccttctta aataacagct tagcatcatt 126600

tgttctatca cgcattgctc ctgctcgtga gaaaaaatct aagttatagt ctaaagtagt 126660

gctaaatgtg ttagccccct tttctgtttt acttaaagta gtctcttctt ttaatttatt 126720

taaaaattct gacataataa tctacccctt ttctttttta taatcttata gctgacctaa 126780

gctatattca ggtcagctat cgtatgtaca ggaagcgttt ttttctcgta catttttttt 126840

ttacatggtt agacaccatg tcgtactaac tgctaatatg cgctgtttta gtttttagta 126900

tttgctgtgt gcttcctaat taatttttac agagagcgtt tttacttttt caaatagtat 126960

aggttagtaa ttgctgttag ctctctattt tatattctgt agattatttt tttatcacag 127020

gaatcatttt tatattttat ccaataatat agtagtaatt gctgttagat tcctaagttt 127080

attttatctt aaatttatga taacatattt tgtctagtat gtcaacatct ttttacattt 127140

tgtttttgtt gtcattctgc cagttttccc aaaattgctc tttttcactt tttcggctct 127200

cgacaaaacc ttgtgtcttc ttacaatatg ggcaatacat atgtttaata tgaccgtcct 127260

tcttttgctt agctgacctt ctgaatatag agactttttc cccacactca gggcatgtta 127320

acgtggtttg tactgtcttt accatagtta taaccagatt gccccgttgg gaatcgaacc 127380

caaaccgagg agttatgagc tccttgctcg accttctagc tacgggacaa tgagggaaac 127440

ttctcttagt tagagaaggc tccaaaatgt tttagttttc caccgagtgc ctttgtcttg 127500

tctgatagtt ctatttttga tgttagacga gacttacctt ctgccacgta cagaaaatct 127560

ttctcaacag taactaactt catttctttt ttgccagtct ctttgttgta tcgttcctgt 127620

aattctttaa caggttcgga aattacgccg taaaattttt tcattgtaaa acaactccta 127680

attttttatt ttttaactaa ctttgtaatt taataataca ataaaagaaa tttggagaga 127740

ttccactctc aactacttcg tgcatcaata tattcagtca cggctaaact agattcttgg 127800

taaggagcta ccttgtcctc catccagtct gtcaattaag actaagttta aacacatcac 127860

gttggaagtg attaaaaatt taccgacagg actattttac ctgtacattt caccaagcat 127920

ggaagagtat atttttatta ataccaattt taaagatgta gattttggct actttgactc 127980

ccatcatccc ttatactcgg ctttcaccga atcaactaca taaatgactt ttagtaagta 128040

gttttaaaag agtacttaca ttcccttcta aaacgcttca tcacgggcag gtaggatact 128100

tttatcttat aacacacgta gcagtacctt tgtctcacac taaccataac tatggctcag 128160

tccccaacgc atactgatat ttaccacata tactaatcga ggcggaaaat ttaagtacat 128220

tagaagagct accttcttgt tatgttacaa atttccttta ttctattatc aatgttctta 128280

gtaacactat accatagatt tcatatagtg tcaagtaata aatcaaggtg gtaggattcg 128340

aacctacgac ttcccgtctc caaagcgggt actctaacca aactgagcta caccctgtcg 128400

gttccgtaca caaacctttt aattgcacgg catatttttt tgactagcta cggacgtcta 128460

gaacgccagc cgacatctcc tgtatcagag aagatgaagc agatatatag tattatctgt 128520

agtttaaggt atgaggatac tagaaccaga aagaagcacc tctattagtt ttcctgttcc 128580

tatagtatcg gaagcaaaac cgaagttata ggtggatttc ctatcttttt tactcgttgt 128640

aactcacttt agttaccgtt tgcttttagc ggtgtgagtg gtaatctctt tactgtacac 128700

ctagtgtacc acactttgcg tttattgtca acacttattt tctaaaattt ttcctgtacc 128760

aattttctat catactattt atatagttat tcatgtagta gtcagtaact ataataccct 128820

tgtctgctgc taatgtatac tcatgaaccc gtaatatata gtaagtactt ataggaagta 128880

caacgttacc tgaataaggt ttagtagttc tctctacatg ttttataaat gactctattt 128940

ctttcatttc ttgttggaaa gataggtatt ttccatgtct agtcctaaaa actagtttgc 129000

ggtaaaaccc accatcaggg atatgctctt ctctagatat tctagacctt tgaagtttat 129060

tcaaccttat tttgtctagt ctcctctcct ttgaatgcca gtccgcttta ctcttgggcg 129120

tcttcttata acttctgctc ataggacacc tcctaatgat tatctaccta gtatttctct 129180

ctgatagtca ttttaccatt cttttcttct gctttacgaa aattctctct aactttagct 129240

actctagctt gctcttcttt tttctcttcg ggtgtcagct ccattaaacc gagaactttc 129300

atagcatggt catactcttc ttgtgagtta cacactactg tatgtttaaa attaatctct 129360

gtcaatattg aagcctccct tgttgataat tatgtaccta tggctgttaa ctcctattca 129420

tttaacttcc agtctataag aagcaccaca gtttgaaaca gatttataag gttcctcaac 129480

ctacatataa gttaacttta ttatgtattt acaaagtcct cagcaggatt cgaacccgcg 129540

ctaagggagt agaaatccct tgtgcatccg ctacaccata aggacactat tctcctatac 129600

acacagcccc gctcttaacc agatgagacg taacggtcac cgaagcaacc gtaagggagt 129660

cgaacccttt ccgagctgtg caaaggaaag taacataggt ctcttattat gttacttagt 129720

ctagtgtccg tccacatagg ttgagactac ctcgggacac gtccgcattc tgttaattca 129780

gatttattta gtattgtgtt gcaacacaac gctgggatga ctaatcccta aagcgggtag 129840

ggggattcga acccccacta catggttgga aaccttgggt actaccctta tactataccc 129900

gcagaggcaa cctacttagg gcaagtaagt taccaataca aaaggagtaa aaattaatta 129960

aaacaaaaaa gcgtatcctg ctggatttga accagcgact tccgacttaa caggacggca 130020

ctctgacctc tgagttaagg acacaaaata ctcggcaagg attttcacct tgcaataacc 130080

ttcatcattc gtttcagcta tcaattcaag catcggatat gctctcagct tacttctatt 130140

ataggttata ccagtcctac tgtgcgatat tttttccgcc acgagtataa aaaatagtaa 130200

tttaagtaca ataggtagca agtttttgta agtgctagca agcgcaatac tgtaccactc 130260

agtattaaaa aaggtgggat atacaattgt aatttcattt tactgtgttt gtaattgtta 130320

tcgtacatgg tttcatacac attgctattg agttaaataa cttgctaccg aggtaactgt 130380

actatttttg cctgctattc ctaaagctct tacaattgac aggtaaggag tcgaacctta 130440

ataactacca ctagcttgtc tttaatactt atcggtgcac cttatagtag acttagggtt 130500

actagacaaa ctaaagtata ctacatactt gacttactac ccgacatttg aagggaggtt 130560

agaaattgat tttagcctct ttttatttgc tgataatagt tttttctact atagcagtaa 130620

ggtgcaccgt ttgtttgtct caccacatag taatcctaat tttaaggaag gtaagggatt 130680

tgaacccttg taccctgtat tccaagggca aactagtttt caagactagc gtattaccaa 130740

gctctaccaa ccttccattt tgttgctatg gagtccgcag gtatcgaacc tgcctctcct 130800

gcttttcaag caagcgctaa tccatctcag ctaggactac gttaatggag ccactaggat 130860

tcgaacctag aataacgggg taaaaacccg cagtgatagc catttcacca taactccttt 130920

gtatagaggt tagagggatt tgaaccctcc gtaactagct atatagtcta gtatgtcgtc 130980

atttcatctt aacctctata ataacattat aacatacttt aagatgtttg tcaactgttt 131040

tacagggata gtaggacttg aacccacctc attggagttg gaaaccaata ttttaccaga 131100

taaactatac ccctaagctg gtctagttgg attcgaacca acgtatcacg gaatcaaaat 131160

ccgttgcctt accgcttggc tacagaccaa tagtaccaag taagctatgc ttgtagtagg 131220

acttgaaccc acgtctgagg cttacaaaac cactgttcta ccaattgaac tatacaagct 131280

tattttttta ttaagtcagc tctctaagtc tccgctcttt acgttagtaa ttattagctg 131340

actcgaagag ctaacctatt atgcgccatc aaggactcga accttggaac cccgaaggga 131400

ggggagttac agtcccctgc aattgcctct atgcgaatgg cacgttgtaa agctagagta 131460

catacttctt gcttattaag ataccctagc taatttttac tacttgttat atacttccca 131520

gtcattagca aatacatcta agttgctaat acaccacatt acatgacttc cagtagctgt 131580

tctcatttgc aagtaaggtt ctactttaaa tagtgcacct tcctctaact cataagcatc 131640

tgctgtttgt ttattagcag ggataccctc tggatagcct ttctgagcta ctacaaacat 131700

gtctttacca ttccaacctg ttctgctagc tttctttcct tttttaatta aaggtaaaat 131760

ctcttcgaat gtcataattt tctcctcctt ctatgtctta ttaataccga gagtgggatt 131820

cgaacccaca tatccttggg acagcagatt ctaagtctac cgtgtatacc agttccacca 131880

tctcggcaag tgtttctact agtctttgat tacattataa tagttagcat aatgagtaga 131940

gataacaaac ccacctcgaa tgtttaagta tggagggatt ctttctaagt cctgtaaatc 132000

aataccatct actaagtaac cgtcattagg ggcatctgtt aatggggata cagcaagaga 132060

gttgtctacc gaaatagttt tcacgttagg atatttttta tcaaaaatag ttttctgaaa 132120

ctttgtgctt acaataacag agtatccaaa ttcctcagct atctccatta tagcctgtgt 132180

ttttcctttt ctcctatact taacagtact gataaagaat agaggctcca ttcgatacat 132240

agccttctta aatcgcctta cttctttact ctctttaaac ttagactttt ttactttctt 132300

atgagtgttc ttttggttca gcatattcat cagtcccttt catttttatt caagcgcacg 132360

taaggggaat cgaaccccta tttctcgata gacagtcgag tataatagcc attatatcat 132420

acgcacagta ctgaggtact tctagctaaa aagttacctc agagtttaac ttggcaatag 132480

actggtcggc tgtgtttctt tagctaagga agccttctac gcactattag taccaacact 132540

tggaattgaa ccaaggacta ccgattatcg gtcggttgct ttgcctccta agctatgctg 132600

gtatagtata ctctctcgtt gactactata cgggggaatg tattaatagg aacgagagaa 132660

tacctatttt taattatacc acactctaac agctttctga gattttaatc tcttgggttt 132720

tcctttttca aggtcaaggt ttctggttta gtgtgtctac tacaaacctt ttttcgaaag 132780

gagagtaagg gattcgaacc cttggtacta ttactagtac aacgagttag caacccgtat 132840

cattaagcct ctctgacaac tctccaagaa acaaaattta aacttatatt tatattataa 132900

cataaattag tacgtttgtc tactaataac ttattttttt tcataaagcg ggtaatggga 132960

attgaaccca tatacgtaga tttgcagtct actacctttc cagttggtta tacccgcaag 133020

ttctagaaga ctaataagac tcctagagtt tttaaatatt tagtgaaggt aaagatataa 133080

acctgatgat aagaaaaacg tgttactaac aatcgtctaa cttacctgta tatacaatat 133140

agcatactta ctgataaaaa gcaacagttt tctataatat ttttcatact gagctaagaa 133200

aatgtatagc aaccctaaaa ctatataatt atctgacaac tgtcctattt aattttattc 133260

gtcccctata ttatattcta atatattata taattattta aagatatgta attagactta 133320

gaatataaaa ggaatataat ataggagaac cttaaaatta aataggacaa ttatctgaat 133380

attttataac gactactatt acttgagtaa caatagaaac ttgatactag tatcaaataa 133440

aaagtaattt aatattgata gtaacccacc tcaggtacct aaatatattc agaaaattta 133500

tactactgtc ctatttaatt ttatctacct cctatattat attctaatat attatataaa 133560

gactataata aattattatt attattatta tttataatat agaagagtcc taaaattaaa 133620

taggacaatt atctgaatat aaagtttaat taacataaaa agacacctgt aaaggcatct 133680

ttattaaaca tctaaaaatt tcttttcgta ggatactaca agtgaggcat ttacataaac 133740

ttcttctcca tctaatgctt tagtagttct aaccttaata aatggtaact cagaacctct 133800

tacagcagag tttaacttag tactagggtc agtaacagta tcaaatacat cttcactgaa 133860

aagataaaat acatctcctg caatagtctt tagttctaaa acagtttgtc ttctagccat 133920

ttttattgtt cctcctctgt tatgtggtat aattaaagta tatcacacat tacttcttaa 133980

ctaaactgaa aacataggta actaccgcac ctagtataat cataaatact ttttctacta 134040

agtctttttt accttcatct tctttttggt tcgtttgtct tagcgcttcc acacctttct 134100

ctaattgacc tatctgatag aagatgtgtg actgcttttc agattgtagg gcagaatctt 134160

tatctagagc cactacaata tctcgtaatt ccttgatagc ttttgttagc tcctcattgt 134220

cagtcttacg tgttagttca cccgtttgta ctttatcttc aaggtttttt attttatcta 134280

gcagactttg aatatttgta tctttttcca ctcacatcct cctcctctca gttctttatt 134340

tttggtaggg tttacgaatg tgttctgata catccgagct ttcagggtag ttatctaaga 134400

agttcctaat tatatcttca taatcaggtg ttacaattct cccgtaatgt gtaacaaagt 134460

ttaggtgaaa gtcaattcta ccgtaggaac ctaggtcaag gtacttacca accatgtcgt 134520

cttcatcaac tttaaaataa ccaaagtcaa aacctataag ggttcccatt gatagagccc 134580

tactaataaa gtagatatga tattcttcac gattttccga gaggaagtta cgtaatatat 134640

ctctaaattc tacttttagc atatccctgt attctttcat tgcagactcc tttctttaca 134700

tactccatgc aaccactact ataaccataa aagatacaac tattacaaaa tatataataa 134760

aggtgtaaac taacgacata agtagtacaa tgttagccac acctgctata aacgttgcta 134820

taattagcgt aaagttttgc gtaatacctt gtatatctcc tagagaaaca tggcgttgca 134880

agtcacttat aacgtggtat gaagtccaaa aaatacccac ttgaaacaag acagaggcta    134940

gtacagctat aaacattata ctcatagtaa gacaacctcc ttaaagctag ttctaatata    135000

gtatacgaca tagaataatt taaagttag                                      135029

<210>  4
<211>  181
<212>  PRT
<213>  Unknown

<220>
<223>  Distal 181 amino acids of the receptor binding protein of
       bacteriophage P100

<400>  4

Val Thr Ala Asn Thr Val Ala Val Glu Arg Val Val Ala Asp Arg Ile
1               5                   10                  15

Ile Thr Thr Leu Gly Val Pro Ser Lys Trp Glu Tyr Arg Ala Lys Ala
            20                  25                  30

Ser Asn Phe Phe Phe Ser Ala Ser Asn Ser Ser Arg Ile Thr Leu Val
        35                  40                  45

Ser Thr Gln Gln Asn Ile Ile Thr Pro Asn Lys Phe Ile Asn Asn Pro
    50                  55                  60

Gly Ser Leu Pro Leu Ser Ala Ile Asn Pro Ala Ile Thr Ile Pro Glu
65                  70                  75                  80

Asp Gly Met Tyr Gln Val Met Ala Ala Leu Asn Ile Asn Gly Ile Leu
                85                  90                  95

Glu Lys Val Tyr Leu Val Ser Glu Leu Thr Leu Leu Ile Asn Asp Val
            100                 105                 110

Val His Pro Ala Thr Phe Gly Met Val Lys Thr Ala Asp Asn Ala Asn
        115                 120                 125

Gly Gln Tyr Ser Leu Ala Gly Asn Gly Ile Tyr Gln Leu Lys Lys Gly
    130                 135                 140

Asp Lys Leu Lys Leu Arg Ser Tyr Cys Asn Asn Thr Gly Asn Asn Pro
145                 150                 155                 160

Tyr Leu Asp Val Asp Lys Leu Tyr Ile Ser Val Gly Lys Ile Ala Asp
                165                 170                 175

Ile Ser Leu Phe Lys

            180

<210>  5
<211>  181
<212>  PRT
<213>  Unknown

<220>
<223>  Distal 181 amino acids of the receptor binding protein of
       bacteriophage A511

<400>  5

Val Thr Ala Asn Thr Val Glu Val Glu Arg Val Val Ala Asp Arg Ile
1               5                   10                  15

Ile Thr Thr Leu Gly Val Pro Ser Lys Trp Glu Tyr Arg Ala Lys Ala
            20                  25                  30

Ser Asn Phe Phe Phe Ser Ala Ser Asn Ser Ser Arg Ile Thr Leu Val
        35                  40                  45

Ser Thr Gln Gln Asn Ile Ile Thr Pro Asn Lys Phe Ile Asn Asn Pro
    50                  55                  60

Asp Ser Leu Pro Leu Ser Ala Ile Asn Pro Ala Ile Thr Ile Pro Glu
65                  70                  75                  80

Asp Gly Met Tyr Gln Val Met Val Thr Leu Asn Ile Asn Gly Ile Leu
                85                  90                  95

Glu Lys Val Tyr Leu Val Ser Glu Leu Thr Leu Leu Val Asn Asp Val
            100                 105                 110

Val His Pro Ala Thr Phe Gly Met Val Lys Thr Val Asp Asn Ala Asn
        115                 120                 125

Gly Gln Tyr Ser Leu Ala Gly Asn Gly Ile Tyr Gln Leu Lys Lys Gly
    130                 135                 140

Asp Lys Leu Lys Leu Arg Ser Tyr Cys Asn Asn Thr Gly Asn Asn Pro
145                 150                 155                 160

Tyr Leu Asp Val Asp Lys Leu Tyr Ile Ser Val Gly Lys Ile Ala Asp
                165                 170                 175

Ile Ser Leu Phe Lys
            180

<210>  6

<211>  181
<212>  PRT
<213>  Unknown

<220>
<223>  Distal 181 amino acids of the receptor binding protein of
       bacteriophage P100+

<400>  6

Val Thr Ala Asn Thr Val Glu Val Glu Arg Val Val Ala Asp Arg Ile
1               5                   10                  15

Ile Thr Thr Leu Gly Val Pro Ser Lys Trp Glu Tyr Arg Ala Lys Ala
            20                  25                  30

Ser Asn Phe Phe Phe Ser Ala Ser Asn Ser Ser Arg Ile Thr Leu Val
        35                  40                  45

Ser Thr Gln Gln Asn Ile Ile Thr Pro Asn Lys Phe Ile Asn Asn Pro
    50                  55                  60

Gly Ser Leu Pro Leu Ser Ala Ile Asn Pro Ala Ile Thr Ile Pro Glu
65                  70                  75                  80

Asp Gly Met Tyr Gln Val Met Ala Ala Leu Asn Ile Asn Gly Ile Leu
                85                  90                  95

Lys Lys Val Tyr Leu Val Ser Asp Leu Thr Leu Leu Ile Asn Asp Val
            100                 105                 110

Val His Pro Ala Thr Phe Gly Met Val Lys Thr Ala Asp Asn Ala Asn
        115                 120                 125

Gly Gln His Ser Leu Ala Gly Asn Gly Ile Tyr Gln Leu Lys Lys Gly
    130                 135                 140

Asp Lys Leu Lys Leu Arg Ser Tyr Cys Asn Asn Thr Gly Asn Asn Pro
145                 150                 155                 160

Tyr Leu Asp Val Asp Lys Leu Tyr Ile Ser Val Gly Lys Ile Ala Asp
                165                 170                 175

Ile Ser Leu Phe Lys
            180


<210>  7
<211>  181
<212>  PRT
<213>  Unknown

<220>
<223>  Distal 181 amino acids of the receptor binding protein of
      bacteriophage P200

<400>  7

Val Thr Ala Asn Thr Val Ala Val Glu Arg Val Val Ala Asp Arg Ile
1               5                   10                  15

Ile Thr Thr Leu Gly Val Pro Ser Lys Trp Glu Tyr Arg Ala Lys Ala
            20                  25                  30

Ser Asn Phe Phe Phe Ser Ala Ser Asn Ser Ser Arg Ile Thr Leu Val
        35                  40                  45

Ser Thr Gln Gln Asn Ile Ile Thr Pro Asn Lys Phe Ile Asn Asn Pro
    50                  55                  60

Gly Ser Leu Pro Leu Ser Ala Ile Asn Pro Ala Ile Thr Ile Pro Glu
65                  70                  75                  80

Asp Gly Met Tyr Gln Val Met Ala Ala Leu Asn Ile Asn Gly Ile Leu
                85                  90                  95

Lys Lys Val Tyr Leu Val Ser Glu Leu Thr Leu Leu Ile Asn Asp Val
            100                 105                 110

Val His Pro Ala Thr Phe Gly Met Val Lys Thr Val Asp Asn Ala Asn
        115                 120                 125

Gly Gln Tyr Ser Leu Ala Gly Asn Gly Ile Tyr Gln Leu Lys Lys Gly
    130                 135                 140

Asp Lys Leu Lys Leu Arg Ser Tyr Cys Asn Asn Thr Gly Asn Asn Pro
145                 150                 155                 160

Tyr Leu Asp Val Asp Lys Leu Tyr Ile Ser Val Gly Lys Ile Ala Asp
                165                 170                 175

Ile Ser Leu Phe Lys
            180


<210>  8
<211>  988
<212>  DNA
<213>  Unknown

<220>
<223>  Recombinant construct encoding the distal part of the P200
      receptor binding protein

<400>  8
   gtgacagcca acactgtagc agtagagagg gtagtcgcag ataggattat tactactcta      60
   ggggtaccga gtaagtggga atacagagct aaagcctcta atttcttctt tagtgcttcc     120
   aattcgtcac gtataacact agtatcgacc caacagaata ttataacacc taataaattt     180
   attaataacc ccggtagttt accactatct gcgataaacc ccgcaataac tattccagaa     240
   gatgggatgt accaagtgat ggcggcccta aacattaacg gtattttaaa aaaagtttac     300
   ttagtttctg aattaactct actaataaat gacgtagtac acccagctac atttggaatg     360
   gttaaaactg ttgataacgc taacggtcaa tactctttag cagggaatgg catatatcaa     420
   ctaaaaaaag gggacaagtt aaaactaaga agttactgta acaatacagg gaataaccct     480
   tacttggatg tagacaaact ttatatatct gttggtaaga ttgcagacat ttcattattt     540
   aaataaactt agtatgctga gaaaggaagt cacaataatg gcagaagaaa aattttcatt     600
   ggacggaagg cagtatgtaa cagcagtcct atatatgtac cctaacctag accctcagta     660
   cgactttgga gttgtatcat ccgaagggaa ggtagaagct tttgaatggc acgtaaaaaa     720
   tataacaaag ccttctattg cacagttaga ccaagcatat aaagactatg taaaatctat     780
   cggaagtcca agtaatccac cagccctaga accagaggta tcaaaagaag cctcagtagg     840
   taagattcta actgaaaaat ttaacacctt agaagaaaca aatgctaata tgttattgga     900
   aaatctagag cttcaagcta gcctagataa aacaaaacaa caaaattcag aactagtatt     960
   tgagctagta gataggggag tcttatga                                      988

<210>  9
<211>  988
<212>  DNA
<213>  Unknown

<220>
<223>  Recombinant construct encoding the distal part of the P100+
       receptor binding protein

<400>  9
   gtgacagcca acactgtaga agtagagagg gtagtcgcag ataggattat tactactcta      60
   ggggtaccga gtaagtggga atacagagct aaagcctcta atttcttctt tagtgcttcc     120
   aactcgtcac gtataacact agtatcgacc caacagaata ttataacacc taataaattt     180
   attaataacc ccgatagttt accactatct gcgataaacc ccgcaataac tattccagaa     240
   gatgggatgt accaagtgat ggcggcccta aacattaacg gtattttaaa aaaagtttac     300
   ttagtttctg atttaactct actaataaat gacgtagtac acccagctac atttggaatg     360
   gttaaaactg ctgataacgc taacggtcaa cattctttag cagggaatgg tatttatcaa     420
   ctaaaaaaag gggacaagtt aaaactaaga agttattgta acaatacagg aaataaccct     480
   tacttggatg tagacaaact ttatatatct gttggtaaga ttgcagacat ttcattattt     540
   aaataaactt agtatgctga gaaaggaagt cacaataatg gcagaagaaa aattttcatt 600
   ggacggaagg cagtatgtaa cagcagtcct atatatgtac cctaacctag accctcagta 660
   cgactttgga gttgtatcat ccgaagggaa ggtagaagct tttgaatggc acgtaaaaaa 720
   tataacaaag ccttctattg cacagttaga ccaagcatat aaagactatg taaaatctat 780
   cggaagtcca agtaatccac cagccctaga accagaggta tcaaaagaag cctcagtagg 840
   taagattcta actgaaaaat ttaacacctt agaagaaaca aatgctaata tgttattgga 900
   aaatctagag cttcaagcta gcctagataa aacaaaacaa caaaattcag aactagtatt 960
   tgagctagta gatagggagg tcttatga 988

## Description

### Field of the invention

The invention relates to the field of microbiology, specifically to a bacteriophage or a combination of bacteriophages, to a composition comprising said bacteriophage or comprising said combination of bacteriophages for preventing, treating or controlling contamination with and/or growth of *Listeria,* including *Listeria monocytogenes* and *Listeria* serovar (SV) 1/2 mutants that are resistant to bacteriophage P100 and/or *Listeria* serovar 3. The invention further relates to the a method for controlling contamination with *Listeria,* including *Listeria monocytogenes,* and *Listeria* serovar (SV) 1/2 mutants that are resistant to bacteriophage P100 and/or *Listeria* serovar 3, in a food product, on food processing equipment, or on food storage containers by applying the bacteriophage or the combination of bacteriophages, to the composition comprising said bacteriophage or said combination of bacteriophages to a food product or food processing equipment to reduce the amount of *Listeria,* including *Listeria monocytogenes,.*

### Background of the invention

Phages, as antibacterial agents, have the advantage of replicating within the bacterial target. Thus, when their progeny lyse the cell and escape into the extracellular milieu, they can infect and multiply in succeeding generations of bacteria, producing progeny levels far greater than that of the binary growth of the target bacteria, thereby increasing the phage population exponentially in numbers at the expense of the bacterial targets. Bacteriophages that are able to lyse several *Listeria* serovars are known to the person skilled in the art. Serovars are determined by variations in the sugar decoration of teichoic acids (cellular antigens) on one hand and the type of flagellum on the other. No flagellotropic *Listeria* phages are known to date and recognition of target bacteria appears to be determined by the type of teichoic acids. The cellular antigen appears in five forms, namely SV 1/2,3, 4, 5 and 6. *L. monocytogenes* isolates are either SV 1/2,3 or 4, whereas *L. seeligeri* isolates are SV 1/2 or 3. *L. welshimeri* isolates are SV 6 as is *L. innocua.* SV 5 is only found in *L. ivanovii.* Bacteriophage P100 (genus Pecentumvirus) is such a bacteriophage and P100 has been used for quite some time to effectively control contamination of food products with Listeria, including *Listeria monocytogenes* (see e.g. WO2004/004495). Bacteriophage P100 is effective against several *Listeria* serovars, including serovars 1/2, 4, 5, and 6. Bacteriophage P100 was deposited at the American Type Culture Collection, 10801 University Blvd., Manassas Va. 20110-2209 on May 23, 2002, 2002, ATCC patent deposit designation number PTA-4383.

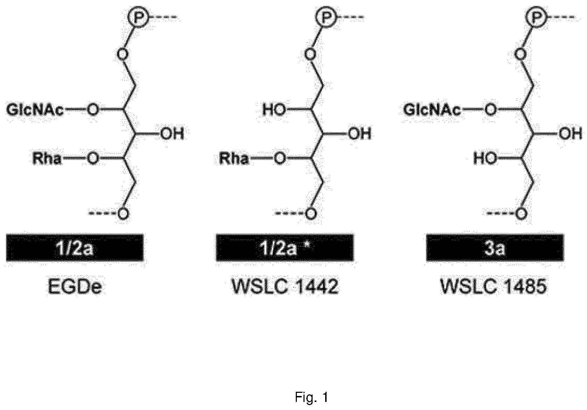

Several mutations in the cell-wall teichoic acids of *Listeria* serovar 1/2, lead to bacteriophage resistance. The cell-wall teichoic acids of *Listeria* serovar 1/2 strain carry sugar decorations, namely an N-acetyglucosamine (GlucNac) residue and a rhamnose (Rha) molecule, strains that have lost the GlucNac are designated SV 1/2*, and those lacking Rha are designated SV 3 (see figure 1). These mutations are quite commonly found and none of the known members of the genus Pecentumviruses (such as bacteriophage P100) are able to recognize, infect and lyse these *Listeria* mutants, rendering the known bacteriophages ineffective against such strains.

Since the mutants described here above (depicted serovar 1/2* and serovar 3) are quite common, there is thus a need for bacteriophages that are able to recognize, infect and lyse depicted *Listeria* serovar 1/2* and/or serovar 3.

**Table 1: Overview of sequences**

| **SEQ ID NO:** | **Name construct** | **Remark** |
|---|---|---|
| 1 | Genome of bacteriophage P100 | DNA |
| 2 | Genome of bacteriophage P100+ | DNA |
| 3 | Genome of bacteriophage P200 | DNA |
| 4 | Distal 181 amino acids of the receptor binding protein of bacteriophage P100 | Protein |
| 5 | Distal 181 amino acids of the receptor binding protein of bacteriophage A511 | Protein |
| 6 | Distal 181 amino acids of the receptor binding protein of bacteriophage P100+ | Protein |
| 7 | Distal 181 amino acids of the receptor binding protein of bacteriophage P200 | Protein |
| 8 | Recombinant construct encoding the distal part of the P200 receptor binding protein | DNA |
| 9 | Recombinant construct encoding the distal part of the P100+ receptor binding protein | DNA |

### Brief description of the figures

Figure 1
   Figure1 depicts the sugar moieties of the cell-wall teichoic acids of various *Listeria* serovars. Serovar 1/2 contains an N-acetyglucosamine (GlucNac) residue and a rhamnose (Rha) residue, strains that have lost the GlucNac are designated SV 1/2*, and those lacking Rha are designated SV 3.
Figure 2

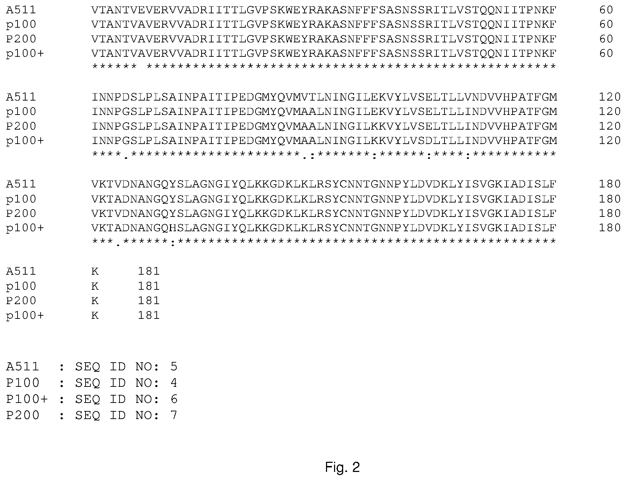

   Figure 2 depicts the distal 181 amino acid residues of the receptor binding proteins of phages A511, P100 and of the two phages with extended host ranges: P100+ and P200.
Figure 3

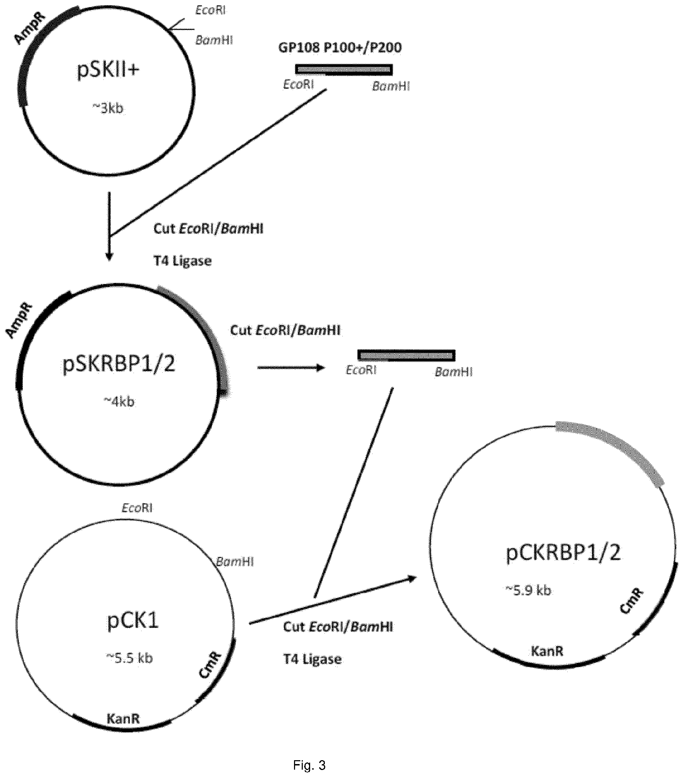

   Figure 3 depicts a schematic overview of the cloning steps in the preparation of bacteriophages A511::P200 and A511::P100+.
Figure 4

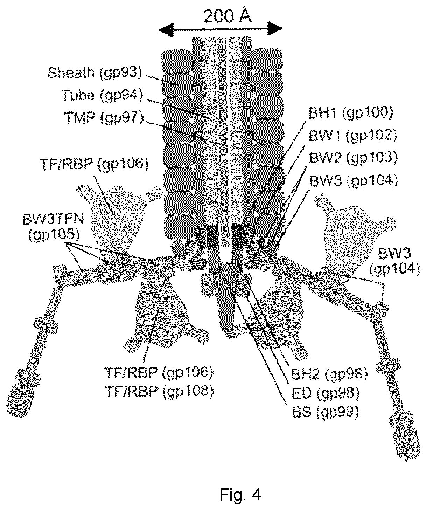

   Figure 4 depicts the location of the receptor binding protein at the end of the phage tail fiber.

### Description of the invention

The inventors have established that specific variant bacteriophages have an extended host range in view of their counterparts known to date; they variants are able to recognize, infect and lyse depicted *Listeria* serovar 1/2* and/or serovar 3.

Accordingly, in a first aspect, there is provided a bacteriophage lytic for at least *Listeria* serovar 1/2* and/or serovar 3, wherein said bacteriophage has a genome that has at least 70% sequence identity with the genome of bacteriophage P100 as set forward in SEQ ID NO: 1 or with the genome of bacteriophage P100, ATCC patent deposit designation number PTA-4383, and wherein the distal part of the receptor binding protein as set forward in SEQ ID NO: 4 has at least one mutation selected from: E97K, E104D and Y131H. Said bacteriophage is herein referred to as a bacteriophage as disclosed herein. In an embodiment, the distal part of the receptor binding protein as set forward in SEQ ID NO: 4 has at least two mutations selected from:
E97K, E104D and Y131H. In an embodiment, the distal part of the receptor binding protein as set forward in SEQ ID NO: 4 has the mutations selected from: E97K; E97K and E104D; E97K and Y131H; E97K, E104D and Y131H; E104D and Y131H.

In an embodiment, the bacteriophage as disclosed herein has a genome that has least 70, 75, 80, 81, 82, 83, 84, 85, 86, 87, 88, 89, 90, 91, 92, 93, 94, 95, 96, 97, 98, at least 99 or 100% sequence identity with the genome of bacteriophage P100 as set forward in SEQ ID NO: 1 or with the genome of bacteriophage P100, ATCC patent deposit designation number PTA-4383.

In an embodiment, the bacteriophage as disclosed herein is bacteriophage P100+ as deposited under number CBS146750 at the Westerdijk Fungal Biodiversity Institute, Uppsalalaan 8, 3584 CT Utrecht, The Netherlands. In an embodiment, the bacteriophage as disclosed herein is bacteriophage P200 as deposited under number CBS146751 at the Westerdijk Fungal Biodiversity Institute. In an embodiment, the bacteriophage as disclosed herein is bacteriophage P100+ having the genome according to GenBank accession nr. MT 178766.1 (corresponding to SEQ ID NO: 3). In an embodiment, the bacteriophage as disclosed herein is bacteriophage P200 having the genome according to GenBank accession nr. MT 178767.1 (corresponding to SEQ ID NO: 4).

In an embodiment, the bacteriophage as disclosed herein is also lytic for at least one of *Listeria* serovars 1/2, 4, 5 and 6, such as *Listeria* serovar 1/2, *Listeria* serovar 4, *Listeria* serovar 5, *Listeria* serovar 6, *Listeria* serovars 1/2 and 4, *Listeria* serovars 1/2 and 5, *Listeria* serovars 1/2 and 6, *Listeria* serovars 1/2, 4 and 5, *Listeria* serovars 1/2, 4 and 6, *Listeria* serovars 1/2, 5 and 6, *Listeria* serovars 1/2, 4, 5 and 6, *Listeria* serovars 4 and 5, *Listeria* serovars 5 and 6, and *Listeria* serovars 4 and 6.

In a second aspect, there is provided a composition comprising at least one bacteriophage according to the first aspect. The features of the second aspect are preferably those of the first aspect. The person skilled in the art will comprehend that such composition as disclosed herein may comprises two or more bacteriophages as disclosed herein, such as a P100+ bacteriophage and a P200 bacteriophage.

The person skilled in the art will comprehend that the composition as disclosed herein may comprise one or more further compounds, such other active agents, and excipients. In an embodiment, the composition as disclosed herein further comprises an agent selected from the group consisting of a bacteriophage endolysin, a surface disinfectant, an antibiotic, a surfactant, an antibacterial enzyme, a further bacteriophage lytic for *Listeria,* such as *Listeria monocytogenes,* and a bacteriophage specific for bacterial contaminants other than *Listeria.* A further bacteriophage lytic for *Listeria,* such as *Listeria monocytogenes,* may be, but is not limited, to bacteriophages phages B054, A118, A502, A006, A500, A511, PSA, P35 and related viruses). These bacteriophages are described in WO2004/004495 which is herein incorporated by reference; the person skilled in the art will comprehend that the subject matter of WO2004/004495 can be combined with the subject matter disclosed herein. A further bacteriophage may also be a virulent bacteriophage specific for *Salmonella enterica* (see e.g. Marti et al, 2013, Mol. Microbiol. 87(4), 818-834). Such bacteriophage may be bacteriophage S16 belonging to the order *Caudovirales.* Phage S16 has a contractile tail, which is the defining morphological feature of the *Myoviridae* family. Phage S16 is the first strictly virulent, non-toxic broad host range T-even like bacteriophage solely infecting *Salmonella* bacteria ever described. Phage S16 lacks any kind of virulence factors as is the case for other T-even phages described in the literature. Phage S16 is a new member of the genus of T4-like viruses, belonging to the T-even type of subgroup and is the first fully characterized member of the T4-like phages limited to infecting *Salmonella.* This bacteriophage and derivatives thereof are extensively described in WO2013/169102, which is herein incorporated by reference; the person skilled in the art will comprehend that the subject matter of WO2013/169102 can be combined with the subject matter disclosed herein. Phage S16 is deposited at the Westerdijk Fungal Biodiversity Centre under number CBS130493.

It has previously been demonstrated that *Listeria* bacteriophages when combined with an organic acid or a salt thereof have increased efficiency of lysing *Listeria,* such as *Listeria monocytogenes*,, especially when applied on food for controlling *Listeria,* such as *Listeria monocytogenes,.* Such combination does not have to be comprised in a single composition, the person skilled in the art will comprehend that the bacteriophage composition as disclosed herein and a composition comprising the organic acid may be administered separately, either consecutively of simultaneously.

Accordingly, in an embodiment there is provided a composition as disclosed herein further comprising an organic acid, or a salt thereof, such as wherein the organic acid is selected from the group consisting of lactic acid, such as L-lactic acid, acetic acid, propionic acid and mixtures thereof, such as wherein the salt of the organic acid is selected from the group consisting of the sodium salt, potassium salt, ammonium salt and mixtures thereof, such as K-(L)lactate, Na-(L)lactate, K-acetate, Na-acetate, K-diacetate, Na-diacetate, K-propionate, Na-propionate and mixtures thereof. Such compositions comprising similar (e.g. P100 and A511) bacteriophages are extensively described in WO2018/141800, which is herein incorporated by reference; the person skilled in the art will comprehend that the subject matter of WO2018/141800 can be combined with the subject matter disclosed herein. In this embodiment, the organic acid may be selected from the group consisting of lactic acid, such as L-lactic acid, acetic acid, propionic acid and mixtures thereof. The salt of the organic acid may be selected from the group consisting of the sodium salt, potassium salt, ammonium salt and mixtures thereof, such as K-(L)lactate, Na-(L)lactate, K-acetate, Na-acetate, K-diacetate, Na-diacetate, K-propionate, Na-propionate and mixtures thereof. The organic acid may be acetic acid in a buffered aqueous solution comprising 2% to 30% acetate, such as comprising 5% to 20% acetate, such as comprising 10% to 20% acetate, such as comprising 15% to 20% acetate, such as comprising 15, 16, 17, 18, 19 or 20% acetate, such as comprising 17% acetate. The pH of the acetic acid in a buffered aqueous solution may be 2 to 7, such as pH 5 to 6.5, such as pH 5.7 to 6.3. Buffering may be performed using sodium acetate, acetic acid, sodium hydroxide, sodium carbonate and/or sodium bicarbonate.

The salt of the organic acid may be in an aqueous solution comprising a mixture of K-(L)lactate and Na-diacetate, such as comprising 50% to 80% K-(L)lactate and 2% to 10% Na-diacetate, such as comprising 60% to 80% K-(L)lactate and 3% to 10% Na-diacetate, such as comprising 70% to 75% K-(L)lactate and 4% to 6% Na-diacetate, such as comprising 72.8% K-(L)lactate and 5.2% Na-diacetate. The aqueous solution may be buffered as described previously herein.

In an embodiment, in the composition as disclosed herein, the bacteriophage as disclosed herein is present in an aqueous liquid and comprises 1 x 10⁷ PFU/ml to 1 x 10¹¹ PFU/ml, such as 1 x 10⁸ PFU/ml to 1 x 10¹⁰ PFU/ml, such as 1 x 10⁹ PFU/ml to 1 x 10¹⁰ PFU/ml, such as 1x 10⁹ PFU/ml. PFU herein is Plaque Forming Unit. The person skilled in the art knows how to calculate and assay PFU's.

A bacteriophage and composition disclosed herein can conveniently be used for controlling *Listeria,* including *Listeria monocytogenes,* contamination in a food product. Accordingly, in a third aspect, there is provided, a method for controlling *Listeria,* including *Listeria monocytogenes,* contamination in a food product, on food processing equipment or on food storage containers, comprising contacting a bacteriophage or a combination of bacteriophages as disclosed herein, or a composition comprising said bacteriophage or comprising said combination of bacteriophages as disclosed herein, to a food product or to food processing equipment to reduce the amount of *Listeria,* including *Listeria monocytogenes.* Said method is herein referred to as a method as disclosed herein. The features of said method as disclosed herein are preferably those of the first and second aspect. The food product may be any food product that is known in the art and is susceptible to bacterial contamination and/or spoilage, the food product may be selected from the group consisting of poultry, such as chicken and turkey, beef, pig, horse, donkey, rabbit, goat, sheep, salmon, trout, lobster, clamps, shrimps, crawfish, cheese, ice cream, sausage, such as frankfurter, bologna, meatloaf, roast beef, ham, sliced meat and mixtures thereof, as known in the art.

In an embodiment, the food product is a processed, non-processed, cured or uncured food product selected from the group consisting of meat, fish, shellfish, pastry, dairy product, vegetables, fruit and mixtures thereof. In an embodiment, the food product is selected from the group consisting of beef, pork, lamb, fruit, vegetables, including but not limited to lettuce, leafy greens, baby leafy greens, sprouts. The processing or curing of the food product may be a process selected from the group consisting of cooking, salting, baking, steaming, smoking, grilling, roasting, drying and brining (e.g. injecting with brine).

In an embodiment, 1 x 10⁵ PFU/ml to 1 x 10⁹ PFU/ml of a bacteriophage as disclosed herein is administered per cm² of food product or food processing equipment, such as 1 x 10⁶ PFU/ml to 1 x 10⁸ PFU/ml, such as 1 x 10⁷ PFU/ml to 1 x 10⁸ PFU/ml, such as 1 x 10⁷ PFU/ml, 2 x 10⁷ PFU/ml, 3 x 10⁷ PFU/ml, 4 x 10⁷ PFU/ml, 5 x 10⁷ PFU/ml, 6 x 10⁷ PFU/ml, 7 x 10⁷ PFU/ml, 8 x 10⁷ PFU/ml, 9 x 10⁷ PFU/ml, or 1 x 10⁸ PFU/ml.

The food product may be packaged, usually immediately after performing a method as disclosed herein or as a step within a method as disclosed herein. The food product may be packaged such that it can be stored and transported from the manufacturer via the distributor, wholesale, retail to the customer. The package may be a package under vacuum or under protective atmosphere using an inert gas. The packaging material may contain oxygen scavengers or the like. The packaged food product may be stored at temperatures below room temperature, such as 4 degrees Celsius / 29 Fahrenheit.

In a method as disclosed herein, a bacteriophage or a composition as disclosed herein may be administered to the food product in any way known to the person skilled in the art, such as by mixing with the food product or dipping the food product in a composition comprising a bacteriophage as disclosed herein. In an embodiment, a method for administering is wherein the administration of a bacteriophage as disclosed herein is performed by spraying or misting said bacteriophage or composition. Spraying of bacteriophage compositions is known in the art and any suitable method known may be used; the person skilled in the art knows to select a proper method.

In an embodiment, the food storage container is a salad bar.

In an embodiment, the food processing equipment is selected from the group consisting of a tube through which milk is being pumped, a high-salt content tank for processing cheese, a container from which cultures are applied to a surface of a cheese, a storage shelve for a food product, a shelve on which a food product is dried and cured, and a floor drain.

In an embodiment, a bacteriophage as disclosed herein or a composition as disclosed herein are sprayed onto a surface selected from the group consisting of food products, food processing equipment and food storage containers.

When the method as disclosed herein comprises administration of a bacteriophage as disclosed herein and of an organic acid or a salt thereof, said bacteriophage and organic acid, or a salt thereof, may be present in different solutions and the spraying may be performed from different nozzles. The spraying of the at least one bacteriophage and of the organic acid, or a salt thereof, is may be performed consecutively or simultaneously. Simultaneously herein means that the spraying of the two compounds is performed at the same time or shortly after each other, e.g. at most 1, 2, 3, 4, 5, 10, 20, 30, 40, 50 or 60 seconds after each other.

Further provided is a food product obtainable of obtained by a method as disclosed herein. Such food product may be packaged as defined previously herein.

Further provided is a food product that comprises at least 1 x 10³ PFU, or at least 1 x 10³ PFU equivalents, per average gram of food product of a bacteriophage as disclosed herein. At least 1 x 10³ PFU may be at least 1 x 10³ PFU or 1 x 10⁴ PFU or 1 x 10⁵ PFU or 1 x 10⁶ PFU or 1 x 10⁷ PFU or 1 x 10⁸ PFU or at least 1 x 10⁹ PFU. At least 1 x 10³ PFU equivalents may be at least 1 x 10³ PFU equivalents or 1 x 10⁴ PFU equivalents or 1 x 10⁵ PFU equivalents or 1 x 10⁶ PFU equivalents or 1 x 10⁷ PFU equivalents or 1 x 10⁸ PFU equivalents or at least 1 x 10⁹ PFU equivalents. PFU equivalent means that said amount of bacteriophage as disclosed herein has been administered by treatment of the food product. The food product maybe any food product, such as the food products described elsewhere herein.

The bacteriophages as disclosed herein can conveniently be used for the detection of *Listeria,* including *Listeria monocytogenes.* Accordingly, in a fourth aspect, there is provided a method for detection of *Listeria,* including *Listeria monocytogenes,* such as serovar 1/2* or serovar 3, comprising obtaining a sample suspected to contain *Listeria,* including *Listeria monocytogenes,* incubating said sample with a bacteriophage as disclosed herein or a bacteriophage as disclosed herein further comprising a detectable label, and detecting a change in said sample caused by said bacteriophage, as an indication of the presence of *Listeria,* including *Listeria monocytogenes.* The features of this aspect are preferably those of the first, second and third aspect.

In an embodiment, said change in the sample is due to lysis of said *Listeria,* including *Listeria monocytogenes,* by said bacteriophage or said change in the sample is due to the detectable label.

### Definitions

"Sequence identity" is herein defined as a relationship between two or more amino acid (peptide, polypeptide, or protein) sequences or two or more nucleic acid (nucleotide, polynucleotide) sequences, as determined by comparing the sequences. In the art, "identity" also means the degree of sequence relatedness between amino acid or nucleotide sequences, as the case may be, as determined by the match between strings of such sequences. "Similarity" between two amino acid sequences is determined by comparing the amino acid sequence and its conserved amino acid substitutes of one peptide or polypeptide to the sequence of a second peptide or polypeptide. In an embodiment, identity or similarity is calculated over the whole SEQ ID NO as identified herein. "Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons. Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. The amino acid change may be conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

A "nucleic acid molecule" or "polynucleotide" (the terms are used interchangeably herein) is represented by a nucleotide sequence. A "polypeptide" is represented by an amino acid sequence. A "nucleic acid construct" is defined as a nucleic acid molecule which is isolated from a naturally occurring gene or which has been modified to contain segments of nucleic acids which are combined or juxtaposed in a manner which would not otherwise exist in nature. A nucleic acid molecule is represented by a nucleotide sequence. Optionally, a nucleotide sequence present in a nucleic acid construct is operably linked to one or more control sequences, which direct the production or expression of said peptide or polypeptide in a cell or in a subject.

"Operably linked" is defined herein as a configuration in which a control sequence is appropriately placed at a position relative to the nucleotide sequence coding for a polypeptide as disclosed herein such that the control sequence directs the production/expression of a polypeptide as disclosed herein in a cell and/or in a subject. "Operably linked" may also be used for defining a configuration in which a sequence is appropriately placed at a position relative to another sequence coding for a functional domain such that a chimeric polypeptide is encoded in a cell and/or in a subject.

"Expression" is construed as to include any step involved in the production of the peptide or polypeptide including, but not limited to, transcription, post-transcriptional modification, translation, post-translational modification and secretion.

A "control sequence" is defined herein to include all components which are necessary or advantageous for the expression of a polypeptide. At a minimum, the control sequences include a promoter and transcriptional and translational stop signals. Optionally, a promoter represented by a nucleotide sequence present in a nucleic acid construct is operably linked to another nucleotide sequence encoding a peptide or polypeptide as identified herein.

The term "transformation" refers to a permanent or transient genetic change induced in a cell following the incorporation of new DNA (i.e. DNA exogenous to the cell). When the cell is a bacterial cell, as is intended herein, the term usually refers to an extrachromosomal, self-replicating vector which harbors a selectable antibiotic resistance.

An "expression vector" may be any vector which can be conveniently subjected to recombinant DNA procedures and can bring about the expression of a nucleotide sequence encoding a polypeptide as disclosed herein in a cell and/or in a subject. As used herein, the term "promoter" refers to a nucleic acid fragment that functions to control the transcription of one or more genes or nucleic acids, located upstream with respect to the direction of transcription of the transcription initiation site of the gene. It is related to the binding site identified by the presence of a binding site for DNA-dependent RNA polymerase, transcription initiation sites, and any other DNA sequences, including, but not limited to, transcription factor binding sites, repressor and activator protein binding sites, and any other sequences of nucleotides known to one skilled in the art to act directly or indirectly to regulate the amount of transcription from the promoter. Within the context herein, a promoter typically ends at nucleotide -1 of the transcription start site (TSS).

A "polypeptide" as used herein refers to any peptide, oligopeptide, polypeptide, gene product, expression product, or protein. A polypeptide is comprised of consecutive amino acids. The term "polypeptide" encompasses naturally occurring or synthetic molecules.

The sequence information as provided herein should not be so narrowly construed as to require inclusion of erroneously identified bases. The skilled person is capable of identifying such erroneously identified bases and knows how to correct for such errors.

In this document and in its claims, the verb "to comprise" and its conjugations is used in its nonlimiting sense to mean that items following the word are included, but items not specifically mentioned are not excluded. In addition the verb "to consist" may be replaced by "to consist essentially of" meaning that a product or a composition or a nucleic acid molecule or a peptide or polypeptide of a nucleic acid construct or vector or cell as defined herein may comprise additional component(s) than the ones specifically identified; said additional component(s) not altering the unique characteristic of the invention. In addition, reference to an element by the indefinite article "a" or "an" does not exclude the possibility that more than one of the elements is present, unless the context clearly requires that there be one and only one of the elements. The indefinite article "a" or "an" thus usually means "at least one". The word "about" or "approximately" when used in association with a numerical value (e.g. about 10) typically means that the value may be the given value (of 10) more or less 10% of the value.

All patent and literature references cited in the present specification are hereby incorporated by reference in their entirety.

The following examples are offered for illustrative purposes only, and are not intended to limit the scope of the present invention in any way.

### Further embodiments

Further embodiments of the invention are listed here below.
1. A bacteriophage lytic for at least *Listeria* serovar 1/2* and/or serovar 3, wherein said bacteriophage has a genome that has at least 70% sequence identity with the genome of bacteriophage P100 as set forward in SEQ ID NO: 1, and wherein the distal part of the receptor binding protein as set forward in SEQ ID NO: 4 has at least one mutation selected from: E97K, E104D and Y131H.
2. A bacteriophage according to embodiment 1, wherein the distal part of the receptor binding protein as set forward in SEQ ID NO: 4 has at least two mutations selected from: E97K, E104D and Y131H.
3. A bacteriophage according to embodiment 1 or 2, wherein the distal part of the receptor binding protein as set forward in SEQ ID NO: 4 has the mutations selected from:
   E97K; E97K and E104D; E97K and Y131 H; E97K, E104D and Y131H; E104D and Y131H.
4. A bacteriophage according to any one of embodiments 1 to 3, wherein said bacteriophage is bacteriophage P100+ as deposited under number CBS146750 or is bacteriophage P200 as deposited under number CBS146751.
5. A bacteriophage according to any one of embodiments 1 to 4, wherein said bacteriophage is bacteriophage P100+ having the genome according to GenBank accession nr. MT 178766.1 (SEQ ID NO: 3) or is bacteriophage P200 having the genome according to GenBank accession nr. MT 178767.1 (SEQ ID NO: 4).
6. A bacteriophage according to any one of embodiments 1 to 5, wherein said bacteriophage is also lytic for at least one of *Listeria* serovars 1/2, 4, 5 and 6.
7. A composition comprising at least one bacteriophage according to any one of embodiments 1 to 6.
8. A composition according to embodiment 7, further comprising an agent selected from the group consisting of a bacteriophage endolysin, a surface disinfectant, an antibiotic, a surfactant, an antibacterial enzyme, a further bacteriophage lytic for *Listeria* and a bacteriophage specific for bacterial contaminants other than *Listeria.*
9. A composition according to embodiment 7 or 8, further comprising an organic acid, or a salt thereof, such as wherein the organic acid is selected from the group consisting of lactic acid, such as L-lactic acid, acetic acid, propionic acid and mixtures thereof, such as wherein the salt of the organic acid is selected from the group consisting of the sodium salt, potassium salt, ammonium salt and mixtures thereof, such as K-(L)lactate, Na-(L)lactate, K-acetate, Na-acetate, K-diacetate, Na-diacetate, K-propionate, Na-propionate and mixtures thereof.
10. A method for controlling *Listeria* contamination in a food product, on food processing equipment or on food storage containers, comprising contacting a bacteriophage or a combination of bacteriophages according to any one of embodiments 1 to 6, or a composition comprising said bacteriophage or comprising said combination of bacteriophages according to any one of embodiments 6 to 9, to a food product or to food processing equipment to reduce the amount of *Listeria.*
11. A method according to embodiment 10, wherein the food product is a processed, non-processed, cured or uncured food product selected from the group consisting of meat, fish, shellfish, pastry, dairy product, vegetables, fruit and mixtures thereof.
12. A method according to embodiment 11, wherein the food product is selected from the group consisting of beef, pork, lamb, fruit, vegetables, including but not limited to lettuce, leafy greens, baby leafy greens, sprouts.
13. A method according to any one of embodiments 10 to 12, wherein said bacteriophage or composition is administered by spraying or misting said bacteriophage or composition to the food product or by dipping or soaking the food product into said composition.
14. A method according to embodiment 10, wherein said food storage container is a salad bar.
15. A method according to embodiment 10, wherein said food processing equipment is selected from the group consisting of a tube through which milk is being pumped, a high-salt content tank for processing cheese, a container from which cultures are applied to a surface of a cheese, a storage shelve for a food product, a shelve on which a food product is dried and cured, and a floor drain.
16. A method according to any one of embodiments 10 to 15, wherein said bacteriophage or composition are sprayed onto a surface selected from the group consisting of food products, food processing equipment and food storage containers.
17. A method for detection of *Listeria,* including serovar 1/2* or serovar 3, comprising obtaining a sample suspected to contain *Listeria,* incubating said sample with a bacteriophage as defined in any one of embodiments 1 to 6 or a bacteriophage as defined in any one of embodiments 1 to 6 further comprising a detectable label, and detecting a change in said sample caused by said bacteriophage, as an indication of the presence of *Listeria.*
18. A method according to embodiment 17, wherein said change in the sample is due to lysis of said *Listeria* by said bacteriophage or wherein said change in the sample is due to the detectable label.

### Examples

### Abstract

This example relates to mutations in the receptor binding protein (tail fiber protein) in view of phage P100 that allow infection of *Listeria* serovar (SV) 1/2 and (SV) 3 mutants that are resistant to P100 and all relatives of P100 (genus Pecentumvirus) reported to date.

The two mutant viruses, designated P100+ (P100plus) and P200, have been deposited under the regulations of the Budapest Treaty in the restricted CBS collection of Westerdijk Fungal Biodiversity Institute (Uppsalalaan 8, 3584 CT Utrecht, The Netherlands) and their sequences have been deposited in GenBank.

CBS 146750 *Listeria* virus P100+ deposited at the GenBank accession nr. MT 178766.1; CBS 146751 *Listeria* virus P200 GenBank accession nr. MT 178767.1.

### Introduction

The cell-wall teichoic acids of *Listeria* serovar (SV) 1/2 strain carry sugar decorations, namely an N-acetyglucosamine (GlucNac) residue and a rhamnose (Rha) molecule, strains that have lost the GlucNac are designated SV 1/2*, and those lacking Rha are designated SV 3 (see Figure 1)

By including the newly identified bacteriophages in a product against *Listeria,* its usefulness would be greatly expanded.

We sequenced newly identified bacteriophages P100+ and P200 and observed changes in a receptor binding protein (CDS 39722-41014 in the GenBank deposits).

Figure 2 depicts the distal 181 residues of the receptor binding protein (the full-length protein has 430 residues) of four phage isolates. Bacteriophage A511 (deposited at ATCC with deposited number PTA-4608; see also Loessner et. al., Applied and Environmental Microbiology, June 1990, p. 1912-1918, 1990) has 94% sequence identity with bacteriophage P100 and has almost identical structural proteins and identical host-range compared to P100. Phage A511 was used for recombination experiments to allow quick differentiation between recombinants and contamination since we work with P200 and P100+ in the same lab). P100, P100+ and P200 are closely similar phages, but the latter two have mutations that broaden their host range. The distal portion of the receptor binding protein is depicted in Figure 2; this is the region of the protein that interacts with the *Listeria* receptor molecule.

In order to demonstrate that the changes observed in this area are responsible for the altered host range we conducted recombination experiments that change this portion of the receptor binding protein in phage A511, resulting in the tail fiber protein being identical to that of either P200 or P100+. The methodology is described here below.

### Materials and Methods

The regions of interest of P100+ and P200 were introduced into phage A511 by homologous recombination of the phage and plasmid pCK1 harboring the P100+ or P200 region including upstream and downstream sequences. Infection by the phage of *Listeria ivanovii* WSLC 3009 strain harboring this plasmid allows for recombination to occur.

The regions of interest of P100+ and P200 were first synthetically generated and inserted into plasmid pCK1 (Gasson MJ, Anderson PH. High copy number plasmid vectors for use in lactic Streptococci. FEMS Microbiol Lett 1985; 30:193-6). The two synthetic sequences included restriction sited allowing insertion into the plasmid (see Figure 3). The resulting plasmids were transformed into *Listeria ivanovii* WSLC 3009.

Cultures of both strains (containing the plasmid with either the P100+ or the P200 regions of interest) were subsequently infected with phage A511. After phage propagation dilutions of the progeny phage were plated on strains *L. monocytogenes* WSLC 1442 (serovar 1/2*) or *L*. *monocytogenes* WSLC1031 (serovar 3) respectively. Only P100+ is able to generate plaques on WSLC1442 while P100 and A511 do not, and P200 is able to generate plaques on WSLC1031 while P100 and A511 do not. The resulting phages were designated A511::P200 and A511::P100+.

Sequencing confirmed that indeed the regions of interest had recombined with A511 in resulting phages. The results are depicted here below.
>A511_P200_recombinant; SEQ ID NO: 8 
GTGACAGCCAACACTGTAGCAGTAGAGAGGGTAGTCGCAGATAGGATTATTACTACTCTAGGG
GTACCGAGTAAGTGGAATACAGAGCTAAAGCCTCTAATTTCTTCTTTAGTGCTTCCAATTCGT
CACGTATAACACTAGTATCGACCCAACAGAATATTATAACACCTAATAAATTTATTAATAACC
CCGGTAGTTTACCACTATCTGCGATAAACCCCGCAATAACTATTCCAGAAGATGGGATGTACC
AAGTGATGGCGGCCCTAAACATTAACGGTATTTAAAAAAAGTTTACTTAGTTTCTGAATTAA
CTCTACTAATAAATGACGTAGTACACCCAGCTACATTTGGAATGGTTAAAACTGTTGATAACG
CTAACGGTCAATACTCTTTAGCAGGGAATGGCATATATCAACTAAAAAAAGGGGACAAGTTA
AAACTAAGAAGTTACTGTAACAATACAGGGAATAACCCTTACTTGGATGTAGACAAACTTTAT
ATATCTGTTGGTAAGATTGCAGACATTTCATTATTTAAATAAACTTAGTATGCTGAGAAAGGA
AGTCACAATAATGGCAGAAGAAAAATTTTCATTGGACGGAAGGCAGTATGTAACAGCAGTCC
TATATATGTACCCTAACCTAGACCCTCAGTACGACTTTGGAGTTGTATCATCCGAAGGGAAGG
TAGAAGCTTTTGAATGGCACGTAAAAAATATAACAAAGCCTTCTATTGCACAGTTAGACCAAG
CATATAAAGACTATGTAAAATCTATCGGAAGTCCAAGTAATCCACCAGCCCTAGAACCAGAGG
TATCAAAAGAAGCCTCAGTAGGTAAGATTCTAACTGAAAAATTTAACACCTTAGAAGAAACA
AATGCTAATATGTTATTGGAAAATCTAGAGCTTCAAGCTAGCCTAGATAAAACAAAACAACAA
AATTCAGAACTAGTATTTGAGCTAGTAGATAGGGGAGTCTTATGA
>A511_P100+_recombinant; SEQ ID NO: 9 
GTGACAGCCAACACTGTAGAAGTAGAGAGGGTAGTCGCAGATAGGATTATTACTACTCTAGG
GGTACCGAGTAAGTGGAATACAGAGCTAAAGCCTCTAATTTCTTCTTTAGTGCTTCCAACTCG
TCACGTATAACACTAGTATCGACCCAACAGAATATTATAACACCTAATAAATTTATTAATAAC
CCCGATAGTTTACCACTATCTGCGATAAACCCCGCAATAACTATTCCAGAAGATGGATGTAC
CAAGTGATGGCGGCCCTAAACATTAACGGTATTTTAAAAAAAGTTTACTTAGTTTCTGATTTAA
CTCTACTAATAAATGACGTAGTACACCCAGCTACATTTGGAATGGTTAAAACTGCTGATAACG
CTAACGGTCAACATTCTTTAGCAGGGAATGGTATTTATCAACTAAAAAAAGGGGACAAGTTAA
AACTAAGAAGTTATTGTAACAATACAGGAAATAACCCTTACTTGGATGTAGACAAACTTTATA
TATCTGTTGGTAAGATTGCAGACATTTCATTATTTAAATAAACTTAGTATGCTGAGAAAGGAA
GTCACAATAATGGCAGAAGAAAAATTTTCATTGGACGGAAGGCAGTATGTAACAGCAGTCCT
ATATATGTACCCTAACCTAGACCCTCAGTACGACTTTGGAGTTGTATCATCCGAAGGGAAGGT
AGAAGCTTTTGAATGGCACGTAAAAAATATAACAAAGCCTTCTATTGCACAGTTAGACCAAGC
ATATAAAGACTATGTAAAATCTATCGGAAGTCCAAGTAATCCACCAGCCCTAGAACCAGAGGT
ATCAAAAGAAGCCTCAGTAGGTAAGATTCTAACTGAAAAATTTAACACCTTAGAAGAAACAA
ATGCTAATATGTTATTGGAAAATCTAGAGCTTCAAGCTAGCCTAGATAAAACAAAACAACAAA
ATTCAGAACTAGTATTTGAGCTAGTAGATAGGGGAGTCTTATGA
>Native_P100; SEQ ID NO: 4 
VTANTVAVERVVADRIITTLGVPSKWEYRAKASNFFFSASNSSRITLVSTQQNIITPNKF
INNPGSLPLSAINPAITIPEDGMYQVMAALNINGILEKVYLVSELTLLINDVVHPATFGM
VKTADNANGQYSLAGNGIYQLKKGDKLKLRSYCNNTGNNPYLDVDKLYISVGKIADISLF
K
>Native_A511; SEQ ID NO: 5 
VTANTVEVERVVADRIITTLGVPSKWEYRAKASNFFFSASNSSRITLVSTQQNIITPNKF
INNPDSLPLSAINPAITIPEDGMYQVMVTLNINGILEKVYLVSELTLLVNDVVHPATFGM
 
VKTVDNANGQYSLAGNGIYQLKKGDKLKLRSYCNNTGNNPYLDVDKLYISVGKIADISLF
K
>A511 P100+ recombinant; SEQ ID NO: 6 
VTANTVEVERVVADRIITTLGVPSKWEYRAKASNFFFSASNSSRITLVSTQQNIITPNKF
INNPGSLPLSAINPAITIPEDGMYQVMAALNINGILKKVYLVSDLTLLINDVVHPATFGM
VKTADNANGQHSLAGNGIYQLKKGDKLKLRSYCNNTGNNPYLDVDKLYISVGKIADISLF
K
>A511_P200_recombinant; SEQ ID NO: 7

VTANTVAVERVVADRIITTLGVPSKWEYRAKASNFFFSASNSSRITLVSTQQNIITPNKF
INNPGSLPLSAINPAITIPEDGMYQVMAALNINGILKKVYLVSELTLLINDVVHPATFGM
VKTVDNANGQYSLAGNGIYQLKKGDKLKLRSYCNNTGNNPYLDVDKLYISVGKIADISLF
K

As can be observed here above, changes in both A511 recombinants resulted in a receptor binding protein identical to that of either P200 or P100+.

Isolation of these phages from either WSLC 1442 or WSLC 1031 already demonstrated that a host range change had occurred, but we conducted further experiments to corroborate that host ranges of the recombinants overlap with their P100 mutant counterparts, and that these changes do not lead to a loss of efficacy on other serovars. To this end we carried out host range experiments on several other strains, including further SV 1/2*, 3, 4, 5 and 6 strains which also include species other than *Listeria monocytogenes.*

Here below, the result of pull-down experiments are depicted. These experiments involved mixing of a small number of phages (∼3000 PFU) with 1 mL of overnight bacterial cultures, incubating for 5 minutes followed by centrifugation. Phages left in the supernatant were enumerated. This type of experiment is far more sensitive to indicate binding of the phage to the host than classical plaque experiments.

| | **P100+** | **A511::P100+** | **P200** | **A511::P200** | **A511** | **P100** |
|---|---|---|---|---|---|---|
| 2627 (L.in SV 6) | **+** | **+** | **+** | **+** | **+** | **+** |
| 1442 (L.mo SV 1/2*) | **+** | **+** | **-** | **-** | **-** | **-** |
| 40137 (L.se SV 3) | **+** | **+** | **+** | **+** | **-** | **-** |
| EGDe WT (L.mo SV 1/2) | **+** | **+** | **+** | **+** | **+** | **+** |
| EGDeΔ2550 (L.mo SV 1/2*) | **+** | **+** | **-** | **-** | **-** | **-** |
| 1031 (L.mo SV 3) | **+** | **+** | **+** | **+** | **-** | **-** |
| 1032 (L. mo SV 3) | **+** | **+** | **+** | **+** | **-** | **-** |
| 1042 (L.mo SV 4) | **+** | **+** | **+** | **+** | **+** | **+** |
| Arl1 (L.mo SV 1/2*) | **+** | **+** | **-** | **-** | **-** | **-** |
| C33R (L.mo SV 1/2*) | **+** | **+** | **-** | **-** | **-** | **-** |
| 1001 (L.mo SV 1/2) | **+** | **+** | **+** | **+** | **+** | **+** |
| 20100 (L.mo SV 6) | **+** | **+** | **+** | **+** | **+** | **+** |
| 40140 (L.se SV 1/2) | **+** | **+** | **+** | **+** | **+** | **+** |
| 3009 (L.iv SV 5) | **+** | **+** | **+** | **+** | **+** | **+** |
| 5008 (L.we SV 1/2) | **+** | **+** | **+** | **+** | **+** | **+** |

| | | | | | | |
|---|---|---|---|---|---|---|
| L.mo= *L. monocytogenes,* L.in = *L.innocua,* L.se = *L. seeligeri,* L. iv = *L.ivanovii,* L.we = *L*. *welshimeri* "+" designates >80% binding of the phages; "-" represents less than 10% binding. | | | | | | |

### Results and discussion

Phages P100+ and P200 are superior in host range to P100 and other described Pecentumviruses. As such, their inclusion in anti-Listerial products for the food industry is highly desirable. Such an extended host-range would also be of interest phage-based bacteria detection.

The ability to recognize serovar 3 strains is the result of a single amino acid change at position 97 of the distal portion of the receptor binding protein from a glutamic acid to a lysine. This change is observed both in P200 and P100+. The ability to recognize SV 1/2* as observed in P100+ requires two (additional) changes. A change from glutamic acid to aspartic acid at position 104 and from tyrosine to histidine at position 131 of the distal part of the receptor binding protein. These positions correspond to residues 276 (for the change in both P100+ and P200), 283 and 310 in the full-length receptor binding protein. Figure 4 indicates the location of the receptor binding protein on the phage tail.

### References

Loessner et. al., Applied and Environmental Microbiology, June 1990, p. 1912-1918, 1990. Gasson MJ, Anderson PH. High copy number plasmid vectors for use in lactic Streptococci. FEMS Microbiol Lett 1985; 30:193-6

## Claims

1. A bacteriophage lytic for at least *Listeria* serovar 1/2* and/or serovar 3, wherein said bacteriophage has a genome that has at least 70% sequence identity with the genome of bacteriophage P100 as set forward in SEQ ID NO: 1, and wherein the distal part of the receptor binding protein as set forward in SEQ ID NO: 4 has at least one mutation selected from: E97K, E104D and Y131H.

2. A bacteriophage according to claim 1, wherein the distal part of the receptor binding protein as set forward in SEQ ID NO: 4 has at least two mutations selected from:
E97K, E104D and Y131H.

3. A bacteriophage according to claim 1 or 2, wherein the distal part of the receptor binding protein as set forward in SEQ ID NO: 4 has the mutations selected from:
E97K; E97K and E104D; E97K and Y131 H; E97K, E104D and Y131 H; E104D and Y131H.

4. A bacteriophage according to any one of claims 1 to 3, wherein said bacteriophage is bacteriophage P100+ as deposited under number CBS146750 or is bacteriophage P200 as deposited under number CBS146751.

5. A bacteriophage according to any one of claims 1 to 4, wherein said bacteriophage is bacteriophage P100+ having the genome according to GenBank accession nr. MT 178766.1 (SEQ ID NO: 3) or is bacteriophage P200 having the genome according to GenBank accession nr. MT 178767.1 (SEQ ID NO: 4).

6. A bacteriophage according to any one of claims 1 to 5, wherein said bacteriophage is also lytic for at least one of *Listeria* serovars 1/2, 4, 5 and 6.

7. A composition comprising at least one bacteriophage according to any one of claims 1 to 6, preferably further comprising an agent selected from the group consisting of a bacteriophage endolysin, a surface disinfectant, an antibiotic, a surfactant, an antibacterial enzyme, a further bacteriophage lytic for *Listeria* and a bacteriophage specific for bacterial contaminants other than *Listeria.*

8. A composition according to claim 7 or 8, further comprising an organic acid, or a salt thereof, such as wherein the organic acid is selected from the group consisting of lactic acid, such as L-lactic acid, acetic acid, propionic acid and mixtures thereof, such as wherein the salt of the organic acid is selected from the group consisting of the sodium salt, potassium salt, ammonium salt and mixtures thereof, such as K-(L)lactate, Na-(L)lactate, K-acetate, Na-acetate, K-diacetate, Na-diacetate, K-propionate, Na-propionate and mixtures thereof.

9. A method for controlling *Listeria* contamination in a food product, on food processing equipment or on food storage containers, comprising contacting a bacteriophage or a combination of bacteriophages according to any one of claims 1 to 6, or a composition comprising said bacteriophage or comprising said combination of bacteriophages according to any one of claims 7 or 8, to a food product or to food processing equipment to reduce the amount of *Listeria.*

10. A method according to claim 9, wherein the food product is a processed, non-processed, cured or uncured food product selected from the group consisting of meat, fish, shellfish, pastry, dairy product, vegetables, fruit and mixtures thereof.

11. A method according to claim 10, wherein the food product is selected from the group consisting of beef, pork, lamb, fruit, vegetables, including but not limited to lettuce, leafy greens, baby leafy greens, sprouts.

12. A method according to claim 10 to 11, wherein said bacteriophage or composition is administered by spraying or misting said bacteriophage or composition to the food product or by dipping or soaking the food product into said composition, wherein said food storage container is preferably a salad bar.

13. A method according to claim 9, wherein said food processing equipment is selected from the group consisting of a tube through which milk is being pumped, a high-salt content tank for processing cheese, a container from which cultures are applied to a surface of a cheese, a storage shelve for a food product, a shelve on which a food product is dried and cured, and a floor drain.

14. A method according to any one of claims 9 to 13, wherein said bacteriophage or composition are sprayed onto a surface selected from the group consisting of food products, food processing equipment and food storage containers.

15. A method for detection of *Listeria,* including serovar 1/2* or serovar 3, comprising obtaining a sample suspected to contain *Listeria,* incubating said sample with a bacteriophage as defined in any one of claims 1 to 6 or a bacteriophage as defined in any one of claims 1 to 6 further comprising a detectable label, and detecting a change in said sample caused by said bacteriophage, as an indication of the presence of *Listeria,* wherein said change in the sample is preferably due to lysis of said *Listeria* by said bacteriophage or wherein said change in the sample is due to the detectable label.
